# EUROPEAN PATENT APPLICATION

(11) **EP 4 843 987 A1**
(43) Date of publication of application: **25.11.2026**
(21) Application number: 25741562.0
(22) Date of filing: 16.01.2025
(51) Int. Cl.: C07D 491/22, A61K 47/68, A61P 35/00

(54) **ANTIBODY-DRUG CONJUGATE CONTAINING THIAZOLO[5,4-B]PYRIDINE STRUCTURE AND USE THEREOF**

(30) Priority: 17.01.2024 CN 202410069495
(71) Applicant: Beijing Tide Pharmaceutical Co., Ltd., Beijing 100176 (CN)
(72) Inventor: WANG, Hongjun, Beijing 100176 (CN); WANG, Yanming, Beijing 100176 (CN); GUO, Tingting, Beijing 100176 (CN); ZHAO, Yanping, Beijing 100176 (CN); LI, Wei, Beijing 100176 (CN); ZHANG, Min, Beijing 100176 (CN); HE, Jingchang, Beijing 100176 (CN); ZHANG, Qi, Beijing 100176 (CN); FENG, Zewang, Beijing 100176 (CN); ZHANG, Lei, Beijing 100176 (CN)
(74) Representative: Novitas Patent AB
(86) International application number: PCT/CN2025/072661
(87) International publication number: WO 2025/153001

(57) **Abstract**

The present invention relates to a new thiol-reactive coupling moiety as shown in formula (I), a linker containing the coupling moiety, a linker-payload conjugate, an antibody-drug conjugate based on the linker and the use thereof, and further relates to a pharmaceutical composition containing the antibody-drug conjugate, and the use of the antibody-drug conjugate for treating and/or preventing a disease.

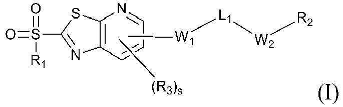

## Description

### TECHNICAL FIELD

The present disclosure belongs to the field of medicinal chemistry, and in particular, relates to a new thiol-reactive coupling moiety, a linker containing the coupling moiety, a linker-payload conjugate, an antibody-drug conjugate based on the linker and the use thereof, and further relates to a pharmaceutical composition containing the antibody-drug conjugate, and the use of the antibody-drug conjugate for treating and/or preventing a disease.

### BACKGROUND

Antibody-drug conjugate (ADC), using a monoclonal antibody with biological targeting to target and transmit the potent cytotoxin to the focus site, combines the advantages of antibody and cytotoxic drug, and has many advantages such as strong targeting property, high activity, low toxic side effects and long half-life. Up to now, there are 15 ADC drugs on the market, and about 200 ADC drugs are in clinical research.

The ADC consists of three components in structure: an antibody, a small-molecule cytotoxin, and a linker. The antibody's role is to achieve target delivery, the cytotoxin's role is to exert its therapeutic effect by killing the target cells, and the linker's role is to achieve the organic binding of the antibody and cytotoxin in structure, so as to form a whole structure. The performance of the linker is a key factor in ensuring the targeted delivery of cytotoxins, which directly determines the drug effect, safety and pharmacokinetic characteristics of the ADC. Therefore, the construction of the linker is of great significance to ADC drugs.

With regard to the development of ADC linkers, there are a number of important considerations, including the selection of antibody conjugation sites, the average number of cytotoxic molecules conjugated per antibody molecule (drug/antibody ratio, DAR), the cleavability of the linkers, and the hydrophilicity of the linkers, and the like. The basic requirements of the ADC for the linker include that it must ensure stability within the human circulatory system throughout the entire treatment cycle after ADC administration, and be able to rapidly and effectively release the payload upon reaching the target tissue, so that the ADC can exert the two advantages of antibody targeting and high toxin potency.

In addition, the design of the linker needs to match the physicochemical properties of the payload connected, thereby adjusting the hydrophilicity of the linker-payload. The proper hydrophilic design of the linker can achieve multiple benefits, including: improving the water solubility of the linker-payload during coupling, improving the coupling yield, reducing the degree of polymerization of the product, improving the stability of the product, and improving the bioavailability and the pharmacokinetics of the product.

Therefore, the selection of the linker and the coupling strategy is extremely critical during the design and development of ADC drugs. The linker of the ADC must ensure effective binding between the antibody and the cytotoxin before reaching the target tissue. If the cytotoxin is prematurely released before the ADC reaches the target tissue, on the one hand, the decrease in the loading rate will greatly reduce the concentration of payload released by the ADC to the target tissue, thereby reducing the efficacy of the ADC; on the other hand, the prematurely released potent cytotoxin will also cause significant off-target toxicity to normal tissues.

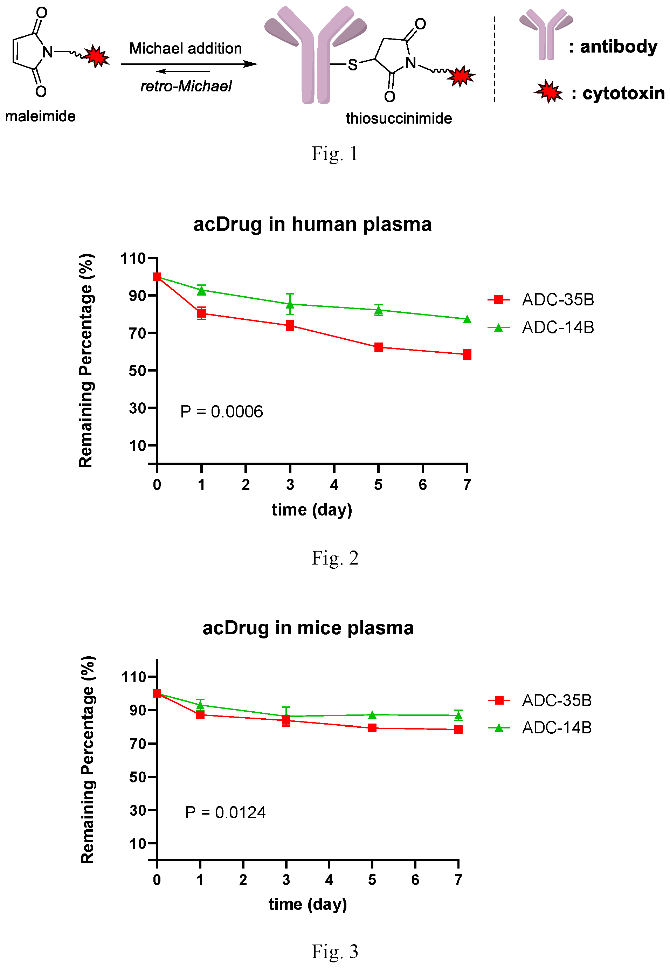

Currently, among the ADC drugs that are on the market or in clinical development, the vast majority still employ the process in which a thiol group undergoes a Michael addition reaction with a maleimide to form a thiosuccinimide during construction (Fig. 1). Because the reaction has many advantages such as fast speed, quantitative and mild condition, no better alternative has yet been found for its application in current ADC research.

However, the thiosuccinimide group generated in the ADC product constructed by the above reaction is unstable. After ADC administration, it will continuously undergo a retro-Michael reaction in vivo, resulting in a continuous off-target process of highly potent cytotoxins from the ADC and release into normal tissues (Fig. 1). The maleimide group generated by off-target process will further react rapidly with substances containing thiol groups such as albumin, glutathione, and cysteine in the blood, further accelerating the off-target process of cytotoxins from the ADC. It is reported that the off-target amount of cytotoxins from ADC can be as high as 50% within 3 days in mouse plasma in vitro, while the off-target amount of cytotoxins can be as high as 60% after one week of ADC administration in the in vivo model of mouse (Nature Biotech., 2014, 32(10): 1059-1062.). The aforementioned metabolism of ADCs in vivo will undoubtedly lead to a decrease in ADC efficacy and an increase in toxicity (Nature Biotech., 2012, 30(2): 184-189.; Bioconjugate Chem., 2008, 19(3): 759-765.; Bioconjug Chem., 2016, 27(7): 1588-1598.; J Med Chem., 2014, 57(19): 7890-7899.).

Therefore, current mainstream ADC linker technology cannot well ensure sufficient stability of the ADC prior to reaching the target tissue, and the construction of the new generation of linkers has become an important problem and major challenge for the development of the ADC. Besides stability, there is still a wide optimization space in many aspects of ADCs, including optimization of the drug release performance of the linker, optimization of the hydrophilicity, and the rational combination of the linker-payload.

### SUMMARY

Specifically, the present disclosure relates to new thiol-reactive coupling moiety based on the 2-(methylsulfonyl)thiazolo[5,4-b]pyridine structure, a linker containing the coupling moiety, a linker-payload conjugate, an antibody-drug conjugate based on the linker, and the use of the linker, and further relates to a pharmaceutical composition containing the antibody-drug conjugate, and the use of the antibody-drug conjugate for treating and/or preventing a disease.

In one aspect, the present application provides an antibody-drug conjugate (ADC), and an intermediate, a preparation method and an application thereof. The antibody-drug conjugate of the present application can realize the wide application of cytotoxic drug in ADC field, and it is mainly used for treating tumor disease. The main technical advantage of the present application lies in the fact that the new thiol-reactive coupling moiety provided is generally suitable for coupling reaction of macromolecule and small molecule containing thiol groups, and is also suitable for coupling with various toxins, and the obtained conjugated products have significantly improved stability. For example, when the new coupling moiety of the present application is applied to the ADC field, the safety of the ADC can be improved on the one hand, and the drug efficacy of the ADC can be improved on the other hand, by reducing the off-target of the payload.

The reaction between the 2-(methylsulfonyl)thiazolo[5,4-b]pyridine model molecule and the thiol model molecule is as follows:

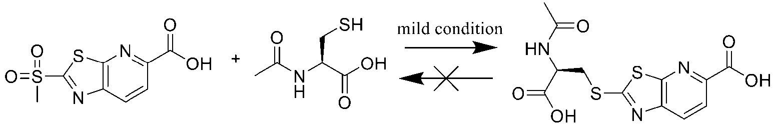

A typical preparation method of 2-(methylsulfonyl)thiazolo[5,4-b]pyridine coupling moiety (mother nucleus) is as follows:

### Preparation method 1.

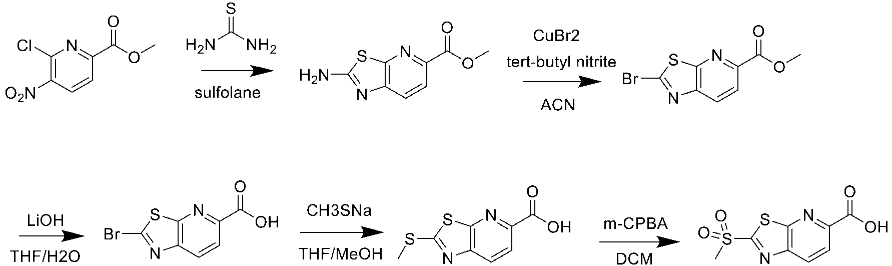

### Preparation method 2.

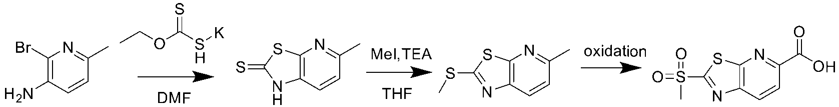

### Preparation method 3.

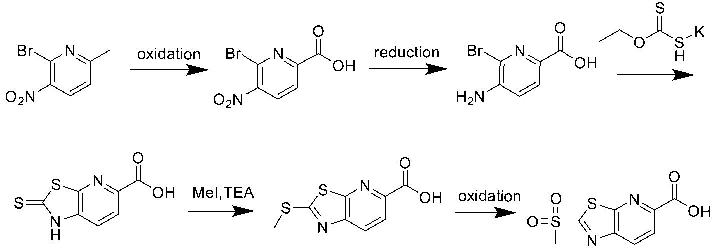

### Preparation method 4.

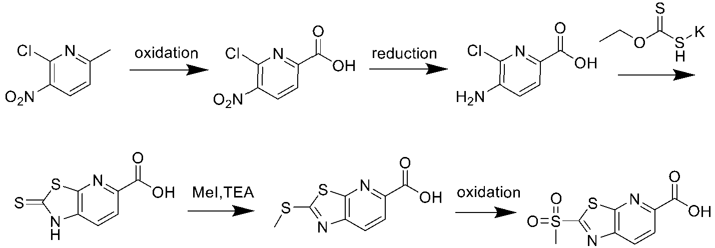

### Preparation method 5.

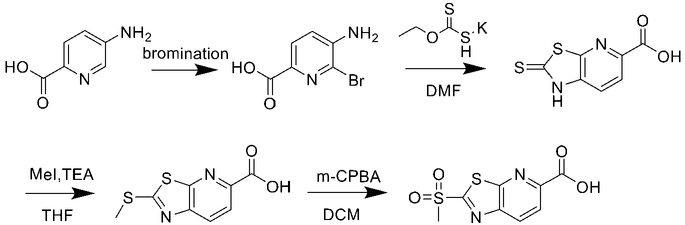

### Preparation method 6.

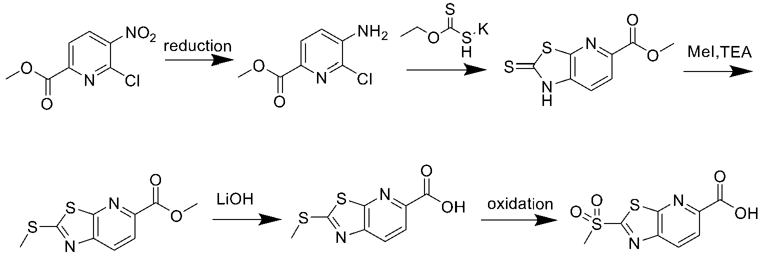

The advantages of the coupling moiety based on the 2-(methylsulfonyl)thiazolo[5,4-b]pyridine structure include:
1. The product after the reaction with thiol groups exhibits higher stability than traditional coupling moiety products;
2. It has higher hydrophilicity than traditional coupling moiety products;
3. It exhibits good reaction kinetics with thiol groups;
4. The reaction with thiol groups under mild conditions exhibits high selectivity;
5. It is generally suitable for all kinds of biomacromolecules and small molecules containing thiol groups;
6. The raw materials for the coupling moiety are readily available, the preparation process is simple, and the reaction yield is high.

Therefore, in one aspect, the present disclosure provides a compound of formula (I), or a pharmaceutically acceptable salt or isotopic variant thereof: 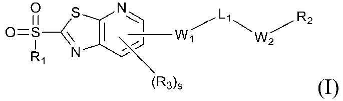 wherein,
R₁ is selected from C₁₋₆ alkyl or C₁₋₆ haloalkyl;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
Li is a chemical bond or -(CH₂)ₘ₁-(OCH₂CH₂)ₙ₁-(CH₂CH₂O)ₙ₂-(CH₂)ᵣ₁-(L)_{q}-(CH₂)ᵣ₂-(OCH₂CH₂)ₙ₃-(CH₂CH₂O)ₙ₄-(CH₂)ₘ₂-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene-, or -5- to 10-membered heteroarylene;
each m1, m2, n1, n2, n3, n4, r1, and r2 is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
R₂ is selected from H, D, halogen, -ORₐ, -NR_{b}R_{c}, or the following groups: 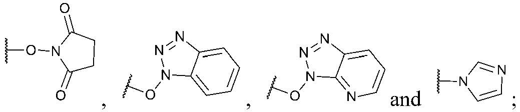
R₃ is selected from H, D, halogen, C₁₋₆ alkyl, or C₁₋₆ haloalkyl;
s = 0, 1, or 2;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

In another aspect, the present disclosure provides the use of the compound of formula (I), or a pharmaceutically acceptable salt or isotopic variant thereof as described above for the manufacture of an antibody-drug conjugate.

In another aspect, the present disclosure provides a compound of formula (II), or a pharmaceutically acceptable salt, prodrug, hydrate, solvate, enantiomer, diastereomer, mesomer, racemate or tautomer thereof: 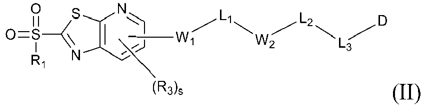 wherein,
R₁ is selected from C₁₋₆ alkyl or C₁₋₆ haloalkyl;
R₃ is selected from H, D, halogen, C₁₋₆ alkyl, or C₁₋₆ haloalkyl;
s = 0, 1, or 2;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
L₁ is a chemical bond or -(CH₂)ₘ-(OCH₂CH₂)ₙ-(CH₂CH₂O)ₙ-(CH₂)ᵣ-(L)_{q}-(CH₂)ᵣ-(OCH₂CH₂)ₙ-(CH₂CH₂O)ₙ-(CH₂)ₘ-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene-, or -5- to 10-membered heteroarylene;
each m, n, and r is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
L₂ is selected from the following amino acid residues or oligopeptide residues consisting of 2 to 10 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄;
wherein N-terminal of amino acid residue or oligopeptide residue is connected to W₂, and the C-terminal is connected to L₃;
R₄ is selected from D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 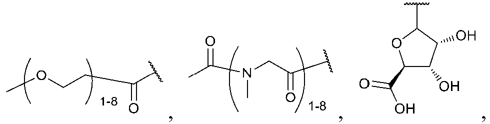 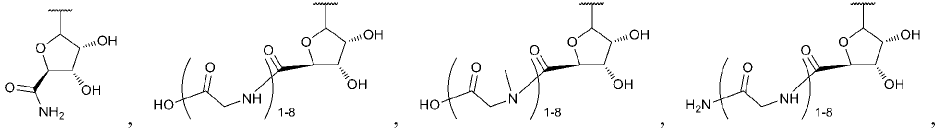 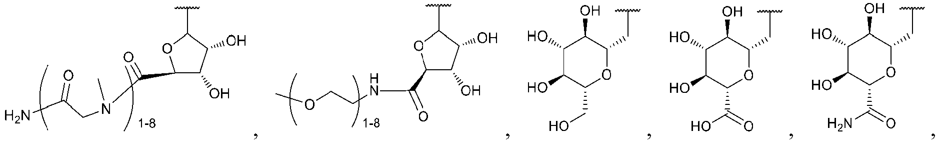 or 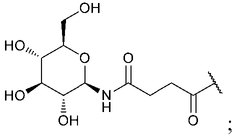
L₃ is selected from the following structures: 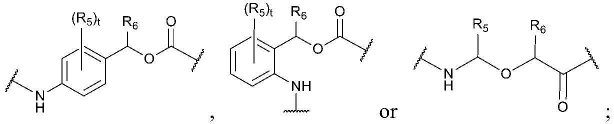
wherein NH is connected to L₂, and C(O) is connected to D;
R₅ is selected from H, D, halogen, NO₂, -ORₐ, -NR_{b}R_{c}, C₁₋₆ alkyl or C₁₋₆ haloalkyl;
t = 0, 1, 2, 3, or 4;
R₆ is selected from H, 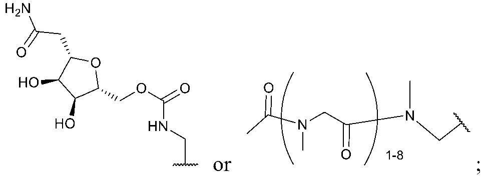
D is an active compound selected from a drug, a cytotoxin, a detection reagent, a diagnostic reagent or a targeting carrier;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

In another aspect, the present disclosure provides a compound of formula (III), or a pharmaceutically acceptable salt, prodrug, hydrate, solvate, enantiomer, diastereomer, mesomer, racemate or tautomer thereof, having the following general formula: 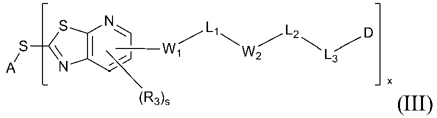 wherein,
A is a targeting molecule;
x = 1, 2, 3, 4, 5, 6, 7 or 8;
R₃ is selected from H, D, halogen, C₁₋₆ alkyl, or C₁₋₆ haloalkyl;
s = 0, 1, or 2;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
L ₁ is a chemical bond or -(CH₂)ₘ-(OCH₂CH₂)ₙ-(CH₂CH₂O)ₙ-(CH₂)ᵣ-(L)_{q}-(CH₂)ᵣ-(OCH₂CH₂)ₙ-(CH₂CH₂O)ₙ-(CH₂)ₘ-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene- or -5- to 10-membered heteroarylene;
each m, n, and r is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
L₂ is selected from the following amino acid residues or oligopeptide residues consisting of 2 to 10 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄;
wherein N-terminal of amino acid residue or oligopeptide residue is connected to W₂, and the C-terminal is connected to L₃;
R₄ is selected from D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 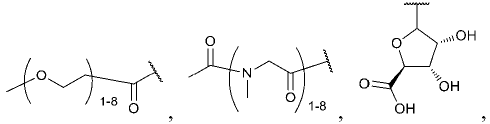 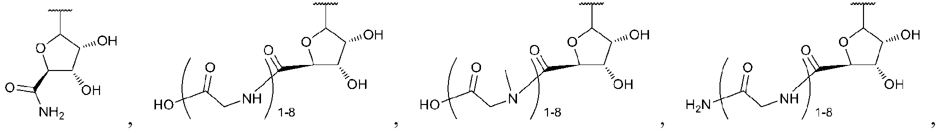 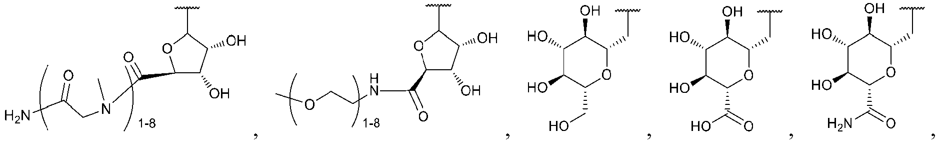 or 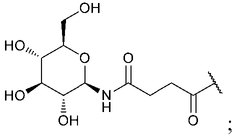
L₃ is selected from the following structures: 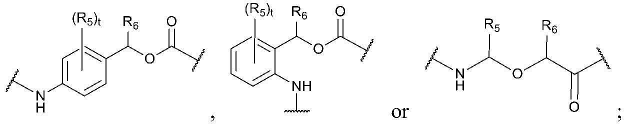
wherein NH is connected to L₂, and C(O) is connected to D;
R₅ is selected from H, D, halogen, NO₂, -ORₐ, -NR_{b}R_{c}, C₁₋₆ alkyl or C₁₋₆ haloalkyl;
t = 0, 1, 2, 3, or 4;
R₆ is selected from H, 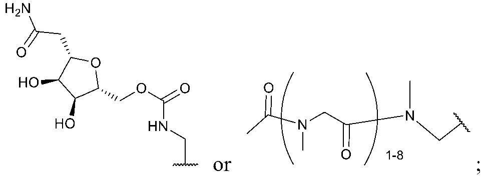
D is an active compound selected from a drug, a cytotoxin, a detection reagent, a diagnostic reagent or a targeting carrier;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

In another aspect, the present disclosure provides a pharmaceutical composition comprising the compound described herein or a pharmaceutically acceptable salt, prodrug, hydrate, solvate, enantiomer, diastereomer, mesomer, racemate, or tautomer thereof, and a pharmaceutically acceptable excipient or adjuvant.

In another aspect, the present disclosure provides the use of the compound described herein or a pharmaceutically acceptable salt, prodrug, hydrate, solvate, enantiomer, diastereomer, mesomer, racemate, or tautomer thereof in the manufacture of a medicament for treating a disease or condition and for reducing the severity of said disease or condition.

In another aspect, the present disclosure provides the compound described herein or a pharmaceutically acceptable salt, prodrug, hydrate, solvate, enantiomer, diastereomer, mesomer, racemate, or tautomer thereof, or the pharmaceutical composition described herein, for use in the treatment of a disease or condition and for use in the reduction of the severity of said disease or condition.

In another aspect, the present disclosure provides a method for treating a disease or condition and for reducing the severity of said disease or condition in a subject, comprising administering to the subject the compound described herein or a pharmaceutically acceptable salt, prodrug, hydrate, solvate, enantiomer, diastereomer, mesomer, racemate or tautomer, or the pharmaceutical composition described herein.

In another aspect, the present application provides a method for preparing an antibody-drug conjugate, comprising reducing the disulfide bond in the hinge region of the antibody or antibody fragment to generate a pair of cysteine residues, and performing substitution reaction between the thiol group in the cysteine residues and the linker of the compound of the disclosure to attach the compound of the present disclosure to the thiol group on cysteine of the antibody or antibody fragment, thereby obtaining an antibody-drug conjugate. The drug-antibody coupling ratio (DAR) can be controlled according to the reaction conditions, for example, typically between 2 and 8.

In another aspect, DAR (that is, the average molar ratio of the drug molecule to the monoclonal antibody molecule in the antibody-drug conjugate obtained by coupling a single monoclonal antibody molecule with a cytotoxic drug) can generally be determined by methods such as Hydrophobic-Interaction Chromatography (HIC), Reverse phase HPLC (RP-HPLC), polyacrylamide-SDS gel electrophoresis (SDS PAGE), liquid chromatograph-mass spectrometer (LC-MS), and ultraviolet/visible spectroscopy (UV/Vis).

### BRIEF DESCRIPTION OF DRAWINGS

Fig. 1: Construction of ADC based on maleimide coupling moiety and the off-target reaction of cytotoxin of the product.
Fig. 2: Stability study of the ADC of the present disclosure in human plasma.
Fig. 3: Stability study of the ADC of the present disclosure in mouse plasma.
Fig. 4: In vitro cytotoxicity studies of MMAE, Dxd, compound-41 and staurosporine.
Fig. 5: In vivo efficacy study of ADC-14B.
Fig. 6: Pharmacodynamic comparison studies of ADC-14B with DS7300-43 and IgG-14B.
Fig. 7: In vivo efficacy study of HDC-14E (DAR4).

### DETAILED DESCRIPTION

### Definitions

### Chemical definitions

The definitions of specific functional groups and chemical terms are described in more detail below.

When a range of values is listed, it is intended to encompass each value and sub-range within the range. For example, "C₁₋₆ alkyl" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"C₁₋₆ alkyl" refers to a linear or branched, saturated hydrocarbon group having 1 to 6 carbon atoms. In some embodiments, C₁₋₄ alkyl is alternative. In some embodiments, C₁₋₂ alkyl is alternative. Examples of C₁₋₆ alkyl include: methyl (C₁), ethyl (C₂), n-propyl (C₃), iso-propyl (C₃), n-butyl (C₄), t-butyl (C₄), sec-butyl (C₄), iso-butyl (C₄), n-pentyl (C₅), 3-pentyl (C₅), pentyl (C₅), neo-pentyl (C₅), 3-methyl-2-butyl (C₅), t-pentyl (C₅) and n-hexyl (C₆). The term "C₁₋₆ alkyl" also includes heteroalkyl, wherein one or more (e.g., 1, 2, 3, or 4) carbon atoms are replaced by heteroatoms (e.g., oxygen (O), sulfur (S), nitrogen (N), boron (B), silicon (Si), phosphorus (P)). The alkyl group may be optionally substituted with one or more substituents, for example, substituted with 1 to 5 substituents, 1 to 3 substituents, or 1 substituent. Conventional alkyl abbreviations include: Me(-CH₃), Et(-CH₂CH₃), iPr(-CH(CH₃)₂), nPr(-CH₂CH₂CH₃), n-Bu(-CH₂CH₂CH₂CH₃), or i-Bu(-CH₂CH(CH₃)₂).

"Halo" or "halogen" refers to fluorine (F), chlorine (Cl), bromine (Br), and iodine (I).

Therefore, "C₁₋₆ haloalkyl" refers to the aforementioned "C₁₋₆ alkyl", which is substituted with one or more halogen groups. In some embodiments, C₁₋₃ haloalkyl is particularly preferred, and C₁₋₂ haloalkyl is more preferred. Exemplary haloalkyl groups include, but are not limited to: -CF₃, -CH₂F, -CHF₂, -CHFCH₂F,-CH₂CHF₂, -CF₂CF₃, -CCl₃, -CH₂Cl, -CHCl₂, 2,2,2-trifluoro-1,1-dimethyl-ethyl, etc. The haloalkyl group can be substituted at any available point of attachment, for example, by 1 to 5 substituents, 1 to 3 substituents, or 1 substituent.

"C₂₋₆ alkenyl" refers to a linear or branched, hydrocarbon group having 2-6 carbon atoms and one or more carbon-carbon double bonds (e.g., 1, 2, or 3 carbon-carbon double bonds). One or more carbon-carbon double bonds can be internal (e.g., in 2-butenyl) or terminal (e.g., in 1-butenyl). In some embodiments, C₂₋₄ alkenyl is alternative. Examples of alkenyl include, but are not limited to, vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. Regardless of whether or not the alkenyl group is modified with "substituted", each alkenyl group is independently optionally substituted, for example, with 1 to 5 substituents, 1 to 3 substituents or 1 substituent. Appropriate substituents are defined as follows.

"C₂₋₆ alkynyl" refers to a linear or branched, hydrocarbon group having 2-6 carbon atoms, one or more carbon-carbon triple bonds (e.g., 1, 2 or 3 carbon-carbon triple bonds) and optionally one or more carbon-carbon double bonds (e.g., 1, 2 or 3 carbon-carbon double bonds). In some embodiments, C₂₋₄ alkynyl is alternative. In some embodiments, alkynyl does not contain any double bond. One or more carbon-carbon triple bonds can be internal (e.g., in 2-butynyl) or terminal (e.g., in 1-butynyl). Examples of alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. Regardless of whether or not the alkynyl group is modified with "substituted", each alkynyl group is independently optionally substituted, for example, with 1 to 5 substituents, 1 to 3 substituents or 1 substituent. Appropriate substituents are defined as follows.

"C₃₋₁₀ cycloalkyl" refers to a non-aromatic cyclic hydrocarbon group having 3-10 ring carbon atoms and zero heteroatoms. In some embodiments, C₃₋₈ cycloalkyl and C₄₋₆ cycloalkyl is alternative, C₃₋₆ cycloalkyl is alternative, and C₅₋₆ cycloalkyl are yet alternative. Cycloalkyl also includes a ring system in which the above cycloalkyl ring is fused with one or more aryl or heteroaryl groups, wherein the point of attachment is on the cycloalkyl ring, and in such instances, the number of carbons continue to designate the number of carbons in the cycloalkyl system. Examples of cycloalkyl include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-1H-indenyl (C₉), decahydronaphthyl (C₁₀), spiro[4.5]decyl (C₁₀), bornyl, adamantyl, etc. Regardless of whether or not the cycloalkyl group is modified with "substituted", each cycloalkyl group is independently optionally substituted, for example, with 1 to 5 substituents, 1 to 3 substituents or 1 substituent. Appropriate substituents are defined as follows.

"C₃₋₈ cycloalkylene" refers to a divalent group formed by removing another hydrogen atom from "C₃₋₈ cycloalkyl".

"3- to 10-membered heterocyclyl" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon. In heterocyclyl groups that contain one or more nitrogen atoms, the point of attachment can be a carbon or nitrogen atom, as valency permits. In some embodiments, 4-to 10-membered heterocyclyl is alternative, and it is a 4- to 10-membered non-aromatic ring system having ring carbon atoms and 1-4 ring heteroatoms; in some embodiments, 5- to 10-membered heterocyclyl is alternative, and it is a 5- to 10-membered non-aromatic ring systems having cyclic carbon atoms and 1-4 cyclic heteroatoms; in some embodiments, 3- to 8-membered heterocyclyl is alternative, and it is a 3- to 8-membered non-aromatic ring systems having cyclic carbon atoms and 1-3 cyclic heteroatoms; in some embodiments, 3- to 6-membered heterocyclyl is alternative, and it is a 3- to 6-membered non-aromatic ring system having ring carbon atoms and 1-3 ring heteroatoms. 5- to 6-membered heterocyclyl is yet alternative, and it is a 5- to 6-membered non-aromatic ring system having ring carbon atoms and 1-3 ring heteroatoms. "Heterocyclyl" also includes ring systems wherein the heterocyclyl, as defined above, is fused with one or more cycloalkyl, aryl or heteroaryl groups wherein the point of attachment is on the heterocyclyl ring, and in such instances, the number of ring members continues to designate the number of ring members in the heterocyclyl ring system. Regardless of whether or not the heterocyclyl group is modified with "substituted", each heterocyclyl group is independently optionally substituted, for example, with 1 to 5 substituents, 1 to 3 substituents or 1 substituent. Appropriate substituents are defined as follows.

"3- to 8-membered heterocyclylene" refers to a divalent group formed by removing another hydrogen atom from "3-8 membered heterocyclyl".

Exemplary 3-membered heterocyclyl groups containing one heteroatom include, without limitation, aziridinyl, oxiranyl, thiiranyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing three heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclyl) include, without limitation, indolinyl, isoindolinyl, dihydrobenzofuranyl, dihydrobenzothienyl, benzoxazolinonyl, and the like. Exemplary 6-membered heterocyclyl groups fused to a C₆ aryl ring (also referred to herein as a 6,6-bicyclic heterocyclyl) include, without limitation, tetrahydroquinolinyl, tetrahydroisoquinolinyl, and the like.

"C₆₋₁₀ aryl" refers to a radical of a monocyclic or polycyclic (e.g., bicyclic or tricyclic) 4n+2 aromatic ring system (e.g., having 6 or 10 π electrons shared in a cyclic array) having 6-10 ring carbon atoms and zero heteroatoms. In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, C₆₋₁₀ aryl is alternative, and C₆ aryl is yet alternative. "Aryl" also includes ring systems wherein the aryl ring, as defined above, is fused with one or more cycloalkyl or heterocyclyl groups wherein the point of attachment is on the aryl ring, and in such instances, the number of carbon atoms continues to designate the number of carbon atoms in the aryl ring system. Regardless of whether or not the aryl group is modified with "substituted", each aryl group is independently optionally substituted, for example, with 1 to 5 substituents, 1 to 3 substituents or 1 substituent. Appropriate substituents are defined as follows.

"C₆₋₁₀ arylene" refers to a divalent group formed by removing another hydrogen atom from "C₆₋₁₀ aryl".

"5- to 10-membered heteroaryl" refers to a radical of a 5- to 10-membered monocyclic or bicyclic 4n+2 aromatic ring system (e.g., having 6 or 10 pi electrons shared in a cyclic array) having ring carbon atoms and 1-4 ring heteroatoms, wherein each heteroatom is independently selected from nitrogen, oxygen and sulfur. In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more cycloalkyl or heterocyclyl groups wherein the point of attachment is on the heteroaryl ring, and in such instances, the number of carbon atoms continues to designate the number of carbon atoms in the heteroaryl ring system. In some embodiments, 5- to 6-membered heteroaryl is alternative, and it is a 5- to 6-membered monocyclic or bicyclic 4n+2 aromatic ring system having ring carbon atoms and 1-4 ring heteroatoms. In some embodiments, 5-membered heteroaryl is alternative, and it is a 5-membered monocyclic or bicyclic 4n+2 aromatic ring system having ring carbon atoms and 1-4 ring heteroatoms. Regardless of whether or not the heteroaryl group is modified with "substituted", each heteroaryl group is independently optionally substituted, for example, with 1 to 5 substituents, 1 to 3 substituents or 1 substituent. Appropriate substituents are defined as follows.

"5- to 10-membered heteroarylene" refers to a divalent group formed by removing another hydrogen atom from "5- to 10-membered heteroaryl".

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzothiazolyl, benzisothiazolyl, benzothiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

In the present application, the term "optional" or "optionally" generally means that the event or circumstance described subsequently may but does not have to occur, and the description includes instances where the event or circumstance occurs and instances where it does not occur. For example, "heterocyclic group optionally substituted with alkyl" means that the alkyl may but does not have to be present, and the description can include the case where the heterocyclic group is substituted with alkyl and the case where the heterocyclic group is not substituted with alkyl.

Exemplary substituents on carbon atoms include, but are not limited to, halo, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H,-CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa},-NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa},-C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa},-SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂,-C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa},-OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂,-B(OR^{cc})₂, -BR^{aa}(OR^{cc}), alkyl, haloalkyl, alkenyl, alkynyl, carbocyclyl, heterocyclyl, aryl and heteroaryl, wherein each of alkyl, alkenyl, alkynyl, carbocyclyl, heterocyclyl, aryl and heteroaryl is independently substituted with 0, 1, 2, 3, 4 or 5 R^{dd} groups;
or two geminal hydrogens on a carbon atom are replaced with the group =O, =S, =NN(R^{bb})₂, -NNR^{bb}C(=O)R^{aa}, -NNR^{bb}C(=O)OR^{aa}, =NNR^{bb}S(=O)₂R^{aa}, =NR^{bb} or =NOR^{cc};
each R^{aa} is independently selected from alkyl, haloalkyl, alkenyl, alkynyl, carbocyclyl, heterocyclyl, aryl and heteroaryl, or two R^{aa} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups;
each R^{bb} is independently selected from: hydrogen, -OH, -OR^{aa}, -N(R^{cc})₂, -CN, -C(=O)R^{aa},-C(=O)N(R^{cc})₂, -CO₂R^{aa}, -SO₂R^{aa}, -C(=NR^{cc})OR^{aa}, -C(-NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, carbocyclyl, heterocyclyl, aryl and heteroaryl, or two R^{bb} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups;
each R^{cc} is independently selected from hydrogen, alkyl, haloalkyl, alkenyl, alkynyl, carbocyclyl, heterocyclyl, aryl and heteroaryl, or two R^{cc} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups;
each R^{dd} is independently selected from: halo, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee},-C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee},-OC(-NR^{ff})R^{ee}, -OC(=NR^{ff})OR^{ee}, -C(-NR^{ff})N(R^{ff})₂, -OC(-NR^{ff})N(R^{ff})₂, -NR^{ff}C(-NR^{ff})N(R^{ff})₂, -NR^{ff}SO₂R^{ee},-SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee},-C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, carbocyclyl, heterocyclyl, aryl, and heteroaryl, wherein each of alkyl, alkenyl, alkynyl, carbocyclyl, heterocyclyl, aryl and heteroaryl is independently substituted with 0, 1, 2, 3, 4 or 5 R^{gg} groups, or two geminal R^{dd} substituents can be bound to form =O or =S;
each R^{ee} is independently selected from alkyl, haloalkyl, alkenyl, alkynyl, carbocyclyl, aryl, heterocyclyl and heteroaryl, wherein each of alkyl, alkenyl, alkynyl, carbocyclyl, heterocyclyl, aryl and heteroaryl is independently substituted with 0, 1, 2, 3, 4 or 5 R^{gg} groups;
each R^{ff} is independently selected from hydrogen, alkyl, haloalkyl, alkenyl, alkynyl, carbocyclyl, heterocyclyl, aryl and heteroaryl, or two R^{ff} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{gg} groups;
each R^{gg} is independently: halo, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl),-NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂,-NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂,-SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ carbocyclyl, C₆-C₁₀ aryl, C₃-C₇ heterocyclyl, or C₅-C₁₀ heteroaryl; or two geminal R^{gg} substituents can be bound to form =O or =S, wherein X⁻ is a counter ion.

Exemplary substituents on the nitrogen atom include but are not limited to, hydrogen, -OH, -OR^{aa},-N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂,-P(=O)₂N(R^{cc})₂, -P(=O)(NR^{cc})₂, alkyl, haloalkyl, alkenyl, alkynyl, carbocyclyl, heterocyclyl, aryl and heteroaryl, or two R^{cc} groups connected to the nitrogen atom are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups, and wherein R^{aa}, R^{bb}, R^{cc} and R^{dd} are as defined above.

"Deuterated" or "deuterium" refers to one or more hydrogen in the compound or group is substituted with deuterium; deuteration can be mono-substituted, di-substituted, poly-substituted, or per-substituted. The term "one or more deuterated" and "one or more deuteration events" are used interchangeably. The deuterium isotope content at the deuterated position is at least 0.015% greater than the natural deuterium isotope content, alternatively greater than 30%, still alternatively greater than 50%, still alternatively greater than 75%, still alternatively greater than 95%, and still alternatively greater than 99%.

In the present application, the term "compound" generally refers to a substance having two or more different elements. For example, the compound of the present application may be an organic compound, for example, the compound of the present application may be a compound with a molecular weight of less than 500, a compound with a molecular weight of less than 1000, a compound with a molecular weight of more than 1000, or a compound with a molecular weight of more than 10,000 or 100,000. In the present application, the compound may also be a compound linked by a chemical bond, for example, the compound may be a compound in which one or more molecules with a molecular weight of less than 1000 are linked by a chemical bond to a biomacromolecule, and the biomacromolecule may be a polysaccharide, a protein, a nucleic acid, a polypeptide, or the like. For example, the compound of the present application may include a compound in which a protein is linked to one or more molecules with a molecular weight of less than 1000, a compound in which a protein is linked to one or more molecules with a molecular weight of less than 10,000, or a compound in which a protein is linked to one or more molecules with a molecular weight of less than 100,000.

As used herein, the term "compound of the present application" refers to the compound of the present application. The term also includes various pharmaceutically acceptable salts, prodrugs, hydrates, solvates, enantiomers, diastereomers, mesomers, racemates, or tautomers of the compound of the present application.

### Biological definition

In the present application, the term "ligand" generally refers to a macromolecular compound that recognizes and binds to cell-associated antigens or receptors. The role of the ligand can be to present a drug to a ligand-bound cell population; these ligands include, but are not limited to, protein hormones, lectins, growth factors, antibodies, or other molecules that can bind to cells, receptors, and/or antigens. In the present application, the ligand can be represented as Ab, and the ligand antigen forms a linking bond with the linking unit via a heteroatom on the ligand. It can be an antibody or an antigen-binding fragment thereof, and the antibody can be selected from a chimeric antibody, a humanized antibody, a fully human antibody or a murine antibody; the antibody can be a monoclonal antibody. For example, the antibody can be an antibody or antigen-binding fragment thereof targeting a target selected from the group consisting of:
HER2, Trop-2, Claudin-6, Claudin-9, Claudin-18.2, EGFR, c-Met, CD19, PSMA, Muc1, BCMA, PD-L1, CD33, CD30, CD22, CD79b, Nectin-4, CD19, tissue factor, FRα, B7-H3, B7-H4, CDH3, CDH6, CDH17, ALPP, CD56, CD37, HER3, ROR1, MSLN, TNF-α, CD25, ENPP3, Muc1, Axl, CD20, ROR2, GPNMB, CEACAM5, CEACAM6, CD138, GC-C, LIV-1, CA6, FUT3, IGF-1R, CTLA4, RNF43, DPEP3, 5T4, ITGB6, EFNA4, CD228, Notch3, CD46, CAIX, SLAMF6, ADAM9, GD3, TDGF1, SLAMF2, CLL-1, CD123, FCRL5, TIM1, sTn, ETB, Globo H, CD38, Ly6E, SLITRK6, GPR20, FGFR2, Muc16, CD51, SLAMF7, LAMP-1, CD74, CCR7, PTK7, SEZ6, LYFD3, TAA, PRL receptor, FGFR3, KAAG1, STEAP1, Flt3, Muc1, LRRC15, CD44, CD70, EphA2, CXCR4, DDR1, DKL1, FOLR, CD45, DSG2, ALK, TRAIL, EpCAM, VEGFR2, CD47, CD49, SSEA-4, DCLK1, OAcGD2, CD73, ENO1, BSG, CD24, GLUT1, and GPRC5D, alternatively HER2, HER3, EGFR, Trop-2, Claudin-6, Claudin-18.2, B7-H3, B7-H4, CDH3, CDH6, CDH17, FRα, ROR1, ALPP, CEACAM5, CEACAM6 or FOLR.

In the present application, the term "conjugate" generally refers to a compound prepared by one or more chemical reactions of a compound of the present application, or a compound that is linked to each other by one or more linking structures such as a bridge, spacer, or linking moiety.

In the present application, the term "HER2" generally refers to human epidermal growth factor receptor 2 (HER2), for example, the term "HER2" refers to any natural HER2 from any human source. The term also encompasses "full-length" and unprocessed HER2, as well as any form of HER2 derived from processing in cells (e.g., mature protein). The term also encompasses naturally occurring variants and isotypes of HER2, such as splice variants or allelic variants. For example, Uniprot accession number P04626 provides a description of HER2 and sequence.

In the present application, the term "B7-H3" and "CD276" generally refer to a type I transmembrane protein belonging to the B7 immune co-stimulatory and co-inhibitory family. The B7-H3 protein is encoded by the chromosome 15q24 gene and structurally consists of 316 amino acids including an extracellular domain, a transmembrane domain, and a short intracellular domain. The intracellular domain of the B7-H3 protein is very short, and there is no known signal motif.

In the present application, the term "chimeric antibody" generally refers to an antibody formed by fusing the variable region of a murine antibody with the constant region of a human antibody, which can alleviate the immune response induced by murine antibodies. To establish a chimeric antibody, a hybridoma that secretes murine-specific monoclonal antibodies can be created. The variable region gene can then be cloned from the murine hybridoma cells, and the constant region gene of a human antibody can be cloned as needed. The murine variable region gene and the human constant region gene can be linked to form a chimeric gene, which is then inserted into an expression vector. The chimeric antibody molecule can then be expressed in eukaryotic or prokaryotic systems.

In the present application, the term "humanized antibody", also known as a CDR-grafted antibody, generally refers to an antibody generated by grafting murine CDR sequences into human antibody variable region frameworks, i.e., framework sequences of different types of human germline antibodies. It can overcome the heterologous reaction resulting from a large amount of murine protein components carried in chimeric antibodies. Such framework sequences can be obtained from public DNA databases or published references containing germline antibody gene sequences. For example, germline DNA sequences of human heavy chain and light chain variable region genes can be found in the "VBase" human germline sequence database.

In the present application, the term "fully human-derived antibody", "fully human antibody", or "whole human-derived antibody", also known as "fully human-derived monoclonal antibody", refer to an antibody whose variable and constant regions can both be human-derived, thus eliminating immunogenicity and toxic side effects. The development of monoclonal antibodies has undergone four stages: murine monoclonal antibodies, chimeric monoclonal antibodies, humanized monoclonal antibodies, and fully human-derived monoclonal antibodies. The antibody or ligand described in the present application can be a fully human-derived monoclonal antibody. The related technology for the preparation of the fully human antibody can be: human hybridoma technology, EBV-transformed B lymphocyte technology, phage display technology, transgenic mouse antibody preparation technology, and single B cell antibody preparation technology, etc.

In the present application, the term "CDR" generally refers to one of the six hypervariable regions within the variable domain of an antibody that primarily facilitate antigen binding. One of the most commonly used definitions of the six CDRs is provided by Kabat EA et al., Chothia et al., and MacCallum et al. As used in the present application, the Kabat definition of CDRs can be applied to CDR1, CDR2, and CDR3 of the light chain variable domain (CDRL1, CDRL2, CDRL3 or LK L2, L3), and CDRK, CDR2, and CDR3 of the heavy chain variable domain (CDRH1, CDRH2, CDRH3 or HI, H2, H3).

In the present application, the term "linker" generally refers to a chemical structural fragment or bond, one end of which is attached to one group and the other end of which is attached to the other group, and it can also be attached to the other linker and then attached to the drug and/or ligand. The direct or indirect connection with a ligand can refer to the group directly connecting to the ligand via a covalent bond, or it can refer to the group connecting to the ligand via a linker. For example, chemical structural fragments or bonds comprising acid-labile coupling moiety structures (e.g., hydrazones), protease-sensitive (e.g., peptidase-sensitive) coupling moiety structures, light-labile coupling moiety structures, dimethyl coupling moiety structures, or disulfide-containing coupling moiety structures can be used as linkers.

In the present application, the term "linking group" generally refers to a group that has the ability to be linked to another group. For example, a compound having a linking group can achieve the linkage of the compound to another group through a coupling reaction between the linking group and another group. For example, a maleimide group can serve as a linking group.

In the present application, the term "pharmaceutical unit" generally refers to a chemical moiety that is directly or indirectly conjugated to an antibody or antigen-binding fragment to form an immunoconjugate. For example, "pharmaceutical unit" includes, but is not limited to, compounds with antitumor activity described herein. For example, pharmaceutical units include topoisomerase inhibitors.

In the present application, the term "disease associated with the expression" of a target generally refers to a disease in which the occurrence and/or progression of the disease is associated with the expression level of the target. For example, high expression means that the expression level of a target is increased in cells from a disease region, such as a specific tissue or organ of a patient, relative to the expression level in normal cells from the tissue or organ. Alternatively, for example, low expression means that the expression level of a target is decreased in cells from a disease region, such as a specific tissue or organ of a patient, relative to the expression level in normal cells from the tissue or organ. Alternatively, for example, positive expression means that cells from a disease region, such as a specific tissue or organ of a patient, express a target. Alternatively, for example, negative expression means that cells from a disease region, such as a specific tissue or organ of a patient, do not express a target. For example, the characteristics of target expression can be determined by standard assays known in the art.

As used herein, the term "antibody" is used in its broadest sense and specifically covers monoclonal antibodies, polyclonal antibodies, dimers, multimers, multispecific antibodies (e.g., bispecific antibodies), and antibody fragments, provided they exhibit the desired biological activity. Antibodies can be mouse, human, humanized, chimeric antibody, or derived from other species. The antibody is a protein produced by an immune system capable of recognizing and binding specific antigens. The target antigen typically has a large number of binding sites, also known as epitopes, recognized by the CDRs of a variety of antibodies. Each antibody that specifically binds to a different epitope has a different structure. Therefore, an antigen can have more than one corresponding antibody. Antibodies include full-length immunoglobulin molecules or the immunoactive portion of full-length immunoglobulin molecules, i.e., molecules that are specifically binding to an antigen or a portion thereof of a target of interest, including, but not limited to, cancer cells or cells that produce autoimmune antibodies associated with autoimmune diseases. The immunoglobulins described in the present application may have any type (e.g., IgG, IgE, IgM, IgD, and IgA), class (e.g., IgG1, IgG2, IgG3, IgG4, IgG1, and IgA2), or subclass of immunoglobulin molecules. Immunoglobulins may be derived from any species. However, in one aspect, the immunoglobulin is derived from human, murine or rabbit. An "antibody fragment" may comprise a portion of a full-length antibody, typically an antigen-binding region or a variable region thereof. Examples of the antibody fragment include: Fab, Fab\F(ab')2, and Fv fragments; a diabody; a linear antibody; a minibody; a fragment prepared from a Fab expression library; an anti-idiotypic (anti-Id) antibody; CDR (complementarity-determining regions); and any of the above epitope-binding fragments that bind to cancer cell antigens, viral antigens, or microbial antigens in an immune-specific manner; a single chain antibody molecule; and a multispecific antibody formed from the antibody fragment. The antibody forming the antibody drug conjugate in the application can keep the antigen binding ability in the original wild state. Therefore, the antibody of the present application may, for example, specifically bind to the antigen. The antigens involved include, for example, tumor-associated antigens (TAAs), cell surface receptor proteins and other cell surface molecules, cell survival regulators, cell proliferation regulators, molecules associated with tissue growth and differentiation (such as those known or anticipated to be functional), lymphokines, cytokines, molecules involved in cell circulation regulation, molecules involved in angiogenesis, and molecules related to angiogenesis (e.g., antigens bound by known antibodies may be one or a subset of the above classes, while other subsets contain other molecules/antigens with specific properties (compared to the antigen of interest)). Antibodies used in antibody-drug conjugates include, but are not limited to, antibodies against cell surface receptors and tumor-associated antigens. Such tumor-associated antigens are well-known in the industry and can be prepared using well-known antibody preparation methods and information in the art. These targets can be specifically expressed on the surface of one or more cancer cells, while being expressed sparingly or not at all on the surface of one or more non-cancer cells. Typically, such tumor-associated peptides can be more overexpressed on the surface of cancer cells than on the surface of non-cancer cells.

In the present application, the term "amino acid" refers to the basic structural unit that make up proteins and is the basis for the biological post-protein modification, with a total of 20 natural amino acids. In addition, on the basis of these basic amino acids, organisms can also synthesize amino acid types derived such as hydroxyproline, hydroxylysine. In fireflies, even D-type amino acids are synthesized. Naturally occurring amino acids are generally L-type, but the present disclosure also includes D-type amino acids. The amino acids used in the present disclosure are shown in the table below:

| | Abbreviation | Name | Nature | Side chain |
|---|---|---|---|---|
| Gly | G | Glycine | hydrophilic | -H |
| Ala | A | Alanine | hydrophobic | -CH₃ |
| Val | V | Valine | hydrophobic | -CH-(CH₃)₂ |
| Leu | L | Leucine | hydrophobic | -CH₂-CH(CH₃)₂ |
| Ile | I | Isoleucine | hydrophobic | -CH(CH₃)-CH₂-CH₃ |
| Phe | F | Phenylalanine | hydrophobic | -CH₂-C₆H₅ |
| Trp | W | Tryptophan | hydrophobic | -C₈NH₆ |
| Tyr | Y | Tyrosine | hydrophilic | -CH₂-C₆H₄-OH |
| Asp | D | Aspartic acid | acidic | -CH₂-COOH |
| Asn | N | Asparagine | hydrophilic | -CH₂-CONH₂ |
| Glu | E | Glutamic acid | acidic | -(CH₂)₂-COOH |
| Lys | K | Lysine | alkaline | -(CH₂)₄-NH₂ |
| Gln | Q | Glutamine | hydrophilic | -(CH₂)₂-CONH₂ |
| Met | M | Methionine | hydrophobic | -(CH₂)₂-S-CH₃ |
| Ser | S | Serine | hydrophilic | -CH₂-OH |
| Thr | T | Threonine | hydrophilic | -CH(CH₃)-OH |
| Cys | C | Cysteine | hydrophilic | -CH₂-SH |
| Pro | P | Proline | hydrophobic | -C₃H₆-, which is linked to the amino. |
| His | H | Histidine | alkaline | -CH₂-C₃H₃N₂ |
| Arg | R | Arginine | alkaline | -(CH₂)₃-NHC(NH)NH₂ |
| Cit | - | Citrulline | alkaline | -(CH₂)₃-NHC(O)NH₂ |

In the present application, the term "polypeptide residue/oligopeptide residue" generally refers to a residue comprising two or more amino acid residues linked by a peptide bond. For example, the two or more amino acids in the polypeptide residue may be optionally substituted. For example, the polypeptide residue in the present application may be selected from the group consisting of: valine-citrulline (Val-Cit), valine-alanine (Val-Ala), valine-lysine (Val-Lys), phenylalanine-lysine (Phe-Lys), lysine-lysine (Lys-Lys), alanine-lysine (Ala-Lys), phenylalanine-citrulline (Phe-Cit), leucine-citrulline (Leu-Cit), isoleucine-citrulline (Ile-Cit), phenylalanine-alanine (Phe-Ala), lysine-valine-citrulline (Lys-Val-Cit), lysine-valine-alanine (Lys-Val-Ala), valine-lysine-glycine (Val-Lys-Gly), glycine-valine-lysine (Gly-Val-Lys), glycine-valine-alanine (Gly-Val-Ala), glutamic acid-valine-alanine (Glu-Val-Ala), glutamic acid-valine-citrulline (Glu-Val-Cit), glutamine-valine-alanine (Gln-Val-Ala), glutamine-valine-citrulline (Gln-Val-Cit), alanine-alanine-alanine (Ala-Ala-Ala), alanine-alanine-asparagine (Ala-Ala-Asn), phenylalanine-phenylalanine-lysine (Phe-Phe-Lys), glycine-phenylalanine-lysine (Gly-Phe-Lys), leucine-alanine-leucine (Leu-Ala-Leu), isoleucine-alanine-leucine (Ile-Ala-Leu), valine-alanine-valine (Val-Ala-Val), glycine-glycine-phenylalanine-glycine (Gly-Gly-Phe-Gly), (Ala-Leu-Ala-Leu) and (Gly-Phe-Leu-Gly), alternatively selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), glutamine-valine-alanine (Gln-Val-Ala), glutamine-valine-citrulline (Gln-Val-Cit), valine-lysine (Val-Lys), alanine-alanine-alanine (Ala-Ala-Ala), alanine-alanine-Asparagine (Ala-Ala-Asn) and glycine-glycine-phenylalanine-glycine (Gly-Gly-Phe-Gly), alternatively selected from the group consisting of: valine-citrulline (Val-Cit), valine-alanine (Val-Ala), valine-lysine (Val-Lys), phenylalanine-lysine (Phe-Lys), lysine-valine-citrulline (Lys-Val-Cit), lysine-valine-alanine (Lys-Val-Ala), glutamine-valine-alanine (Gln-Val-Ala), glutamine-valine-citrulline (Gln-Val-Cit) and glycine-glycine-phenylalanine-glycine (Gly-Gly-Phe-Gly), alternatively selected from the group consisting of: valine-citrulline (Val-Cit), valine-alanine (Val-Ala), valine-lysine (Val-Lys), lysine-valine-citrulline (Lys-Val-Cit), lysine-valine-alanine (Lys-Val-Ala) and glycine-glycine-phenylalanine-glycine (Gly-Gly-Phe-Gly).

In the present application, the term "polyethylene glycol group/PEG group" generally refers to a residue comprising one or more ethylene glycol residues linked together. For example, a polyethylene glycol group may contain -(CH₂CH₂O)ₚ-, wherein p is a number of at least 1. For example, the polyethylene glycol group in the present application may be optionally substituted.

In the present application, the term "polysarcosine residue" generally refers to a residue comprising one or more sarcosine residues linked together. For example, a polysarcosine residue may contain - (COCH₂N(CH₃))_{q}-, wherein q is a number of at least 1.

In the present application, the term "pentose" refers to a monosaccharide having five carbon atoms in the molecule, also referred to as a five-carbon sugar. The pentose is widely distributed in the biological field and plays an important role in life activity. It mainly comprises D-xylose, L-arabinose, D-ribose, and its derivative D-2-deoxyribose. Pentuloses that are intermediate products of sugar metabolism include D-ribulose and D-xylulose.

In the present application, the term "hexose" refers to a monosaccharide containing six carbon atoms, also known as a six-carbon sugar. The hexose is the most widely distributed and abundant in nature, and is most closely related to the body's nutritional metabolism. The hexose containing an aldehyde group at position 1 is referred to as an aldohexose, and the hexose containing a ketone group at position 2 is referred to as a ketohexose. Five of the six carbon atoms in a hexose are attached to hydroxyl groups (-OH), and the remaining carbon atom belongs to an aldehyde group (-COH) or a ketone group (-CO-). The important aldohexose include D-glucose, D-galactose, and D-mannose; ketohexose include D-fructose.

In the present application, the term "tumor" generally refers to any new pathological tissue hyperplasia. For the purposes of the present application, angiogenesis is a part of tumor characteristics. The tumor may be benign or malignant. The term "tumor" is generally used to refer to a benign or malignant tumor, while the term "cancer" is generally used to refer to a malignant tumor, which may be metastatic cancer or non-metastatic cancer. The tumor that can be treated with the methods of the present application is selected from the following group: breast cancer, ovarian cancer, non-Hodgkin lymphoma, Hodgkin lymphoma, acute lymphoblastic leukemia, anaplastic large cell lymphoma, multiple myeloma, prostate cancer, malignant melanoma, squamous cell carcinoma, glioblastoma, renal cell carcinoma, gastrointestinal tumors, pancreatic cancer, prostate cancer, rectal cancer, gastric cancer, glioma, and mesothelioma. When used for research purposes, these tissues can be isolated from readily available resources using methods well known to those skilled in the art.

### Other definitions

In the present application, the terms "comprising", "including", and "containing" generally refer to the inclusion of explicitly specified features, but does not exclude other elements. The terms "above" and "below" generally refer to the case in which the stated number is included.

In the present application, the term "about" generally refers to a variation within a range of 0.5% to 10% above or below a specified value, such as a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

As used herein, the term "pharmaceutically acceptable salt" refers to an acid addition salt or a base addition salt of a compound of the present disclosure that, within the scope of sound medical judgment, is suitable for use in contact with patient tissues without undue toxicity, irritation, allergic reaction, etc., commensurate with a reasonable benefit/risk ratio, and effective for its intended use, including, where possible, the zwitterionic form of a compound of the present disclosure..

Pharmaceutically acceptable salts can be sulfates, pyrosulfates, bisulfates, sulfites, bisulfites, nitrates, borates, phosphates, monohydrogen phosphates, dihydrogen phosphates, metaphosphates, pyrophosphates, chlorides, bromides, iodides, etc., prepared from inorganic acids. Representative salts include hydrobromides, hydrochlorides, sulfates, bisulfates, nitrates, borates, and phosphates and the like. Salts can also be prepared from organic acids, such as aliphatic monocarboxylic and dicarboxylic acids, phenyl-substituted alkanoic acids, hydroxyalkanoic acids, alkanedioic acids, aromatic acids, aliphatic and aromatic sulfonic acids. Representative salts include acetates, propionates, valerates, oleates, palmitates, stearates, laurates, caprylates, isobutyrates, oxalates, malonates, succinates, suberates, sebacates, fumarates, mandelates, benzoates, chlorobenzoates, methylbenzoates, dinitrobenzoates, naphthoates, benzenesulfonates, toluenesulfonates, phenylacetates, citrates, lactates, maleates, tartrates, methanesulfonates, gluceptates, lactobionates, laurylsulfonates, and hydroxyethanesulfonates. Pharmaceutically acceptable salts may include alkali metal and alkaline earth metal-based cations, such as sodium, lithium, potassium, calcium, and magnesium, as well as non-toxic ammonium, quaternary ammonium, and amine cations, including but not limited to ammonium, tetramethylammonium, tetraethylammonium, methylamine, dimethylamine, trimethylamine, triethylamine, and ethylamine, and the like. Also encompassed are salts of amino acids, such as arginine salts, gluconates, galacturonates and the like (see, e.g., Berge S. M. et al., "Pharmaceutical Salts,"J. Pharm. Sci., 1977; 66: 1-19, incorporated herein by reference).

The present disclosure also comprises compounds that are labeled with isotopes (isotopic variants), which are equivalent to those general formulas or specific compounds described in the present application, but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds disclosed herein that comprise the above isotopes and/or other isotopes of other atoms, prodrugs thereof and pharmaceutically acceptable salts of said compounds or prodrugs all are within the scope of the present disclosure. Certain isotope-labeled compounds disclosed herein, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are alternative, because they are easy to prepare and detect. Furthermore, replaced by heavier isotopes, such as deuterium (i.e., ²H) may provide therapeutic benefits due to the higher metabolic stability, such as prolonging the half-life *in vivo* or decreasing the dosage requirements, and thus is alternative in some cases. Isotope-labeled compounds of the present disclosure and prodrugs thereof can be prepared generally by using readily available isotope-labeled reagents to replace non-isotope-labeled reagents in the following schemes and/or the procedures disclosed in the examples and preparation examples.

The compounds disclosed herein may include one or more asymmetric centers, and thus may exist in a variety of stereoisomeric forms, for example, enantiomers and/or diastereomers. For example, the compounds disclosed herein may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

In the present application, the term "pharmaceutical composition" generally refers to a mixture containing one or more of the compounds described in the present application, or physiologically/pharmaceutically acceptable salts or prodrugs thereof, and other chemical components, as well as other components such as physiologically/pharmaceutically acceptable carriers and excipients. The pharmaceutical composition can facilitate administration to an organism, which is beneficial for the absorption of the active ingredient so as to exert the biological activity. Conventional methods for preparing pharmaceutical compositions can be seen in the common technology of the art.

In the present application, the term "pharmaceutically acceptable carrier" generally refers to a carrier for administering therapeutic agents, such as antibodies or peptides, genes, and other therapeutic agents. The term refers to any pharmaceutical carrier which itself does not induce antibody production that is detrimental to an individual receiving the composition and which may be administered without excessive toxicity. For example, a pharmaceutically acceptable carrier may be distinguished from a nucleic acid vector used in genetic engineering for containing genes. Suitable carriers can be large, slowly metabolized macromolecules, such as proteins, polysaccharides, polylactic acids, polyglycolic acids, polyamino acids, amino acid copolymers, lipid aggregates, and inactivated viral particles. These carriers are well known to those skilled in the art. Pharmaceutically acceptable carriers in therapeutic compositions may include liquids such as water, saline, glycerol, and ethanol. Auxiliary substances such as wetting agents or emulsifiers, pH buffering substances and the like may also be present in these carriers.

In the present application, the term "compound with anti-tumor activity" generally refers to a compound that has the ability to reduce the proliferation rate, viability, or metastatic activity of tumor cells. For example, anti-tumor activity may be indicated by a reduction in growth rate of abnormal cells occurring during treatment or by a stable or reduced tumor size, or by a longer survival period due to treatment as compared to a control in the absence of treatment. The anti-tumor activity can be assessed using recognized in vitro or in vivo tumor models, such as xenograft models.

In some embodiments of the present disclosure, the bioactive molecule in the conjugate is a compound with antitumor activity, such as: radioactive isotopes, such as radioactive isotopes of At211, I131, I125, Y90, Re186, Re188, Sm153, Bi212, P32, Pb212 or Lu; metal complexes, such as metal platinum complexes (e.g., oxaliplatin) or metal gold complexes; glycopeptide antibiotics, such as bleomycin or pingyangmycin; topoisomerase inhibitors; drugs that interfere with DNA synthesis, such as methotrexate, 5-fluorouracil, cytarabine, gemcitabine, mercaptopurine, pentostatin, fludarabine, cladribine or nelarabine, etc.; drugs that act on structural proteins, such as microtubulin inhibitors (e.g., vinca alkaloids, vincristine, vinblastine, paclitaxel, maytansines, auristatin, Tubulysin B or eribulin, etc.); tumor signaling pathway inhibitors, such as serine/threonine kinase inhibitors, tyrosine kinase inhibitors, aspartate kinase inhibitors, or histidine kinase inhibitors, etc.; proteasome inhibitors; epigenetic-related target inhibitors; tumor neovascularization inhibitors; cell cycle protein inhibitors.

In the present application, the term "topoisomerase inhibitor" generally refers to a compound or derivative thereof comprising a topoisomerase I inhibitor and a topoisomerase II inhibitor. Examples of topoisomerase I inhibitors include, but are not limited to, camptothecin and analogs thereof; and examples of topoisomerase II inhibitors include, but are not limited to, actinomycin D, doxorubicin, doxorubicin, duocarmycin, daunorubicin, mitoxantrone, podophyllotoxin, or etoposide, etc. The topoisomerase can refer to an enzyme that corrects the linking number of DNA by cleaving phosphodiester bonds in one or two strands of DNA and then re-coiling and resealing them.

In the present application, the term "camptothecin analogue" generally refers to a compound similar to camptothecin structure or derived from camptothecin. For example, the structure of camptothecin can be described in CAS No. 7689-03-4. For example, camptothecin analogues can refer to Exatecan (CAS No. 171335-80-1) or Belotecan (CAS No. 256411-32-2). The term "non-camptothecin topoisomerase I inhibitor" generally refers to a heterocyclic molecule having topoisomerase I inhibitory activity, such as indolocarbazoles, indenoisoquinolones, benzophenanthridines, and dibenzonaphthyridinones, mainly referring to Genz-644282 (CAS No. 529488_28-6).

In the present application, the term "effective amount" generally refers to an amount of therapeutic agent that treats, alleviates or prevents a disease or condition of interest, or that exhibits a detectable therapeutic or prophylactic effect. The precise effective amount for a subject depends on the subject's body type and health condition, the nature and extent of the condition, and the selection of therapeutic agents and/or combinations of therapeutic agents to be administered. Therefore, it is useless to pre-specify an accurate, effective amount. However, for a given condition, the effective amount can be determined by routine experimentation, and can be determined by the clinician.

The term "patient" includes both mammals and non-mammals. Examples of mammals include, but are not limited to, any member of the class Mammalia: humans, non-human primates such as chimpanzees and other apes and monkeys; farm animals such as cattle, horses, sheep, goats, and pigs; domesticated animals such as rabbits, dogs, and cats; laboratory animals, including rodents such as rats, mice, and guinea pigs, and including in utero mammals. Examples of non-mammals include, but are not limited to, birds and fish, and the like.

The term "patient" includes diagnosed patients, but the "patient" does not need to have any particular identity to a hospital, clinic or research device (such as being a newly diagnosed patient, research participant, etc.).

In the present application, the term "hydrophobic chromatography" generally refers to an analytical technique based on the differences in hydrophobicity of substances.

In the present application, the term "liquid chromatography-mass spectrometry" generally refers to an analytical method for identifying the components of a substance. For example, liquid chromatography-mass spectrometry can analyze the molecular weight of an analyte by liquid chromatography-mass spectrometry analysis.

### Specific embodiment

In one aspect, the present disclosure provides a compound of formula (I), or a pharmaceutically acceptable salt or isotopic variant thereof: 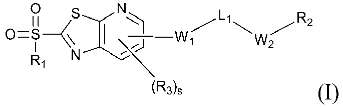 wherein,
R₁ is selected from C₁₋₆ alkyl or C₁₋₆ haloalkyl;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
L₁ is a chemical bond or -(CH₂)ₘ₁-(OCH₂CH₂)ₙ₁-(CH₂CH₂O)ₙ₂-(CH₂)ᵣ₁-(L)_{q}-(CH₂)ᵣ₂-(OCH₂CH₂)ₙ₃-(CH₂CH₂O)ₙ₄-(CH₂)ₘ₂-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene- or -5- to 10-membered heteroarylene;
each m1, m2, n1, n2, n3, n4, r1, and r2 is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
R₂ is selected from H, D, halogen, -ORₐ, -NR_{b}R_{c}, or the following groups: 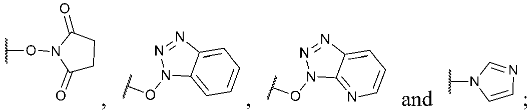
R₃ is selected from H, D, halogen, C₁₋₆ alkyl, or C₁₋₆ haloalkyl;
s = 0, 1, or 2;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

In another aspect, the present disclosure provides a compound of formula (II), or a pharmaceutically acceptable salt, prodrug, hydrate, solvate, enantiomer, diastereomer, mesomer, racemate or tautomer thereof: 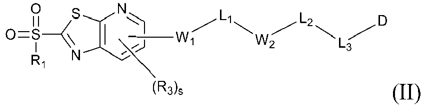 wherein,
R₁ is selected from C₁₋₆ alkyl or C₁₋₆ haloalkyl;
R₃ is selected from H, D, halogen, C₁₋₆ alkyl, or C₁₋₆ haloalkyl;
s = 0, 1, or 2;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
L₁ is a chemical bond or -(CH₂)ₘ-(OCH₂CH₂)ₙ-(CH₂CH₂O)ₙ-(CH₂)ᵣ-(L)_{q}-(CH₂)ᵣ-(OCH₂CH₂)ₙ-(CH₂CH₂O)ₙ-(CH₂)ₘ-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene- or -5- to 10-membered heteroarylene;
each m, n, and r is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
L₂ is selected from the following amino acid residues or oligopeptide residues consisting of 2 to 10 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄;
wherein N-terminal of amino acid residue or oligopeptide residue is connected to W₂, and the C-terminal is connected to L₃;
R₄ is selected from D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 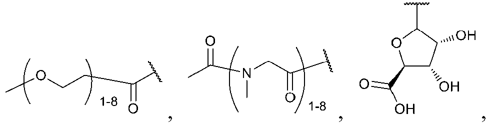 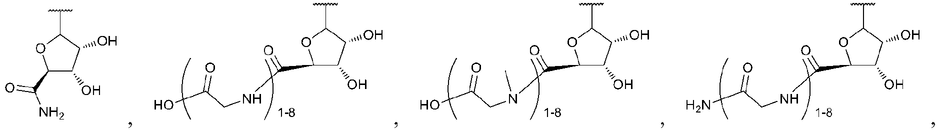 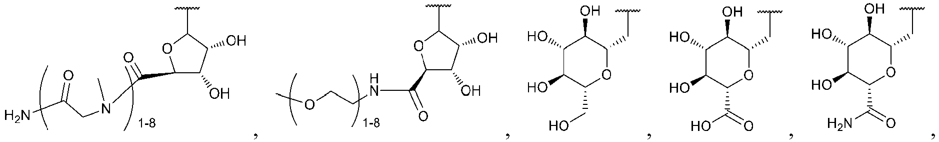 or 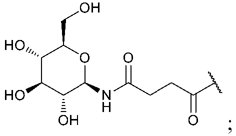
L₃ is selected from the following structures: 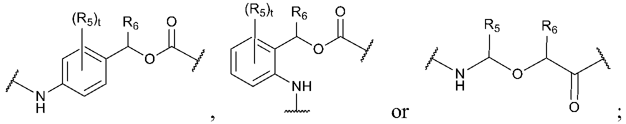
wherein NH is connected to L₂, and C(O) is connected to D;
R₅ is selected from H, D, halogen, NO₂, -ORₐ, -NR_{b}R_{c}, C₁₋₆ alkyl or C₁₋₆ haloalkyl;
t = 0, 1, 2, 3, or 4;
R₆ is selected from H, 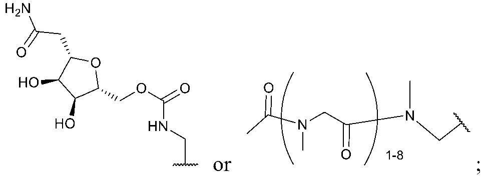
D is an active compound selected from a drug, a cytotoxin, a detection reagent, a diagnostic reagent or a targeting carrier;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

In another aspect, the present disclosure provides a compound of formula (III), or a pharmaceutically acceptable salt, prodrug, hydrate, solvate, enantiomer, diastereomer, mesomer, racemate or tautomer thereof, having the following general formula: 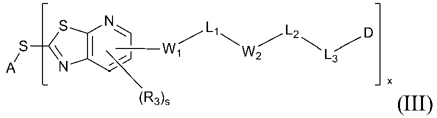 wherein,
A is a targeting molecule;
x = 1, 2, 3, 4, 5, 6, 7 or 8;
R₃ is selected from H, D, halogen, C₁₋₆ alkyl, or C₁₋₆ haloalkyl;
s = 0, 1 or 2;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
L₁ is a chemical bond or -(CH₂)ₘ-(OCH₂CH₂)ₙ-(CH₂CH₂O)ₙ-(CH₂)ᵣ-(L)_{q}-(CH₂)ᵣ-(OCH₂CH₂)ₙ-(CH₂CH₂O)ₙ-(CH₂)ₘ-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene- or -5- to 10-membered heteroarylene;
each m, n, and r is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
L₂ is selected from the following amino acid residues or oligopeptide residues consisting of 2 to 10 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄;
wherein N-terminal of amino acid residue or oligopeptide residue is connected to W₂, and the C-terminal is connected to L₃;
R₄ is selected from D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 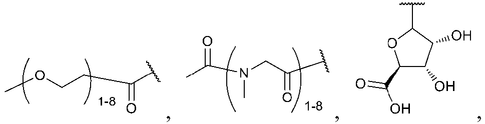 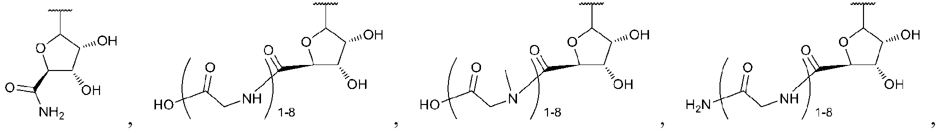 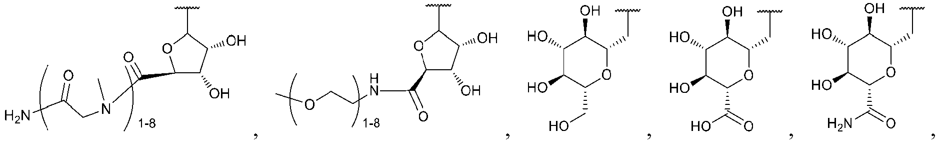 or 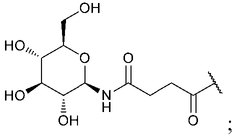
L₃ is selected from the following structures: 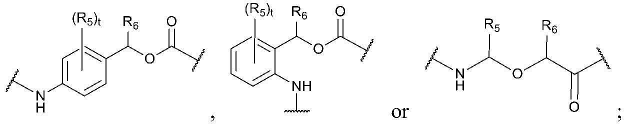
wherein NH is connected to L₂, and C(O) is connected to D;
R₅ is selected from H, D, halogen, NO₂, -ORₐ, -NR_{b}R_{c}, C₁₋₆ alkyl or C₁₋₆ haloalkyl;
t = 0, 1, 2, 3, or 4;
R₆ is selected from H, 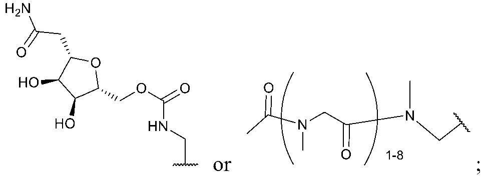
D is an active compound selected from a drug, a cytotoxin, a detection reagent, a diagnostic reagent or a targeting carrier;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

### A

In one embodiment, A is a targeting molecule; in a specific embodiment, A is selected from a protein, an antibody, an antibody fragment, a fusion protein, a polypeptide, an enzyme, and a small molecule; in a specific embodiment, A is a protein; in another specific embodiment, A is an antibody, alternatively a monoclonal antibody, such as a monospecific monoclonal antibody and a bispecific monoclonal antibody; in another specific embodiment, A is an IgG antibody and an HCAb antibody targeting tumor-associated antigens; in a specific embodiment, A is an antibody fragment; in a specific embodiment, A is a fusion protein; in a specific embodiment, A is a peptide; in a specific embodiment, A is an enzyme; in a specific embodiment, A is a small molecule.

### x

In one embodiment, x=1; in another embodiment, x=2; in another embodiment, x=3; in another embodiment, x=4; in another embodiment, x=5; in another embodiment, x=6; in another embodiment, x=7; in another embodiment, x=8; in another embodiment, x=2, 3, 4, 5, 6, 7 or 8; in another embodiment, x=2, 4, 6 or 8; in another embodiment, x=4, 6 or 8.

### R₃

In one embodiment, R₃ is H; in another embodiment, R₃ is D; in another embodiment, R₃ is halogen; in another embodiment, R₃ is C₁₋₆ alkyl; in another embodiment, R₃ is C₁₋₆ haloalkyl.

### s

In one embodiment, s=0; in another embodiment, s=1; in another embodiment, s=2.

### W₁

In one embodiment, W₁ is -O-; in another embodiment, W₁ is -S-; in another embodiment, W₁ is -NR_{b}-; in another embodiment, W₁ is -C(O)O-; in another embodiment, W₁ is -C(O)NR_{b}-; in another embodiment, W₁ is -O-C(O)-; in another embodiment, W₁ is -NR_{b}-C(O)-; in another embodiment, W₁ is -S(O)ₚO-; in another embodiment, W₁ is -O-S(O)ₚ-.

In one embodiment, p=1; in another specific embodiment, p=2.

### L₁

In one embodiment, L₁ is a chemical bond; in another embodiment, L₁ is -(CH₂)ₘ₁-(OCH₂CH₂)ₙ₁-(CH₂CH₂O)ₙ₂-(CH₂)ᵣ₁-(L)_{q}-(CH₂)ᵣ₂-(OCH₂CH₂)ₙ₃-(CH₂CH₂O)ₙ₄-(CH₂)ₘ₂-.

In another embodiment, L₁ is -(CH₂)ₘ₁-(CH₂CH₂O)ₙ₁-(CH₂)ᵣ₁-L-(CH₂)ᵣ₂-(CH₂CH₂O)ₙ₂-(CH₂)ₘ₂-; in another embodiment, L₁ is -(CH₂)ₘ₃-(OCH₂CH₂)ₙ₃-(CH₂)ᵣ₃-L-(CH₂)ᵣ₄-(OCH₂CH₂)ₙ₄-(CH₂)ₘ₄-.

In another embodiment, L₁ is -(CH₂CH₂O)ₙ₅-(CH₂)ᵣ₅-L-(CH₂)ₘ₅-; in another embodiment, L₁ is-(OCH₂CH₂)ₙ₆-(CH₂)ᵣ₆-L-(CH₂)ₘ₆-; in another embodiment, L₁ is -(CH₂)ₘ₇-L-(CH₂CH₂O)ₙ₇-(CH₂)ᵣ₇-; in another embodiment, L₁ is -(CH₂)ₘ₈-L-(OCH₂CH₂)ₙ₈-(CH₂)ᵣ₈-.

In another embodiment, L₁ is -(CH₂)ₘ₉-(CH₂CH₂O)ₙ₉-(CH₂)ᵣ₉-; in another embodiment, L₁ is-(CH₂)ₘ₁₀-(OCH₂CH₂)ₙ₁₀-(CH₂)ᵣ₁₀-.

In another embodiment, L₁ is -(CH₂CH₂O)ₙ₁₁-(CH₂)ₘ₁₁-; in another embodiment, L₁ is -(CH₂)ₘ₁₂-(CH₂CH₂O)ₙ₁₂-; in another embodiment, L₁ is -(OCH₂CH₂)ₙ₁₃-(CH₂)ₘ₁₃-; in another embodiment, L₁ is - (CH₂)ₘ₁₄-(OCH₂CH₂)ₙ₁₄-.

In another embodiment, L₁ is -(CH₂)ₘ₁₅-O-(CH₂)ₘ₁₆-; in another embodiment, L₁ is -(CH₂)ₘ₁₇-O-,-(CH₂)ₘ₁₈-NH-; in another embodiment, L₁ is -O-(CH₂)ₘ₁₉-; in another embodiment, L₁ is -NR_{b}-(CH₂)ₘ₂₀-; in another embodiment, L₁ is -(CH₂)ₘ₂₁-; in another embodiment, L₁ is -L-; in another embodiment, L₁ is -(CH₂)ₘ₂₂-L-; in another embodiment, L₁ is -L-(CH₂)ₘ₂₃-.

In another embodiment, L₁ is -(CH₂CH₂O)ₙ₁₁-(CH₂)ₘ₁₁-, alternatively -(CH₂CH₂O)ₙ₁₁-; in another embodiment, L₁ is -(CH₂)ₘ₂₁-.

In a specific embodiment, -L- is -O-; in another specific embodiment, -L- is -NR_{b}-; in another specific embodiment, -L- is -C(O)NR_{b}-; in another specific embodiment, -L- is -C(O)O-; in another specific embodiment, -L- is -NR_{b}-C(O)-; in another specific embodiment, -L- is -O-C(O)-; in another specific embodiment, -L- is -C₃₋₈ cycloalkylene-; in another specific embodiment, -L- is -3- to 8-membered heterocyclylene-; in another specific embodiment, -L- is -C₆₋₁₀ arylene-; in another specific embodiment, -L- is -5- to 10-membered heteroarylene.

In a specific embodiment, m=0; in another specific embodiment, m=1; in another specific embodiment, m=2; in another specific embodiment, m=3; in another specific embodiment, m=4; in another specific embodiment, m=5; in another specific embodiment, m=6; in another specific embodiment, m=7; in another specific embodiment, m=8; in another specific embodiment, m=9; in another specific embodiment, m=10.

In a specific embodiment, n=0; in another specific embodiment, n=1; in another specific embodiment, n=2; in another specific embodiment, n=3; in another specific embodiment, n=4; in another specific embodiment, n=5; in another specific embodiment, n=6; in another specific embodiment, n=7; in another specific embodiment, n=8; in another specific embodiment, n=9; in another specific embodiment, n=10.

In a specific embodiment, r=0; in another specific embodiment, r=1; in another specific embodiment, r=2; in another specific embodiment, r=3; in another specific embodiment, r=4; in another specific embodiment, r=5; in another specific embodiment, r=6; in another specific embodiment, r=7; in another specific embodiment, r=8; in another specific embodiment, r=9; in another specific embodiment, r=10.

In a specific embodiment, q=0; in another specific embodiment, q=1; in another specific embodiment, q=2.

In another specific embodiment, m1, m2, m3, m4, m5, m6, m7, m8, m9, m10, m11, m12, m13, m14, m15, m16, m17, m18, m19, m20, m21, m22, m23 are each independently selected from 0, 1, 2, 3, 4, 5, 6, 7, 8, 9, or 10; n1, n2, n3, n4, n5, n6, n7, n8, n9, n10, n11, n12, n13, n14 are each independently selected from 0, 1, 2, 3, 4, 5, 6, 7, 8, 9, or 10; r1, r2, r3, r4, r5, r6, r7, r8, r9, r10 are each independently selected from 0, 1, 2, 3, 4, 5, 6, 7, 8, 9, or 10.

### W₂

In one embodiment, W₂ is a chemical bond; in another embodiment, W₂ is -O-; in another embodiment, W₂ is -S-; in another embodiment, W₂ is -NR_{b}-; in another embodiment, W₂ is -C(O)-.

### L₂

In one embodiment, L₂ is selected from the following amino acid residues or oligopeptide residues consisting of 2 to 10 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄; in another embodiment, L₂ is selected from the following amino acid residues or oligopeptide residues consisting of 2 to 8 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄. In another embodiment, L₂ is selected from the following amino acid residues or oligopeptide residues consisting of 2 to 6 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄; in another embodiment, L₂ is selected from the following amino acid residues or oligopeptide residues consisting of 2 to 5 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄; in another embodiment, L₂ is selected from the following amino acid residues or oligopeptide residues consisting of 2 to 4 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄.

In a specific embodiment, L₂ is selected from the following amino acid residues or oligopeptide residues consisting of 2 to 5 following amino acids, wherein the amino acid is selected from valine, citrulline, alanine, glycine, phenylalanine, lysine, arginine, aspartic acid, glutamic acid and serine, and the amino acid is optionally substituted with 1, 2, 3, 4, 5, or 6 R₄.

In another specific embodiment, L₂ is selected from dipeptide, tripeptide, or tetrapeptide residues consisting of the following amino acids, wherein the amino acid is selected from valine, citrulline, alanine, glycine, phenylalanine, lysine, arginine, aspartic acid, glutamic acid, and serine, and the amino acid is optionally substituted with 1, 2, 3, 4, 5, or 6 R₄.

In another specific embodiment, L₂ is selected from valine-citrulline, valine-alanine, valine-lysine, phenylalanine-lysine, lysine-lysine, alanine-lysine, phenylalanine-citrulline, leucine-citrulline, isoleucine-citrulline, phenylalanine-alanine, lysine-valine-citrulline, lysine-valine-alanine, valine-lysine-glycine, glycine-valine-lysine, glycine-valine-alanine, glutamine-valine-alanine, glutamine-valine-citrulline, glutamic acid-valine-alanine, glutamic acid-valine-citrulline, alanine-alanine-alanine, alanine-alanine-asparagine, phenylalanine-phenylalanine-lysine, glycine-phenylalanine-lysine, leucine-alanine-leucine, isoleucine-alanine-leucine, valine-alanine-valine, glycine-glycine-phenylalanine-glycine, alanine-leucine-alanine-leucine, and glycine-phenylalanine-leucine-glycine, and the amino acid is optionally substituted with 1, 2, 3, 4, 5, or 6 R₄.

In another specific embodiment, L₂ is selected from glutamine-valine-alanine, glutamine-valine-citrulline, valine-citrulline, valine-alanine, valine-lysine, lysine-valine-citrulline, lysine-valine-alanine, and glycine-glycine-phenylalanine-glycine, and the amino acid is optionally substituted with 1, 2, 3, 4, 5, or 6 R₄.

In another specific embodiment, L₂ is selected from valine-citrulline, valine-alanine, valine-lysine, lysine-valine-citrulline, lysine-valine-alanine, and glycine-glycine-phenylalanine-glycine, and the amino acid is optionally substituted with 1, 2, 3, 4, 5, or 6 R₄.

### R₄

In one embodiment, R₄ is D; in another embodiment, R₄ is halogen; in another embodiment, R₄ is NO₂; in another embodiment, R₄ is -ORₐ; in another embodiment, Rₐ is -NR_{b}R_{c}; in another embodiment, R₄ is C₁₋₆ alkyl; in another embodiment, R₄ is C₁₋₆ haloalkyl; in another embodiment, R₄ is polyethylene glycol; in another embodiment, R₄ is polysarcosine; in another embodiment, R₄ is pentose; in another embodiment, R₄ is hexose; in another embodiment, R₄ is sulfonic group; in another embodiment, R₄ is methylsulfonyl; in another embodiment, R₄ is phosphate group; in another embodiment, R₄ is phosphite group; in another embodiment, R₄ is quaternary ammonium salt; in another embodiment, R₄ is 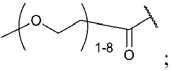 in another embodiment, R₄ is 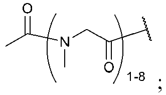 in another embodiment, R₄ is 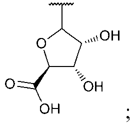 in another embodiment, R₄ is 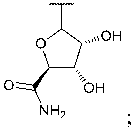 in another embodiment, R₄ is 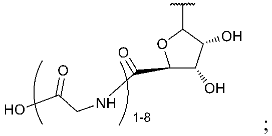 in another embodiment, R₄ is 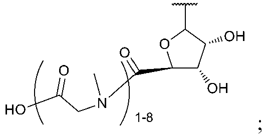 in another embodiment, R₄ is 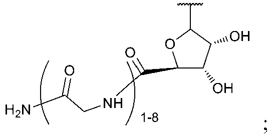 in another embodiment, R₄ is 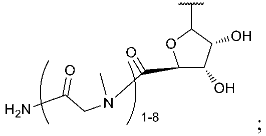 in another embodiment, R₄ is 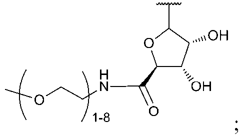 in another embodiment, R₄ is 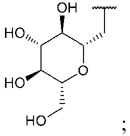 in another embodiment, R₄ is 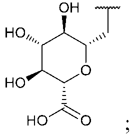 in another embodiment, R₄ is 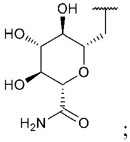 in another embodiment, R₄ is

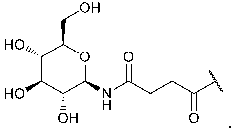

### L₃

In one embodiment, L₃ is 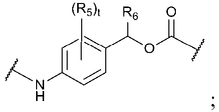 In another embodiment, L₃ is 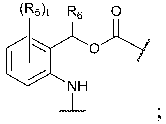 in another embodiment, L₃ is

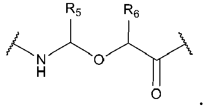

### R₅

In one embodiment, R₅ is H; in another embodiment, R₅ is D; in another embodiment, R₅ is halogen; in another embodiment, R₅ is NO₂; in another embodiment, R₅ is -ORₐ; in another embodiment, R₅ is -NR_{b}R_{c}; in another embodiment, R₅ is C₁₋₆ alkyl; in another embodiment, R₅ is C₁₋₆ haloalkyl.

### t

In one embodiment, t=0; in another embodiment, t=1; in another embodiment, t=2; in another embodiment, t=3; in another embodiment, t=4.

### R₆

In one embodiment, R₆ is H; in another embodiment, R₆ is 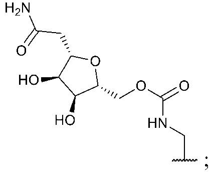 in another embodiment, R₆ is

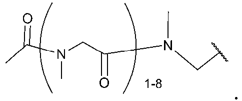

### D

In one embodiment, D is an active compound; in another embodiment, D is a drug; in another embodiment, D is a cytotoxin; in another embodiment, D is a detection reagent; in another embodiment, D is a diagnostic reagent; in another embodiment, D is a targeting carrier.

Specifically, the present disclosure relates to the linker selected from the following:

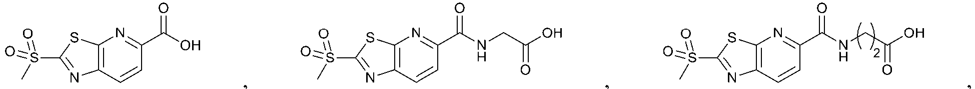

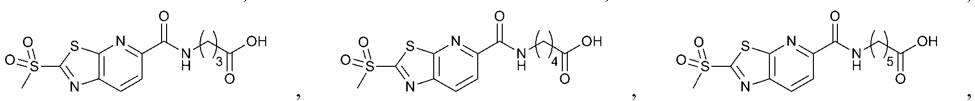

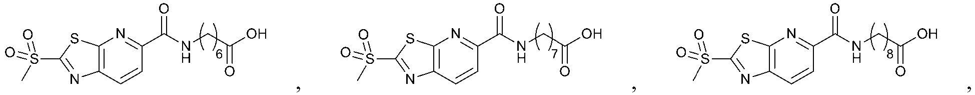

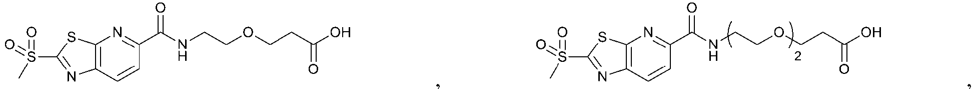

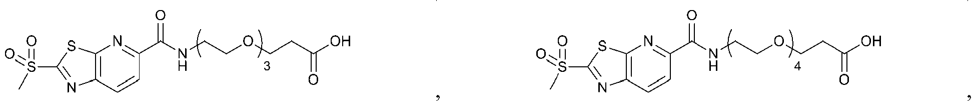

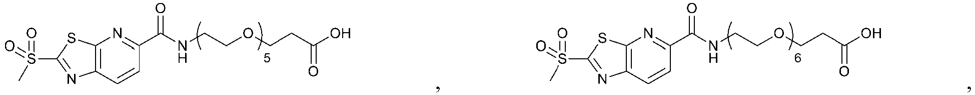

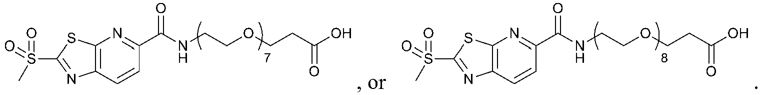

Specifically, the present disclosure relates to the linker-payload selected from the following table:

| Compoun d number | Linker-Payload Structure |
|---|---|
| 1 | 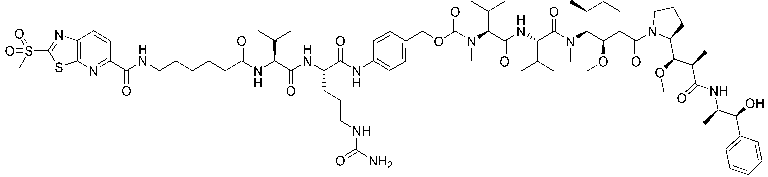 |
| 2 | 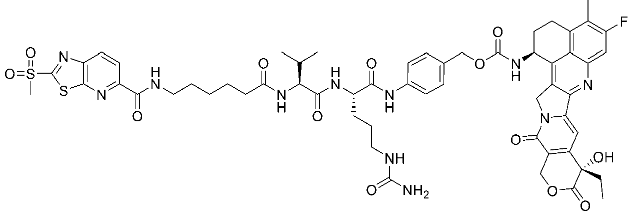 |
| 3 | 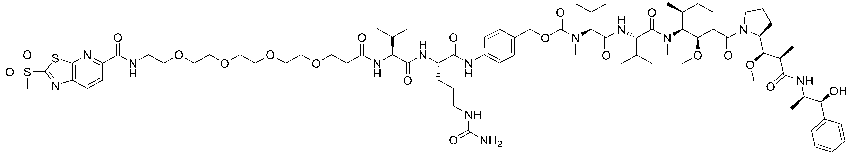 |
| 4 | 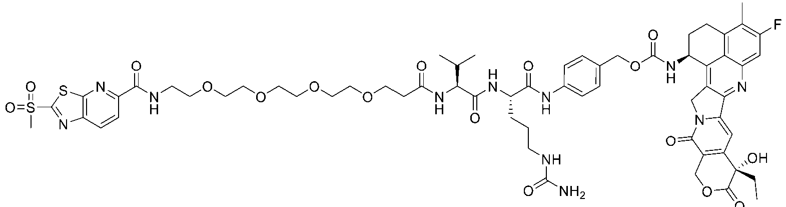 |
| 5 | 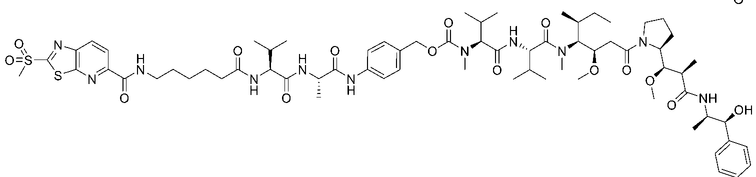 |
| 6 | 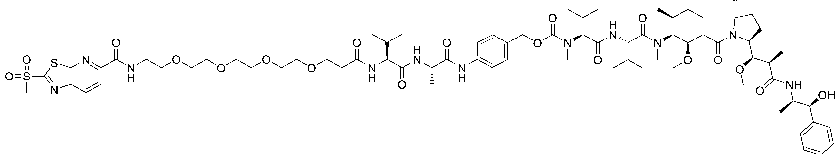 |
| 7 | 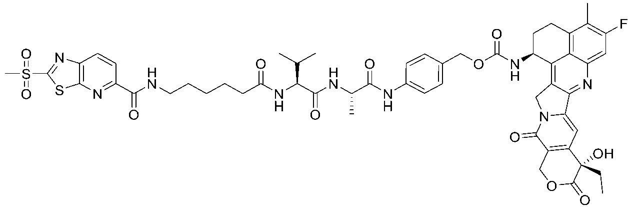 |
| 8 | 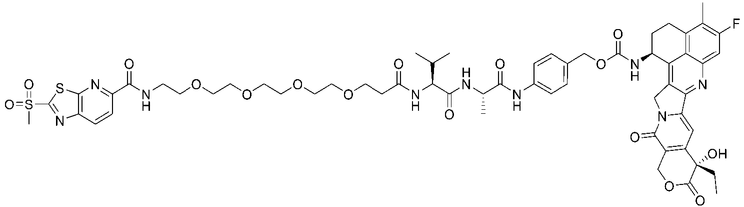 |
| 9 | 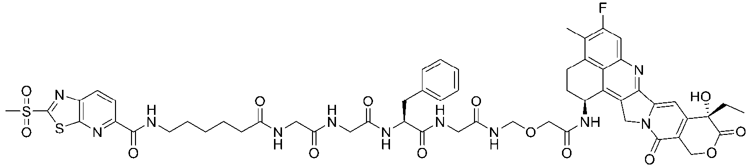 |
| 10 | 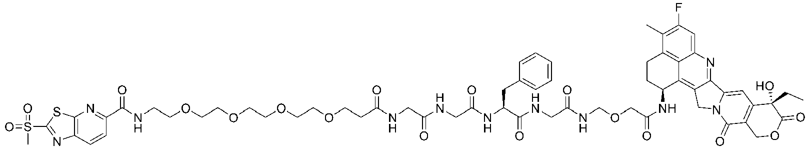 |
| 11 | 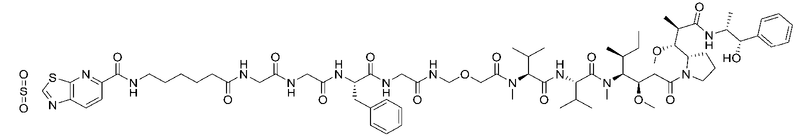 |
| 12 | 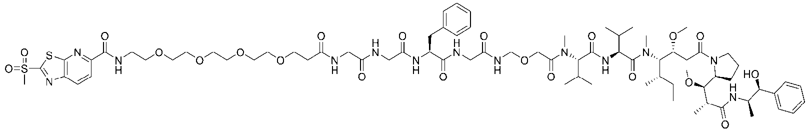 |
| 13 | 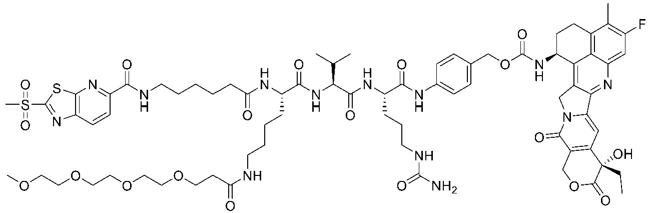 |
| 14 | 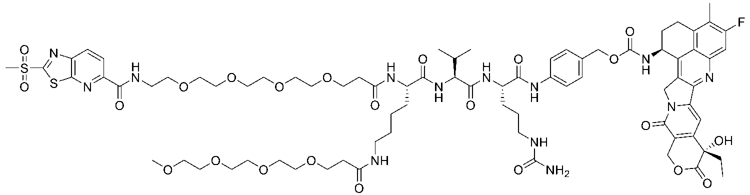 |
| 15 | 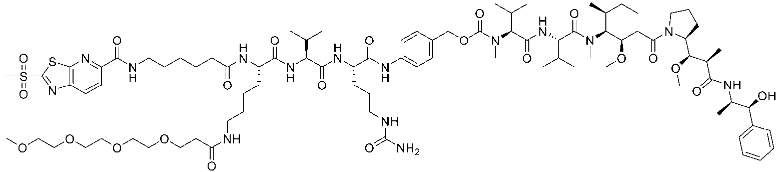 |
| 16 | 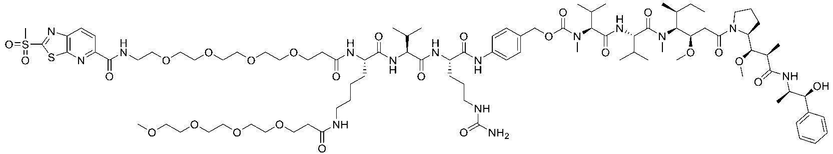 |
| 17 | 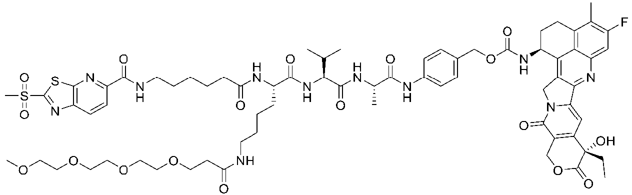 |
| 18 | 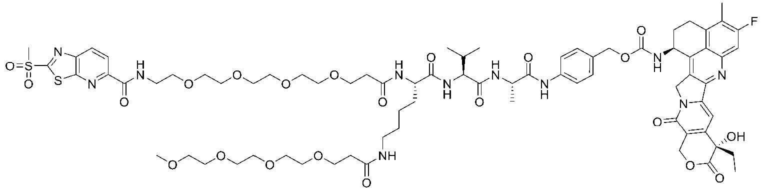 |
| 19 | 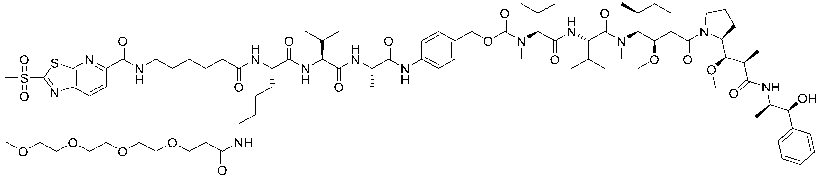 |
| 20 | 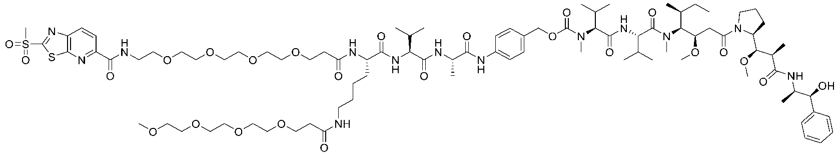 |
| 21 | 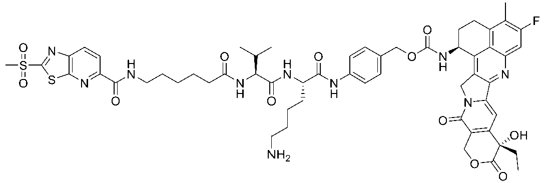 |
| 22 | 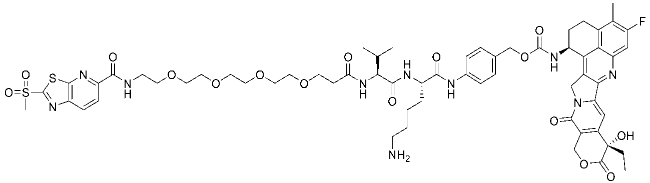 |
| 23 | 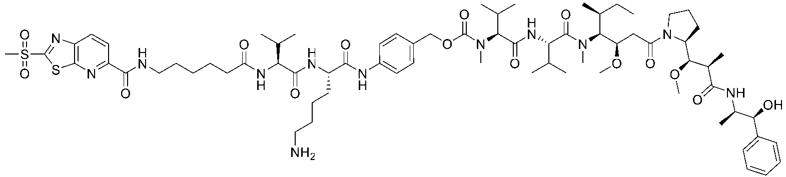 |
| 24 | 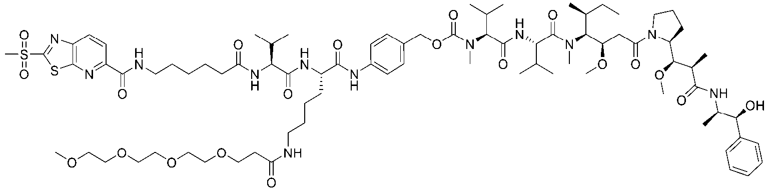 |
| 25 | 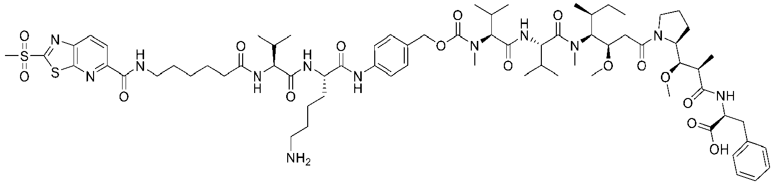 |
| 26 | 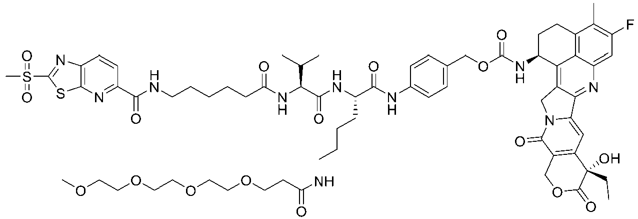 |
| 27 | 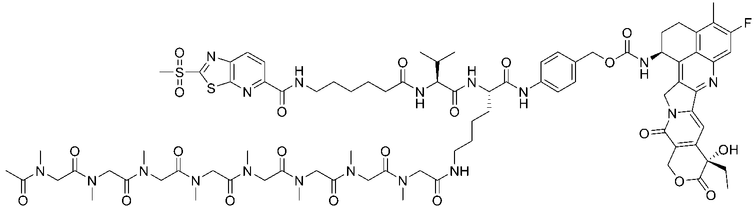 |
| 28 | 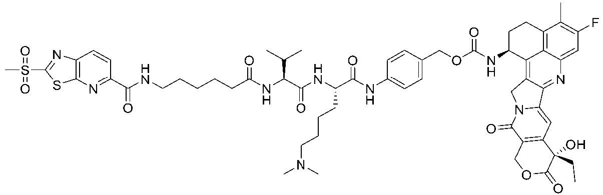 |
| 29 | 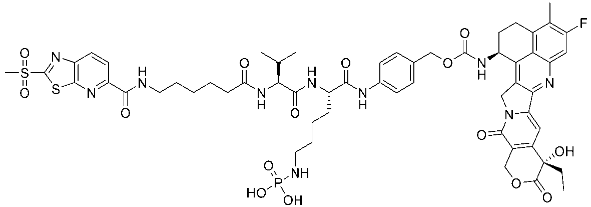 |
| 30 | 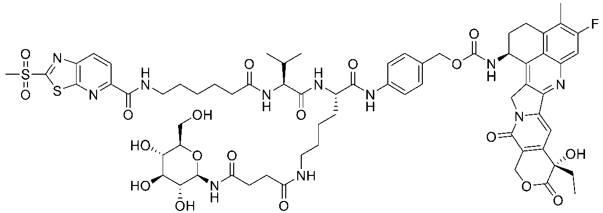 |
| 31 | 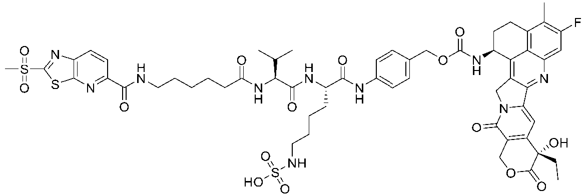 |
| 39 | 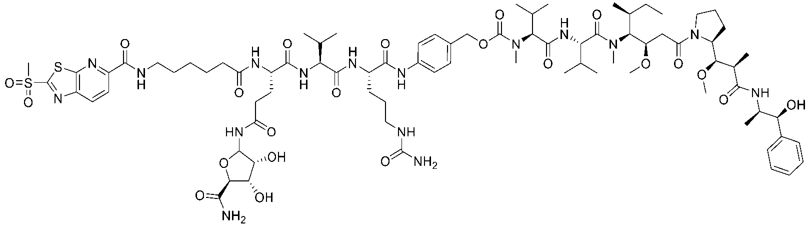 |
| 40 | 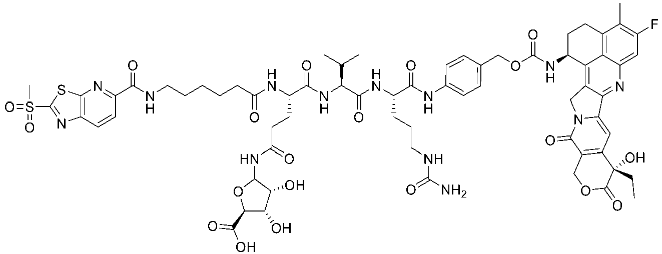 |
| 41 | 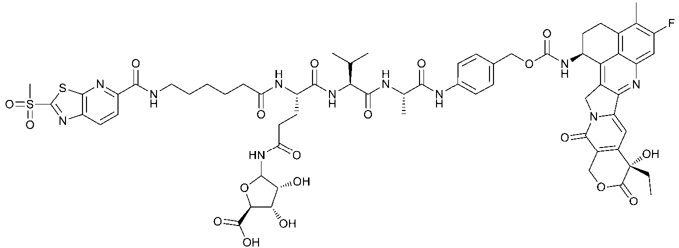 |
| 42 | 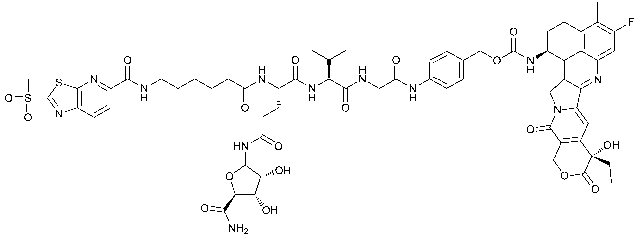 |
| 43 | 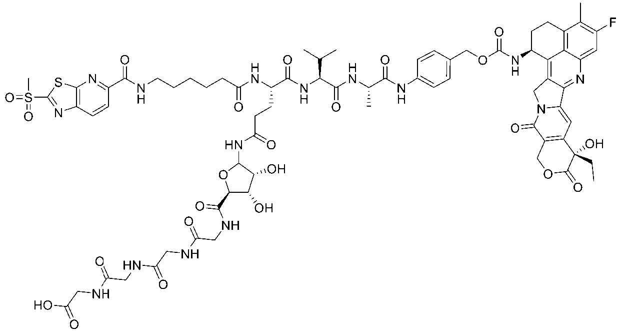 |
| 44 | 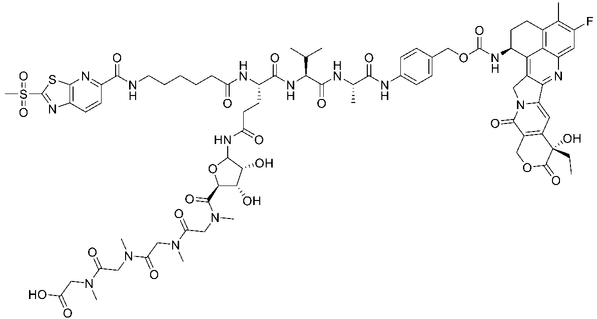 |
| 45 | 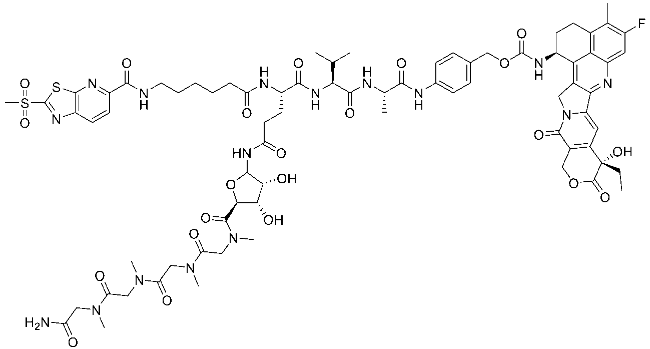 |
| 46 | 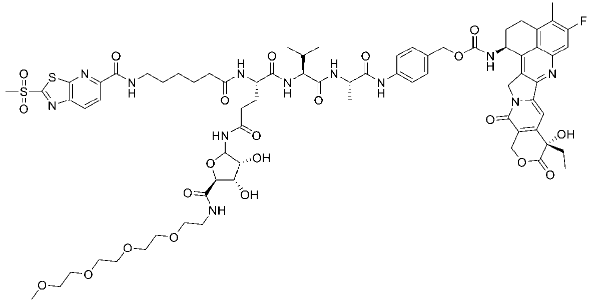 |
| 47 | 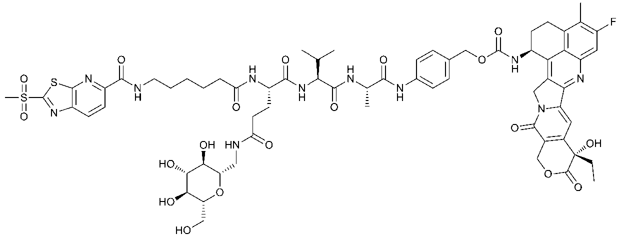 |
| 48 | 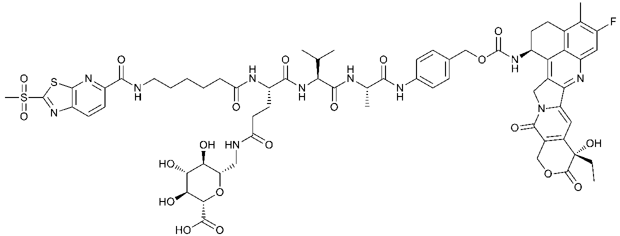 |
| 49 | 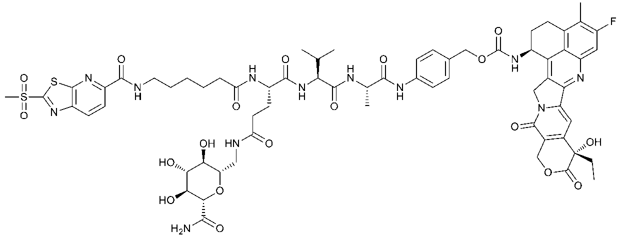 |
| 50 | 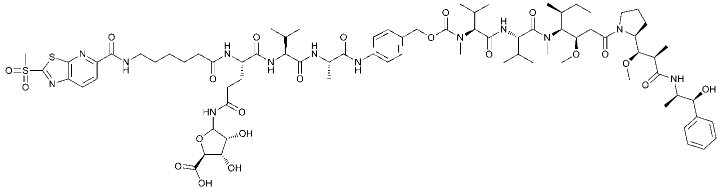 |
| 51 | 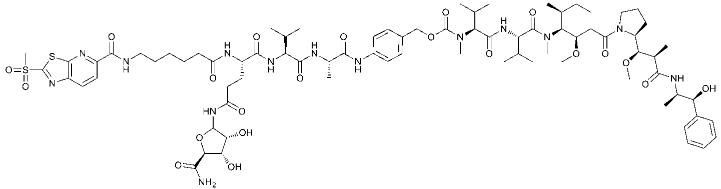 |
| 52 | 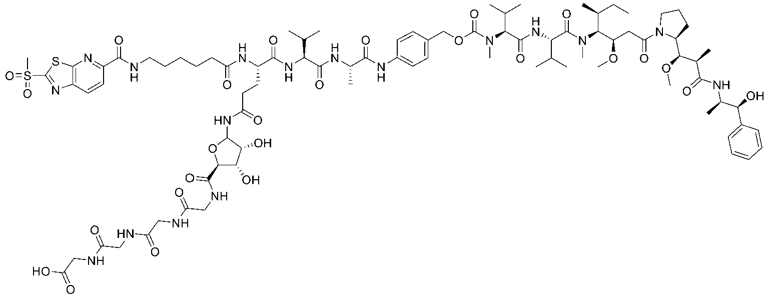 |
| 53 | 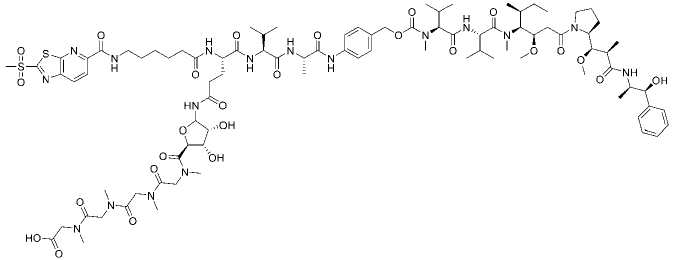 |
| 54 | 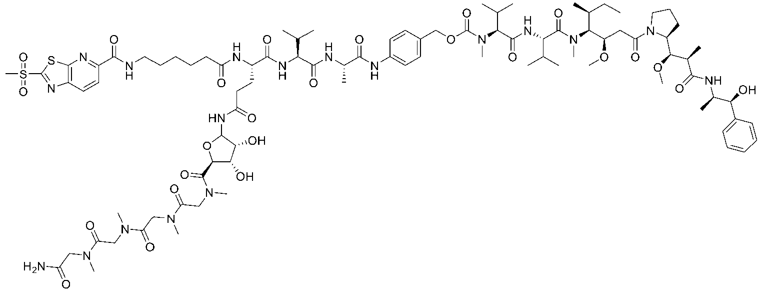 |
| 55 | 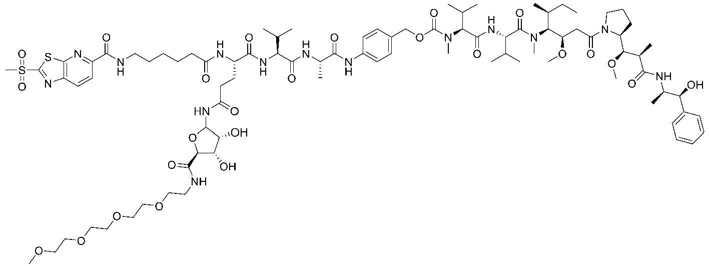 |
| 56 | 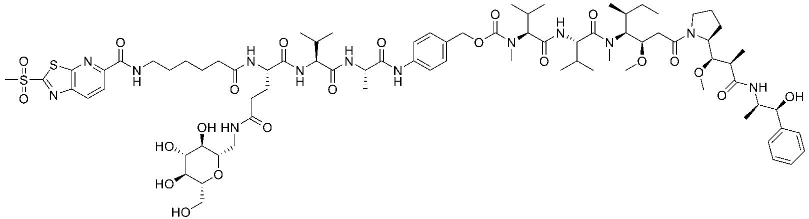 |
| 57 | 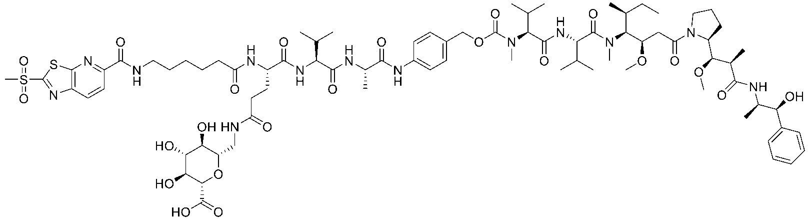 |
| 58 | 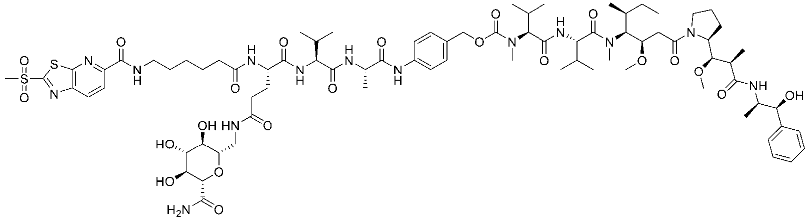 |
| 59 | 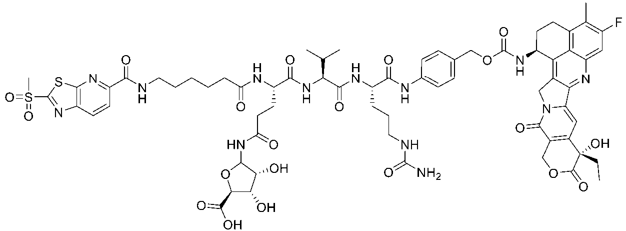 |
| 60 | 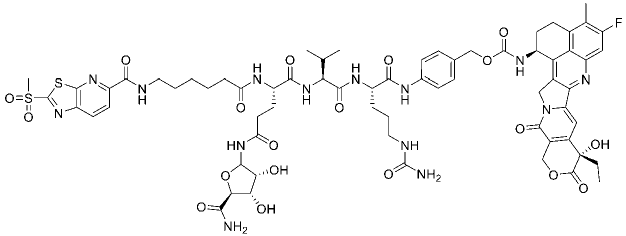 |
| 61 | 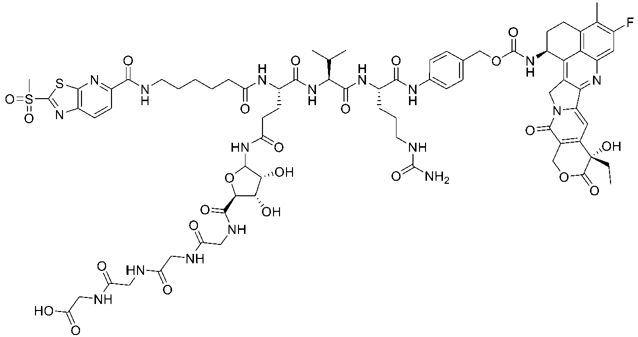 |
| 62 | 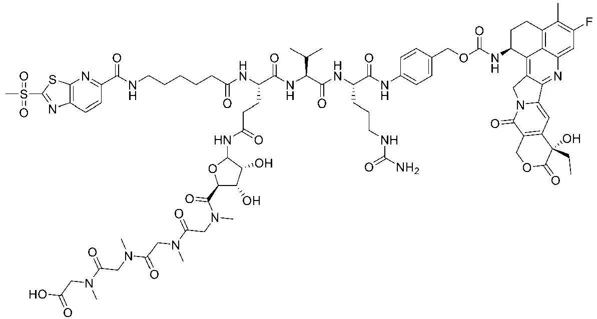 |
| 63 | 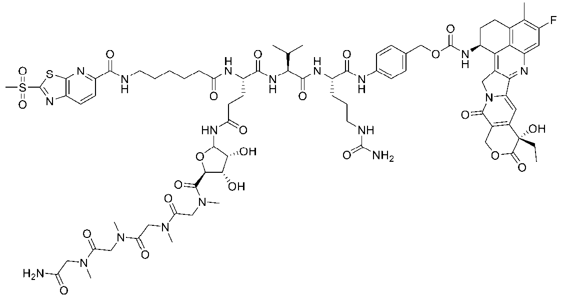 |
| 64 | 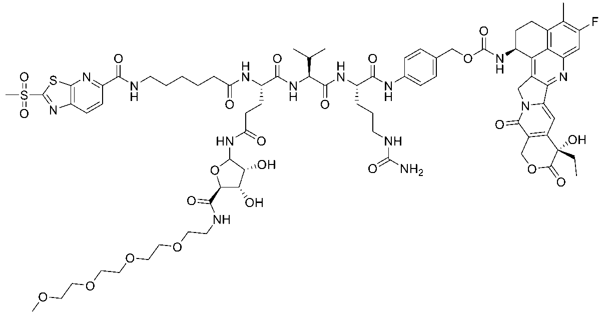 |
| 65 | 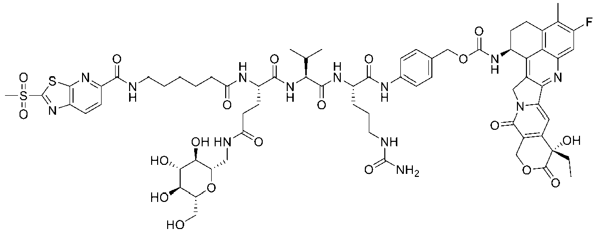 |
| 66 | 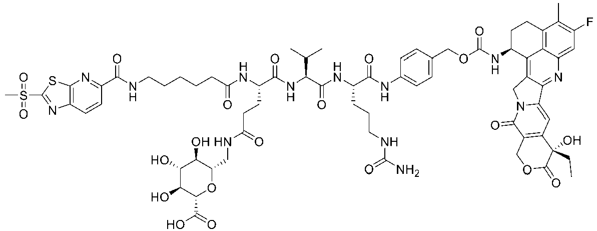 |
| 67 | 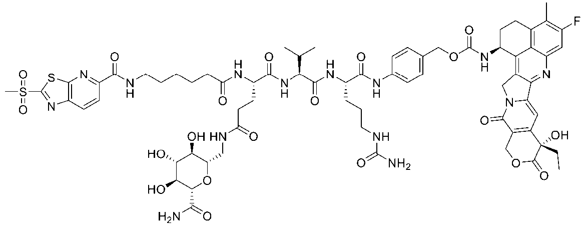 |
| 68 | 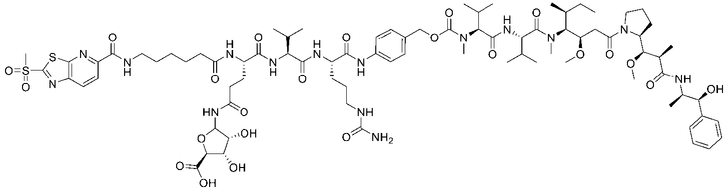 |
| 69 | 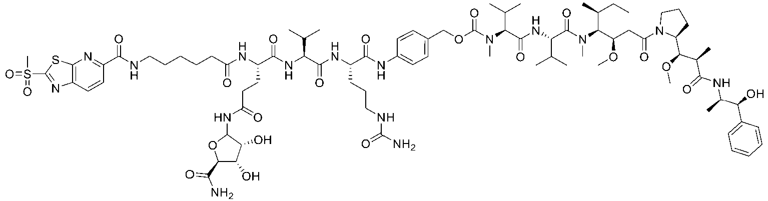 |
| 70 | 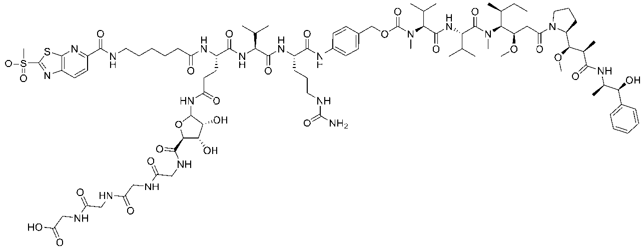 |
| 71 | 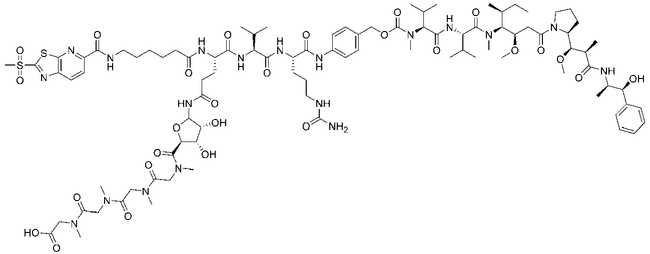 |
| 72 | 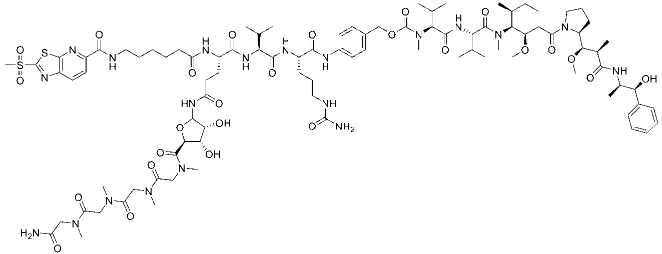 |
| 73 | 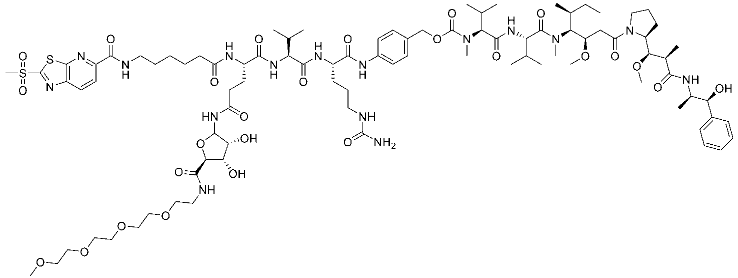 |
| 74 | 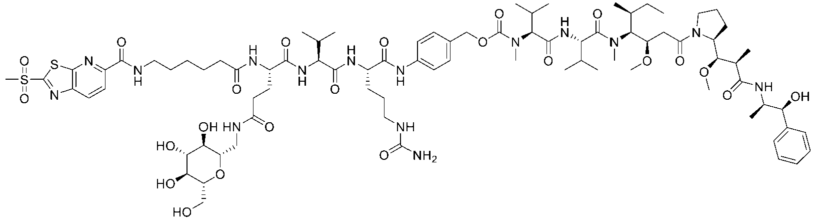 |
| 75 | 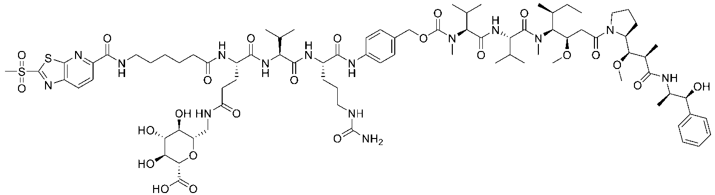 |
| 76 | 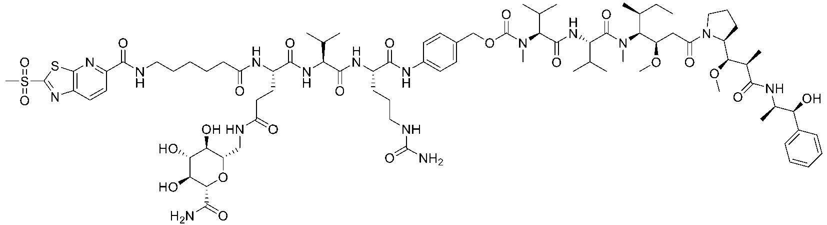 |
| 77 | 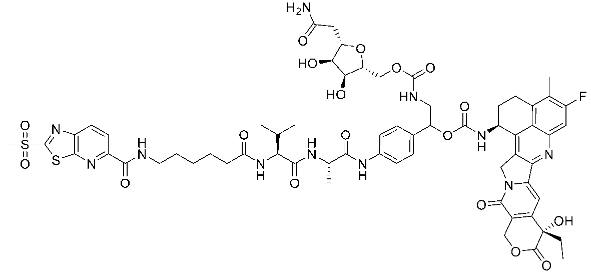 |
| 78 | 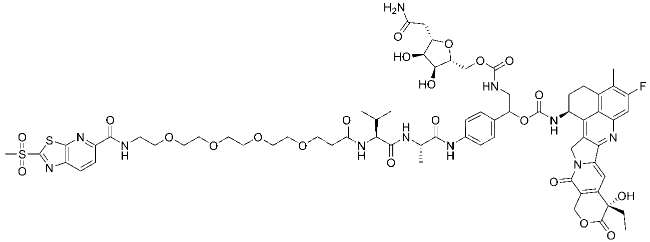 |
| 79 | 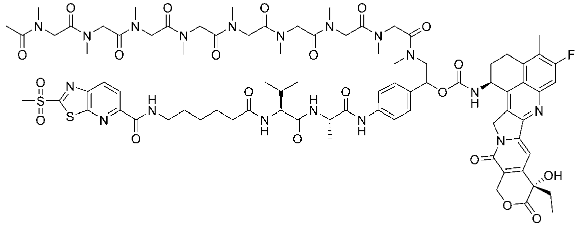 |
| 35 | 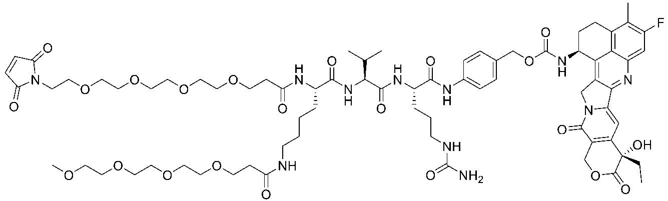 |

Specifically, the present disclosure relates to ADC selected from the following table:

| ADC number | ADC structure |
|---|---|
| 1 | 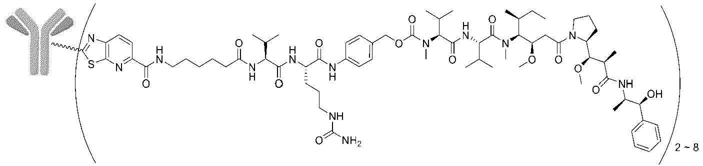 |
| 2 | 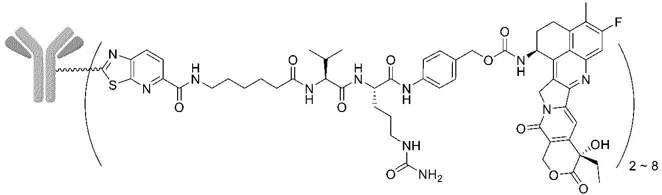 |
| 3 | 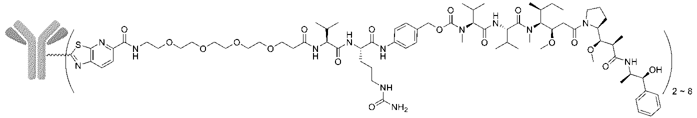 |
| 4 | 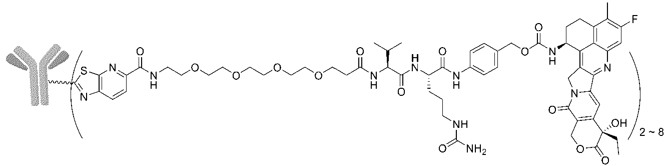 |
| 5 | 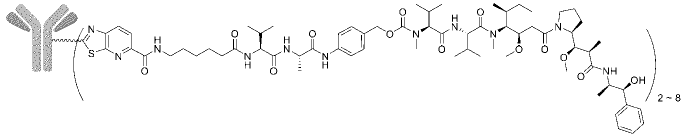 |
| 6 | 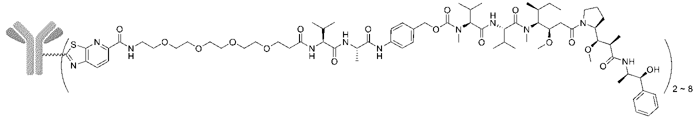 |
| 7 | 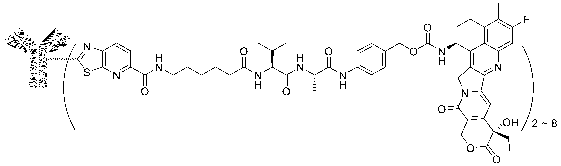 |
| 8 | 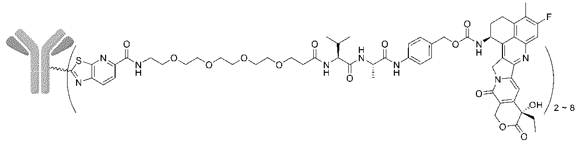 |
| 9 | 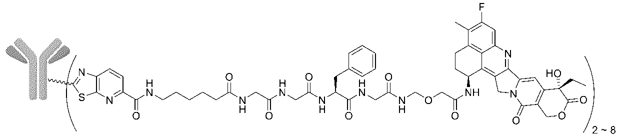 |
| 10 | 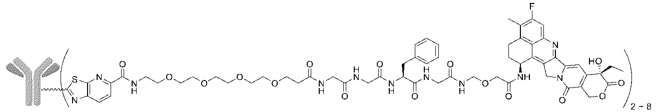 |
| 11 | 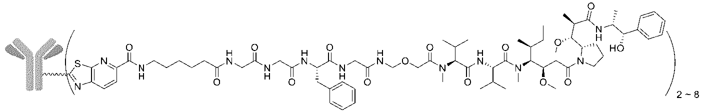 |
| 12 | 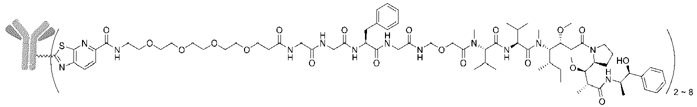 |
| 13 | 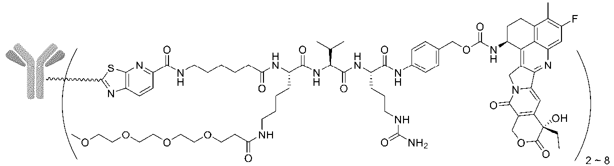 |
| 14 | 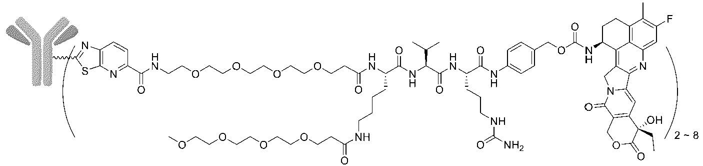 |
| 15 | 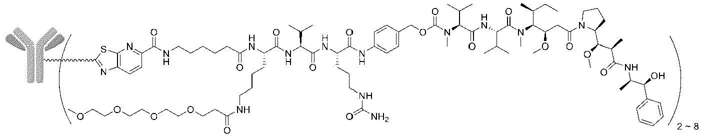 |
| 16 | 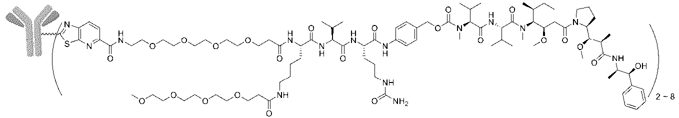 |
| 17 | 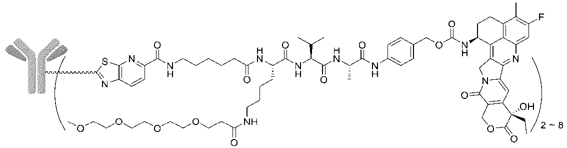 |
| 18 | 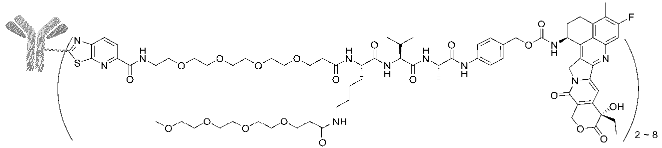 |
| 19 | 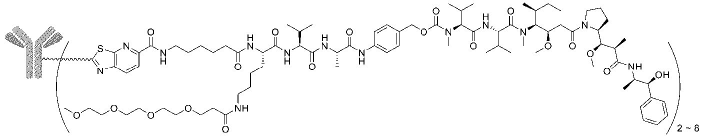 |
| 20 | 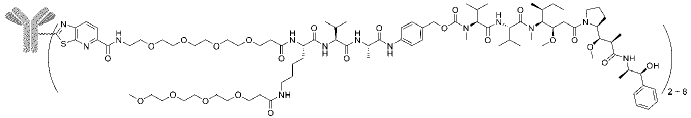 |
| 21 | 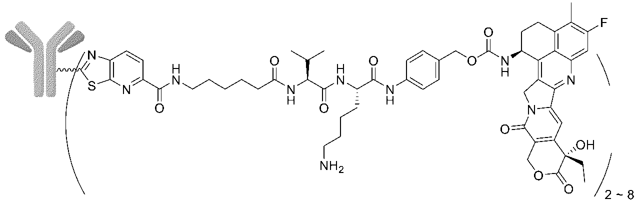 |
| 22 | 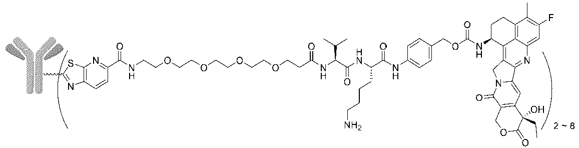 |
| 23 | 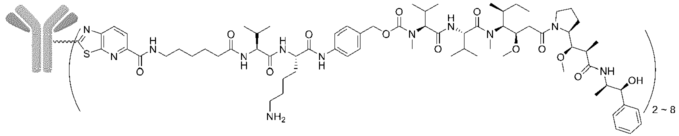 |
| 24 | 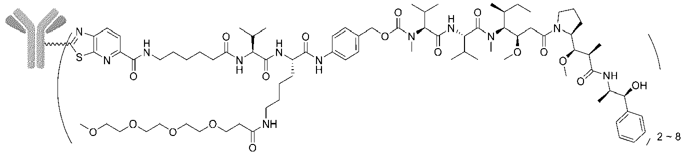 |
| 25 | 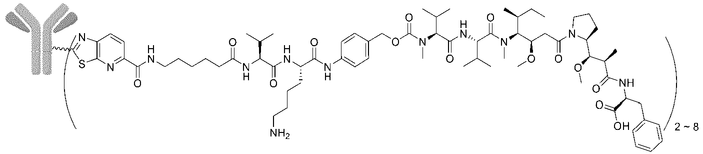 |
| 26 | 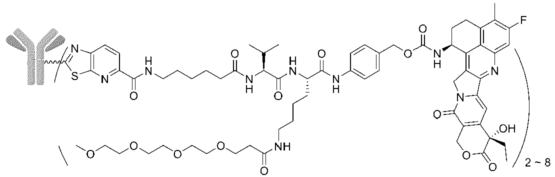 |
| 27 | 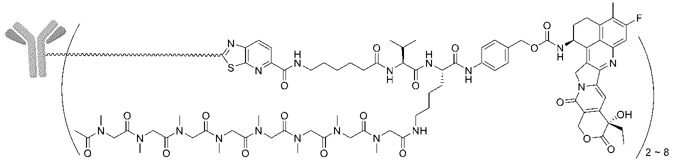 |
| 28 | 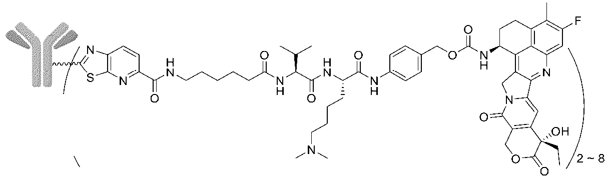 |
| 29 | 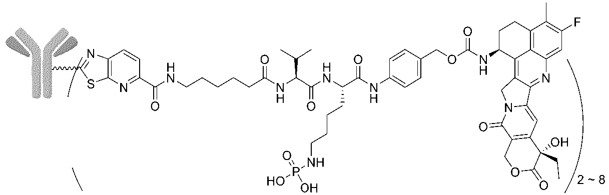 |
| 30 | 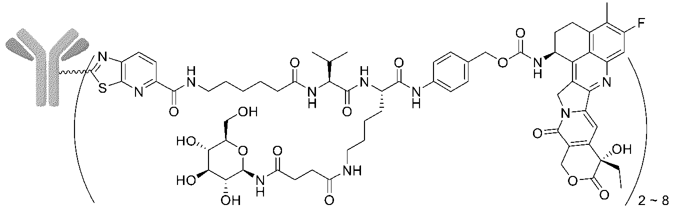 |
| 31 | 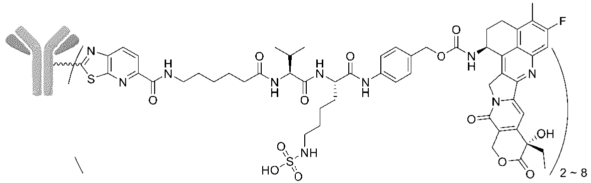 |
| 35 | 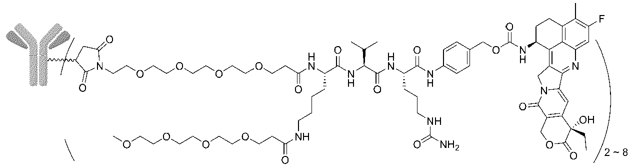 |
| 39 | 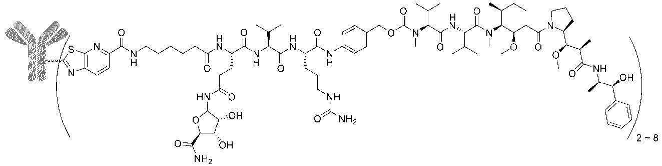 |
| 40 | 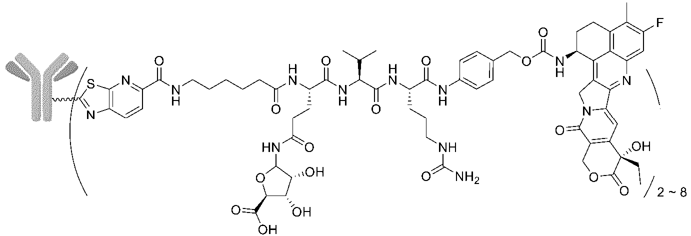 |

wherein, 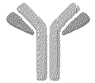 represents a monoclonal antibody, alternatively an IgG1 or HCAb type monoclonal antibody against HER2 and B7-H3.

### Method of Treatment

The targeting molecules described in the present application may be protein hormones, lectins, growth factors, antibodies, or other molecules that can bind to cells, receptors, and/or antigens. For example, the targeting molecules in the present application may be anti-HER2 antibodies, anti-B7-H3 antibodies, including IgG type antibodies and HCAb type antibodies, or antigen-binding fragments thereof.

Because the antibody-drug conjugate provided in the present application can target specific cell populations and bind to cell surface-specific proteins (antigens), the drug is released into the cells in its active form via endocytosis of the conjugate or infiltration of the drug. Therefore, the antibody-drug conjugate of the present application can be used for the treatment of diseases. The antibody-drug conjugate of the present application can be administered to a subject (e.g., a human) in a therapeutically effective amount via an appropriate route. The subject in need of treatment may be a patient at risk or suspected of having a condition associated with the activity or expression level of a particular antigen. Such patients can be identified through routine physical examinations.

When treated with the antibody-drug conjugate of the present application, delivery can be performed using methods conventional in the art. For example, it can be introduced into cells using liposomes, hydrogels, cyclodextrins, biodegradable nanocapsules, or bioadhesive microspheres. Alternatively, the nucleic acid or carrier can be delivered locally by direct injection or by using an infusion pump. Other methods may include the use of various transport and carrier systems by the use of conjugates and biodegradable polymers.

As is well known to those skilled in the art, the dosage of a drug depends on a variety of factors, including but not limited to: the activity of the specific compound used, the patient's age, the patient's weight, the patient's health status, the patient's behavior, the patient's diet, the timing of administration, the route of administration, the rate of excretion, and the combination of drugs. In addition, the optimal method of treatment, such as the mode of treatment, the daily dosage of the compound described in the present application or pharmaceutically acceptable salts, prodrugs, hydrates, solvates, enantiomers, diastereomers, mesomers, racemates, or tautomers thereof, or mixtures thereof, or the type of pharmaceutically acceptable salt, can be verified according to conventional treatment regimens.

The compounds of the present application can be administered alone or in combination with other pharmaceutically acceptable therapeutic agents. When the pharmaceutical composition is used, it could be administered a safe and effective amount of the compound of the present application to a mammal (such as a human) in need of treatment, wherein the dose administered may be a pharmaceutically considered effective dose, and the specific dose may also take into account factors such as the route of administration and the patient's health condition.

The present application provides the use of a compound of the present application, or a pharmaceutically acceptable salt, prodrug, hydrate, solvate, enantiomer, diastereomer, mesomer, racemate, or tautomer thereof, or a pharmaceutical composition of the present application, in the manufacture of a medicament for treating and/or preventing a tumor. For example, the tumor may be selected from a tumor associated with the expression of targets such as HER2 and B7-H3. For example, the tumor associated with the expression of targets such as HER2 and B7-H3 include tumors with high expression of these targets and/or tumors positive for these targets. For example, the tumor may include solid tumors and/or hematologic malignancies. For example, the tumors include breast cancer, ovarian cancer, non-Hodgkin lymphoma, Hodgkin lymphoma, acute lymphoblastic leukemia, anaplastic large cell lymphoma, multiple myeloma, prostate cancer, lung cancer, malignant melanoma, squamous cell carcinoma, glioblastoma, renal cell carcinoma, gastrointestinal tumors, pancreatic cancer, prostate cancer, colorectal cancer, gastric cancer, glioma, or mesothelioma.

The present application provides a compound, or a pharmaceutically acceptable salt, prodrug, hydrate, solvate, enantiomer, diastereomer, mesomer, racemate, or tautomer thereof, or a pharmaceutical composition comprising the compound of the present application, for use in the treatment and/or prevention of a tumor. For example, the tumor may be selected from a tumor associated with the expression of the following targets: HER2, B7-H3, etc. For example, the tumor associated with the expression of the targets include tumors with high expression of these targets and/or tumors positive for these targets. For example, the tumor includes solid tumors and/or hematologic malignancies. For example, the tumor is selected from the group consisting of breast cancer, ovarian cancer, non-Hodgkin lymphoma, Hodgkin lymphoma, acute lymphoblastic leukemia, anaplastic large cell lymphoma, multiple myeloma, prostate cancer, lung cancer, malignant melanoma, squamous cell carcinoma, glioblastoma, renal cell carcinoma, gastrointestinal tumors, pancreatic cancer, prostate cancer, colorectal cancer, gastric cancer, glioma, and mesothelioma.

The present application provides a method for preventing and/or treating a tumor, which may include administering to a subject a compound of the present application, or a pharmaceutically acceptable salt, prodrug, hydrate, solvate, enantiomer, diastereomer, mesomer, racemate, or tautomer, or a pharmaceutical composition of the present application. For example, the tumor may be selected from a tumor associated with the expression of the following targets: HER2, B7-H3, etc. For example, the tumor associated with the expression of the targets include tumors with high expression of these targets and/or tumors positive for these targets. For example, the tumor includes solid tumors and/or hematologic malignancies. For example, the tumor is selected from the group consisting of breast cancer, ovarian cancer, non-Hodgkin lymphoma, Hodgkin lymphoma, acute lymphoblastic leukemia, anaplastic large cell lymphoma, multiple myeloma, prostate cancer, lung cancer, malignant melanoma, squamous cell carcinoma, glioblastoma, renal cell carcinoma, gastrointestinal tumors, pancreatic cancer, prostate cancer, colorectal cancer, gastric cancer, glioma, and mesothelioma.

### Pharmaceutical Composition

In addition to the active compound, the pharmaceutical composition described in the present application may contain one or more adjuvants, the adjuvants may be selected from the group consisting of fillers (diluents), binders, wetting agents, disintegrants, and excipients. The composition may contain 0.1 to 99% by weight of the active compound depending on the method of administration.

The active ingredient-containing pharmaceutical composition may be in a form suitable for oral, such as tablets, lozenges, troches, aqueous or oil suspensions, dispersible powders or granules, emulsions, hard or soft capsules, or syrups. An oral composition may be prepared according to any method known in the art for preparing pharmaceutical compositions, and the composition may contain binders, fillers, lubricants, disintegrants, or pharmaceutically acceptable wetting agents, or the like, and may also contain one or more ingredients selected from the group consisting of sweeteners, flavoring agents, colorants, and preservatives.

The aqueous suspension may contain an active substance and excipients suitable for the preparation of an aqueous suspension. The aqueous suspension may also contain one or more preservatives, such as one or more colorants, one or more flavoring agents, and one or more sweeteners. The oil suspension may be formulated by suspending the active ingredient in a vegetable oil. The oil suspension may contain thickeners. The aforementioned sweeteners and flavoring agents may also be added.

The pharmaceutical composition may also be dispersible powders or granules that provide the active ingredient, for preparing an aqueous suspension by adding water and mixing with one or more of dispersants, wetting agents, suspending agents, or preservatives. Other excipients such as sweeteners, flavoring agents, and colorants may also be added. These compositions are preserved by adding antioxidants such as ascorbic acid. The pharmaceutical composition of the present application may also be in the form of an oil-in-water emulsion.

The pharmaceutical composition may be in the form of a sterile injectable aqueous solution. The acceptable vehicles or solvents that can be used are water, Ringer's solution and isotonic sodium chloride solution. The sterile injectable formulation may be a sterile injectable oil-in-water microemulsion in which the active ingredient is dissolved in the oil phase. For example, an active ingredient is dissolved in a mixture of soybean oil and lecithin. The oil solution may then be added to a mixture of water and glycerol to form a microemulsion. The injection solution or microemulsion may be injected into the patient's bloodstream via local large-volume injection. Alternatively, the solution and microemulsion may be administered in such a manner that the constant cyclic concentration of the compounds of the present application can be maintained. To maintain such a constant concentration, a continuous intravenous delivery device may be used. For example, the device may be an intravenous infusion pump.

The pharmaceutical composition may be in the form of a sterile injectable aqueous or oil suspension for intramuscular and subcutaneous administration. The suspension may be formulated using suitable dispersants or wetting agents and suspending agents described above, according to known techniques. The sterile injectable formulation may also be a sterile injectable solution or suspension prepared in a parenterally acceptable nontoxic diluent or solvent. Alternatively, a sterile fixed oil can be conveniently used as a solvent or suspension medium.

The compounds of the present application may be administered in the form of suppositories for rectal administration. These pharmaceutical compositions can be prepared by mixing the drug with a suitable, nonirritating excipient which is solid at normal temperatures but liquid in the rectum, and thus will melt in the rectum to release the drug. Such materials include cocoa butter, glycerin gelatin, hydrogenated vegetable oils, mixtures of polyethylene glycol of various molecular weights and fatty acid esters of polyethylene glycol.

The pharmaceutical composition of the present application may contain a safe and effective amount of the antibody-drug conjugate of the present application and a pharmaceutically acceptable carrier. Such carriers may include (but are not limited to) saline, buffer solution, glucose, water, glycerol, ethanol, and combinations thereof. Generally, the pharmaceutical formulation should be matched to the route of administration. The pharmaceutical composition of the present application may be formulated in the form of solution, for example, prepared by conventional methods using physiological saline or an aqueous solution containing glucose and other adjuvants. The pharmaceutical composition can be produced under sterile conditions. The dosage of the active ingredient may be a therapeutically effective amount.

The effective amount of the antibody-drug conjugate described in the present application can vary depending on the mode of administration and the severity of the disease to be treated. The selection of the effective amount can be determined by those skilled in the art based on various factors (e.g., through clinical trials). The factors may include, but are not limited to: the pharmacokinetic parameters of the bifunctional antibody-drug conjugate, such as bioavailability, metabolism, and half-life; the severity of the disease to be treated, the patient's weight, the patient's immune status, and the route of administration, and the like. Generally, satisfactory results can be obtained when the antibody-drug conjugate of the present application is administered daily at an appropriate dose. For example, due to the urgency of the condition treated, a separate dose may be administered several times a day, or the dose may be reduced proportionally.

### EXAMPLE

The specific embodiments of the present application will be described below by specific examples, and those skilled in the art will readily appreciate from the disclosure of this specification the other advantages and advantages of the present application. Without being limited by any theory, the examples described below are merely for illustrating the compounds, preparation methods, and uses of the present application, and are not intended to limit the scope of the present disclosure.

The abbreviations used herein are defined as follows:
ADC: antibody-drug conjugate;
Ala: Alanine;
Boc: *t*-Butyloxy carbonyl;
Cit: Citrulline;
CD276 (Cluster of Differentiation 276): B7-H3;
DAR (Drug to antibody ratio): The molar ratio of drug to antibody;
DCC: Dicyclohexylcarbodiimide;
DCM: Dichloromethane;
DIPEA: *N,N*-Diisopropylethylamine;
DMAC (Dimethylacetamide): *N,N*-dimethylacetamide;
DMF: *N,N*-Dimethylformamide;
DMSO: Dimethyl Sulphoxide;
DX-8951f: Exatecan Mesylate;
EA: Ethyl acetate;
EEDQ: *N*-Ethoxycarbonyl-2-ethoxy-1,2-dihydroquinoline;
Fmoc: Fluorenylmethyloxycarbonyl;
HATU: N,N,N',N'-Tetramethyl-O-(7-azabenzotriazol-1-yl)uronium hexafluorophosphate
HER2: Human epidermal growth factor receptor 2;
HIC: Hydrophobic Interaction Chromatography;
His: Histidine;
HOBt (*N-Hydroxybenzotriazole*): 1-Hydroxybenzotriazole;
m-CPBA: m-Chloroperbenzoic Acid;
mM: Millimoles;
Me: methyl;
mAB: Monoclonal Antibody;
MMAE: Monomethyl auristatin E;
MMAF: Monomethyl auristatin F;
NHS: *N*-Hydroxysuccinimide;
NAC: *N*-Acetyl-*L*-cysteine;
NPC: Bis(4-nitrophenyl)carbonate;
PAB: *p*-Aminobenzyl alcohol;
Su/SuOH: Succinimide;
TCEP: Tris(2-carboxyethyl)phosphine;
TFA: Trifluoroacetic acid;
THF: Tetrahydrofuran;
VA (Valine-Alanine, Val-Ala): Valine-Alanine dipeptide;
Val: Valine;
VC (Valine-Citrulline, Val-Cit): Valine-Citrulline dipeptide;
DCU: Dicyclohexylurea;
EDCI: 1-(3-Dimethylaminopropyl)-3-ethylcarbodiimide hydrochloride;
TEA: Triethylamine;
Lys: Lysine;
Gly: Glycine;
Thr: Threonine;
Leu: Leucine;
Ile: Isoleucine;
Asn: Asparagine;
Phe: Phenylalanine.

In the examples, where specific conditions are not indicated, the procedures were carried out under conventional conditions or under conditions recommended by the manufacturer. Any reagents or instruments whose manufacturers are not specified are conventional products commercially available.

### Example 1: Preparation of Compound-1

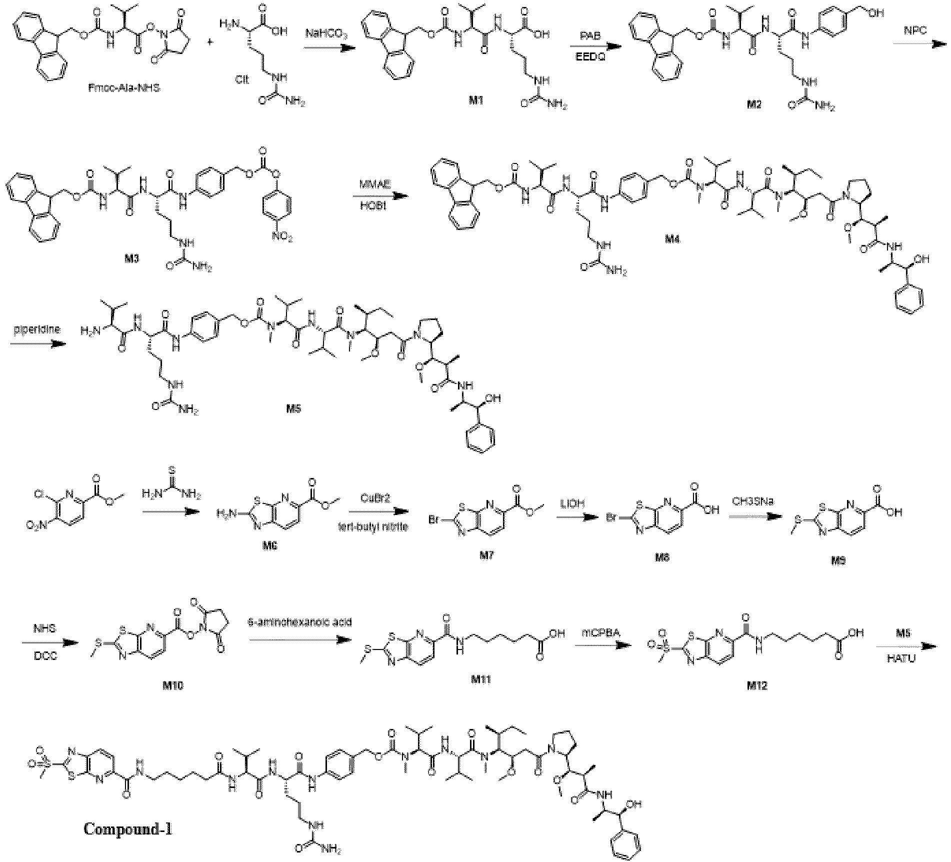

The specific synthesis steps are as follows.

### Step 1-1: Preparation of M1

Fmoc-Ala-NHS (25 g, 57.28 mM) was dissolved in a DME/THF mixed solution (2:1, 300 mL). After complete dissolution, a solution of citrulline (Cit; 13 g, 74.46 mM) in NaHCO₃ was added, and the reaction was stirred at room temperature for 20 hours. LC/MS monitoring showed that the reaction of compound Fmoc-Ala-NHS was complete, with M1 as the main product. After the reaction was complete, the reaction solution was poured into a 15% citric acid aqueous solution under stirring, and the jelly-like insoluble substance was precipitated. After filtering, the filter cake was thoroughly dried under vacuum and further dried by heating before being slurried twice with diethyl ether to obtain product M1 as a white solid powder, 27.88 g, with a yield of about 98%. ¹H NMR (400 MHz, DMSO-d6): δ 0.89 (dd, 6H), 1.42 (m, 2H), 1.58 (m, 1H), 1.72 (m, 1H), 1.98 (m, 1H), 2.96 (q, 2H), 3.94 (t, 1H), 4.16 (q, 1H), 4.25 (m,3H), 5.43 (s, 2H), 5.98 (t, 1H), 7.33 (t, 2H), 7.43 (q, 3H), 7.76 (t, 2H), 7.89 (d, 2H), 8.21 (d, 1H), 12.61 (s, 1H). MS (EI) m/z: 497.52 [M+H]⁺; 495.62 [M-H]⁻.

### Step 1-2: Preparation of M2

Compound M1 (15 g, 30.2 mM; 1 eq) was dissolved in a DCM/MeOH mixed solution (2:1, 450 mL). After complete dissolution, p-aminobenzyl alcohol (PAB; 7.44 g, 60.4 mM) and EEDQ (14.94 g, 60.4 mM) were added. The reaction was stirred for more than 36 hours at room temperature in the dark. After the reaction was completed, the solvent was removed by concentration under reduced pressure. The obtained residue was slurried three times with diethyl ether to obtain product M2 as a white solid powder, 17 g, with a yield of about 95%. ¹H NMR (400 MHz, DMSO-d6): δ 0.86 (dd, 6H), 1.41 (m, 2H), 1.57 (m, 1H), 1.69 (m, 1H), 1.98 (m, 1H), 3.00 (m, 2H), 3.92 (q, 1H), 4.23 (q, 2H), 4.30 (q, 1H), 4.41 (q, 3H), 5.12 (t, 1H), 5.42 (s, 2H), 5.98 (t, 1H), 7.23 (d, 2H), 7.32 (m, 2H), 7.41 (m, 2H), 7.46 (d, 1H), 7.54 (d, 2H), 7.74 (t, 2H), 7.89 (d, 2H), 8.12 (d, 1H), 9.99 (s, 1H). MS (EI) m/z: 602.67 [M+H]⁺; 624.48 [M+Na]⁺.

### Step 1-3: Preparation of M3

Compound M2 (1.5 g, 2.49 mM) was dissolved in DMF (40 mL). After complete dissolution, p-nitrophenol carbonate (NPC; 1.52 g, 4.99 mM) and DIPEA (0.48 g, 3.74 mM) were added. The reaction was stirred overnight at room temperature. After the reaction was complete, the solvent was removed by concentration under reduced pressure. The obtained residue was slurried three times with diethyl ether. Product M3 was a white solid powder, 1.2 g, with a yield of about 68%. The obtained material was directly used for the next reaction without further purification. MS (EI) m/z: 767.05 [M+H]⁺; 789.26 [M+Na]⁺.

### Step 1-4: Preparation of M4

Compound M3 (128 mg, 0.167 mM) was dissolved in DMF (40 mL). After complete dissolution, MMAE (100 mg, 0.139 mM), HOBt (18.82 mg, 0.139 mM), and DIPEA (36 mg, 0.279 mM) were added. The reaction was stirred overnight at room temperature. After the reaction was complete, the solvent was removed by concentration under reduced pressure. The obtained residue was slurried three times with ethyl acetate (EA). After filtration, the white solid powder product M4 was obtained in a total of 150 mg, with a yield of about 80%. MS (EI) m/z: 673.80 [M+2H]²⁺.

### Step 1-5: Preparation of M5

Compound M4 (135 mg, 0.1 mM) was dissolved in DMF (20 mL), and piperidine (1 mL) was added. The reaction was stirred at room temperature for 2 hours, and the starting material had disappeared by LC/MS monitoring. The solvent was removed by concentration under reduced pressure, and the obtained residue was slurried with diethyl ether and filtered. The filter cake was further purified by column chromatography to obtain product M5 as a pale yellow solid powder, 83 mg, with a yield of about 74%. MS (EI) m/z: 1123.68 [M+H]⁺; MS (EI) m/z: 562.41 [M+2H]²⁺.

### Step 1-6: Preparation of M6

Methyl 5-chloro-4-nitro-2-pyridinecarboxylate (5 g, 23 mM) and thiourea (5 g, 2.8 eq) were dissolved in sulfolane (30 mL), and the reaction was heated to 130 °C and stirred for 2-4 hours. After the reaction was complete, the reaction solution was allowed to stand and slowly cooled, and then purified directly by column chromatography to obtain M6 as an orange-yellow solid powder, 3.68 g, with a yield of about 77%. ¹H NMR (400 MHz, DMSO-*d*₆) δ 8.26 (s, 2H), 7.95 (d, *J =* 8.3 Hz, 1H), 7.68 (d, *J =* 8.3 Hz, 1H), 3.86 (s, 3H). MS (EI) m/z: 209.29 [M+H]⁺.

### Step 1-7: Preparation of M7

M6 (3.77 g, 18 mM) was dispersed in acetonitrile, and copper bromide (8.4 g, 36 mM) was added under stirring at room temperature. After complete dissolution, tert-butyl nitrite (3.71 g, 36 mM) was added dropwise in portions. After the addition, the reaction was stirred at room temperature for about 5 hours. After the reaction was complete, the acetonitrile was removed by concentration under reduced pressure. The residue was redissolved with water and extracted with ethyl acetate twice. The organic phase was washed twice with water and saturated NaCl solution, respectively, then dried over Na₂SO₄, and concentrated to obtain a yellow solid residue. The crude product was further purified by silica gel column chromatography to obtain product M7 as a white solid powder, 4.03 g, with a yield of about 82%. ¹H NMR (400 MHz, DMSO-*d*₆) δ 8.57 (d, *J* = 8.5 Hz, 1H), 8.24 (d, *J =* 8.5 Hz, 1H), 3.94 (s, 3H). MS (EI) m/z: 272.74 / 274.84 [M+H]⁺.

### Step 1-8: Preparation of M8

M7 (2.05 g, 7.47 mM) was dissolved in tetrahydrofuran (THF, 100 mL). LiOH monohydrate (313 mg, 7.47 mM) was added with stirring at room temperature, followed by about 20 mL of water. The reaction was carried out for about 10 minutes. LC/MS monitoring showed that the reaction of the compound M7 is complete, with compound M8 as the main product. The solvent was removed by concentration under reduced pressure. The residue was redissolved with water, and the pH was adjusted to 3-4 with diluted hydrochloric acid. The suspension was allowed to stand for 15 minutes and then filtered. The filter cake was washed 2-3 times with distilled water and thoroughly dried to obtain product M8 as a pale yellow solid powder, 1.8 g, with a yield of about 93%. ¹H NMR (400 MHz, DMSO-*d*₆) δ 13.61 (s, 1H), 8.55 (d, *J =* 8.4 Hz, 1H), 8.23 (d, *J =* 8.5 Hz, 1H). MS (EI) m/z: 258.83 / 260.73 [M+H]⁺.

### Step 1-9: Preparation of M9

M8 (1.80 g, 6.95 mM) was dissolved in a mixed solvent of tetrahydrofuran and MeOH (1:1, 80 mL). An aqueous solution of sodium methanethiol (20%, 0.974 g, 13.9 mM) was added with stirring at room temperature. After addition, the reaction was stirred for about 10 minutes at room temperature. LC/MS monitoring showed that the reaction of compound M8 is complete, with compound M9 as the main product. The solvent was removed by concentration under reduced pressure. The residue was redissolved with water. The pH was first adjusted to ~10 using diluted NaOH solution, and then sonicated to fully dissolve the residue. The pH was then adjusted to about 3-4 using diluted hydrochloric acid. The suspension was allowed to stand for 15 minutes and then filtered. The filter cake was washed 2-3 times with distilled water and thoroughly dried to obtain product M9 as a pale yellow solid powder, 1.5 g, with a yield of about 95%. ¹H NMR (400 MHz, DMSO-*d*₆) δ 13.26 (s, 1H), 8.31 (d, *J =* 8.4 Hz, 1H), 8.16 (d, *J* = 8.4 Hz, 1H), 2.85 (s, 3H). MS (EI) m/z: 226.90 [M+H]⁺; MS (EI) m/z: 224.78 [M-H]⁻.

### Step 1-10: Preparation of M10

M9 (1.3 g, 5.74 mM) was dissolved in DMF (30 mL). After complete dissolution, NHS (1.32 g, 11.49 mM), DCC (1.78 g, 8.62 mM), and DIPEA (cat., 0.2 mL) were added. The reaction was carried out overnight at room temperature with stirring. LC/MS monitoring showed that the reaction of the compound M9 is complete, with compound M10 as the major product. The insoluble DCU was removed by filtration, and the solvent was removed by concentration under reduced pressure. The obtained residue was directly mixed with crude silica gel, and purified by silica gel column chromatography to obtain the product M10 as an off-white solid powder, 1.78 g, with a yield of about 96%. ¹H NMR (400 MHz, DMSO-d₆) δ 8.43 (d, *J* = 8.5 Hz, 1H), 8.33 (d, *J* = 8.5 Hz, 1H), 2.93 (s, 4H), 2.88 (s, 3H). MS (EI) m/z: 323.98 [M+H]⁺.

### Step 1-11: Preparation of M11

M10 (1.0 g, 3.1 mM) was dissolved in DMF (30 mL). After complete dissolution, 6-aminohexanoic acid (0.61 g, 4.65 mM) and a catalytic amount of DIPEA were added. The reaction was carried out overnight at room temperature with stirring. After the reaction was completed, the solvent was removed by concentration under reduced pressure to obtain a pale yellow solid residue. The obtained residue was purified by silica gel column chromatography to obtain product M11 as a white solid powder, 916 mg, with a yield of about 87%. ¹H NMR (500 MHz, DMSO-*d6*): δ 12.01 (br, 1H), 8.16 (d, *J =* 7.7 Hz, 1H), 8.06 (d, *J =* 7.5 Hz, 1H), 8.01 (t, 1H), 3.35 (q, *J* = 5.4 Hz, 2H), 2.77 (s, 3H), 2.26 (t, *J =* 8.9 Hz, 2H), 1.62 - 1.56 (m, 2H), 1.59 - 1.51 (m, 2H), 1.42 - 1.32 (m, 2H). MS (EI) m/z: 340.06 [M+H]⁺; MS (EI) m/z: 338.08 [M-H]⁻.

### Step 1-12: Preparation of M12

M11 (500 mg, 1.47 mM) was dissolved in DCM (30 mL). After complete dissolution, m-chloroperoxybenzoic acid (m-CPBA, 85%; 1.0 g, 5.89 mM) was added. The reaction was carried out overnight at room temperature with stirring. LC/MS monitoring showed that the reaction of the compound M11 is complete, with compound M12 as the major product. The solvent was removed by concentration under reduced pressure. The obtained residue was mixed with crude silica gel and purified by silica gel column chromatography to obtain product M12 as a white solid powder, 509 mg, with a yield of about 93%. ¹H NMR (500 MHz, DMSO-*d6*) δ 8.21 (d, *J =* 7.5 Hz, 1H), 8.09 (d, *J* = 7.5 Hz, 1H), 8.01 (t, 1H), 3.48 (s, 3H), 3.35 (q, *J* = 5.4 Hz, 2H), 2.26 (t, *J* = 8.9 Hz, 2H), 1.61 - 1.55 (m, 2H), 1.58 - 1.51 (m, 2H), 1.42 - 1.32 (m, 2H). MS (EI) m/z: 372.08 [M+H]⁺; MS (EI) m/z:370.05 [M-H]⁻.

### Step 1-13: Preparation of Compound-1

M5 (50 mg, 0.0445 mM) and M12 (20 mg, 0.0534 mM) were dissolved in DMF (10 mL). After complete dissolution by stirring, the reaction system was transferred to an ice bath for cooling. Then, HATU (25.4 mg, 0.668 mM) and a catalytic amount of DIPEA were added to the reaction solution. After the addition, the reaction was stirred in an ice bath for 30 minutes, then gradually brought to room temperature and stirred overnight at room temperature. After the reaction was complete, the solvent was removed by concentration under reduced pressure. The obtained residue was directly mixed with crude silica gel and purified by silica gel column chromatography to obtain compound-1 as a white solid, which was further lyophilized to obtain 36.8 mg of a fluffy white solid, with a yield of about 56%. MS (EI) m/z: 738.38 [M+2H]²⁺; MS (ESI) m/z: 1476.7643 [M+H]⁺.

### Example 2: Preparation of Compound-2

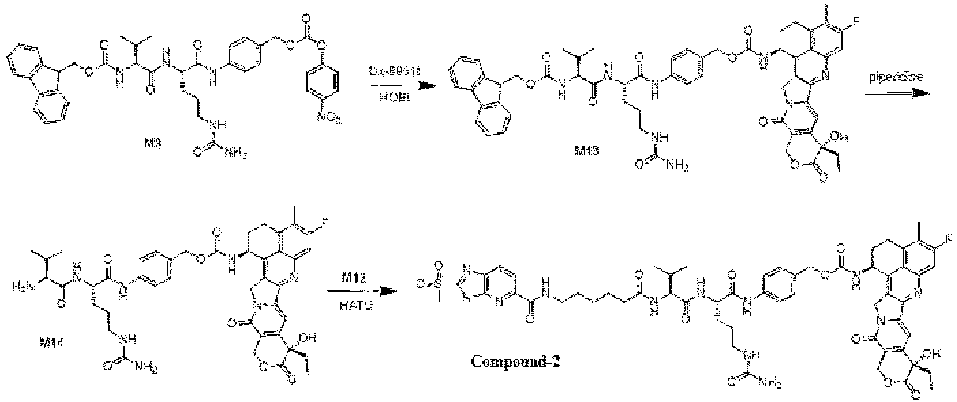

The specific synthesis steps are as follows.

### Step 2-1: Preparation of M13

Following the synthesis method of M4, Dx-8951 mesylate was used in place of the MMAE to react with M3, to obtain M13 as a pale yellow solid powder with a yield of about 83%. MS (EI) m/z: 1063.45 [M+H]⁺.

### Step 2-2: Preparation of M14

Following the synthesis method of M5, M13 was used in place of M4 to undergo a deprotection reaction in piperidine solution, to obtain M14 as a pale yellow solid powder with a yield of about 70%. MS (EI) m/z: 841.41 [M+H]⁺.

### Step 2-3: Preparation of Compound-2

Following the synthetic method of compound-1, M14 was used in place of M5 to react with M12, to obtain compound-2 as a pale yellow solid powder with a yield of about 56%. MS (EI) m/z: 597.68 [M+2H]²⁺; MS (ESI) m/z: 1194.4175 [M+H]⁺.

### Example 3: Preparation of Compound-3

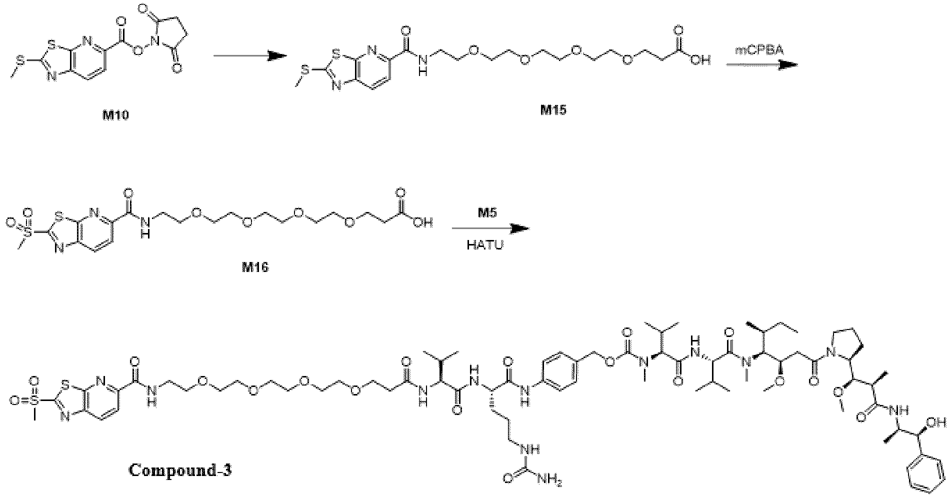

The specific synthesis steps are as follows.

### Step 3-1: Preparation of M15

Following the synthesis method of M11, 1-amino-3,6,9,12-tetraoxapentadecan-15-oic acid was used in place of 6-aminocaproic acid to react with M10, to obtain M15 as a light yellow viscous oil with a yield of about 92%. ¹H NMR (500 MHz, Chloroform-*d*) δ 12.04 (br, 1H), 8.26 (t, 1H), 8.16 (d, *J* = 7.7 Hz, 1H), 8.06 (d, *J =* 7.5 Hz, 1H), 3.69 - 3.59 (m, 15H), 3.59 - 3.53 (m, 2H), 3.40 (dt, *J =* 5.6, 4.2 Hz, 2H), 2.77 (s, 3H), 2.48 (t, *J* = 6.4 Hz, 2H). MS (EI) m/z: 474.07 [M+H]⁺.

### Step 3-2: Preparation of M16

Following the synthesis method of M12, M15 was used in place of M11 to react with m-CPBA, to obtain M16 as a light yellow viscous oil with a yield of about 80%. ¹H NMR (500 MHz, Chloroform-d) δ 12.04 (br, 1H), 8.26 (t, 1H), 8.16 (d, *J =* 7.7 Hz, 1H), 8.06 (d, *J* = 7.5 Hz, 1H), 3.69 - 3.59 (m, 15H), 3.59 - 3.53 (m, 2H), 3.40 (dt, *J* = 5.6, 4.2 Hz, 2H), 2.77 (s, 3H), 2.48 (t, *J =* 6.4 Hz, 2H). MS (EI) m/z: 505.98 [M+H]⁺; 528.10 [M+Na]⁺.

### Step 3-3: Preparation of Compound-3

Following the synthetic method of compound-1, M16 was used in place of M12 to react with M5, to obtain compound-3 as a white solid powder with a yield of about 63%. MS (EI) m/z: 805.92 [M+2H]²⁺; MS (ESI) m/z: 1610.8215 [M+H]⁺.

### Example 4: Preparation of Compound-4

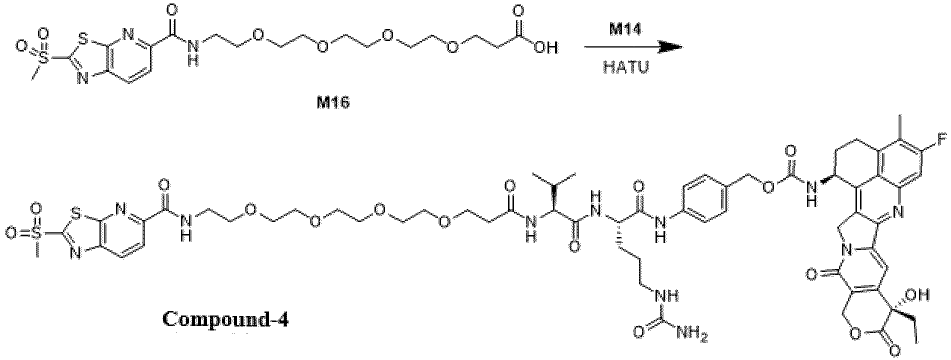

The specific synthesis steps are as follows.

### Step 4-1: Preparation of Compound-4

Following the synthetic method of compound-2, M16 was used in place of M12 to react with M14, to obtain compound-4 as a pale yellow solid powder with a yield of about 55%. MS (EI) m/z: 664.71 [M+2H]²⁺; MS (ESI) m/z: 1328.4768 [M+H]⁺.

### Example 5: Preparation of Compound-5

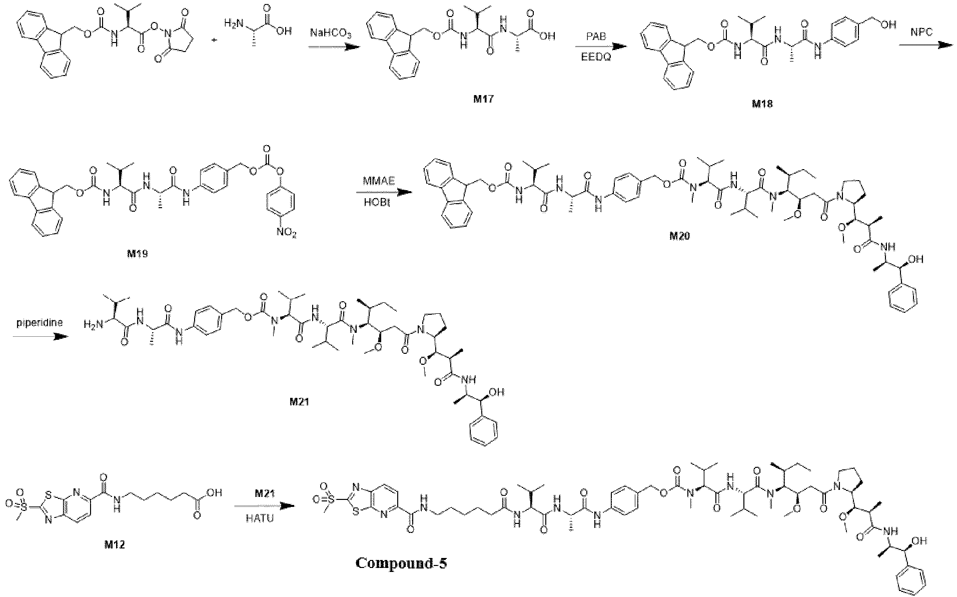

The specific synthesis steps are as follows.

### Step 5-1: Preparation of M17

Following the synthetic method of M1, L-alanine was used in place of L-citrulline to react with Fmoc-Ala-NHS, to obtain compound M17 as a white solid powder with a yield of about 83%. ¹H NMR (400 MHz, DMSO-d6): δ 0.88 (dd, 6H), 1.27 (d, 3H), 1.96 (m, 1H), 3.89 (q, 1H), 4.22 (m, 4H), 7.33 (t, 2H), 7.43 (m, 3H), 7.74 (t 2H), 7.89 (d, 2H), 8.25 (d, 1H), 12.48 (s, 1H). MS (EI) m/z: 411.23 [M+H]⁺; 455.05 [M+Na]⁺.

### Step 5-2: Preparation of M18

Following the synthesis method of M2, M17 was used in place of M1 to undergo a condensation reaction with PAB by using EEDQ, to obtain compound M18 as a white solid powder with a yield of about 87%. ¹H NMR (400 MHz, DMSO-d6): δ 0.88 (dd, 6H), 1.30 (d, 3H), 2.00 (m, 1H), 3.91 (t, 1H), 4.22 (q, 2H), 4.30 (t, 1H), 4.40 (br, 1H), 4.42 (d, 2H), 5.13 (t, 1H), 7.24 (t, 2H), 7.34 (t, 2H), 7.41 (t, 2H), 7.52 (q, 3H), 7.75 (t, 2H), 7.89 (d, 2H), 8.22 (d, 1H), 9.96 (s, 1H). MS (EI) m/z: 516.29 [M+H]⁺; 538.20 [M+Na]⁺.

### Step 5-3: Preparation of M19

Following the synthetic method of M3, M18 was used in place of M2 to react with p-nitrophenol carbonate, to obtain compound M19 as a white solid powder with a yield of about 92%. The obtained material was directly used for the next reaction without further purification. MS (EI) m/z: 681.24 [M+H]⁺; 703.20 [M+Na]⁺.

### Step 5-4: Preparation of M20

Following the synthetic method of M4, M19 was used in place of M3 to react with MMAE, to obtain compound M20 as a pale yellow solid powder with a yield of about 67%. MS (EI) m/z: 630.34 [M+2H]²⁺; MS (ESI) m/z: 1259.7341 [M+H]⁺.

### Step 5-5: Preparation of M21

Following the synthetic method of M5, M20 was used in place of M4 to undergo a deprotection reaction in piperidine solution to remove the Fmoc protecting group, to obtain compound M21 as a pale yellow solid powder with a yield of about 55%. The product has a certain hygroscopicity and is used directly for the next reaction without being left to stand. MS (EI) m/z: 1123.68 [M+H]⁺.

### Step 5-6: Preparation of Compound-5

Following the synthetic method of compound-1, M21 was used in place of M5 to react with compound M12, to obtain compound-5 as an off-white solid powder with a yield of about 53%. MS (EI) m/z: 695.86 [M+2H]²⁺; MS (ESI) m/z: 1390.7162 [M+H]⁺.

### Example 6: Preparation of Compound-6

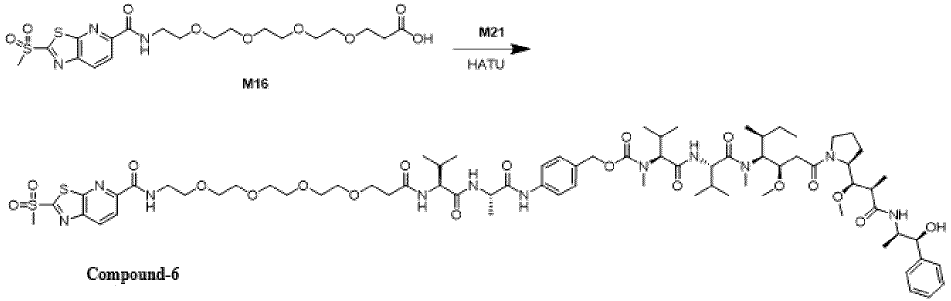

The specific synthesis steps are as follows.

### Step 6-1: Preparation of Compound-6

Following the synthetic method of compound-5, M16 was used in place of M12 to react with M21, to obtain compound-6 as an off-white solid powder with a yield of about 61%. MS (EI) m/z:762.93 [M+2H]²⁺; MS (ESI) m/z: 1524.7745 [M+H]⁺.

### Example 7: Preparation of Compound-7

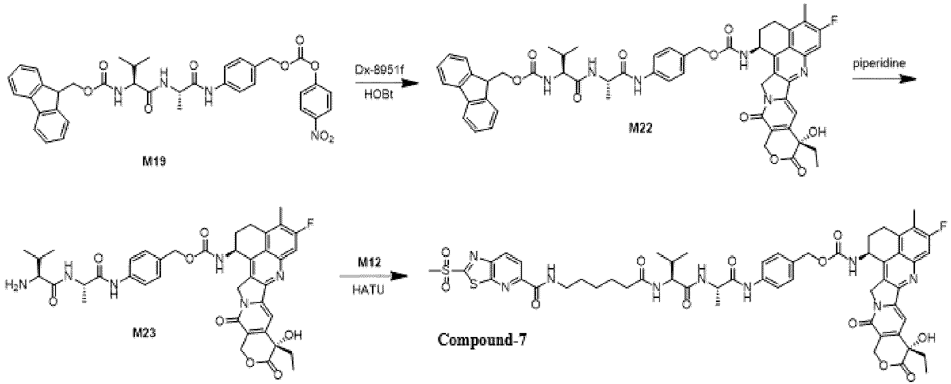

The specific synthesis steps are as follows.

### Step 7-1: Preparation of M22

Following the synthesis method of M13, M19 was used in place of M3 to undergo a condensation reaction with Dx-8951f, to obtain M22 as a pale yellow solid powder with a yield of about 85%. MS (EI) m/z:977.40 [M+H]⁺.

### Step 7-2: Preparation of M23

Following the synthesis method of M14, M22 was used in place of M13 to undergo a deprotection reaction in piperidine solution to remove the Fmoc protecting group, to obtain M23 as a pale yellow solid powder with a yield of about 75%. MS (EI) m/z:755.36 [M+H]⁺.

### Step 7-3: Preparation of Compound-7

Following the synthetic method of compound-2, M23 was used in place of M14 to undergo a condensation reaction with M12 in the presence of HATU, to obtain compound-7 as a pale yellow solid powder with a yield of about 63%. MS (EI) m/z: 1108.33 [M+H]⁺; MS (ESI) m/z: 1130.32 [M+Na]⁺.

### Example 8: Preparation of Compound-8

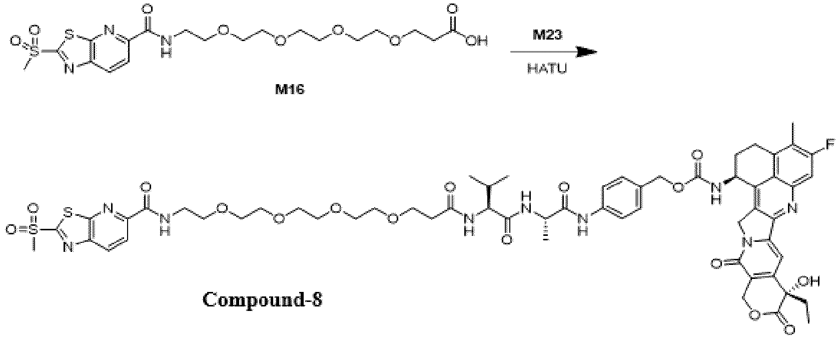

The specific synthesis steps are as follows.

### Step 8-1: Preparation of Compound-8

Following the synthetic method of compound-7, M16 was used in place of M12 to undergo a condensation reaction with M23 in the presence of HATU, to obtain compound-8 as a pale yellow solid powder with a yield of about 56%. MS (EI) m/z: 621.74 [M+2H]²⁺; MS (ESI) m/z: 1242.4294 [M+H]⁺.

### Example 9: Preparation of Compound-9

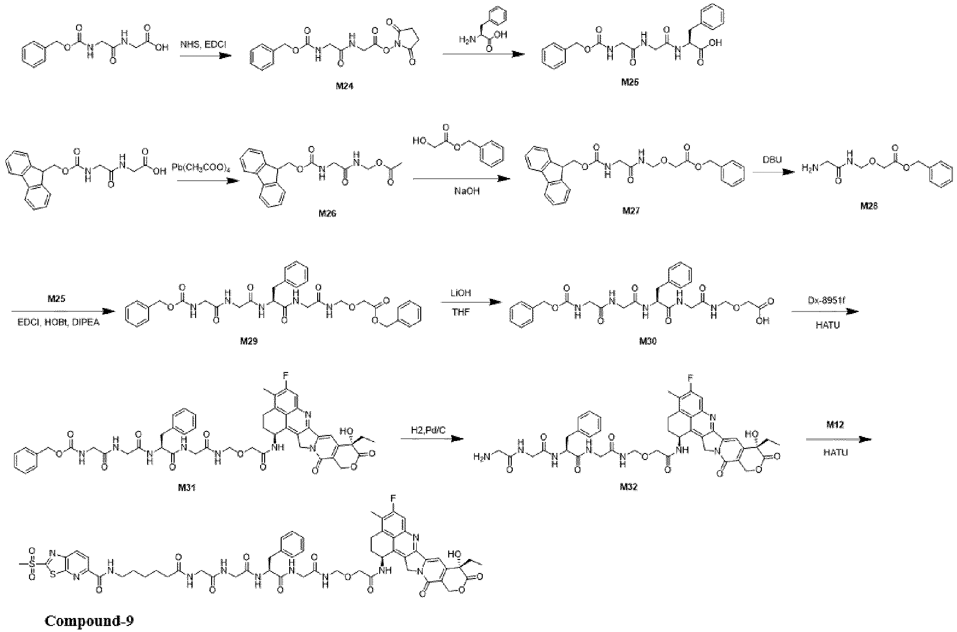

The specific synthesis steps are as follows.

### Step 9-1: Preparation of M24

The benzyloxycarbonyl protected diglycine (10 g, 37.56 mM) was dissolved in acetonitrile (100 mL), and NHS (4.75 g, 41.31 mM) and EDCI (8.64 g, 45.07 mM) were added with stirring. The mixture was stirred overnight under nitrogen protection. After the reaction was complete, the solvent was removed by concentration under reduced pressure to obtain a colorless oil residue. The oil was redissolved in ethyl acetate, washed with distilled water, and separated, repeated three times. The final organic phase was washed three times with saturated brine, and the reaction solution was concentrated to about 100 mL. After standing at room temperature for 24 hours, a white insoluble substance precipitated. The filter cake was filtered and washed with EA. After the filter cake was thoroughly dried, product M24 was obtained as a white solid powder, 13 g, with a yield of about 96%. M24 is an active ester intermediate, which is directly used in the next reaction without being placed for a long time. MS (EI) m/z: 364.18 [M+H]⁺; MS (EI) m/z: 386.10 [M+Na]⁺.

### Step 9-2: Preparation of M25

Phenylalanine (4.55 g, 27.52 mM) was dissolved in a mixture of acetonitrile and water (1:1; 100 mL). Triethylamine (TEA, 2.78 g, 27.52 mM) was added, and the mixture was stirred at room temperature for 5 minutes until the solid was completely dissolved. M24 (10 g, 27.52 mM) was added, and the reaction was continued to be stirred at room temperature for 3 hours. After the reaction was complete, water (50 mL) was added to the reaction solution, and hydrochloric acid was added dropwise under stirring to adjust the pH to ~2. The solution was concentrated under reduced pressure to remove most of the acetonitrile, and the remaining liquid was left to stand at room temperature overnight to precipitate a white solid. The filter cake was filtered, and washed with diethyl ether, and dried thoroughly to obtain product M25 as a white solid powder, 13.2 g, with a yield of about 84%. ¹H NMR (400 MHz, DMSO-*d6*) δ 7.77 (d, *J* = 9.1 Hz, 1H), 7.48 (t, *J =* 6.0 Hz, 1H), 7.38 - 7.18 (m, 10H), 5.89 (t, *J* = 6.6 Hz, 1H), 5.10 (d, *J =* 0.8 Hz, 2H), 4.45 (dt, *J* = 9.2, 7.0 Hz, 1H), 3.86 (d, *J* = 6.6 Hz, 2H), 3.84 - 3.71 (m, 2H), 3.12 - 3.06 (m, 2H). MS (EI) m/z: 414.19 [M+H]⁺; MS (EI) m/z: 412.13 [M-H]⁻.

### Step 9-3: Preparation of M26

Fmoc-protected diglycine (12.49 g, 35.24 mM) was dispersed in a mixed solution of tetrahydrofuran (200 mL) and acetic acid (50 mL). The mixture was heated to 40 °C with stirring until the solid dissolved completely. Lead tetraacetate (25 g, 56.39 mmol) was added, and the mixture was then heated to reflux for 2 hours. After the reaction was complete, heating was stopped and the mixture was allowed to cool naturally to room temperature. With stirring, about 100 mL of 20% sodium citrate solution was added to the mixture, resulting in the precipitation of a pale yellow precipitate. After the insoluble matter was removed by filtration, the filtrate was washed 2-3 times with 20% sodium citrate solution. The separated organic phase was concentrated to about 60 mL, and about 100 mL of distilled water was added to the concentrated solution. After standing overnight at room temperature, the mixture was filtered, and the filter cake was washed 3 times with distilled water and thoroughly dried under vacuum, to obtain M26 as a white solid powder, 13.2 g, with a yield of about 99%.

¹H NMR (400 MHz, Chloroform-*d*) δ 7.82 (dd, *J* = 8.2, 1.3 Hz, 2H), 7.62 (dd, *J* = 7.6, 1.4 Hz, 2H), 7.53 - 7.46 (m, 3H), 7.41 (td, *J* = 7.8, 1.5 Hz, 2H), 5.81 (t, *J =* 6.6 Hz, 1H), 5.44 - 5.39 (m, 1H), 5.04 (d, *J =* 3.5 Hz, 2H), 4.42 (d, *J* = 4.8 Hz, 2H), 3.84 (d, *J =* 6.6 Hz, 2H), 2.19 (s, 3H). MS (EI) m/z: 369.21 [M+H]⁺.

### Step 9-4: Preparation of M27

M26 (12.2 g, 33.12 mM) was dissolved in glycol dimethyl ether (DME; 200 mL), and benzyl glycolate (11 g, 66.23 mM) was added to the reaction mixture. After the solid was completely dissolved, the reaction mixture was cooled in an ice bath. 10 M NaOH solution (10 eq, 1.32 g) was added dropwise to the reaction mixture, and the reaction was stirred at low temperature for 1 hour. About 1.3 mL of glacial acetic acid was added dropwise to the reaction mixture, and the mixture was stirred at low temperature for another 1 hour. After the reaction was complete, 150 mL of water was added to the reaction system, and the mixture was stirred at low temperature for another 2.5 hours. The mixture was filtered, and the filter cake was washed three times with a mixture of water and glycol dimethyl ether (1:1). After thorough drying, M27 was obtained as a white solid powder, 13.94 g, with a yield of about 89%. The obtained solid product was used directly in the next reaction. MS (EI) m/z: 475.22 [M+H]⁺.

### Step 9-5: Preparation of M28

M27 (13 g, 23.4 mM) was dissolved in acetonitrile (400 mL), and DBU (4.59 g, 30.14 mM) was added to the reaction mixture. The mixture was stirred at room temperature for 4 hours. After the reaction was complete, the solvent was removed by concentration under reduced pressure to obtain a viscous oil residue. The residue was further purified by silica gel column chromatography to obtain a product with a yield >100%, which was suspected of containing trapped residual solvent. The obtained free amine product M28 exhibited poor stability and was directly used in the next reaction. MS (EI) m/z: 253.23 [M+H]⁺.

### Step 9-6: Preparation of M29

M25 (9.0 g, 22 mM) and M28 (6.3 g, 25 mM) were dissolved in acetonitrile (200 mL). A catalytic amount of DIPEA (0.5 eq) was added, and the reaction system was cooled to about 0 °C in an ice bath. EDCI (5.75 g, 30 mM) was added in portions, and the reaction was continued at low temperature for 1 hour. Then, the mixture was transferred to room temperature and reacted for another 2 hours. After the reaction was complete, the solvent was removed by concentration under reduced pressure to obtain a yellow oil residue. The residue was redissolved in ethyl acetate, washed with water, and separated three times. The organic solvent was then concentrated to about 100 mL. The mixture was stirred overnight at room temperature, and the precipitated paste-like insoluble matter was filtered out, thoroughly dried under vacuum, and then dried completely by heating to obtain M29 as a pale yellow solid powder, 13.4 g, with a yield of about 94%. MS (EI) m/z: 648.33 [M+H]⁺; MS (EI) m/z: 670.26 [M+Na]⁺.

### Step 9-7: Preparation of M30

M29 (1.0 g, 1.54 mM) was dissolved in methanol (50 mL), and LiOH monohydrate (77.7 mg, 1.85 mM) and a catalytic amount of water were added with stirring at room temperature. The reaction was carried out overnight at room temperature, and the solvent was removed by concentration under reduced pressure. The obtained solid residue was mixed with crude silica gel and purified by silica gel column chromatography to obtain product M30 as a white solid powder, 300 mg, with a yield of about 35%. MS (EI) m/z: 580.31 [M+Na]⁺; MS (EI) m/z: 556.24 [M-H]⁻.

### Step 9-8: Preparation of M31

M30 (100 mg, 0.179 mM) was dissolved in DMF (20 mL). After the solid was completely dissolved, Dx-8951f methanesulfonate (64 mg, 0.1196 mM), HATU (136.39 mg, 0.359 mM), and DIPEA (92.73 mg, 0.7174 mM) were added sequentially. After the reaction was stirred at room temperature for 4 hours, the solvent was removed by concentration under reduced pressure. The obtained oil residue was purified by silica gel column chromatography to obtain product M31, 210 mg, yield >100%, which was suspected of containing a small amount of trapped silica gel. The obtained crude product was directly used for the next step without further purification. MS (EI) m/z: 975.41 [M+H]⁺.

### Step 9-9: Preparation of M32

Following the synthetic method of M14, M31 was used in place of M13 to undergo a deprotection reaction in piperidine solution to remove the Fmoc protecting group, to obtain M32 as a pale yellow solid powder with a yield of about 57%. The obtained amino product exhibited poor stability and was directly used in the next reaction without further purification. MS (EI) m/z:841.45 [M+H]⁺.

### Step 9-10: Preparation of Compound-9

Following the synthetic method of compound-2, M32 was used in place of M14 to undergo a condensation reaction with M12 in the presence of HATU, to obtain compound-9 as a pale yellow solid powder with a yield of about 45%. MS (EI) m/z: 597.72 [M+2H]²⁺; MS (ESI) m/z: 1194.3832 [M+H]⁺.

### Example 10: Preparation of Compound-10

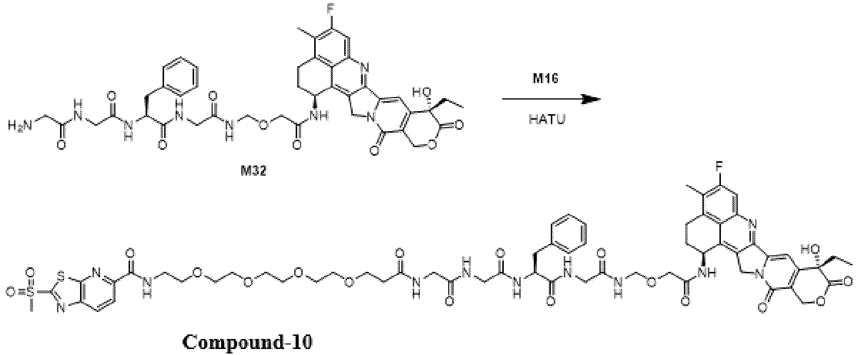

The specific synthesis steps are as follows.

### Step 10-1: Preparation of Compound-10

Following the synthetic method of compound-9, M16 was used in place of M12 to undergo a condensation reaction with M32 in the presence of HATU, to obtain compound-10 as a pale yellow solid powder with a yield of about 47%. MS (EI) m/z: 664.69 [M+2H]²⁺; MS (ESI) m/z: 1328.4436 [M+H]⁺.

### Example 11: Preparation of Compound-11

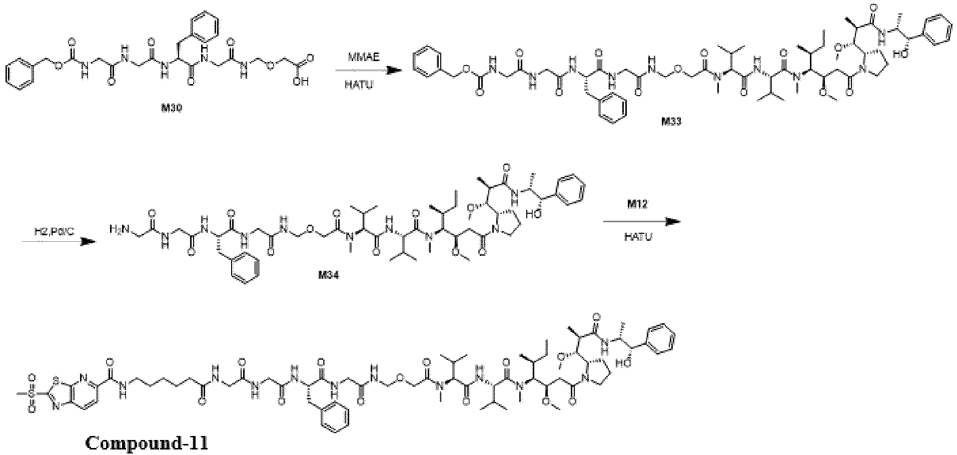

The specific synthesis steps are as follows.

### Step 11-1: Preparation of M33

Following the synthetic method of compound M31, MMAE was used in place of Dx-8951f methanesulfonate to undergo a condensation reaction with M30 in the presence of HATU, to obtain intermediate product M33 as a white solid powder with a yield of about 78%. MS (EI) m/z: 629.33 [M+2H]²⁺; MS (ESI) m/z: 1257.7135 [M+H]⁺.

### Step 11-2: Preparation of M34

Compound M33 (100 mg, 0.08 mM) was dissolved in methanol (10 mL), and a catalytic amount of Pd/C (~15 mg) was added. The reaction was stirred under a hydrogen atmosphere. After reacting at room temperature for 3 hours, the hydrogen balloon was removed, and the mixture was filtered to remove insoluble matter. The solution was then concentrated under reduced pressure to obtain a residue as a pale yellow glassy. The residue was further purified by silica gel column chromatography to obtain compound M34 as a white solid powder, 61 mg, in about 67% yield. MS (ESI) m/z: 1123.68 [M+H]⁺.

### Step 11-3: Preparation of Compound-11

Following the synthetic method of compound-1, M34 was used in place of M5 to undergo a condensation reaction with M12 in the presence of HATU, to obtain compound-11 as a white solid powder with a yield of about 46%. MS (EI) m/z: 738.79 [M+2H]²⁺; MS (ESI) m/z: 1476.7281 [M+H]⁺.

### Example 12: Preparation of Compound-12

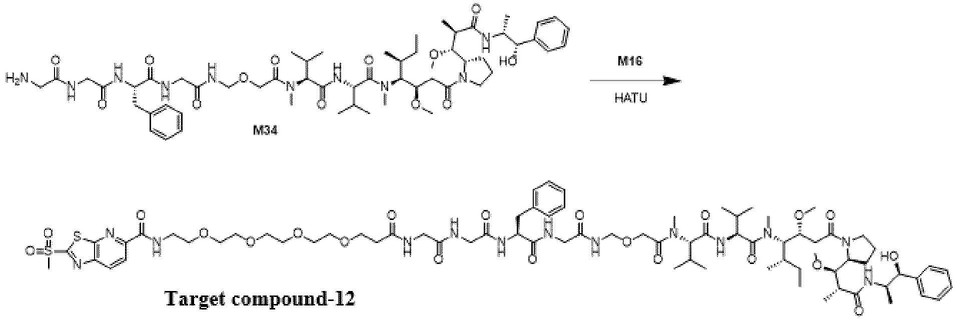

The specific synthesis steps are as follows.

### Step 12-1: Preparation of Compound-12

Following the synthetic method of compound-11, M16 was used in place of M12 to undergo a condensation reaction with M34 in the presence of HATU, to obtain compound-12 as a white solid powder with a yield of about 53%. MS (EI) m/z: 805.95 [M+2H]²⁺; MS (ESI) m/z: 1610.7848 [M+H]⁺.

### Example 13: Preparation of Compound-13

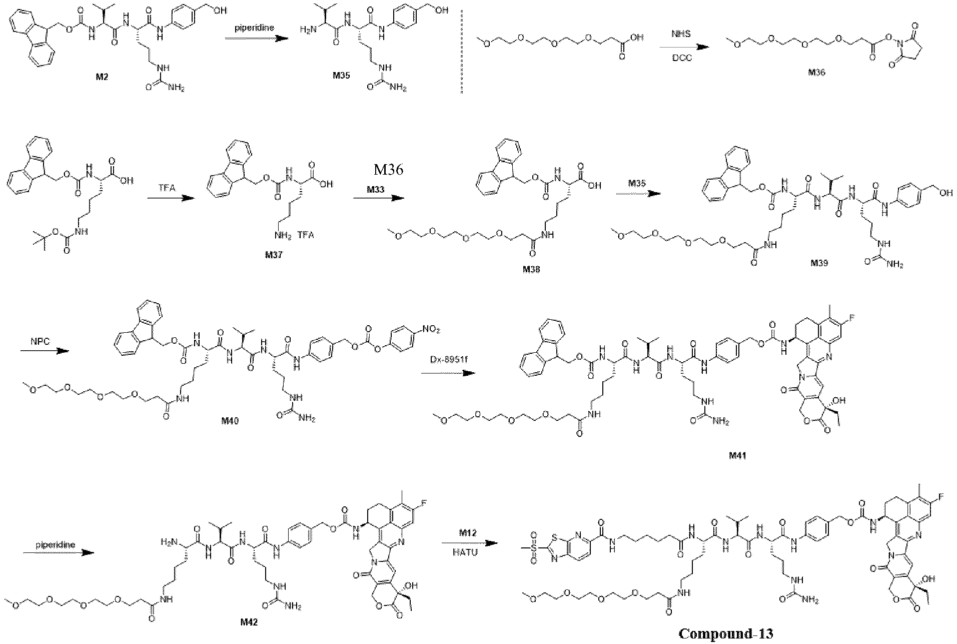

The specific synthesis steps are as follows.

### Step 13-1: Preparation of M35

M2 (1.0 g, 1.66 mM) was dissolved in DMF (15 mL). After the solid was completely dissolved, piperidine (1 mL) was added, and the reaction was stirred at room temperature for 2 hours. After the reaction was complete, the solvent was removed by concentration under reduced pressure. The obtained oil residue was purified by silica gel column chromatography to obtain product as a colorless viscous liquid. The liquid was further dried under reduced pressure and allowed to stand to obtain M35 as a white solid, 530 mg, with a yield of about 84%. ¹H NMR (400 MHz, DMSO-d6): δ 0.86 (dd, 6H), 1.38 (m, 2H), 1.64 (m, 2H), 1.94 (m, 1H), 2.95 (m, 2H), 3.05 (d, 1H), 4.43 (s, 2H), 4.47 (s, 1H), 5.13 (br, 1H), 5.44 (d, 2H), 6.01 (br, 1H), 7.23 (d, 2H), 7.54 (d, 2H), 8.17 (br, 1H), 10.07 (s, 1H). MS (EI) m/z: 380.32 [M+H]⁺; MS (EI) m/z: 402.31 [M+Na]⁺.

### Step 13-2: Preparation of M36

2,5,8,11-tetraoxatetradecane-14 acid (5.0 g, 21.16 mM) was dissolved in dichloromethane (50 mL), and NHS (3.65 mg, 31.74 mM) and EDCI (4.78 g, 25.4 mM) were added sequentially. The reaction was stirred overnight at room temperature. After the reaction was complete, the solvent was removed by concentration under reduced pressure. The obtained colorless oil residue was further purified by column chromatography to obtain M36 as a colorless viscous oil, 8.0 g, yield >100%. The oil contained a small amount of solvent that could not be completely evaporated. The obtained oil was used directly for the next reaction as soon as possible without further purification. MS (EI) m/z: 334.42 [M+H]⁺.

### Step 13-3: Preparation of M37

Fmoc-²N-Boc-⁶N-*L*-Lys (10.0 g, 21.3 mM) was dispersed in DCM (100 mL), which was not completely dissolved. After addition of TFA (10 mL), the reaction was completely dissolved and showed pale yellow. After the reaction was stirred at room temperature for 3 hours, the solvent was removed by concentration under reduced pressure. Ethyl acetate was added to redissolve the residue, and then the solution was evaporated to dryness again. This process was repeated three times to remove residual TFA, which was then thoroughly dried under vacuum using a diaphragm pump to obtain 17 g of a viscous oil. The yield was >100%, but still contained a small amount of residual solvent. The obtained colorless oil, M37, was used directly in the next step. MS (EI) m/z: 369.21 [M+H]⁺.

### Step 13-4: Preparation of M38

Compound M37 (crude, ~15 mM) and M36 (crude, ~15 mM) were dispersed in DCM (100 mL), and DIPEA (1.94 g, 15 mM) was added. The reaction was stirred overnight at room temperature. The mixture was concentrated under reduced pressure and then completely dried under vacuum using a diaphragm pump to obtain an oil crude product. The crude was further purified by silica gel column chromatography to obtain product M38 as a colorless viscous oil, 13 g, yield >100%, which still containing a small amount of unremoved solvent. The obtained colorless oil M38 was used directly in the next reaction. MS (EI) m/z: 587.32 [M+H]⁺.

### Step 13-5: Preparation of M39

Compound M38 (~2.0 g, 3.4 mM) and M35 (1.0 g, 2.6 mM) were dissolved in DMF (20 mL). After complete dissolution at room temperature with stirring, HATU (1.50 g, 3.93 mM) and DIPEA (0.508 g, 3.93 mM) were added sequentially. The reaction was stirred overnight at room temperature, and the solvent was removed by concentration under reduced pressure to obtain a glassy residue. Ethyl acetate was added to the residue, ultrasonically dispersed, and slurried. The paste was filtered, and the filter cake was washed with ethyl acetate (repeated 3 times). After the filter cake was thoroughly dried, M39 was obtained as a pale yellow solid powder, 2.5 g, with a yield of about 95%. MS (EI) m/z: 948.56 [M+H]⁺; MS (EI) m/z: 983.17 [M+Cl]⁻.

### Step 13-6: Preparation of M40

Following the synthetic method of compound M3, M39 was used in place of M2 to react with p-nitrophenol carbonate, to obtain intermediate product M40 as a white solid powder with a yield of about 74%. MS (EI) m/z: 1113.69 [M+H]⁺; MS (ESI) m/z: 1135.49 [M+Na]⁺.

### Step 13-7: Preparation of M41

Following the synthetic method of compound M13, M40 was used in place of M3 to react with Dx-8951f methanesulfonate, to obtain intermediate product M41 as a pale yellow solid powder, with a yield of about 57%. MS (EI) m/z: 705.27 [M+2H]²⁺; MS (ESI) m/z: 1409.6475 [M+H]⁺.

### Step 13-8: Preparation of M42

Following the synthetic method of compound M14, M41 was used in place of M13 to undergo a deprotection reaction in piperidine solution to remove the Fmoc protecting group, to obtain intermediate product M42 as an earth-gray solid powder with a yield of about 68%. MS (EI) m/z: 594.37 [M+2H]²⁺; MS (ESI) m/z: 1187.5790 [M+H]⁺.

### Step 13-9: Preparation of Compound-13

Following the synthetic method of compound-2, M42 was used in place of M14 to undergo a condensation reaction with M12 in the presence of HATU, to obtain compound-13 as a white solid powder with a yield of about 59%. MS (EI) m/z: 770.86 [M+2H]²⁺; MS (ESI) m/z: 1540.6295 [M+H]⁺.

### Example 14: Preparation of Compound-14

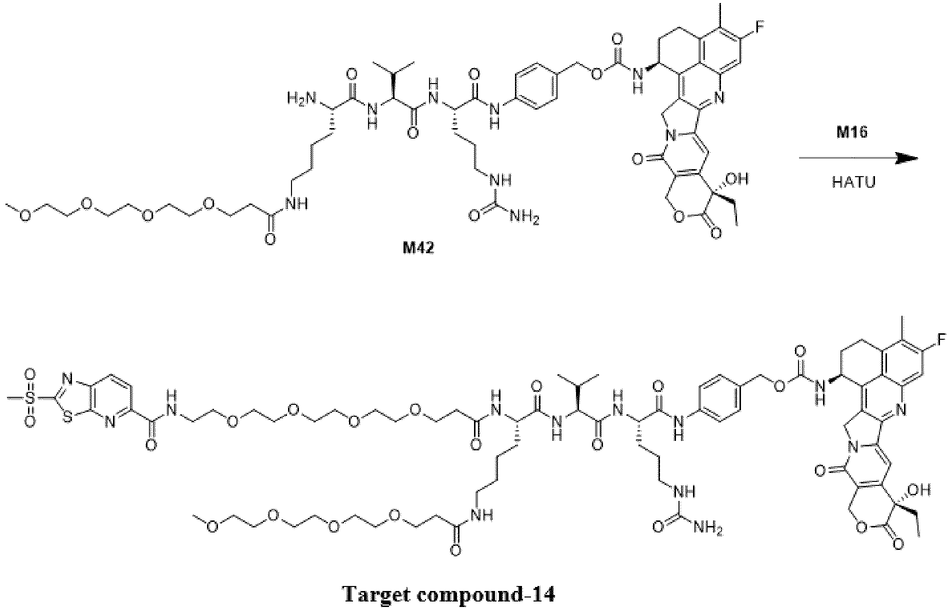

The specific synthesis steps are as follows.

### Step 14-1: Preparation of Compound-14

Following the synthetic method of compound-13, M16 was used in place of M12 to undergo a condensation reaction with M42 in the presence of HATU, to obtain compound-14 as a white solid powder with a yield of about 43%. MS (EI) m/z: 838.13 [M+2H]²⁺; MS (ESI) m/z: 1674.6877 [M+H]⁺.

### Example 15: Preparation of Compound-15

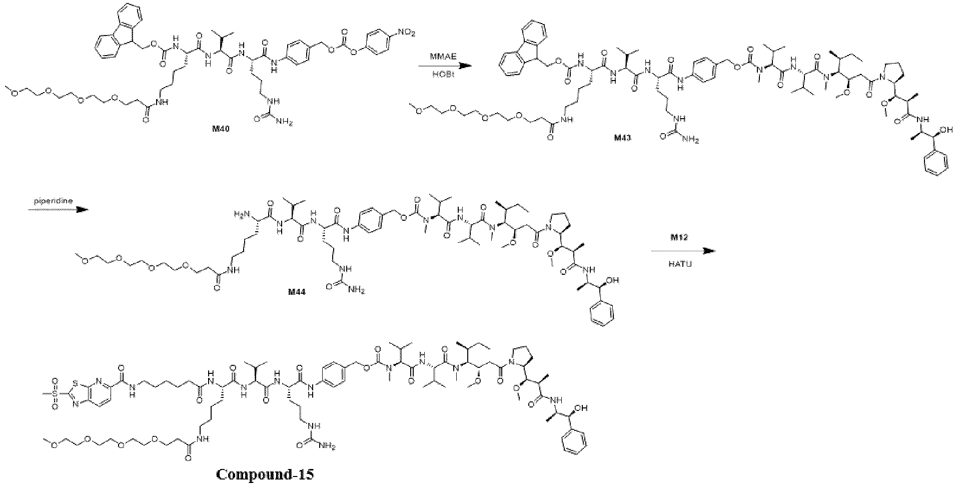

The specific synthesis steps are as follows.

### Step 15-1: Preparation of M43

Following the synthetic method of compound M4, M40 was used in place of M3 to react with MMAE, to obtain M43 as a white solid powder with a yield of about 76%. MS (EI) m/z: 846.57 [M+2H]²⁺; MS (ESI) m/z: 1691.9921 [M+H]⁺.

### Step 15-2: Preparation of M44

Following the synthetic method of compound M5, M43 was used in place of M4 to undergo a deprotection reaction in piperidine solution to remove the Fmoc protecting group, to obtain M44 as a glassy solid powder with a yield of about 61%. MS (EI) m/z: 735.54 [M+2H]²⁺; MS (ESI) m/z: 1469.9233 [M+H]⁺.

### Step 15-3: Preparation of Compound-15

Following the synthetic method of compound-1, M44 was used in place of M5 to undergo a condensation reaction with M12 in the presence of HATU, to obtain compound-15 as a white solid powder with a yield of about 48%. MS (EI) m/z: 911.95 [M+2H]²⁺; MS (ESI) m/z: 1822.9741 [M+H]⁺.

### Example 16: Preparation of Compound-16

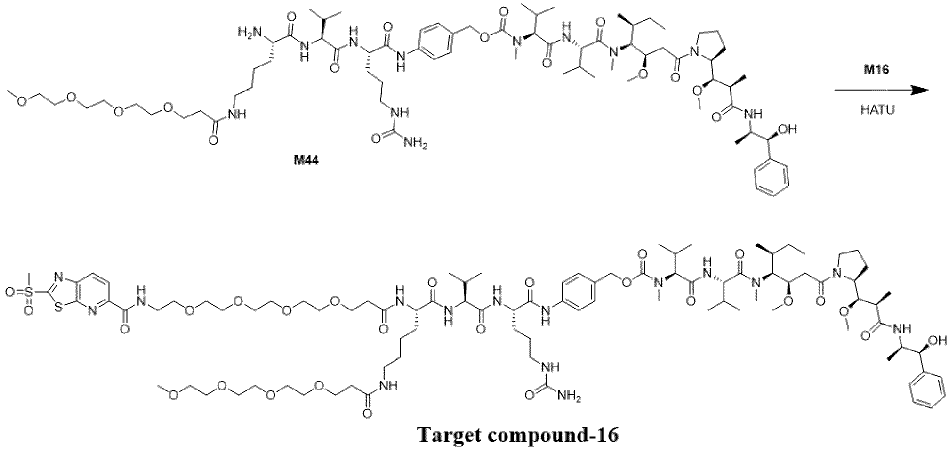

The specific synthesis steps are as follows.

### Step 16-1: Preparation of Compound-16

Following the synthetic method of compound-15, M16 was used in place of M12 to undergo a condensation reaction with M44 in the presence of HATU, to obtain compound-16 as a white solid powder with a yield of about 59%. MS (EI) m/z: 979.23 [M+2H]²⁺; MS (ESI) m/z: 1957.0324 [M+H]⁺.

### Example 17: Preparation of Compound-17

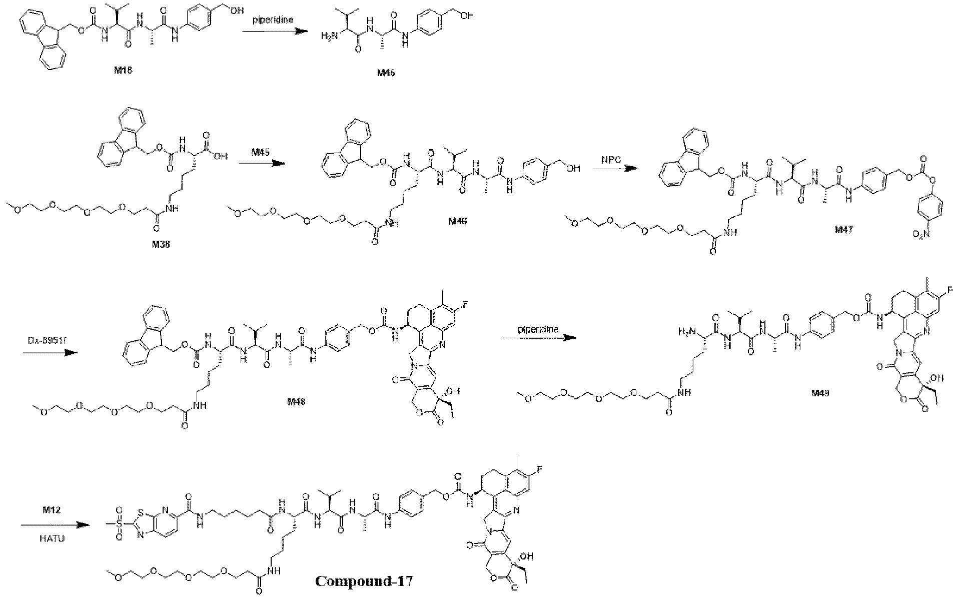

The specific synthesis steps are as follows.

### Step 17-1: Preparation of M45

Following the synthesis method of M35, M18 was used in place of M2 to undergo a deprotection reaction in piperidine solution to remove the Fmoc protecting group, to obtain M45 as a colorless viscous oil with a yield of about 75%. M45 is unstable at room temperature and should be used in the next reaction as soon as possible. ¹H NMR (400 MHz, DMSO-d6): δ 0.85 (dd, 6H), 1.29 (d, 3H), 1.92 (m, 1H), 2.80 (d, 1H), 3.00 (d, 1H), 4.43 (s, 1H), 4.48 (t, 1H), 5.13 (s, 1H), 7.24 (d, 2H), 7.53 (d, 2H), 8.18 (s, 1H), 10.0 (s, 1H). MS (EI) m/z: 294.23 [M+H]⁺; MS (EI) m/z: 316.22 [M+Na]⁺.

### Step 17-2: Preparation of M46

Following the synthesis method of M39, M45 was used in place of M35 to undergo an acylation reaction with M38 in the presence of HATU, to obtain M46 as a pale yellow solid powder with a yield of about 68%. MS (EI) m/z: 862.48 [M+H]⁺; MS (EI) m/z: 884.46 [M+Na]⁺.

### Step 17-3: Preparation of M47

Following the synthesis method of M40, M46 was used in place of M39 to react with p-nitrophenol carbonate, to obtain M47 as a pale yellow solid powder with a yield of about 85%. MS (EI) m/z: 1027.47 [M+H]⁺; MS (EI) m/z: 1049.45 [M+Na]⁺.

### Step 17-4: Preparation of M48

Following the synthesis method of M41, M47 was used in place of M40 to react with Dx-8951f methanesulfonate, to obtain M48 as a grayish-white solid powder with a yield of about 44%. MS (EI) m/z: 662.32 [M+2H]²⁺; MS (ESI) m/z: 1323.5974 [M+H]⁺.

### Step 17-5: Preparation of M49

Following the synthesis method of M42, M48 was used in place of M41 to undergo a deprotection reaction in DMF solution of piperidine to remove the Fmoc protecting group, to obtain M49 as an earth-gray solid powder with a yield of about 56%. MS (EI) m/z: 1101.55 [M+H]⁺; MS (EI) m/z: 1123.54 [M+Na]⁺.

### Step 17-6: Preparation of Compound-17

Following the synthetic method of compound-13, M49 was used in place of M42 to undergo an acylation reaction with M12 in the presence of HATU, to obtain compound-17 as a grayish-white solid powder with a yield of about 49%. MS (EI) m/z: 727.72 [M+2H]²⁺; MS (ESI) m/z: 1454.5831 [M+H]⁺.

### Example 18: Preparation of Compound-18

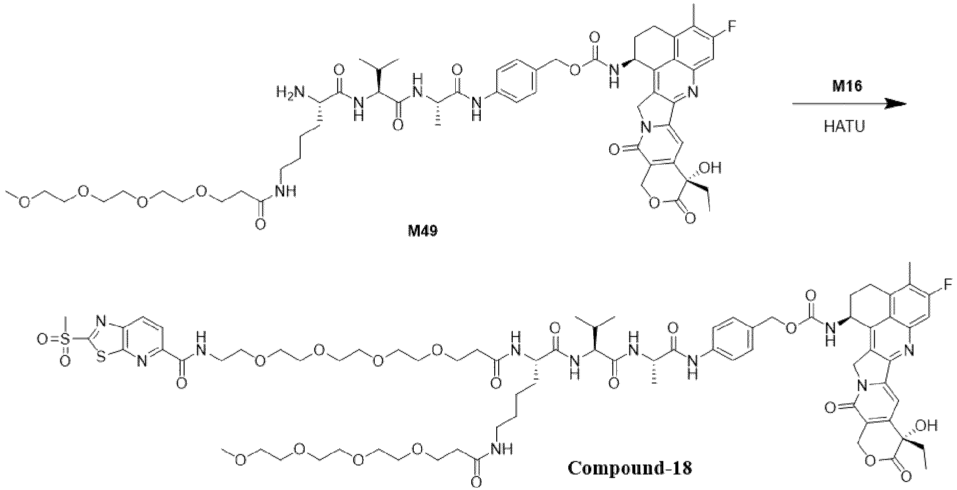

The specific synthesis steps are as follows.

### Step 18-1: Preparation of Compound-18

Following the synthetic method of compound-17, M16 was used in place of M12 to undergo an acylation reaction with M49 in the presence of HATU, to obtain compound-18 as a grayish-white solid powder with a yield of about 50%. MS (EI) m/z: 794.76 [M+2H]²⁺; MS (ESI) m/z: 1588.6387 [M+H]⁺.

### Example 19: Preparation of Compound-19

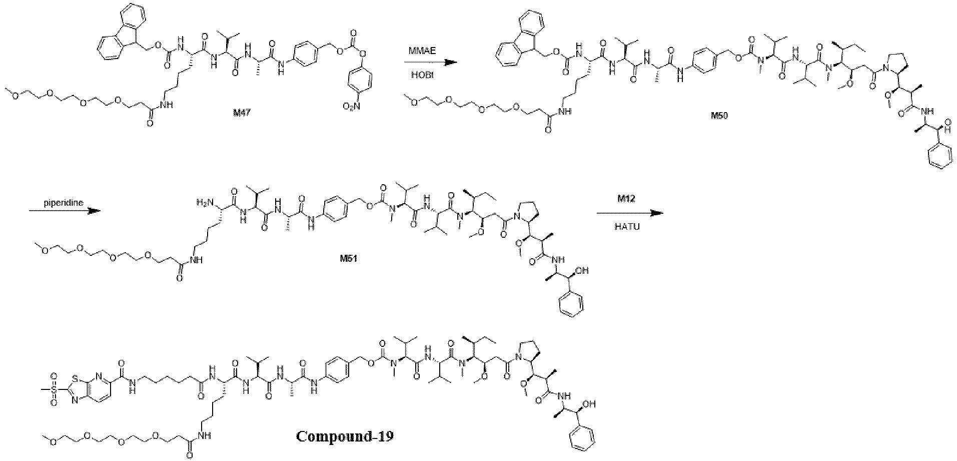

The specific synthesis steps are as follows.

### Step 19-1: Preparation of M50

Following the synthesis method of M48, MMAE was used in place of Dx-8951f methanesulfonate to undergo an acylation reaction with M47, to obtain M50 as a white solid powder with a yield of about 61%. MS (EI) m/z: 803.49 [M+2H]²⁺.

### Step 19-2: Preparation of M51

Following the synthetic method of M5, M50 was used in place of M4 to undergo a deprotection reaction in a solution of piperidine in DMF to remove the Fmoc protecting group, to obtain M51 as a white waxy solid with a yield of about 68%. MS (EI) m/z: 692.43 [M+2H]²⁺; MS (ESI) m/z: 1383.8751 [M+H]⁺.

### Step 19-3: Preparation of Compound-19

Following the synthetic method of compound-1, M51 was used in place of M5 to undergo an acylation reaction with M12 in the presence of HATU, to obtain compound-19 as a white solid powder with a yield of about 57%. MS (EI) m/z: 868.89 [M+2H]²⁺; MS (ESI) m/z: 1736.9256 [M+H]⁺.

### Example 20: Preparation of Compound-20

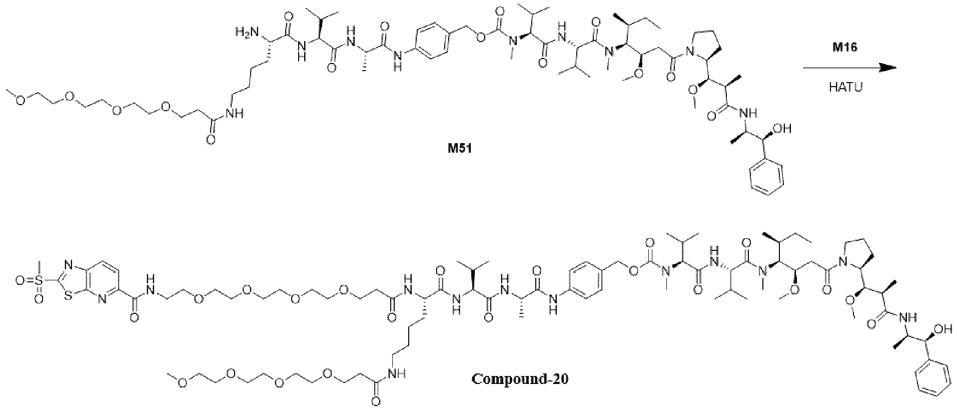

The specific synthesis steps are as follows.

### Step 20-1: Preparation of Compound-20

Following the synthetic method of compound-19, M16 was used in place of M12 to undergo an acylation reaction with M51 in the presence of HATU, to obtain compound-20 as a white solid powder with a yield of about 60%. MS (EI) m/z: 936.02 [M+2H]²⁺; MS (ESI) m/z: 1870.9833 [M+H]⁺.

### Example 21: Preparation of Compound-21

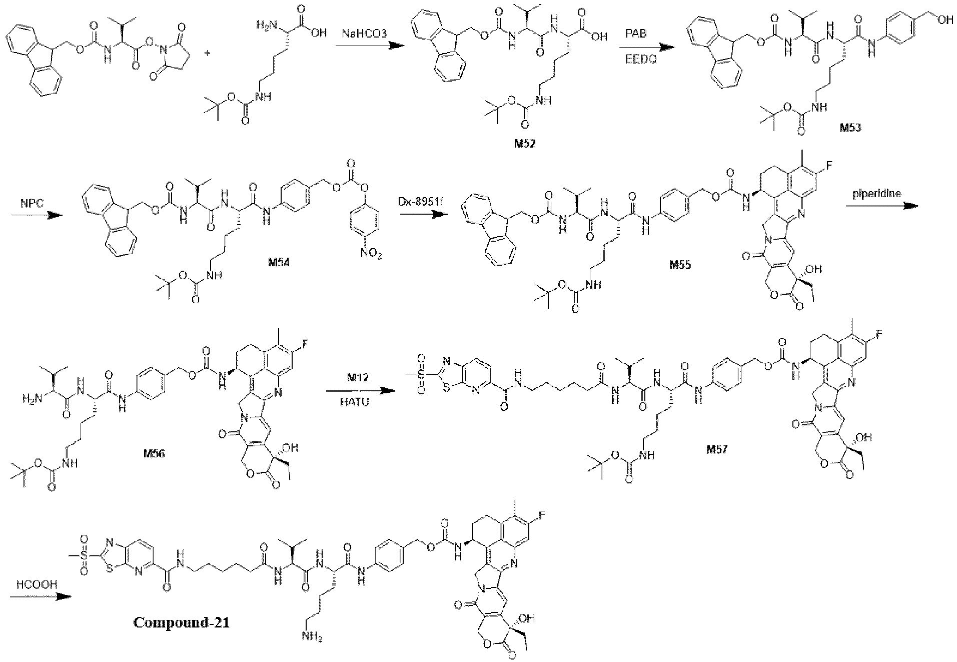

The specific synthesis steps are as follows.

### Step 21-1: Preparation of M52

Following the synthetic method of M1, Boc-⁶N-L-Lys was used in place of citrulline to react with Fmoc-Val-NHS, to obtain M52 as a white solid powder with a yield of about 96%. MS (EI) m/z: 568.35 [M+H]⁺; MS (EI) m/z: 590.28 [M+Na]⁺.

### Step 21-2: Preparation of M53

Following the synthesis method of M2, M52 was used in place of M1 to undergo a condensation reaction with PAB in the presence of EEDQ, to obtain M53 as a white solid powder. The yield was about 89%. MS (EI) m/z: 673.33 [M+H]⁺; MS (EI) m/z: 695.31 [M+Na]⁺.

### Step 21-3: Preparation of M54

Following the synthesis method of M3, M53 was used in place of M2 and react with p-nitrophenol carbonate, to obtain M54 as a white solid powder. The yield was about 91%. MS (EI) m/z: 838.35 [M+H]⁺.

### Step 21-4: Preparation of M55

Following the synthesis method of M13, M54 was used in place of M3 to undergo an acylation reaction with Dx-8951f, to obtain M55 as a gray solid powder. The yield was about 49%. MS (EI) m/z: 1134.52 [M+H]⁺; MS (EI) m/z: 1156.51 [M+Na]⁺.

### Step 21-5: Preparation of M56

Following the synthesis method of M14, M55 was used in place of M13 to undergo a deprotection reaction in a solution of piperidine in DMF to remove the Fmoc protecting group, to obtain M56 as a grayish-white glassy solid. The yield was about 55%. MS (EI) m/z: 912.43 [M+H]⁺; MS (EI) m/z: 934.41 [M+Na]⁺.

### Step 21-6: Preparation of M57

Following the synthetic method of compound-2, M56 was used in place of M14 to undergo an acylation reaction with M12 in the presence of HATU, to obtain M57 as a grayish-white solid powder with a yield of about 46%. MS (EI) m/z: 633.28 [M+2H]²⁺; MS (ESI) m/z: 1265.4821 [M+H]⁺.

### Step 21-7: Preparation of Compound-21

Compound M57 (50 mg, 0.079 mM) was dispersed in DCM (10 mL), and HCOOH (1 mL) was added under stirring to completely dissolve M57. After stirring at room temperature for 4 hours, the solvent was removed by concentration under reduced pressure, to obtain a light yellow semi-solid oil residue. After redissolving, the residue was mixed with crude silica gel and further purified by silica gel column chromatography to obtain compound-21 as a grayish-white glassy solid, 47 mg, with a yield of about 51%. MS (EI) m/z: 583.23 [M+2H]²⁺; MS (ESI) m/z: 1165.4287 [M+H]⁺.

### Example 22: Preparation of compound-22

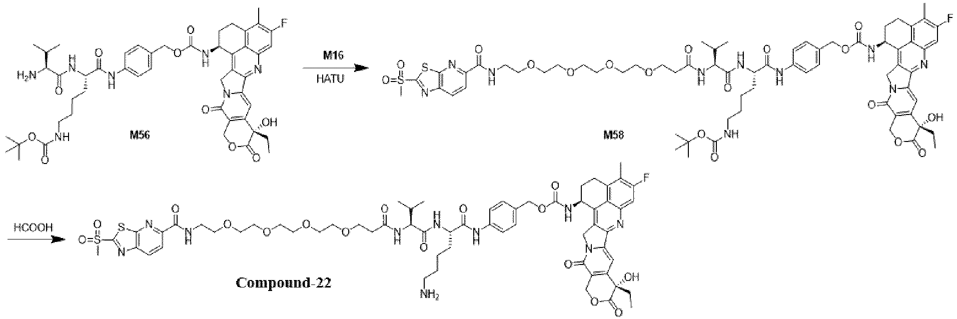

The specific synthesis steps are as follows.

### Step 22-1: Preparation of M58

Following the synthesis method of M57, M16 was used in place of M12 to undergo an acylation reaction with M56 in the presence of HATU, to obtain M58 as a grayish-white glassy solid with a yield of about 49%. MS (EI) m/z: 700.32 [M+2H]²⁺; MS (ESI) m/z: 1399.5392 [M+H]⁺.

### Step 22-2: Preparation of compound-22

Following the synthetic method of compound-21, M58 was used in place of M57 to undergo a deprotection reaction in formic acid solution to remove the Boc protecting group, to obtain compound-22 as a gray glassy solid with a yield of about 52%. MS (EI) m/z: 650.29 [M+2H]²⁺; MS (ESI) m/z: 1299.4873 [M+H]⁺.

### Example 23: Preparation of compound-23

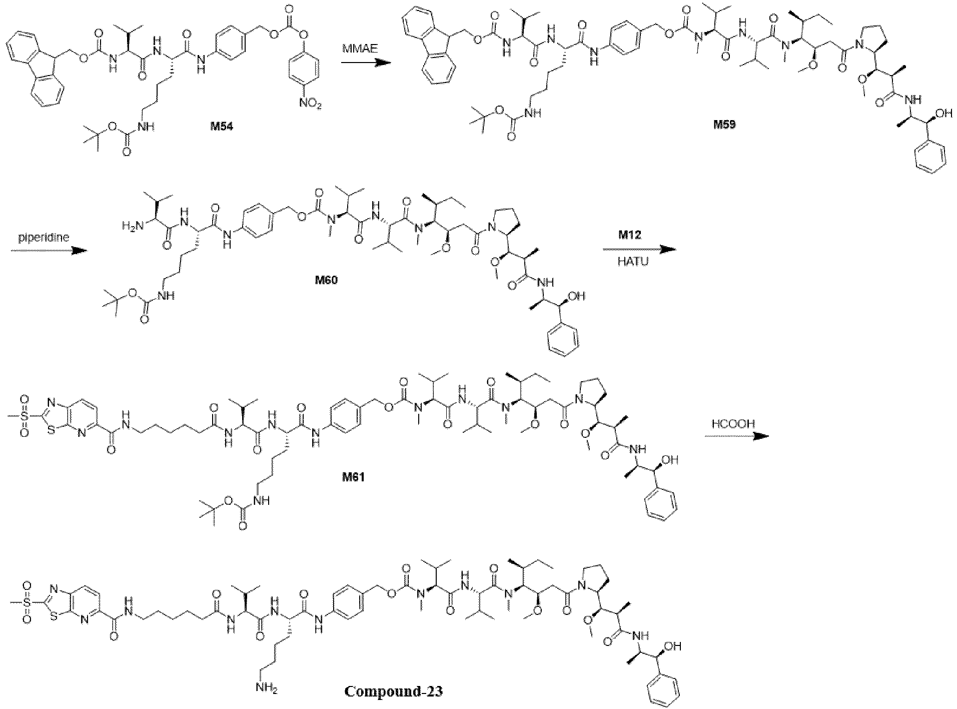

The specific synthesis steps are as follows.

### Step 23-1: Preparation of M59

Following the synthesis method of M4, M54 was used in place of M3 to undergo an acylation reaction with MMAE, to obtain M59 as a white solid powder with a yield of about 87%. MS (EI) m/z: 708.95 [M+2H]²⁺; MS (ESI) m/z: 1416.843 [M+H]⁺.

### Step 23-2: Preparation of M60

Following the synthesis method of M5, M59 was used in place of M4 to undergo a deprotection reaction in a solution of piperidine in DMF to remove the Fmoc protecting group, to obtain M60 as a white solid powder with a yield of about 68%. The product is prone to hygroscopicity and was used directly in the next step after preparation. MS (EI) m/z: 597.84 [M+2H]²⁺; MS (ESI) m/z: 1194.7512 [M+H]⁺.

### Step 23-3: Preparation of M61

Following the synthetic method of compound-1, M60 was used in place of M5 to undergo an acylation reaction with M12 under the catalysis of HATU, to obtain M61 as a white solid powder with a yield of about 49%. MS (EI) m/z: 774.42 [M+2H]²⁺; MS (ESI) m/z: 1547.8262 [M+H]⁺.

### Step 23-4: Preparation of compound-23

Following the synthetic method of compound-21, M61 was used in place of M57 to undergo a deprotection reaction in formic acid solution to remove the Boc protecting group, to obtain compound-23 as a white glassy solid with a yield of about 43%. MS (EI) m/z: 724.43 [M+2H]²⁺; MS (ESI) m/z: 1447.7741 [M+H]⁺.

### Example 24: Preparation of compound-24

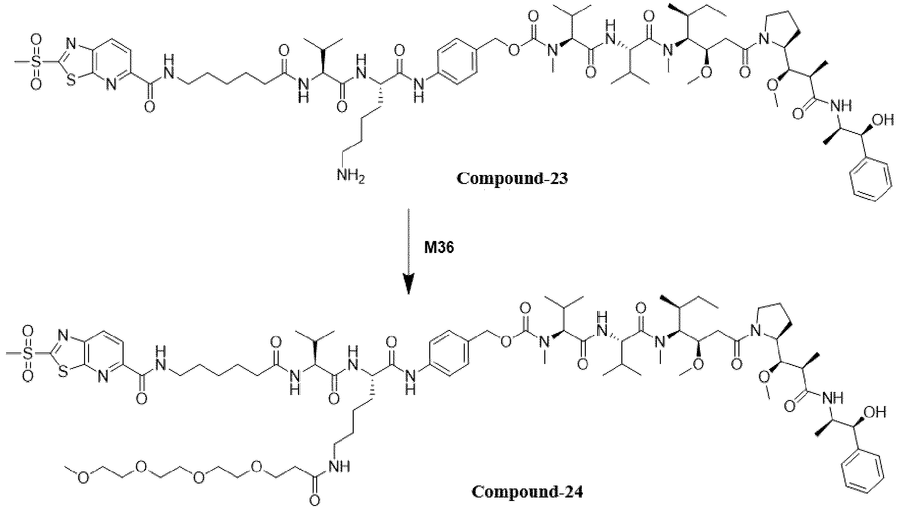

The specific synthesis steps are as follows.

### Step 24-1: Preparation of Compound-24

Compound-23 (45 mg, ~30 µM) was dissolved in DMF (10 mL). After complete dissolution, a solution of M36 (20 mg, 60 µM) in DMF (5 mL) was added, and the mixture was stirred until homogeneous. Then, a catalytic amount of DIPEA (10 µL) was added, and the reaction was stirred overnight at room temperature. After the reaction was complete, the solvent was removed by concentration under reduced pressure. The obtained oil residue was further purified by silica gel column chromatography to obtain compound-24 as a waxy white solid, 31 mg, with a yield of about 62%. MS (EI) m/z: 833.41 [M+2H]²⁺; MS (ESI) m/z: 1665.8876 [M+H]⁺.

### Example 25: Preparation of Compound-25

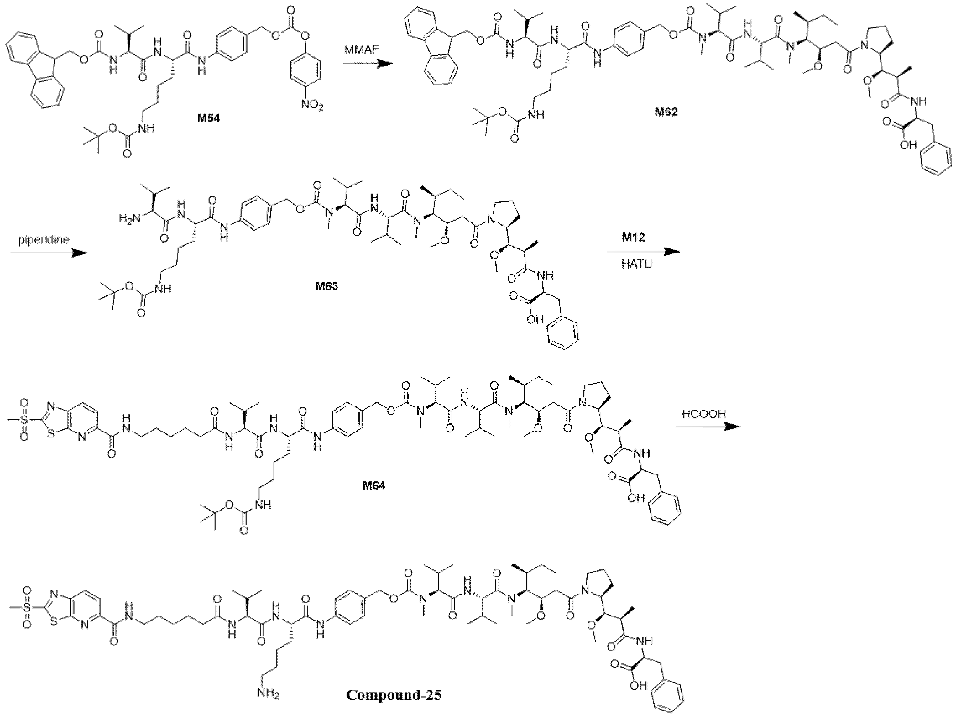

The specific synthesis steps are as follows.

### Step 25-1: Preparation of M62

Following the synthesis method of M59, MMAF was used in place of MMAE to undergo an acylation reaction with M54, to obtain M62 as a white solid powder with a yield of about 68%. MS (ESI) m/z: 1430.8232 [M+H]⁺.

### Step 25-2: Preparation of M63

Following the synthesis method of M5, M62 was used in place of M4 to undergo a deprotection reaction in a solution of piperidine in DMF to remove the Fmoc protecting group, to obtain M63 as a white solid powder with a yield of about 59%. The product was used directly in the next step after preparation. MS (ESI) m/z: 1208.7549 [M+H]⁺.

### Step 25-3: Preparation of M64

Following the synthesis method of M61, M63 was used in place of M60 to undergo an acylation reaction with M12 under the catalysis of HATU, to obtain M64 as a white solid powder with a yield of about 53%. MS (ESI) m/z: 1561.8053 [M+H]⁺.

### Step 25-4: Preparation of Compound-25

Following the synthetic method of compound-23, M64 was used in place of M61 to undergo a deprotection reaction in formic acid solution to remove the Boc protecting group, to obtain compound-25 as a white glassy solid with a yield of about 37%. MS (EI) m/z: 724.43 [M+2H]²⁺; MS (ESI) m/z: 1461.7533 [M+H]⁺.

### Example 26: Preparation of Compound-26

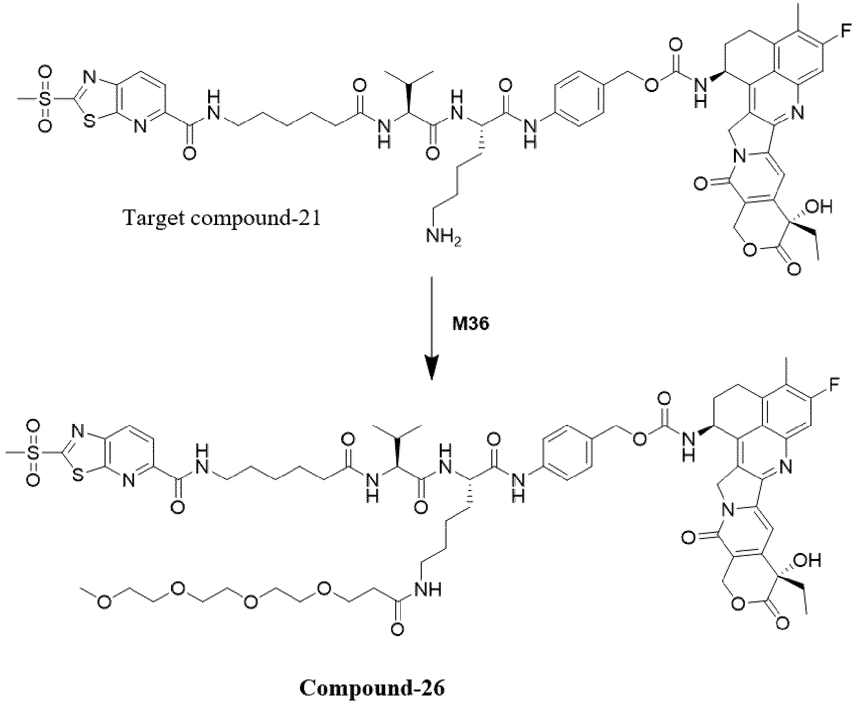

The specific synthesis steps are as follows.

### Step 26-1: Preparation of Compound-26

Following the synthetic method of compound-24, compound-21 was used in place of compound-23 to undergo an acylation reaction with M36, to obtain compound-26 as a white solid powder with a yield of about 47%. MS (ESI) m/z: 1383.5443 [M+H]⁺.

### Example 27: Preparation of Compound-27

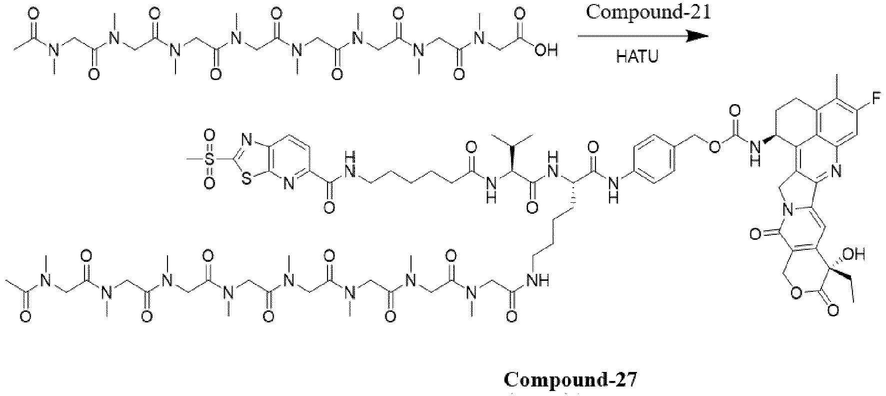

The specific synthesis steps are as follows.

### Step 27-1: Preparation of Compound-27

Following the synthetic method of compound-26, acetyloctapolysarcosine was used in place of M36 to undergo an acylation reaction with compound-21, to obtain compound-27 as an off-white solid powder with a yield of about 61%. MS (ESI) m/z: 1775.7363 [M+H]⁺.

### Example 28: Preparation of Compound-28

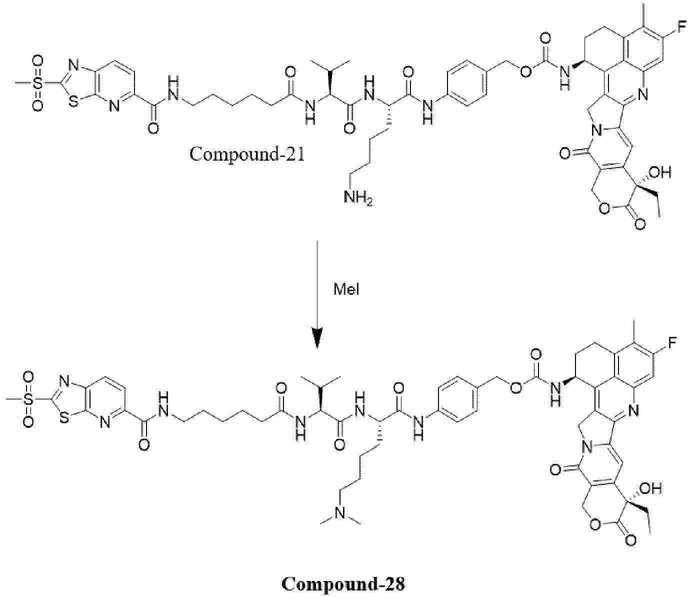

The specific synthesis steps are as follows.

### Step 28-1: Preparation of compound-28

Compound-21 (50 mg, 0.043 mM) was dissolved in anhydrous tetrahydrofuran (50 mL). After complete dissolution by sonication, the mixture was cooled to 0 °C in an ice bath. A catalytic amount of K₂CO₃ was added, and a solution of MeI (12 mg, 0.086 mM) in tetrahydrofuran (10 mL) was slowly added dropwise. The reaction was stirred at low temperature for 1 hour, and then the solvent was removed by concentration under reduced pressure. The obtained residue was purified by C-18 reversed-phase chromatography to obtain compound-28 as a off-white solid powder, 14.5 mg, with a yield of about 28%. MS (ESI) m/z: 1193.4611 [M+H]⁺.

### Example 29: Preparation of compound-29

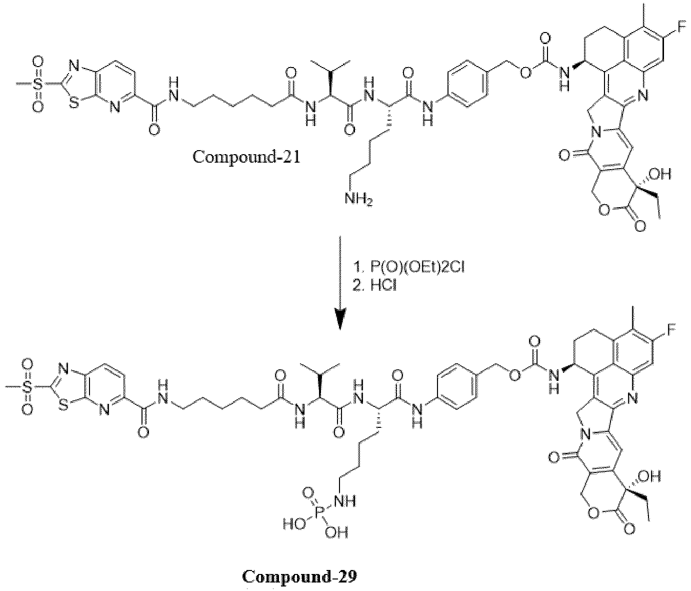

The specific synthesis steps are as follows.

### Step 29-1: Preparation of compound-29

Compound-21 (50 mg, 0.043 mM) was dissolved in anhydrous tetrahydrofuran (50 mL). After complete dissolution by sonication, the mixture was cooled to 0 °C in an ice bath. P(O)(OEt)₂Cl (0.045 mM) was added dropwise, and the reaction was stirred at low temperature for 1 hour, and then transferred to room temperature and stirred for another 2 hours. After the reaction was complete, hydrochloric acid (10 eq) was added, and the reaction continued at room temperature for 3 hours. After the reaction was complete, the solvent was removed by concentration under reduced pressure. The obtained residue was purified by C-18 reversed-phase chromatography to obtain compound-29 as a off-white solid powder, 17 mg, with a yield of about 32%. MS (ESI) m/z: 1245.3962 [M+H]+.

### Example 30: Preparation of Compound-30

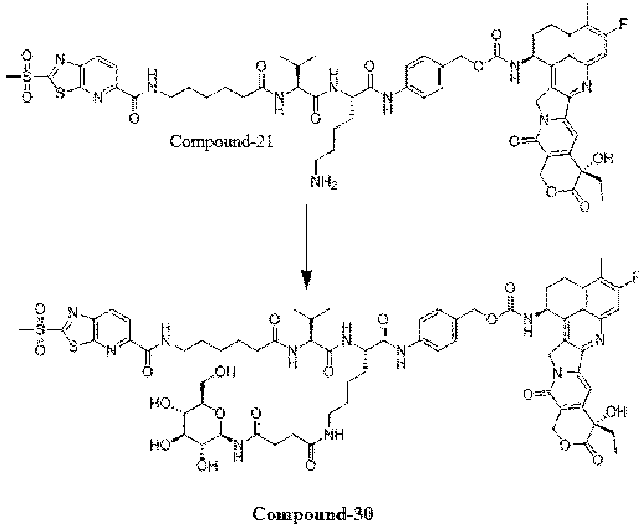

The specific synthesis steps are as follows.

### Step 30-1: Preparation of Compound-30

4-(P-D-glucopyranosylamino)-4-oxobutyric acid (CAS No.: 896730-79-3) was prepared according to the method described in the reference (Carbohydrate Research (2006), 341(8), 947-956) with a yield of about 57%. It was used directly in the next reaction without being left to stand for a long time. Following the synthesis method of compound-26, the prepared 4-(β-D-glucopyranosylamino)-4-oxobutyric acid was used in place of M36 to undergo an acylation reaction with compound-21, to obtain compound-30 as a white solid powder with a yield of about 45%. MS (ESI) m/z: 1426.5133 [M+H]⁺.

### Example 31: Preparation of compound-31

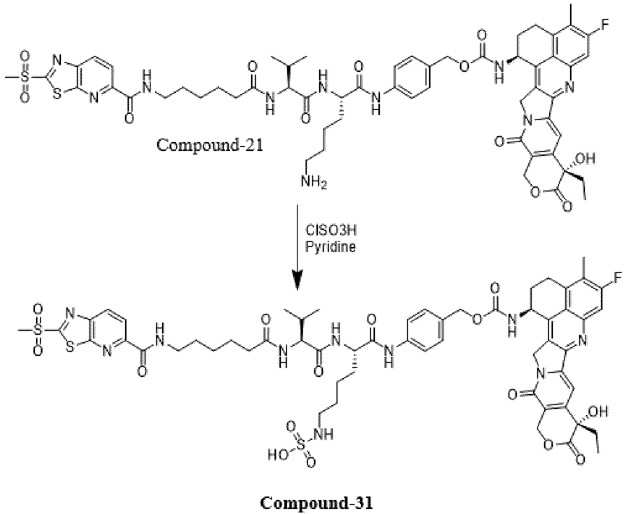

The specific synthesis steps are as follows.

### Step 31-1: Preparation of compound-31

Compound-21 (50 mg, 0.043 mM) was dissolved in anhydrous tetrahydrofuran (50 mL). After complete dissolution by sonication, the pyridine (2 eq) was added, and then the mixture was cooled to 0 °C in an ice bath. ClSO₃H (1 eq) was slowly added dropwise. The reaction was stirred at low temperature for 1 hour, then transferred to room temperature and stirred for another 2 hours. After the reaction was complete, the solvent was removed by concentration under reduced pressure. The obtained residue was purified by C-18 reversed-phase chromatography to obtain compound-31 as a grayish-white solid powder, 15 mg, with a yield of about 28%. MS (ESI) m/z: 1245.3857 [M+H]⁺.

### Example 32: Preparation of Compound-35

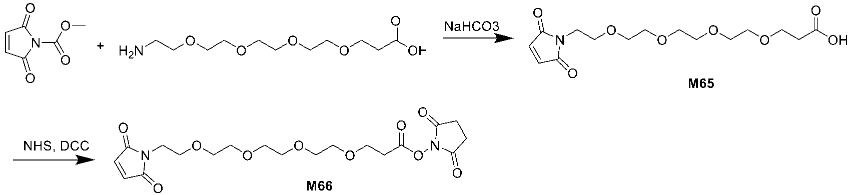

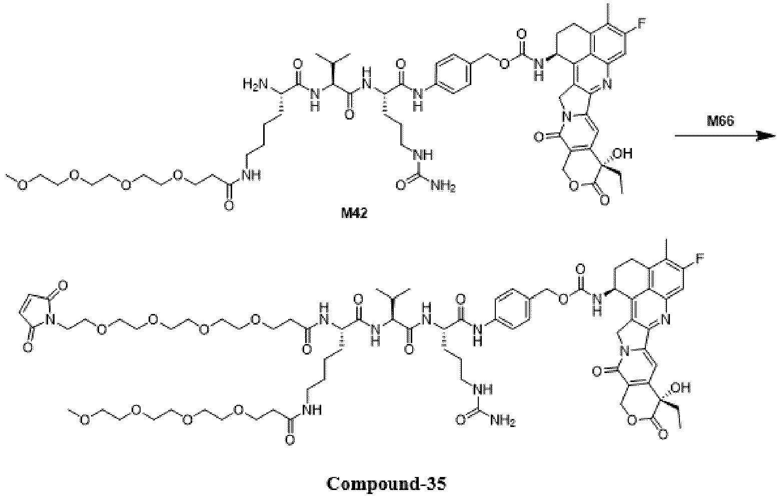

The specific synthesis steps are as follows.

### Step 35-1: Preparation of M65

1-Amino-3,6,9,12-tetraoxapentadecan-15-oic acid (5 g, 18.85 mM) was dissolved in 50 mL of saturated NaHCO₃ and dispersed evenly. The reaction system was then cooled in an ice bath. Methyl 2,5-dioxo-2,5-dihydro-1H-pyrrole-1-carboxylate (3.22 g, 20.73 g) was added. The reaction was stirred at low temperature for half an hour, and then transferred to room temperature for another 2 hours. The pH of the reaction solution was adjusted to ~2 with HCl, and the reaction solvent was removed by concentration. The residue was redissolved with methanol, and then mixed with crude silica gel, eluted with ethyl acetate to obtain product M65 as a colorless oil, 5.31 g, with a yield of about 75%. MS (EI) m/z: 346.16 [M+H]⁺; MS (EI) m/z: 368.15 [M+Na]⁺.

### Step 35-2: Preparation of M66

M65 (5 g, 14.48 mM) was dissolved in tetrahydrofuran (40 mL), and NHS (2.17 g, 18.82 mM) and DCC (3.88 g, 18.82 mM) were added sequentially. The reaction solution was stirred at low temperature for half an hour, and then transferred to room temperature for another 2 hours. After the reaction was completed, the insoluble DCU was removed by filtration, and the filtrate was concentrated to obtain a light yellow oil residue. The residue was further purified by silica gel column chromatography to obtain M66 as a light yellow viscous oil, 4.70 g, with a yield of about 74%. MS (EI) m/z:443.19 [M+H]⁺; MS (EI) m/z: 465.20 [M+Na]⁺.

### Step 35-3: Preparation of Compound-35

Following the synthetic method of compound-14, M66 was used in place of M16 to undergo a condensation reaction with M42, to obtain compound-35 as an off-white solid powder with a yield of about 50%. MS (EI) m/z: 757.74 [M+2H]²⁺; MS (ESI) m/z: 1514.7115 [M+H]⁺.

### Example 33: Preparation of compound-39

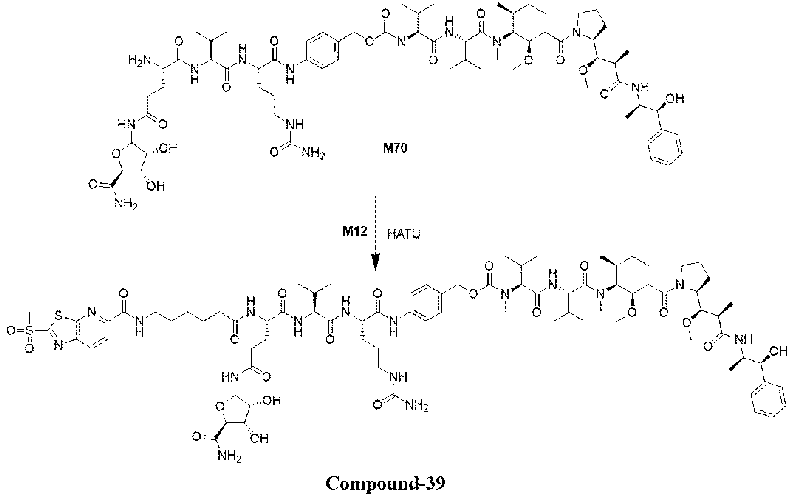

The specific synthesis steps are as follows.

### Step 39-1: Preparation of compound-39

M70 was prepared according to the method described in reference (WO2023125530A1; P44, compound 2i). Then, following the synthesis method of compound-36, M12 was used in place of McOSu to undergo a condensation reaction with M70, to obtain compound-39 as a light yellow solid powder with a yield of about 39%. MS (EI) m/z: 875.38 [M+2H]²⁺; MS (ESI) m/z: 1749.8582 [M+H]⁺.

### Example 34: Preparation of Compound-40

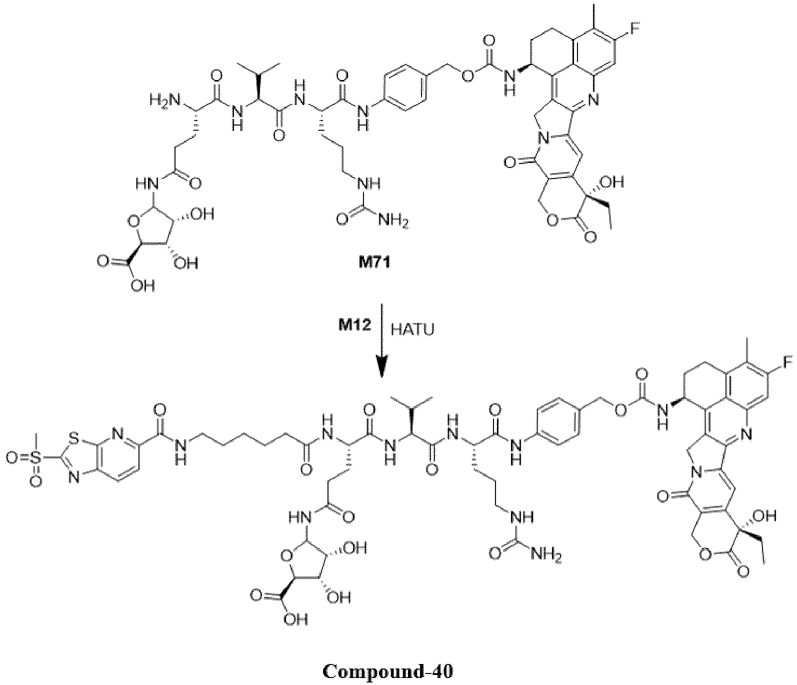

The specific synthesis steps are as follows.

### Step 40-1: Preparation of Compound-40

M71 was prepared according to the method described in reference (WO2023125530A1; P84, compound 27h). Then, following the synthesis method of compound-39, M71 was used in place of M70 to undergo a condensation reaction with M12, to obtain compound-40 as a light yellow solid powder with a yield of about 36%. MS (EI) m/z: 734.77 [M+2H]²⁺; MS (ESI) m/z: 1468.4982 [M+H]⁺.

### Example 35: Structure of compound-41 and preparation method thereof

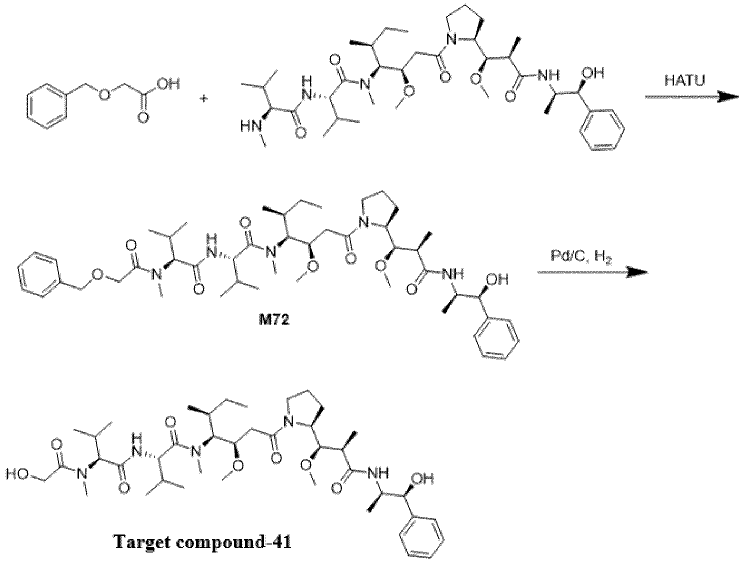

The specific synthesis steps are as follows.

### Step 41-1: Preparation of M72

Benzyloxyacetic acid (46.29 mg, 0.279 mM) was dissolved in DMF (8 mL), followed by HATU (105.92 mg, 0.279 mM) and DIPEA (36 mg, 0.279 mM) was added sequentially. After complete dissolution of the solids, the reaction system was cooled in an ice bath. MMAE (100 mg, 0.139 mM) was added, and the reaction was carried out at low temperature for 1 hour, then slowly warmed to room temperature and reacted for another 4 hours. After the reaction was complete, the solvent was removed by concentration under reduced pressure, to obtain an oil residue. The residue was purified by C-18 reversed-phase column chromatography and further lyophilized to obtain M72 as a white solid powder, 73 mg, with a yield of about 61%. MS (EI) m/z: 866.80 [M+H]⁺.

### Step 41-2: Preparation of compound-41

M72 (70 mg, 0.081 mM) was dissolved in methanol (15 mL). After complete dissolution, 10% palladium on carbon (0.5 eq) was added, and the reaction was stirred overnight at room temperature under a hydrogen atmosphere. After the reaction was complete, the solvent was removed by concentration under reduced pressure. The obtained residue was purified by C-18 reversed-phase column chromatography and further lyophilized to give compound-41 as a white solid powder, 60 mg, with a yield of about 96%. MS (EI) m/z: 776.75 [M+H]⁺; MS (EI) m/z: 798.66 [M+Na]⁺.

### Example 36: Preparation of antibody-drug conjugates

During the preparation of the ADC, the antibody of the ADC selected the humanized IgG antibody of anti-HER-2, Trastuzumab, and the humanized IgG antibody against B7-H3 (CD276), CE4-Z3, to verify the coupling performance, druggability and in vitro and in vivo pharmacodynamic characteristics of the prepared new linker-payload.

### Step 36-1: General preparation method of DAR4 ADC

(1) Displacement of Antibody: Before conjugation, the selected antibody (IgG) was desalted by an AKTA system with a G25 dextran gel column, and the protein was displaced into the coupling system buffer (PBS: 20 mM; EDTA: 2 mM; pH 6.5 to 8.0, or L-HIs: 20 mM; EDTA: 2 mM; pH 6.5 to 8.0). The absorbance of the protein was measured using a UV spectrophotometer, and the concentration of the antibody after buffer displacement was calculated by extinction coefficient method. If the antibody concentration was lower than the conjugation concentration, ultrafiltration concentration is performed to increase the concentration of the protein.
(2) Preparation of the conjugation reaction solution: Based on the required amount of conjugated antibody (1 eq), a pipette was used to accurately transfer the corresponding conjugation system buffer for the antibody, so that the antibody concentration is the initial concentration for conjugation, typically 1-10 mg/mL.
(3) Reduction of antibody: 2.0 to 2.5 eq of TCEP • HCl solution (1.0 mg/mL to 5.73 mg/mL) was added to the reaction vial containing the antibody solution under slow stirring; after the addition was complete, the reaction was stirred slowly at room temperature for 60 to 180 min.
(4) Conjugation of Antibody: The volume of organic solvent (DMAC or DMSO) to be added was calculated, so that it accounts for 5% to 15% of the total volume (usually, ≤10%); the mass of the small molecule loading (linker-payload) to be added was simultaneously calculated, typically in slight excess (usually, 8 eq), and then the concentration of the organic solution of the loading to be added was calculated. After accurately preparing the solution of loading, it was slowly added to the reduced antibody reaction solution dropwise. The mixture was stirred slowly at room temperature for 0.25 to 1.0 h depending on the specific conjugation situation.
(5) Quenching of the reaction: After the reaction solution reaches the predetermined coupling time, an excess of water-soluble small molecule N-acetylcysteine (NAC) solution containing reducing thiol groups (1.0 to 3.26 mg/mL) was added, and the reaction was slow stirred for another 15 to 60 min.
(6) Purification of ADC product: After the coupling reaction was quenched, the reaction solution was first filtered, and then desalted using the AKTA system with a G25 dextran gel column. The first part (about 80%) of the component eluent was collected, concentrated again by ultrafiltration, aseptically filtered, and then the sample was divided into aliquots. The other ADC products were stored at -80 °C for future use, except that the reserved portion of the ADC samples for analysis were stored at 4 °C for a short period of time.

### Step 36-2: General preparation method of DAR8 ADC

Following the procedure in step 36-1, the amount of TCEP•HCl in (3) (Reduction of antibody) was adjusted to 4.0 to 8.0 eq, and the amount of linker-payload in (4) (Conjugation of Antibody) and the amount of NAC in step (5) (Quenching of the reaction) were adjusted to 8 to 12 eq. An ADC product with DAR ≈ 8.0 was obtained.

### Step 36-3: General method for DAR value analysis of ADC

### (1) DAR value analysis of ADCs with MMAE as payload

ADCs with different numbers of drugs for each antibody were separated at room temperature using a butyl HIC column (TSK gel butyl NPR 4.6×35mm 2.5µm, Tosoh Bioscience). HIC was also performed on an Agilent 1260 Infinity II HPLC system or a Shimadzu HPLC system, with UV detection at 280 nm. Mobile phase A is 1.5 mmol/L (NH₄)₂SO₄, 50 mmol/L K₂HPO₄, pH 7.0, and mobile phase B is 21.3 mmol/L KH₂PO₄, 28.6 mmol/L K₂HPO₃, 25% (v/v) isopropanol, pH 7.0. The gradient program is as follows: B%: 0% to 25% (0 - 1 min, 0.8 mL/min), 25% (1 - 3 min, 0.6 mL/min). The corresponding chromatographic peak areas of DAR= 0, DAR= 2, DAR= 4, DAR= 6, DAR= 8 are integrated and weighted, and the DAR value of the measured ADC is calculated.

### (2) DAR value analysis of ADCs with Dx-8951f or Dxd as payload

The absorbance of the ADC to be tested at the wavelengths of 280 nm and 360 nm was measured by using an ultraviolet spectrophotometer, respectively, and the measurement was performed in parallel three times. The average absorbance at each wavelength was then calculated. Since the antibody does not exhibit UV absorption at 360 nm of the characteristic absorption wavelength of the linker-payload, the relationship between the mass concentration of the linker-payload and A360 (a linear equation in one variable), and the multiple relationship between A360 and A280, were calculated using the standard curve method. The mass concentration of the linker-payload and the A280 value of the linker-payload in the ADC were calculated by the calculated A360. Finally, according to Beer-Lambert Law, the total absorbance at any given wavelength is equal to the sum of the absorbances at that wavelength of all light-absorbing chemical substances present in the system. The absorbance value of the antibody at A280, A_{mAb}, was calculated by using A_{mAb} = A_{average} - A_{linker-payload}. Finally, the DAR value of the ADC was calculated by determining the molar ratio of the linker-payload to the antibody in the solution.

### (3) ADC-DAR value analysis by LC-MS method

To 100 µg of ADC or naked antibody sample was added 1 µl of PNGaseF 500U, and digest overnight at 37°C to obtain the test sample. Liquid chromatography-mass spectrometry parameters set as follows: the liquid chromatography system used was an ACQUITY UPLC H-Class (Waters), the column was ACQUITY UPLC Protein, BEH SEC, 200Å, 1.7 µm, 2.1 mm × 100 mm, the column temperature was 25°C, the mobile phase was 100 mM ammonium acetate aqueous solution, isocratic elution at a flow rate of 0.1 ml/min. The mass spectrometry detection system was a Xevo G2-XS Qtof (Waters), the detection mode was positive ion, using full scan mode. Data acquisition was performed using MassLynx 4.1 (Waters) software, and deconvolution was performed using MaxEnt I. The model parameters were set with a resolution of 2-3.5 Da. The minimum intensity ratio was set to 60%, and the output resolution was set to 1 Da. The algorithm iteration parameter was set to 20. The DAR value of the tested ADC was calculated by weighting the TIC peak heights corresponding to DAR=0, DAR=2, DAR=4, DAR=6, and DAR=8 of the ADC after deconvolution processing.

### Step 36-4: General method for polymerization degree analysis of ADC

The aggregation of ADC products was evaluated by size exclusion chromatography, which was performed on an Agilent 1260 Infinity II HPLC system or a Shimadzu HPLC system with UV detection at 280 nm. The column used was a TSKgel G3000SWXL column (Tosoh Bioscience). Samples were injected at a load of 20 to 50 µg. The mobile phase was 200 mmol/L sodium phosphate and 150 mmol/L sodium chloride (pH 7.0). Additionally, 10% (v/v) isopropanol was added to the mobile phase to minimize secondary hydrophobic interactions with the stationary phase and to prevent bacterial growth. The column temperature was set to room temperature. The peak areas corresponding to the monomer peak, fragment peak, and aggregate peak under the SEC chromatogram of the tested ADC were integrated, and the percentage of monomer content in the tested ADC was calculated using area normalization.

The structure of the prepared ADC is shown in the table below:

| ADC Number | Antibody | ADC structure (DAR ≈ 4) |
|---|---|---|
| anti-HER2: **ADC-1A** and anti-CD276: **ADC-1B** | anti-HER2: Trastuzumab and anti-CD276: CE4-Z3 | 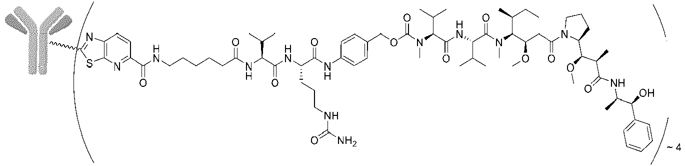 |
| anti-HER2: **ADC-2A** and anti-CD276: **ADC-2B** | anti-HER2: Trastuzumab and anti-CD276: CE4-Z3 | 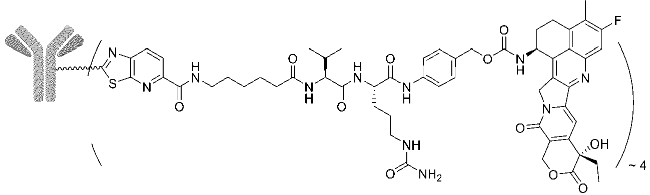 |
| anti-HER2: **ADC-3A** and anti-CD276: **ADC-3B** | anti-HER2: Trastuzumab and anti-CD276: CE4-Z3 | 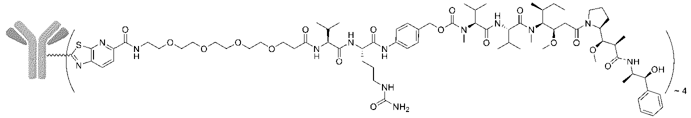 |
| anti-HER2: **ADC-4A** and anti-CD276: **ADC-4B** | anti-HER2: Trastuzumab and anti-CD276: CE4-Z3 | 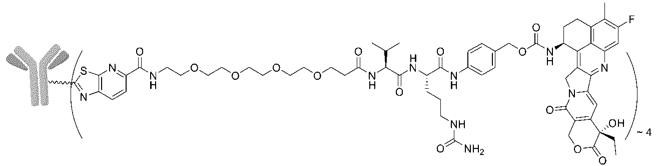 |
| anti-HER2: **ADC-5A** and anti-CD276: **ADC-5B** | anti-HER2: Trastuzumab and anti-CD276: CE4-Z3 | 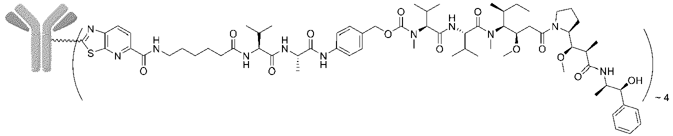 |
| anti-HER2: **ADC-6A** and anti-CD276: **ADC-6B** | anti-HER2: Trastuzumab and anti-CD276: CE4-Z3 | 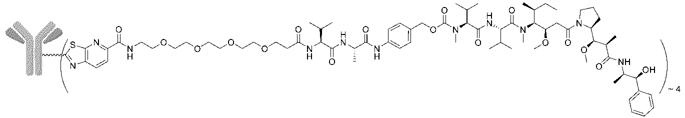 |
| anti-HER2: **ADC-7A** and anti-CD276: **ADC-7B** | anti-HER2: Trastuzumab and anti-CD276: CE4-Z3 | 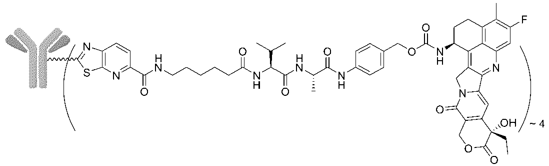 |
| anti-HER2: **ADC-8A** and anti-CD276: **ADC-8B** | anti-HER2: Trastuzumab and anti-CD276: CE4-Z3 | 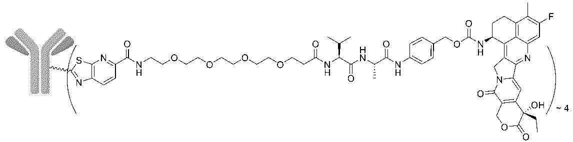 |
| anti-HER2: **ADC-9A** and anti-CD276: **ADC-9B** | anti-HER2: Trastuzumab and anti-CD276: CE4-Z3 | 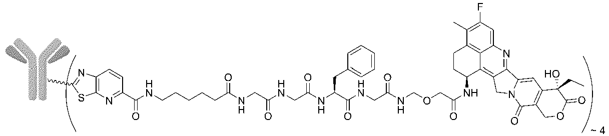 |
| anti-HER2: **ADC-10A** and anti-CD276: **ADC-10B** | anti-HER2: Trastuzumab and anti-CD276: CE4-Z3 | 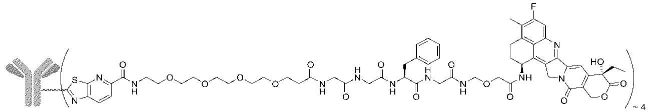 |
| anti-HER2: **ADC-11A** and anti-CD276: **ADC-11B** | anti-HER2: Trastuzumab and anti-CD276: CE4-Z3 | 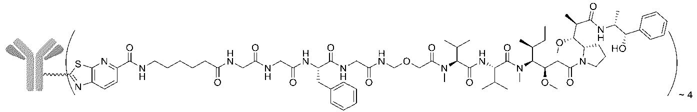 |
| anti-HER2: **ADC-12A** and anti-CD276: **ADC-12B** | anti-HER2: Trastuzumab and anti-CD276: CE4-Z3 | 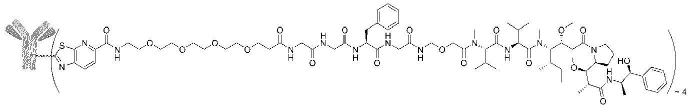 |
| anti-HER2: **ADC-13A** and anti-CD276: **ADC-13B** | anti-HER2: Trastuzumab and anti-CD276: CE4-Z3 | 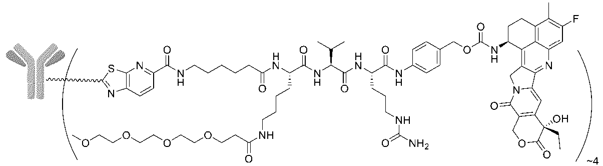 |
| anti-HER2: **ADC-14A** and anti-CD276: **ADC-14B** | anti-HER2: Trastuzumab and anti-CD276: CE4-Z3 | 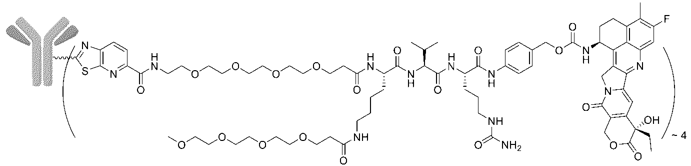 |
| anti-HER2: **ADC-15A** and anti-CD276: **ADC-15B** | anti-HER2: Trastuzumab and anti-CD276: CE4-Z3 | 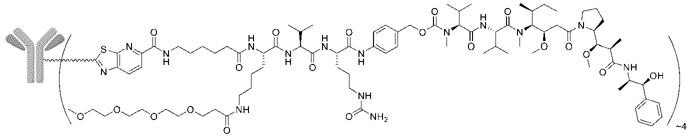 |
| anti-HER2: **ADC-16A** and anti-CD276: **ADC-16B** | anti-HER2: Trastuzumab and anti-CD276: CE4-Z3 | 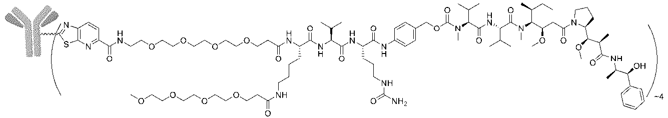 |
| anti-HER2: **ADC-17A** and anti-CD276: **ADC-17B** | anti-HER2: Trastuzumab and anti-CD276: CE4-Z3 | 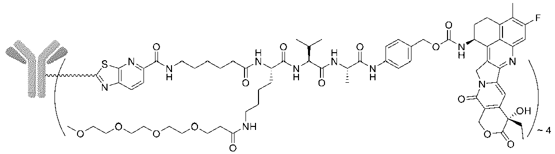 |
| anti-HER2: **ADC-18A** and anti-CD276: **ADC-18B** | anti-HER2: Trastuzumab and anti-CD276: CE4-Z3 | 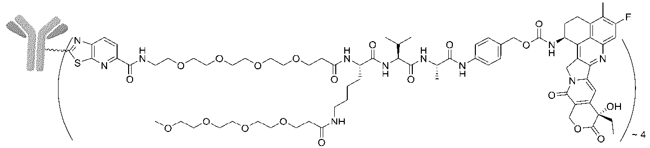 |
| anti-HER2: **ADC-19A** and anti-CD276: **ADC-19B** | anti-HER2: Trastuzumab and anti-CD276: CE4-Z3 | 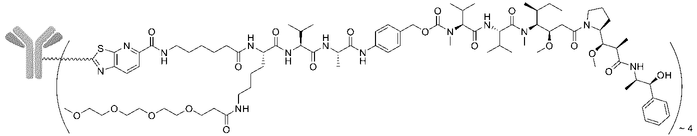 |
| anti-HER2: **ADC-20A** and anti-CD276: **ADC-20B** | anti-HER2: Trastuzumab and anti-CD276: CE4-Z3 | 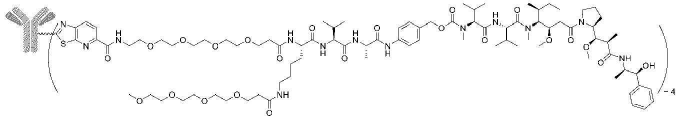 |
| anti-HER2: **ADC-21A** and anti-CD276: **ADC-21B** | anti-HER2: Trastuzumab and anti-CD276: CE4-Z3 | 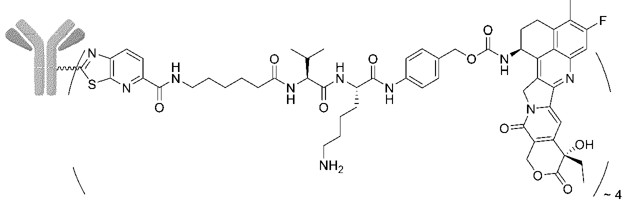 |
| anti-HER2: **ADC-22A** and anti-CD276: **ADC-22B** | anti-HER2: Trastuzumab and anti-CD276: CE4-Z3 | 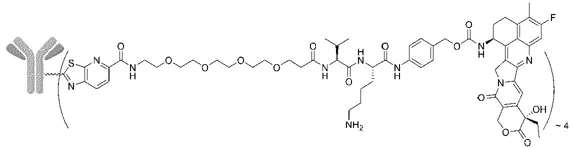 |
| anti-HER2: **ADC-23A** and anti-CD276: **ADC-23B** | anti-HER2: Trastuzumab and anti-CD276: CE4-Z3 | 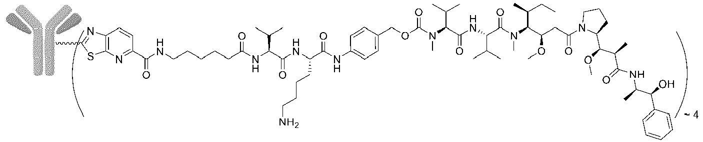 |
| anti-HER2: **ADC-24A** and anti-CD276: **ADC-24B** | anti-HER2: Trastuzumab and anti-CD276: CE4-Z3 | 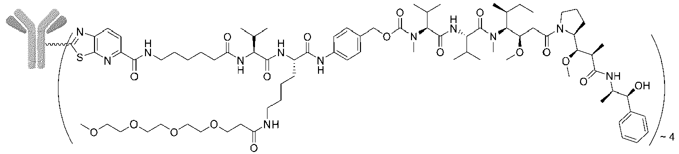 |
| anti-HER2: **ADC-25A** and anti-CD276: **ADC-25B** | anti-HER2: Trastuzumab and anti-CD276: CE4-Z3 | 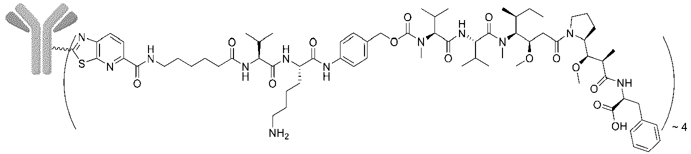 |
| anti-HER2: **ADC-26A** and anti-CD276: **ADC-26B** | anti-HER2: Trastuzumab and anti-CD276: CE4-Z3 | 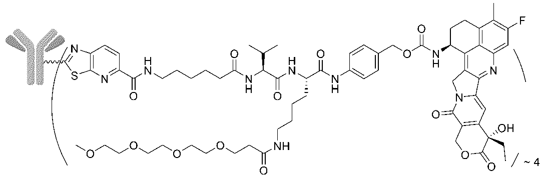 |
| anti-HER2: **ADC-27A** and anti-CD276: **ADC-27B** | anti-HER2: Trastuzumab and anti-CD276: CE4-Z3 | 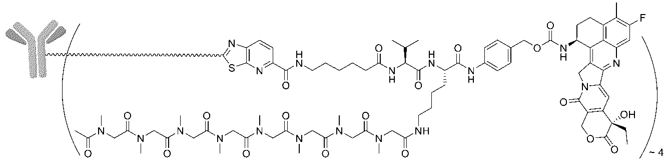 |
| anti-HER2: **ADC-28A** and anti-CD276: **ADC-28B** | anti-HER2: Trastuzumab and anti-CD276: CE4-Z3 | 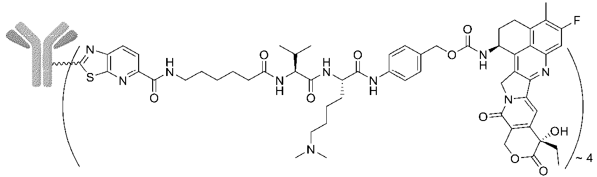 |
| anti-HER2: **ADC-29A** and anti-CD276: **ADC-29B** | anti-HER2: Trastuzumab and anti-CD276: CE4-Z3 | 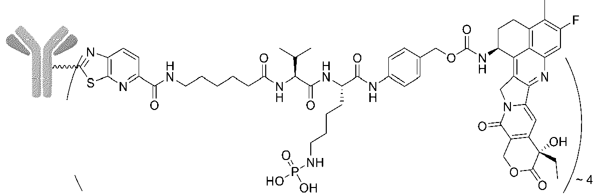 |
| anti-HER2: **ADC-30A** and anti-CD276: **ADC-30B** | anti-HER2: Trastuzumab and anti-CD276: CE4-Z3 | 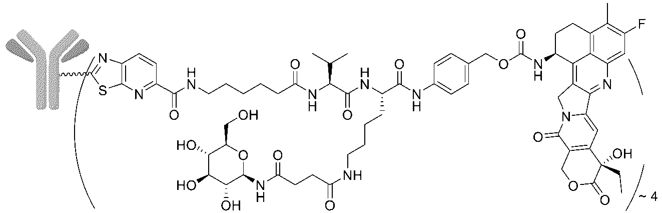 |
| anti-HER2: **ADC-31A** and anti-CD276: **ADC-31B** | anti-HER2: Trastuzumab and anti-CD276: CE4-Z3 | 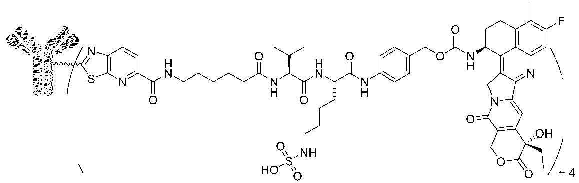 |
| anti-HER2: **ADC-35A** and anti-CD276: **ADC-35B** | anti-HER2: Trastuzumab and anti-CD276: CE4-Z3 | 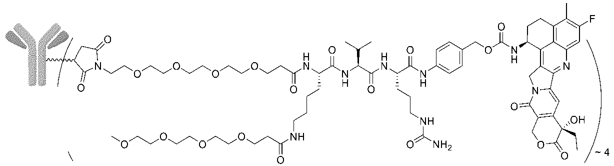 |
| anti-HER2: **ADC-39A** and anti-CD276: **ADC-39B** | anti-HER2: Trastuzumab and anti-CD276: CE4-Z3 | 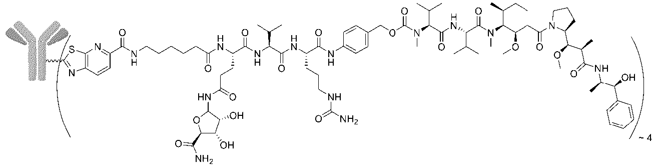 |
| anti-HER2: **ADC-40A** and anti-CD276: **ADC-40B** | anti-HER2: Trastuzumab and anti-CD276: CE4-Z3 | 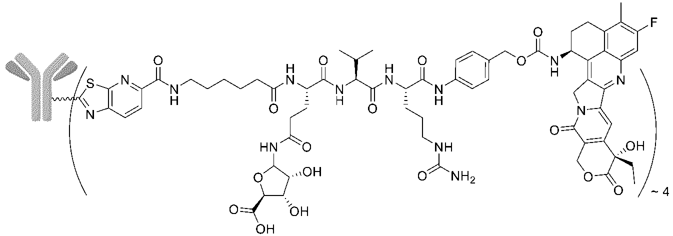 |

wherein, 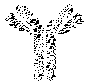 represents trastuzumab or CE4-Z3.

| ADC Number | Antibody | ADC structure (DAR ≈ 8) |
|---|---|---|
| anti-HER2: **ADC-3C** and anti-CD276: **ADC-3D** | anti-HER2: Trastuzumab and anti-CD276: CE4-Z3 | 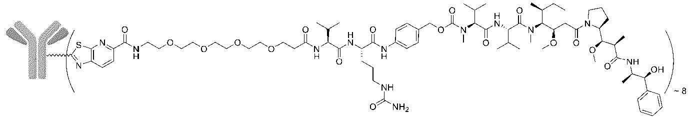 |
| anti-HER2: **ADC-4C** and anti-CD276: **ADC-4D** | anti-HER2: Trastuzumab and anti-CD276: CE4-Z3 | 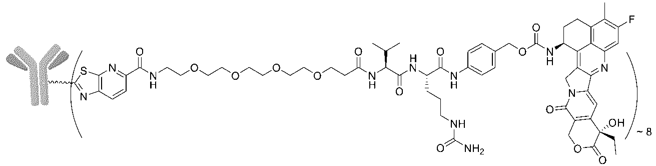 |
| anti-HER2: **ADC-6C** and anti-CD276: **ADC-6D** | anti-HER2: Trastuzumab and anti-CD276: CE4-Z3 | 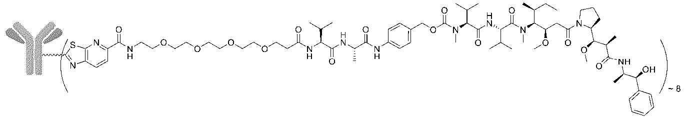 |
| anti-HER2: **ADC-8C** and anti-CD276: **ADC-8D** | anti-HER2: Trastuzumab and anti-CD276: CE4-Z3 | 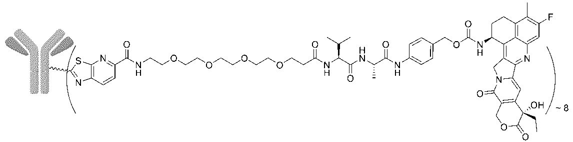 |
| anti-HER2: **ADC-9C** and anti-CD276: **ADC-9D** | anti-HER2: Trastuzumab and anti-CD276: CE4-Z3 | 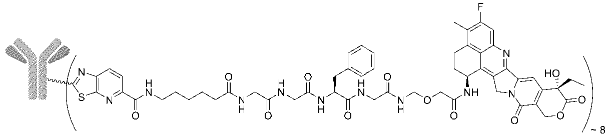 |
| anti-HER2: **ADC-10C** and anti-CD276: **ADC-10D** | anti-HER2: Trastuzumab and anti-CD276: CE4-Z3 | 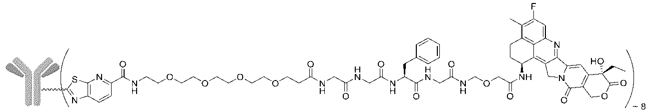 |
| anti-HER2: **ADC-12C** and anti-CD276: **ADC-12D** | anti-HER2: Trastuzumab and anti-CD276: CE4-Z3 | 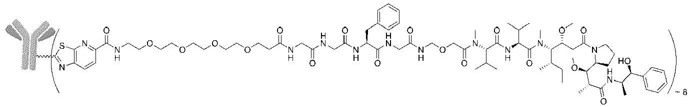 |
| anti-HER2: **ADC-14C** and anti-CD276: **ADC-14D** | anti-HER2: Trastuzumab and anti-CD276: CE4-Z3 | 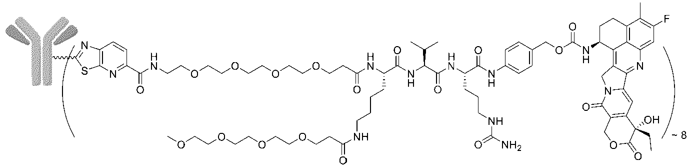 |
| anti-HER2: **ADC-16C** and anti-CD276: **ADC-16D** | anti-HER2: Trastuzumab and anti-CD276: CE4-Z3 | 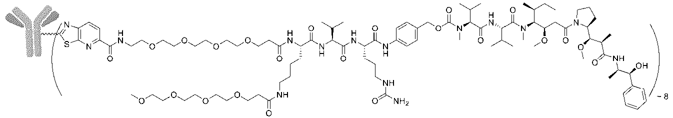 |
| anti-HER2: **ADC-18C** and anti-CD276: **ADC-18D** | anti-HER2: Trastuzumab and anti-CD276: CE4-Z3 | 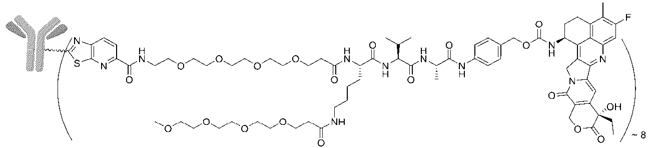 |
| anti-HER2: **ADC-20C** and anti-CD276: **ADC-20D** | anti-HER2: Trastuzumab and anti-CD276: CE4-Z3 | 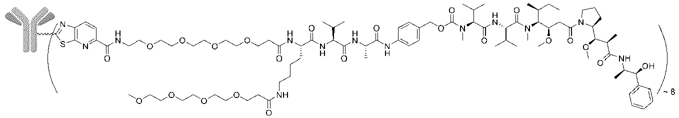 |
| anti-HER2: **ADC-35C** and anti-CD276: **ADC-35D** | anti-HER2: Trastuzumab and anti-CD276: CE4-Z3 | 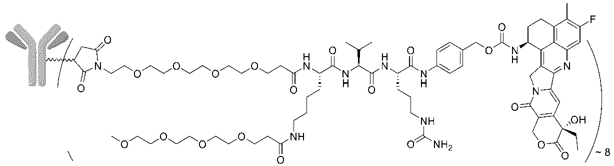 |

wherein, 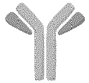 represents trastuzumab or CE4-Z3.

### Example 37: Preparation of antibody-drug conjugates

A humanized heavy chain camel antibody (HCAb) against B7-H3 (CD276) was used to replace the traditional IgG antibody in Example 36. This HCAb was conjugated with a new linker-payload to obtain a small antibody HCAb-drug conjugates (HDCs) to verify the universality of the linker-payload. The conjugation methods and post-conjugation characterization were performed as described in Example 36.

In order to compare with the linker-payload of the traditional structure, MC-VC-PABC-MMAE (Cas No.: 646502-53-6) and MC-GGFG-Dxd (Cas No.: 1599440-13-7) were purchased directly through the commercial route of the InnoChem Reagents platform and directly coupled to the protein. The corresponding compound codes of MC-VC-PABC-MMAE and MC-GGFG-Dxd in the present disclosure are compound-42 and compound-43, respectively. The chemical structures of MC-VC-PABC-MMAE and MC-GGFG-Dxd are as follows:

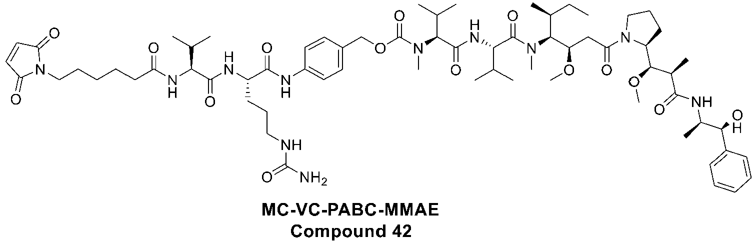

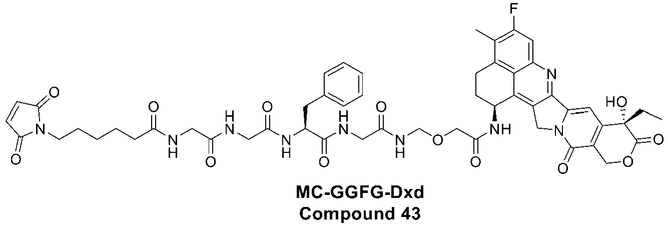

The structure of the prepared HDC is as follows:

| HDC Number | Antibody | HDC structure |
|---|---|---|
| anti-CD276: **HDC-14E** | CA2-VHH25 | 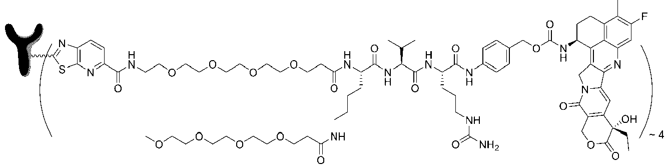 |
| anti-CD276: **HDC-14F** | CA2-VHH25 | 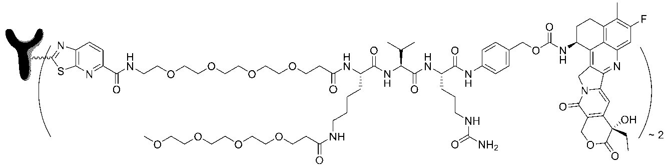 |
| anti-CD276: **HDC-35E** | CA2-VHH25 | 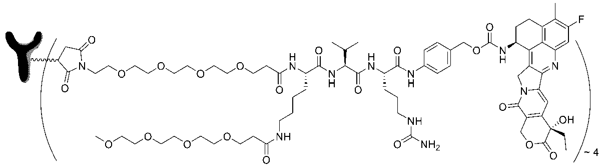 |
| anti-CD276: **HDC-42E** | CA2-VHH25 | 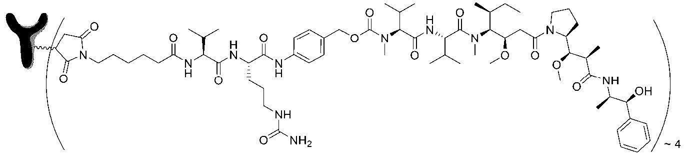 |
| anti-CD276: **HDC-43E** | CA2-VHH25 | 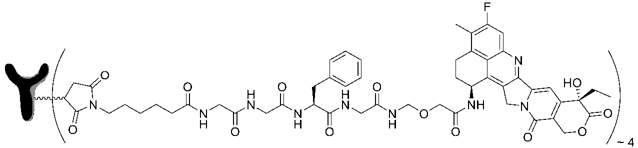 |

wherein, 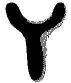 represents CA2-VHH25.

### Example 38: Hydrophilicity/Hydrophobicity Study of Antibody-Drug Conjugates

To directly compare the hydrophilicity/hydrophobicity differences between the ADC based on the new 2-(methylsulfonyl)thiazolo[5,4-b]pyridine coupling moiety in the present disclosure and the ADC based on the traditional succinimide coupling moiety, the present disclosure pioneered a comparative study between ADCs containing the 2-(methylsulfonyl)thiazolo[5,4-b]pyridine coupling moiety and ADCs containing the traditional succinimide coupling moiety, using the HCAb conjugate with simpler structure to replace the IgG conjugate.

### (I) Hydrophilicity/hydrophobicity analysis of HDC based on HCAb coupling

Under the same coupling conditions, stronger hydrophobicity generally leads to higher ADC polymerization. In the case where the DAR value is substantially equivalent, higher hydrophilicity of the linker-payload generally results in a higher monomer content in the coupling product. The present disclosure first detected the degree of polymerization of the prepared HDC to preliminarily determine the hydrophilicity/hydrophobicity of the prepared linker-payload. Typically, a monomer content of at least 95% is required after coupling.

| HDC Number | Monomer content percentage | Whether the coupling requirements are met |
|---|---|---|
| **HDC-14E** | ∼ 95% | Yes |
| **HDC-14F** | > 95% | Yes |
| **HDC-35E** | ∼ 90% | No |
| **HDC-42E** | < 80% | No |
| **HDC-43E** | ∼ 95% | Yes |

The analysis of the data in the above table can be seen as follows:
(1) By comparing the polymerization degree data of HDC-14E and HDC-14F, it can be seen that reducing the DAR value of HCAb can improve the monomer content of HDC.
(2) By comparing the polymerization degree data of HDC-14E and HDC-35E, it can be seen that the ADC based on the 2-(methylsulfonyl)thiazolo[5,4-b]pyridine coupling moiety has higher hydrophilicity than the ADC based on the traditional succinimide coupling moiety.

### (II) HIC Analysis of HDC Based on HCAb Coupling

Typically, in hydrophobic interaction chromatography (HPLC-HIC), each component of the antibody-drug conjugate HDC coupled with different payloads will also elute in sequence according to the hydrophilic size of the component (naked antibody (DAR= 0) with the shortest retention time, followed by the component of DAR= 2, DAR= 4). Therefore, the hydrophobicity of the corresponding linker-payload can be determined based on the retention time of HDCs on HPLC-HIC.

| HDCs Number | Retention time |
|---|---|
| **HDC-14E** | 27.5 min |
| **HDC-35E** | 33.7 min |

The analysis of the data in the above table can be seen as follows: under the condition that the DAR value is substantially equivalent (DAR=4), **HDC-14E** based on the new 2-(methylsulfonyl)thiazolo[5,4-b]pyridine coupling moiety has a significantly closer retention time to that of the naked antibody (DAR=0) on HPLC-HIC than **HDC-35E** based on the traditional succinimide coupling moiety, demonstrating that the linker based on the 2-(methylsulfonyl)thiazolo[5,4-b]pyridine coupling moiety has higher hydrophilicity than the linker based on the traditional succinimide coupling moiety.

### Example 39: Stability Study of ADC

### Improved stability of the ADCs based on the new coupling moiety

To verify the stability characteristics of ADCs based on the new 2-(methylsulfonyl)thiazolo[5,4-b]pyridine coupling moiety, the present disclosure used the ADC based on the conventional succinimide coupling moiety as a comparison, selected ADC-14B and ADC-35B as model ADC molecules, and carried out stability studies of the above two ADCs with different coupling moiety structures in human plasma and mouse plasma. The linkers in the evaluated ADCs all contained a PEG fragment. The parameter for assessing the stability of the ADC coupling moiety is the change in the remaining content of the antibody-conjugated drug (acDrug) over plasma incubation time. The results are shown in Fig. 2 and 3.

The stability test results of the above ADC-14B and ADC-35B in human plasma and mouse plasma show that: replacing the traditional succinimide coupling moiety with the new 2-(methylsulfonyl)thiazolo[5,4-b]pyridine coupling moiety developed in the present disclosure at coupling moiety position of the antibody end in the linker can significantly increase the content of the antibody-conjugated drug (P values were 0.0006 and 0.0124, respectively), that is, the ADC based on the 2-(methylsulfonyl)thiazolo[5,4-b]pyridine coupling moiety has a lower linker-payload release rate in plasma.

### Example 40: In vitro cytotoxicity study of ADC

The present disclosure aims to screen and match more reasonable linker-payload combinations based on new 2-(methylsulfonyl)thiazolo[5,4-b]pyridine coupling moiety.

The present disclosure evaluated the in vitro activity of free MMAE, Dxd, compound-41, and staurosporine (used as a control) in the aforementioned tumor cell lines. Regarding the selection of cell lines, HER2 high-expression cell lines included breast cancer cell lines BT-474, SK-BR-3, and gastric cancer cell line NCI-N87; HER2 medium-expression cell lines included MDA-MB-453; HER2 low-expression cell lines included MCF-7; and HER2 non-expression cell lines included MDA-MB-231.

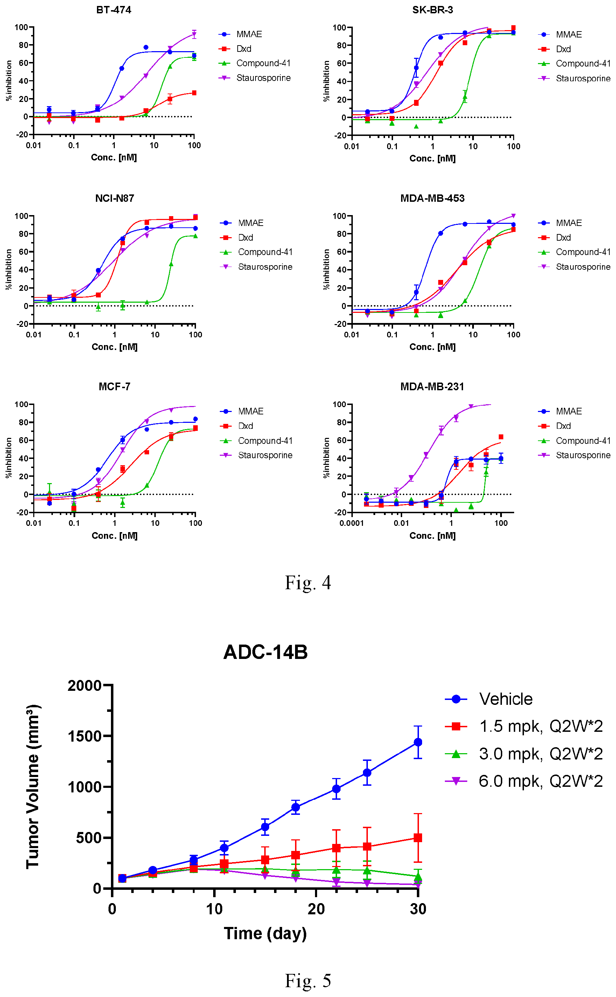

As shown in Fig. 4, the order of the active strength of the payload in the free form was as follows: MMAE > Dxd > compound-41. The EC50 value of the free compound-41 is much worse than that of Dxd, while the in vitro activity difference between the ADC obtained after conjugation of Compound-41 (DAR4) and the ADC based on Dxd (DAR8) is small. Therefore, using Compound-41 according to the present disclosure as a payload of ADC has the application potential to improve the drug therapeutic window.

Based on the new 2-(methylsulfonyl)thiazolo[5,4-b]pyridine coupling moiety, the present disclosure prepared compound-1 to compound-31, as well as compound-39 and compound-40, and conjugated them with various types of antibodies (taking IgG type antibody of anti-HER2 and anti-CD276 and/or HCAb as examples) to obtain antibody-drug conjugates (ADC or HCAb), which were then used to conduct further druggability studies.

### Example 41: In vivo pharmacodynamic study of ADC in a mouse model

To verify the in vivo efficacy of the antibody-drug conjugates prepared using the prepared new linker-payload, the present disclosure selected the antibody-drug conjugates corresponding to the humanized IgG1 antibody CE4-Z3 (abbreviated as Z3) and the humanized HCAb antibody CA2-VHH25 (abbreviated as VHH25) against B7-H3, respectively, and evaluated the in vivo efficacy characteristics of these ADCs (including HDC) in a human lung cancer Calu-6 xenograft CDX model with high B7-H3 expression.

The in-vivo efficacy test aimed to study the antitumor effect of the test ADCs in a female mouse animal model of subcutaneous xenograft of human lung cancer Calu-6 cell line. The test animals selected were six- to eight-week-old female Balb/c-nude mice and housed in an environment with a temperature of 20-26 °C and humidity of 40-70%, with each test group/box housed separately. Calu-6 cells were passaged in MEM (+0.01 mM NEAA) + 10% FBS medium, and cells in the exponential growth phase were collected, resuspended to an appropriate concentration, and then used for subcutaneous tumor inoculation in mice. The experimental mice were subcutaneously inoculated with 5 × 10⁶ Calu-6 cells on the right back. When the tumors grew to an average volume of about 100 mm³, the mice were grouped using a randomized block design (grouping was performed using StudyDirector^{™} software, version 3.1.399.19, supplier Studylog System, Inc., S. San Francisco, CA, USA) based on tumor size and mouse body weight. On the same day after grouping, the mice were administered at the set dose, and the dosing volume was calculated as V = 10 µL/g. Tumor volume was calculated according to V (mm³) = 1/2 × (major diameter × minor diameter²), while the weight of the animal was measured twice a week. The effect of the tested drug on the normal behavior of the animal is observed and recorded at the same time when measuring the volume of the tumor and the weight of the mouse. Data are expressed as mean + SD. The indicators were statistically analyzed using the unpaired two-tailed t-test, with a statistical significance level set at p < 0.05. Statistical analysis was performed using GraphPad software (Version 8.0.2, La Jolla, CA, USA).

The present disclosure conducted an in vivo pharmacodynamic study of ADC-14B. The results showed that, compared with the vehicle group, ADC-14B exhibited significant in vivo antitumor efficacy at all doses (P < 0.0001). At the same time, the in vivo antitumor efficacy of ADC-14B showed a significant dose-effect-dependent relationship at low, medium, and high doses (1.5 mpk, 3.0 mpk, and 6.0 mpk). Specifically, when comparing 1.5 mpk dose group with 3.0 mpk dose group, the in vivo efficacy of 3.0 mpk dose group showed a significant advantage (P = 0.0092); when comparing 1.5 mpk dose group with 6.0 mpk dose group, the in vivo efficacy of 6.0 mpk dose group showed a significant advantage (P = 0.0028); and when comparing 3.0 mpk dose group with 6.0 mpk dose group, the in vivo efficacy of 6.0 mpk dose group showed a significant advantage (P = 0.0428). The results are shown in Fig. 5.

The purchased MC-GGFG-Dxd (compound code: 43) was coupled with DS7300 reported by Daiichi Sankyo Company Limited to obtain an ADC with DAR=4 (code: DS7300-43). At the same time, nonspecific isotype IgG1 was coupled with compound 14 to obtain an ADC with DAR4 (code: IgG-14B).

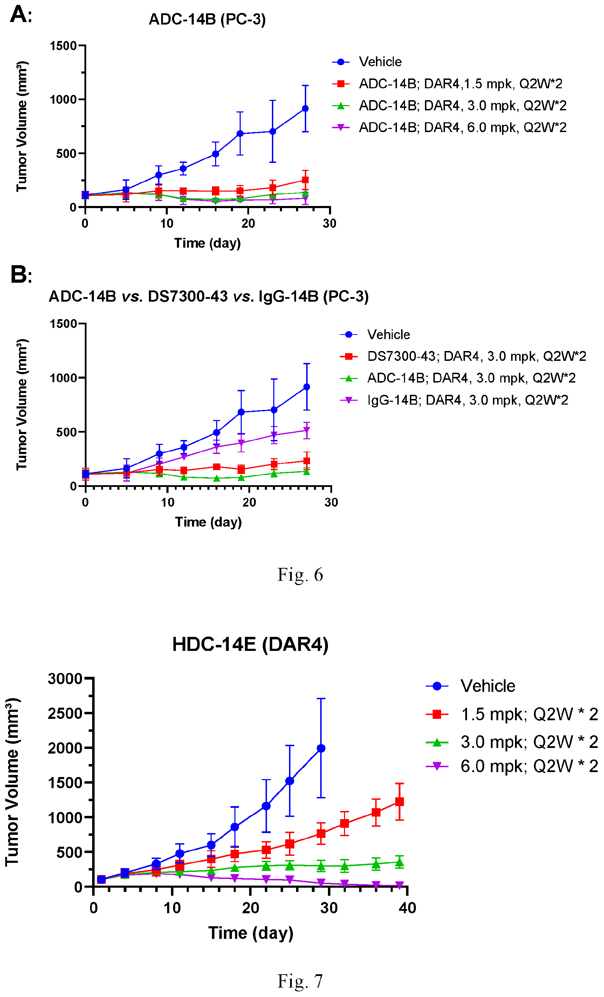

Furthermore, the present disclosure compared the in vivo efficacy characteristics of ADC-14B, DS7300-43, and IgG-14B in a xenograft mouse model of PC3 prostate cancer cells with medium expression of antigen B7-H3. On the one hand, as shown in Fig. 6A, compared with the vehicle group, ADC-14B exhibited significant in vivo tumor inhibitory effect at all doses (P < 0.0001). At the same time, ADC-14B showed a significant dose-effect-dependent relationship at low, medium, and high doses (1.5 mpk, 3.0 mpk, and 6.0 mpk). Specifically, when comparing 1.5 mpk dose group with 3.0 mpk dose group, the in vivo efficacy of 3.0 mpk dose group showed a significant advantage (P = 0.0038); when comparing 3.0 mpk dose group with 6.0 mpk dose group, the in vivo efficacy of 6.0 mpk dose group showed a significant advantage (P = 0.0356). On the other hand, as shown in Fig. 6B, at the same dose (3.0 mpk), ADC-14B showed a significant advantage in in vivo efficacy over DS7300-43 and IgG-14B (P values were 0.0076 and <0.0001, respectively).

The compound-14 in the present disclosure was coupled with the humanized HCAb CA2-VHH25 against B7-H3 screened by our researchers, to obtain HDC-14E with DAR ≈ 4. In vivo efficacy results showed that, compared with the vehicle group, tumor growth was significantly inhibited in all HDC-14E (DAR4) test groups (P < 0.0001). Furthermore, the in vivo tumor-inhibiting effect of HDC-14E (DAR4) exhibited a significant dose-effect-dependent relationship at low, medium, and high doses (1.5 mpk, 3.0 mpk, and 6.0 mpk). Specifically, when comparing 1.5 mpk dose group with 3.0 mpk dose group, the 3.0 mpk dose group showed a significant advantage (P = 0.0001); when comparing 1.5 mpk dose group with 6.0 mpk dose group, the 6.0 mpk dose group showed a significant advantage (P < 0.0001); and when comparing 3.0 mpk dose group with 6.0 mpk dose group, the 6.0 mpk dose group showed a significant advantage (P < 0.0001). The results are shown in Fig. 7.

### Example 42: Safety study of anti-B7-H3 ADC in mouse model

To directly compare the drug safety differences between the ADC based on the new 2-(methylsulfonyl)thiazolo[5,4-b]pyridine coupling moiety and the ADC based on the traditional succinimide coupling moiety, the present disclosure used ADC-14B (DAR4) based on the new 2-(methylsulfonyl)thiazolo[5,4-b]pyridine coupling moiety and ADC-35B (DAR4) based on the traditional succinimide coupling moiety as model ADC molecules. A preliminary comparison of their tolerability studies at high doses (200 mg/kg) in B-hB7-H3 (C57BL6-Cd276*^{tm1(CD276)}*/Bcgen, BIOCYTOGEN, Beijing, CN) transgenic mice was conducted, with each test group containing 3 animals.

The test results showed that after 4 days of dosing, none of the test animals died after administration, and at the same time, none of the test animals showed serious abnormalities in physical signs and behavior.

After 4 days of dosing, the average body weight of the test animals in the ADC-14B group decreased by 11.0% compared with that before administration, while the average body weight of the test animals in the ADC-35B group decreased by 18.4% compared with that before administration. The percentage data of body weight decrease in animals of the ADC-14B test group (9.4%, 9.4%, 14.4%) were significantly different (P = 0.0154) from the percentage data of body weight decrease in animals of the ADC-35B test group (18.6%, 19.5%, 17.1%).

After 7 days of dosing, the average body weight of the test animals in the ADC-14B group and the ADC-35B group recovered to 98.53% and 68.73% compared with that before administration, respectively. The percentage data of body weight decrease in animals of the ADC-14B test group (-3.53%, 1.56%, 6.38%) was significantly different (P = 0.0006) from the percentage data of body weight decrease in animals of the ADC-35B test group (32.79%, 31.61%, 29.41%).

In addition, it was observed that the animals in the ADC-35B test group had serious adverse effects such as gait instability, dull coat, perianal soiling, bradykinesia and so on, while the tested animals in the ADC-14B group had no obvious abnormality.

The above data indicate that the dose of 200 mg/kg has not reached the maximum tolerated dose of ADC-14B, while the maximum tolerated dose of ADC-35B is below 200 mg/kg. This demonstrates that ADC-14B based on the new 2-(methylsulfonyl)thiazolo[5,4-b]pyridine coupling moiety has a potential safety advantage compared to ADC-35B based on the conventional succinimide coupling moiety.

| ADC | Mouse Number | Body weight of Mouse (g) (Day 0) | Body weight of Mouse (g) (Day 4) | Body weight of Mouse (g) (Day 7) |
|---|---|---|---|---|
| ADC-14B | 1 | 17.0 | 15.4 | 17.6 |
| | 2 | 19.2 | 17.4 | 18.9 |
| | 3 | 18.8 | 16.1 | 17.6 |
| ADC-35B | 4 | 18.3 | 14.9 | 12.3 |
| | 5 | 17.4 | 14.0 | 11.9 |
| | 6 | 17.0 | 14.1 | 12.0 |

The above content is a further detailed description of the present disclosure in conjunction with specific preferred embodiments, and it is not to be understood that the specific embodiments of the present disclosure are limited to these descriptions. For a person of ordinary skill in the technical field to which the present disclosure pertains, without departing from the inventive concept of the present disclosure, several simple deductions or substitutions may be made, all of which should be considered within the protection scope of the present disclosure.

## Claims

1. A compound of formula (I), or a pharmaceutically acceptable salt or isotopic variant thereof: 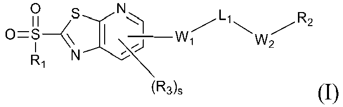 wherein,
R₁ is selected from C₁₋₆ alkyl or C₁₋₆ haloalkyl;
W₁ is selected from -O-, -S-, -NR_{b}-, -C(O)O-, -C(O)NR_{b}-, -O-C(O)-, -NR_{b}-C(O)-, -S(O)ₚO-, or -O-S(O)ₚ-;
wherein p = 1 or 2;
Li is a chemical bond or -(CH₂)ₘ₁-(OCH₂CH₂)ₙ₁-(CH₂CH₂O)ₙ₂-(CH₂)ᵣ₁-(L)_{q}-(CH₂)ᵣ₂-(OCH₂CH₂)ₙ₃-(CH₂CH₂O)ₙ₄-(CH₂)ₘ₂-;
wherein -L- is selected from -O-, -NR_{b}-, -C(O)NR_{b}-, -C(O)O-, -NR_{b}-C(O)-, -O-C(O)-, -C₃₋₈ cycloalkylene-, -3- to 8-membered heterocyclylene-, -C₆₋₁₀ arylene- or -5- to 10-membered heteroarylene;
each m1, m2, n1, n2, n3, n4, r1, and r2 is independently selected from 0, 1, 2, 3, 4, 5, 6, 7, 8, 9, or 10;
q = 0, 1, or 2;
W₂ is selected from a chemical bond, -O-, -S-, -NR_{b}-, or -C(O)-;
R₂ is selected from H, D, halogen, -ORₐ, -NR_{b}R_{c}, or the following groups: 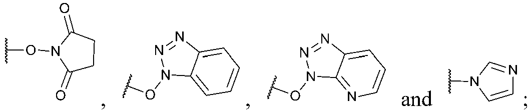
R₃ is selected from H, D, halogen, C₁₋₆ alkyl, or C₁₋₆ haloalkyl;
s = 0, 1, or 2;
wherein Rₐ, R_{b}, and R_{c} are independently selected from H, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl, or 5- to 10-membered heteroaryl; or R_{b} and R_{c} together with the N atom to which they are attached form a 3- to 10-membered heterocyclyl;
wherein the above group is optionally substituted with one or more deuterium atoms, up to completely deuterated.

2. The compound of formula (I) of claim 1, or a pharmaceutically acceptable salt or isotopic variant thereof, wherein R₁ is selected from C₁₋₄ alkyl or C₁₋₄ haloalkyl, alternatively methyl.

3. The compound of formula (I) of claim 1 or 2, or a pharmaceutically acceptable salt or isotopic variant thereof, wherein W₁ is selected from -C(O)O-, -C(O)NR_{b}-, -O-C(O)-, or -NR_{b}-C(O)-, alternatively -C(O)O-, or -C(O)NR_{b}-.

4. The compound of formula (I) of any one of claims 1-3, or a pharmaceutically acceptable salt or isotopic variant thereof, wherein,
Li is selected from a chemical bond, -(CH₂)ₘ₁-(CH₂CH₂O)ₙ₁-(CH₂)ᵣ₁-L-(CH₂)ᵣ₂-(CH₂CH₂O)ₙ₂-(CH₂)ₘ₂-, or -(CH₂)ₘ₃-(OCH₂CH₂)ₙ₃-(CH₂)ᵣ₃-L-(CH₂)ᵣ₄-(OCH₂CH₂)ₙ₄-(CH₂)ₘ₄-;
alternatively, Li is selected from a chemical bond, -(CH₂CH₂O)ₙ₅-(CH₂)ᵣ₅-L-(CH₂)ₘ₅-, -(OCH₂CH₂)ₙ₆-(CH₂)ᵣ₆-L-(CH₂)ₘ₆-, -(CH₂)ₘ₇-L-(CH₂CH₂O)ₙ₇-(CH₂)ᵣ₇-, or -(CH₂)ₘ₈-L-(OCH₂CH₂)ₙ₈-(CH₂)ᵣ₈-;
alternatively, L₁ is selected from a chemical bond, -(CH₂)ₘ₉-(CH₂CH₂O)ₙ₉-(CH₂)ᵣ₉-, or -(CH₂)ₘ₁₀-(OCH₂CH₂)ₙ₁₀-(CH₂)ᵣ₁₀-;
alternatively, Li is selected from a chemical bond, -(CH₂CH₂O)ₙ₁₁-(CH₂)ₘ₁₁-, -(CH₂)ₘ₁₂-(CH₂CH₂O)ₙ₁₂-, - (OCH₂CH2)ₙ₁₃-(CH₂)ₘ₁₃-, or -(CH₂)ₘ₁₄-(OCH₂CH₂)ₙ₁₄-;
alternatively, L₁ is selected from a chemical bond, -(CH₂)ₘ₁₅-O-(CH₂)ₘ₁₆-, -(CH₂)ₘ₁₇-O-, -(CH₂)ₘ₁₈-NH-, - O-(CH₂)ₘ₁₉-, or -NR_{b}-(CH₂)ₘ₂₀-; alternatively, L₁ is selected from a chemical bond, -(CH₂)ₘ₂₁-, -L-, -(CH₂)ₘ₂₂-L-, or -L-(CH₂)ₘ₂₃-;
alternatively, L₁ is selected from a chemical bond, -(CH₂CH₂O)ₙ₁₁-(CH₂)ₘ₁₁-, or -(CH₂)ₘ₂₁-;
wherein m1, m2, m3, m4, m5, m6, m7, m8, m9, m10, m11, m12, m13, m14, m15, m16, m17, m18, m19, m20, m21, m22, m23 are independently selected from 0, 1, 2, 3, 4, 5, 6, 7, 8, 9, or 10;
n1, n2, n3, n4, n5, n6, n7, n8, n9, n10, n11, n12, n13, n14 are independently selected from 0, 1, 2, 3, 4, 5, 6, 7, 8, 9 or 10;
r1, r2, r3, r4, r5, r6, r7, r8, r9, r10 are independently selected from 0, 1, 2, 3, 4, 5, 6, 7, 8, 9 or 10.

5. The compound of formula (I) of any one of claims 1-4, or a pharmaceutically acceptable salt or isotopic variant thereof, wherein -L- is selected from -C(O)NR_{b}-, -C(O)O-, -NR_{b}-C(O)-, or -O-C(O)-.

6. The compound of formula (I) of any one of claims 1-5, or a pharmaceutically acceptable salt or isotopic variant thereof, wherein W₂ is selected from a chemical bond or -C(O)-.

7. The compound of formula (I) of any one of claims 1-6, or a pharmaceutically acceptable salt or isotopic variant thereof, wherein R₂ is selected from H, -OH, or

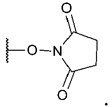

8. The compound of formula (I) of any one of claims 1-7, or a pharmaceutically acceptable salt or isotopic variant thereof, having the following general formula: 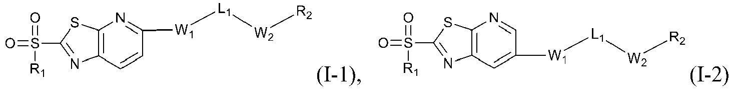 or 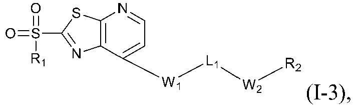 wherein each of the groups is defined as any one of claims 1-7.

9. The compound of any one of claims 1-8, or a pharmaceutically acceptable salt or isotopic variant thereof, wherein,
R₁ is Me;
W₁ is -C(O)O- or -C(O)NH-;
Li is selected from a chemical bond, -(CH₂CH₂O)ₙ₁₁-(CH₂)ₘ₁₁-, or -(CH₂)ₘ₂₁-;
wherein n11 = 1, 2, 3, 4, 5, 6, 7, 8, 9 or 10;
m11 = 0, 1, 2, 3, 4, 5, 6, 7, 8, 9 or 10;
m21 = 1, 2, 3, 4, 5, 6, 7, 8, 9 or 10;
W₂ is selected from a chemical bond or -C(O)-;
R2 is selected from H, -OH, or

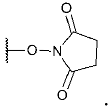

10. The compound of any one of claims 1-9, or a pharmaceutically acceptable salt or isotopic variant thereof, wherein the compound is selected from:

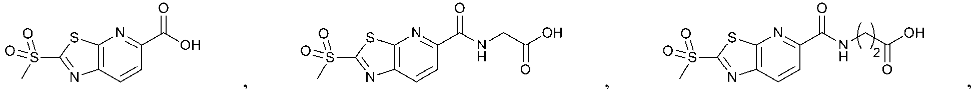

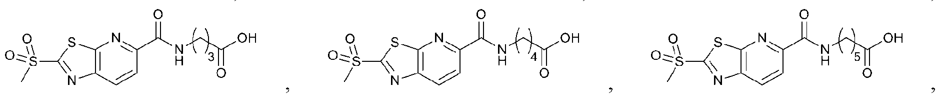

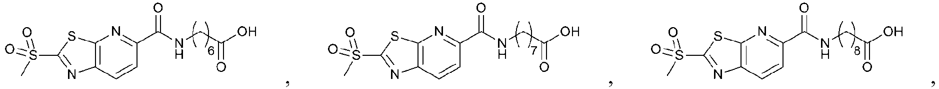

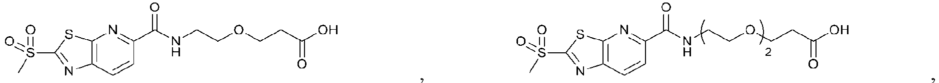

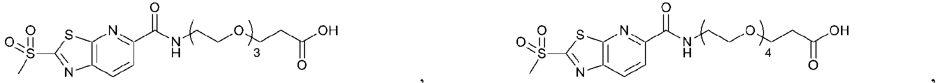

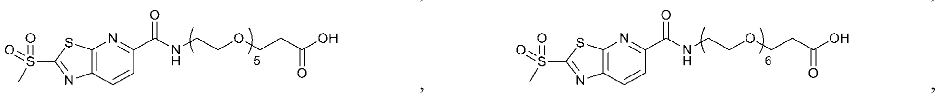

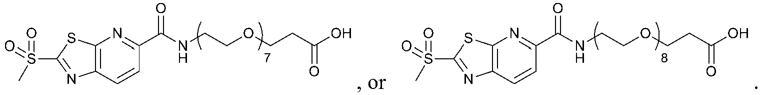

11. Use of the compound of any one of claims 1-10 or a pharmaceutically acceptable salt or isotopic variant thereof in the manufacture of an antibody-drug conjugate.

12. A compound of formula (II), or a pharmaceutically acceptable salt, prodrug, hydrate, solvate, enantiomer, diastereomer, mesomer, racemate, or tautomer thereof: 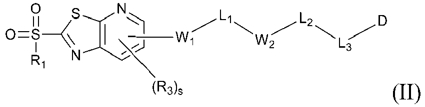 wherein,
L₂ is selected from the following amino acid residues or oligopeptide residues consisting of 2 to 10 following amino acids, wherein the amino acid is selected from cysteine, phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine, and the amino acid is optionally substituted with 1, 2, 3, 4, 5, or 6 R₄;
wherein N-terminal of amino acid residue or oligopeptide residue is connected to W₂, and the C-terminal is connected to L₃;
R₄ is selected from D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 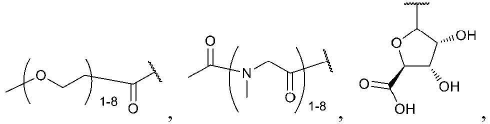 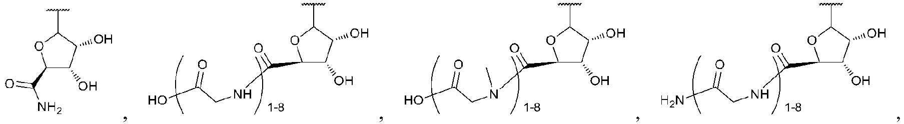 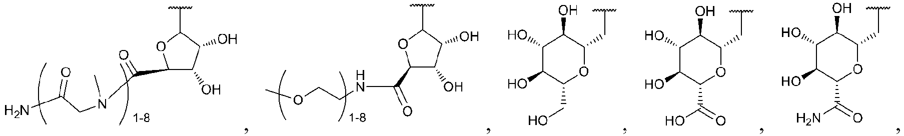 or 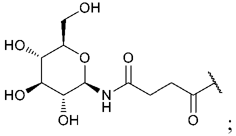
L₃ is selected from the following structures: 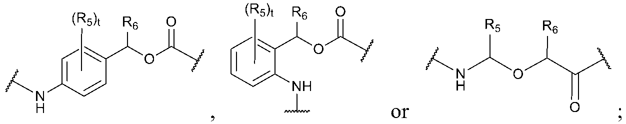
wherein NH is connected to L₂, and C(O) is connected to D;
R₅ is selected from H, D, halogen, NO₂, -ORₐ, -NR_{b}R_{c}, C₁₋₆ alkyl or C₁₋₆ haloalkyl;
t = 0, 1, 2, 3, or 4;
R₆ is selected from H, 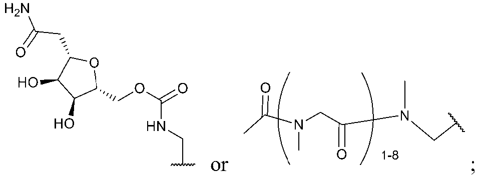
D is an active compound selected from a drug, a cytotoxin, a detection reagent, a diagnostic reagent or a targeting carrier;
wherein the above group is optionally substituted with one or more deuterium atoms, up to completely deuterated.
other groups are defined as any one of claims 1-10.

13. The compound of formula (II) of claim 12, or a pharmaceutically acceptable salt, prodrug, hydrate, solvate, enantiomer, diastereomer, mesomer, racemate, or tautomer thereof, wherein,
L₂ is selected from the following amino acid residues or oligopeptide residues consisting of 2 to 5 following amino acids, wherein the amino acid is selected from phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine;
alternatively, L₂ is selected from the following amino acid residues or oligopeptide residues consisting of 2-5 following amino acids, wherein the amino acid is selected from valine, citrulline, alanine, glycine, phenylalanine, lysine, arginine, aspartic acid, glutamic acid and serine;
alternatively, L₂ is selected from dipeptide, tripeptide or tetrapeptide residues consisting of the following amino acids, wherein the amino acid is selected from valine, citrulline, alanine, glycine, phenylalanine, lysine, arginine, aspartic acid, glutamic acid and serine;
alternatively, L₂ is selected from valine-citrulline, valine-alanine, valine-lysine, phenylalanine-lysine, lysine-lysine, alanine-lysine, phenylalanine-citrulline, leucine-citrulline, isoleucine-citrulline, phenylalanine-alanine, lysine-valine-citrulline, lysine-valine-alanine, valine-lysine-glycine, glycine-valine-lysine, glycine-valine-alanine, glutamine-valine-alanine, glutamine-valine-citrulline, glutamic acid-valine-alanine, glutamic acid-valine-citrulline, alanine-alanine-alanine, alanine-alanine-asparagine, phenylalanine-phenylalanine-lysine, glycine-phenylalanine-lysine, leucine-alanine-leucine, isoleucine-alanine-leucine, valine-alanine-valine, glycine-glycine-phenylalanine-glycine, alanine-leucine-alanine-leucine, and glycine-phenylalanine-leucine-glycine;
alternatively, L₂ is selected from glutamine-valine-alanine, glutamine-valine-citrulline, valine-citrulline, valine-alanine, valine-lysine, lysine-valine-citrulline, lysine-valine-alanine, and glycine-glycine-phenylalanine-glycine;
alternatively, L₂ is selected from valine-citrulline, valine-alanine, valine-lysine, lysine-valine-citrulline, lysine-valine-alanine, and glycine-glycine-phenylalanine-glycine.

14. The compound of formula (II) of claim 12 or 13, or a pharmaceutically acceptable salt, prodrug, hydrate, solvate, enantiomer, diastereomer, mesomer, racemate, or tautomer thereof, wherein,
R₄ is selected from H, D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups:

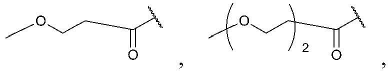

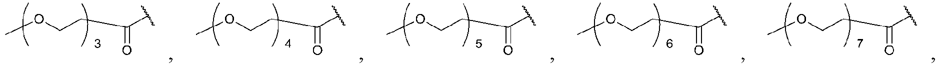

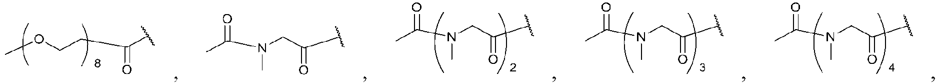

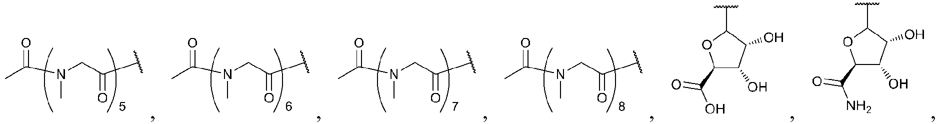

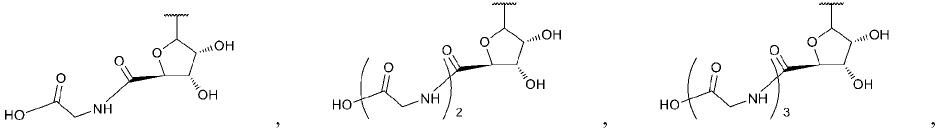

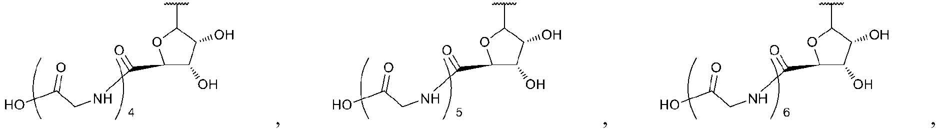

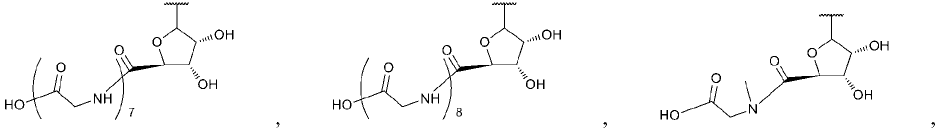

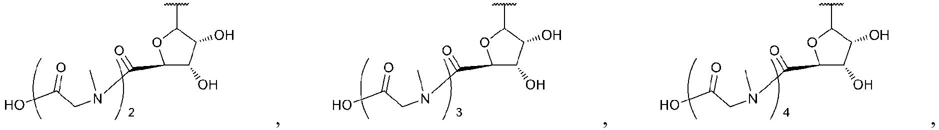

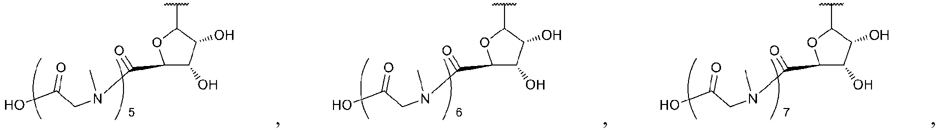

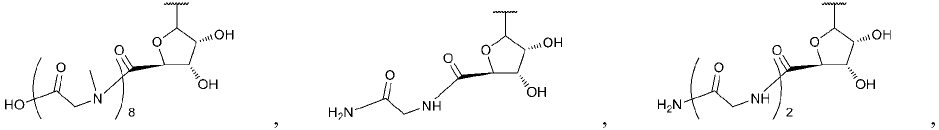

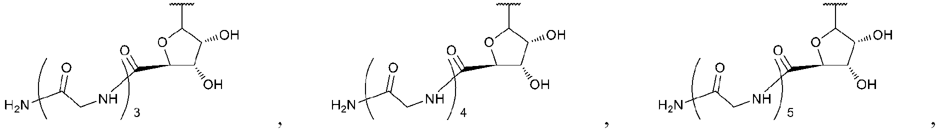

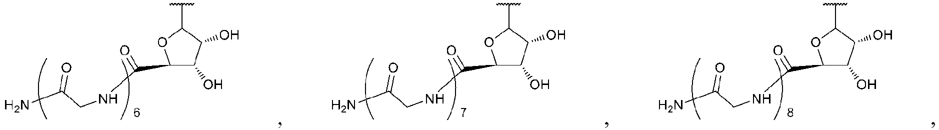

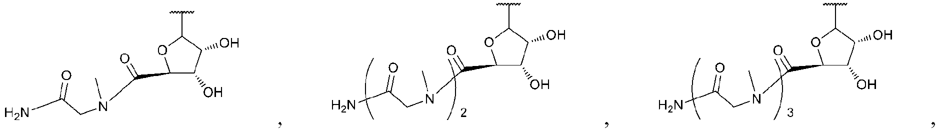

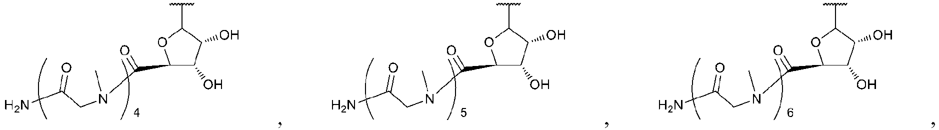

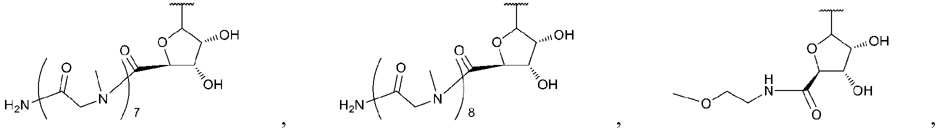

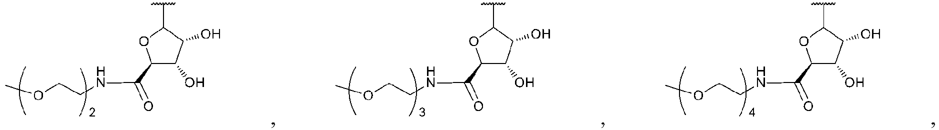

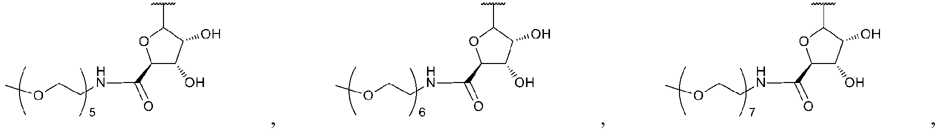

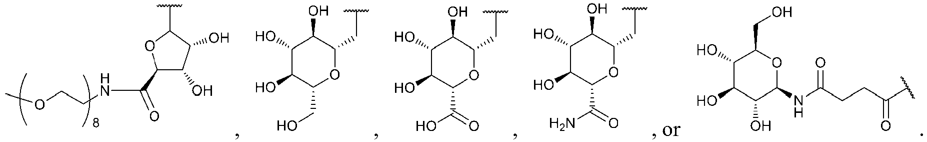

15. The compound of formula (II) of any one of claims 12-14, or a pharmaceutically acceptable salt, prodrug, hydrate, solvate, enantiomer, diastereomer, mesomer, racemate, or tautomer thereof, wherein,
L₃ is selected from the following structures: 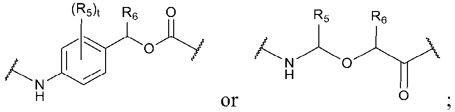
alternatively,
R₆ is selected from H,

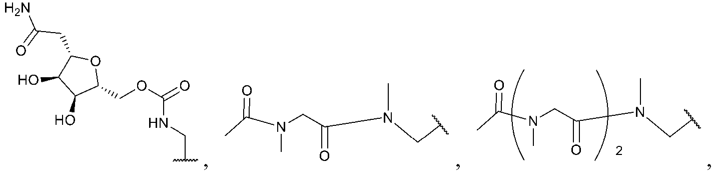

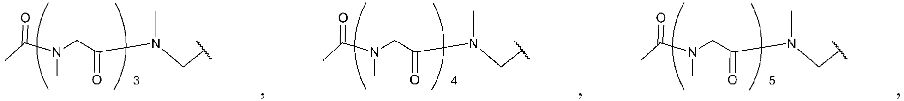

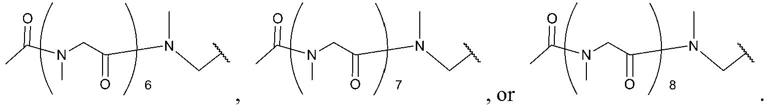

16. The compound of formula (II) of any one of claims 12-15, or a pharmaceutically acceptable salt, prodrug, hydrate, solvate, enantiomer, diastereomer, mesomer, racemate, or tautomer thereof, wherein,
D is selected from a cytotoxin, an antitumor drug, an anti-infective drug, or an immunomodulatory drug;
alternatively, D is a cytotoxin, such as a tubulin inhibitor, topoisomerase inhibitor, DNA alkylating agent, DNA intercalating agent, enzyme inhibitor, immunomodulator, PROTAC, antimetabolite, active peptide, active nucleotide sequence;
alternatively, D is selected from microtubule inhibitors, DNA topoisomerase inhibitors, drugs that interfere with DNA synthesis, DNA alkylating agents, DNA intercalating agents, enzyme inhibitors, RNA inhibitors, Bcl-xL inhibitors, NAMPT inhibitors, proteasome inhibitors, plicamycin, immunomodulators, PROTAC, antimetabolites, active peptides, nucleotides, tumor signaling pathway inhibitors, histone deacetylase inhibitors, tumor angiogenesis inhibitors, cyclin inhibitors, drugs that act on structural proteins, metal complexes, or glycopeptide antibiotics;
alternatively, D is a cytotoxin, alternatively selected from maytansine and its derivatives, auristatin and its derivatives, tubulysins and its derivatives, tubulin and its derivatives, cryptophycin and its derivatives, eribulin and its derivatives, pyrrolobenzodiazepines (PBD) and its derivatives, enediyne molecules and its derivatives, duocarmycin and its derivatives, and other active substances that inhibit tumor cell growth, promote tumor cell apoptosis or necrosis;
alternatively, D is selected from auristatin and its derivatives, camptothecin and its derivatives, maytansine and its derivatives, calicheamicin and its derivatives, paclitaxel and its derivatives, pyrrolobenzodiazepines and its derivatives, duocarmycin, doxorubicin, melphalan, mitomycin C, chlorambucil, and other active substances that inhibit tumor cell growth, promote tumor cell apoptosis or necrosis;
alternatively, D is selected from monomethyl auristatin E (MMAE), monomethyl auristatin F (MMAF), DM1, DM4, Dxd (CAS: 1599440-33-1), exatecan (Dx-8951f), oxaliplatin, bleomycin, pingyangmycin, camptothecin, hydroxycamptothecin, 9-aminocamptothecin, SN-38, irinotecan, topotecan, belotecan, Rubitecan, actinomycin D, doxorubicin, duocarmycin, daunorubicin, mitoxantrone, podophyllotoxin, etoposide, methotrexate, 5-fluorouracil, cytarabine, gemcitabine, mercaptopurine, Pentostatin, fludarabine, Cladribine, nelarabine, vinca alkaloids, vincristine, vinblastine, paclitaxel, docetaxel, Cabazitaxel, serine/threonine kinase inhibitors, tyrosine kinase inhibitors, aspartate kinase inhibitors, or histidine kinase inhibitors;
alternatively, D is selected from MMAE, MMAF, Dxd or Dx-8951f.

17. The compound of formula (II) of any one of claims 12-16, or a pharmaceutically acceptable salt, prodrug, hydrate, solvate, enantiomer, diastereomer, mesomer, racemate, or tautomer thereof, having the following general formula: 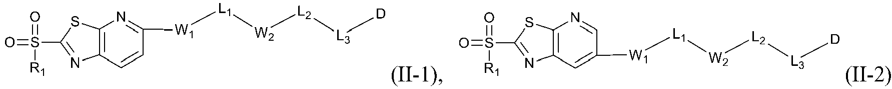 or 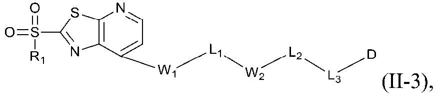 wherein each of the groups is defined as any one of claims 1-16.

18. The compound of any one of claims 12-17, or a pharmaceutically acceptable salt, prodrug, hydrate, solvate, enantiomer, diastereomer, mesomer, racemate, or tautomer thereof, wherein
R₁ is Me;
W₁ is -C(O)O- or -C(O)NH-;
L₁ is selected from -(CH₂CH₂O)ₙ₁₁-(CH₂)ₘ₁₁-, or -(CH₂)ₘ₂₁-;
wherein n11 = 1, 2, 3, 4, 5, 6, 7, 8, 9 or 10;
m11 = 0, 1, 2, 3, 4, 5, 6, 7, 8, 9 or 10;
m21 = 1, 2, 3, 4, 5, 6, 7, 8, 9 or 10;
W₂ is -C(O)-;
L₂ is selected from dipeptide, tripeptide, or tetrapeptide residues consisting of the following amino acids, wherein the amino acid is selected from valine, citrulline, alanine, glycine, phenylalanine, lysine, arginine, aspartic acid, glutamic acid, and serine, and the amino acid is optionally substituted with 1, 2, 3, 4, 5, or 6 R₄;
alternatively, L₂ is selected from valine-citrulline, valine-alanine, valine-lysine, phenylalanine-lysine, lysine-lysine, alanine-lysine, phenylalanine-citrulline, leucine-citrulline, isoleucine-citrulline, phenylalanine-alanine, lysine-valine-citrulline, lysine-valine-alanine, valine-lysine-glycine, glycine-valine-lysine, glycine-valine-alanine, glutamine-valine-alanine, glutamine-valine-citrulline, glutamic acid-valine-alanine, glutamic acid-valine-citrulline, alanine-alanine-alanine, alanine-alanine-asparagine, phenylalanine-phenylalanine-lysine, glycine-phenylalanine-lysine, leucine-alanine-leucine, isoleucine-alanine-leucine, valine-alanine-valine, glycine-glycine-phenylalanine-glycine, alanine-leucine-alanine-leucine, and glycine-phenylalanine-leucine-glycine, and the amino acid is optionally substituted with 1, 2, 3, 4, 5, or 6 R₄;
R₄ is selected from H, D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 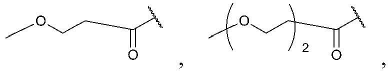 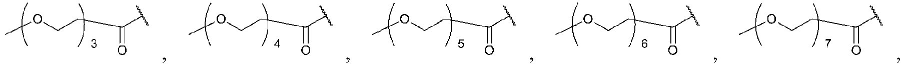 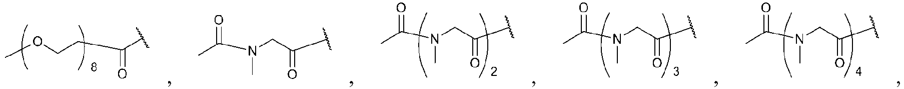 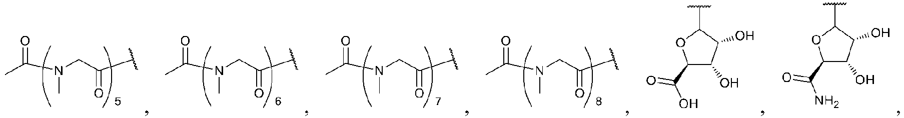 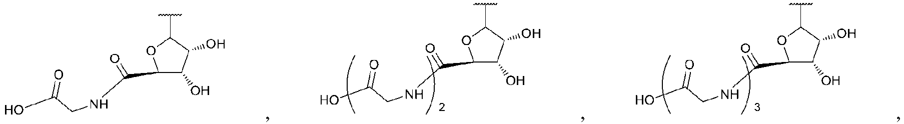 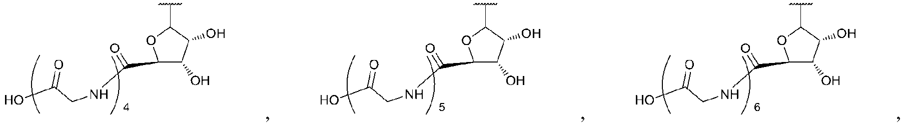 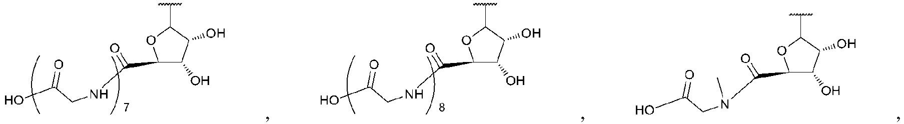 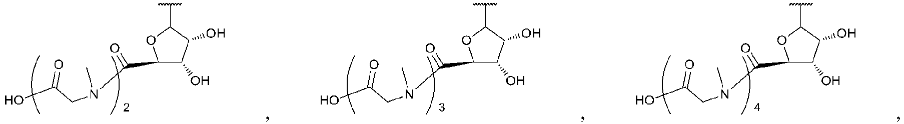 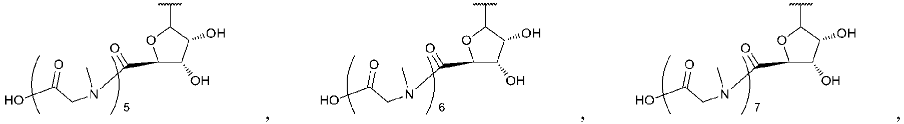 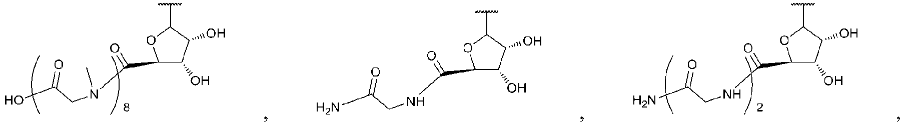 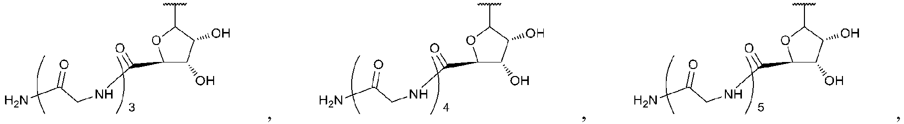 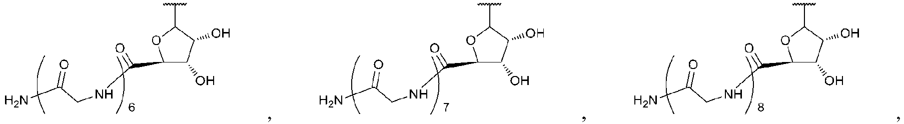 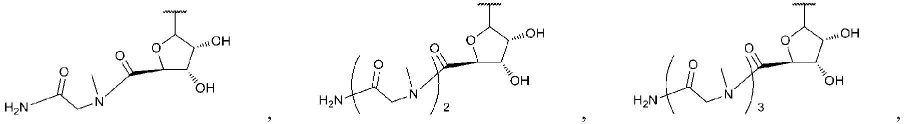 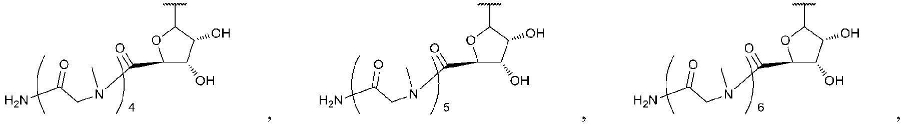 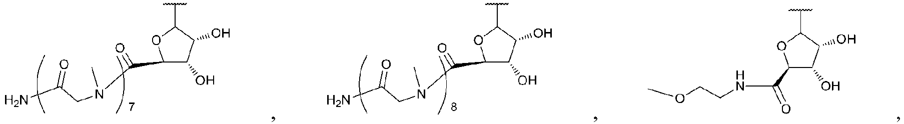 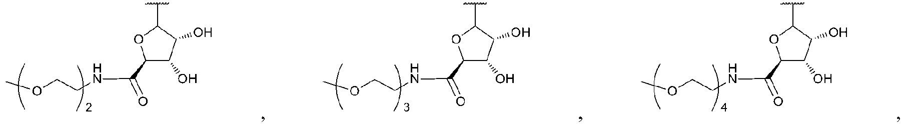 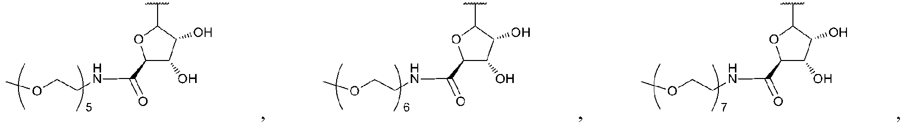 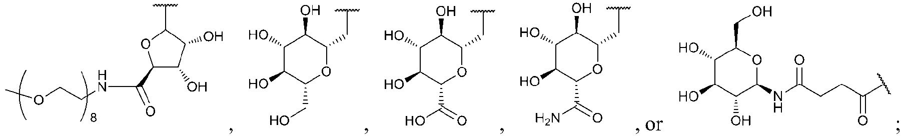
L₃ is selected from the following structures: 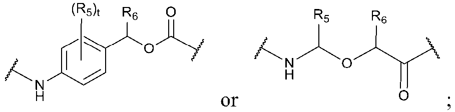
wherein R₅ is selected from H, D, halogen, C₁₋₆ alkyl or C₁₋₆ haloalkyl;
t = 0, 1, 2, 3, or 4;
R₆ is selected from H, 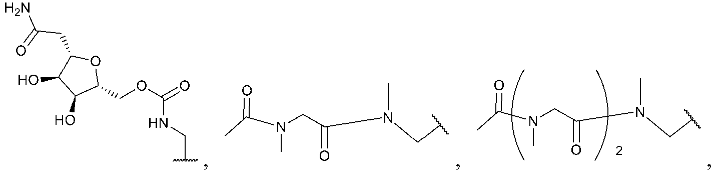 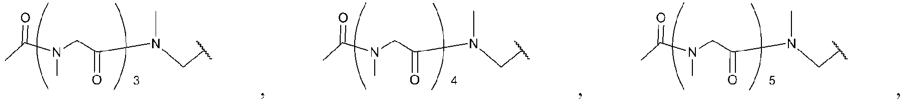 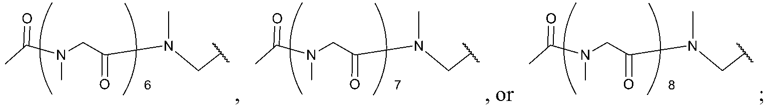
D is selected from microtubule inhibitors, topoisomerase I inhibitors, and DNA alkylating agents, such as MMAE, MMAF, Dxd, or Dx-8951f.

19. The compound of any one of claims 12-18, or a pharmaceutically acceptable salt, prodrug, hydrate, solvate, enantiomer, diastereomer, mesomer, racemate, or tautomer thereof, wherein the compound is selected from the group consisting of:

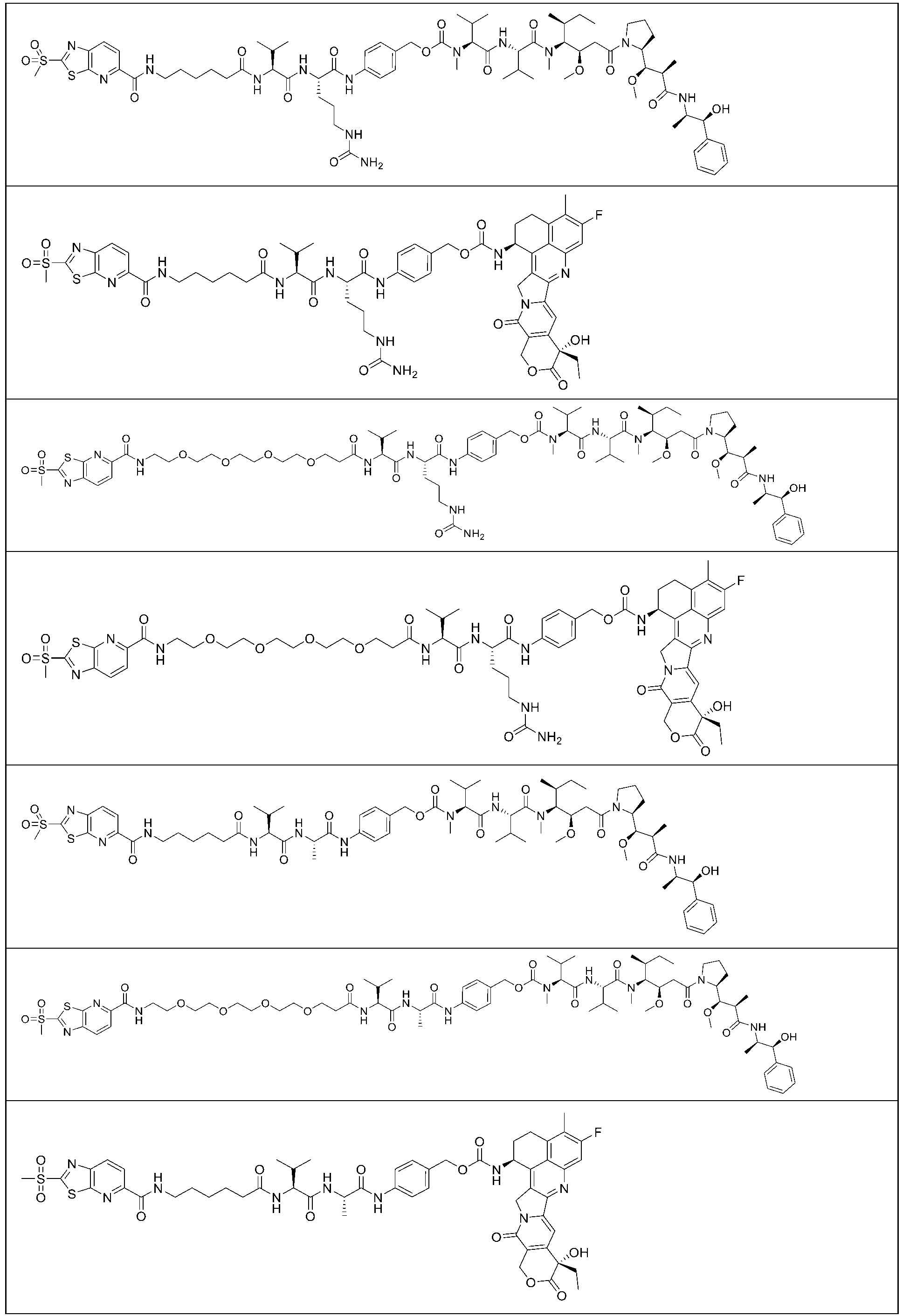

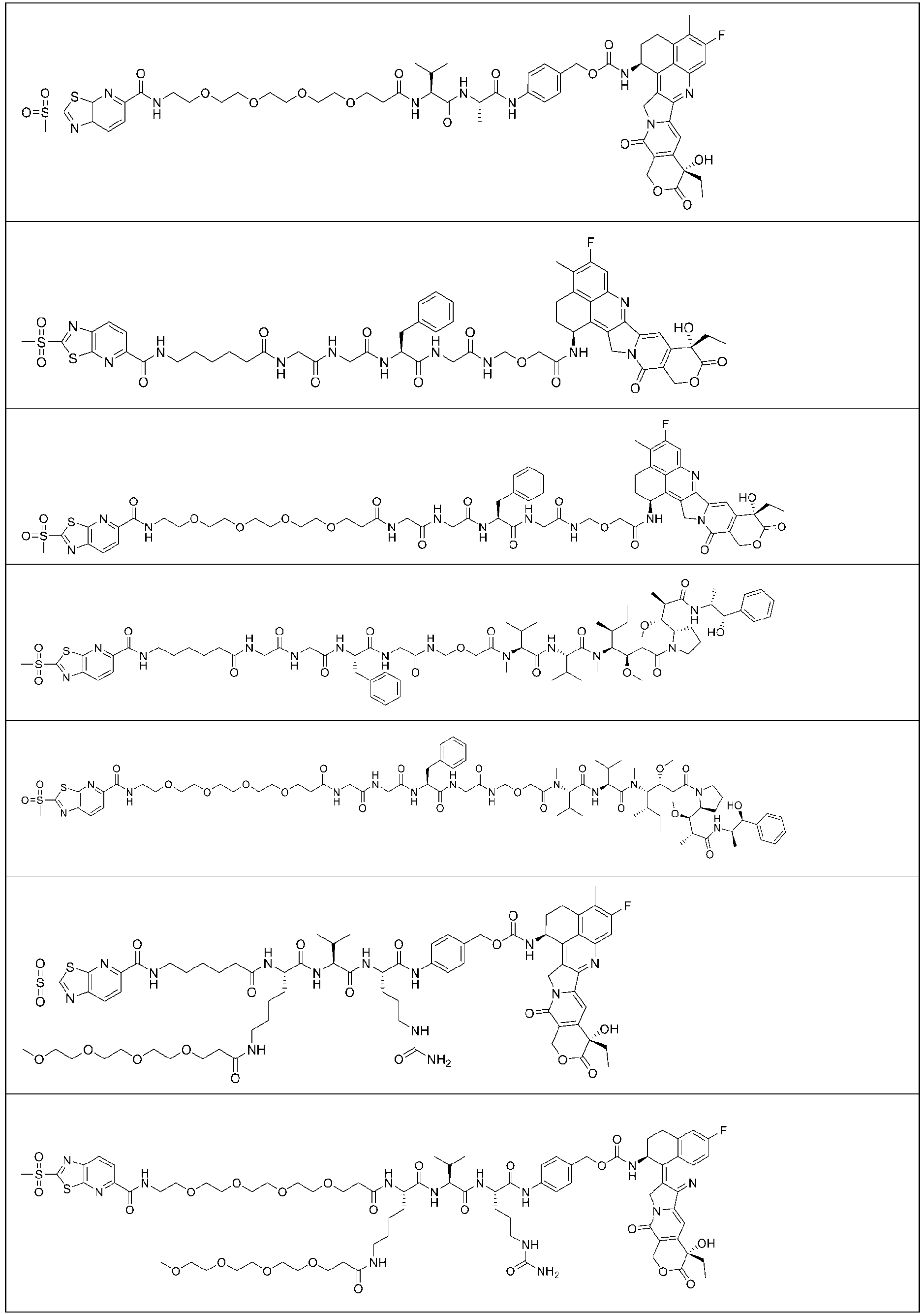

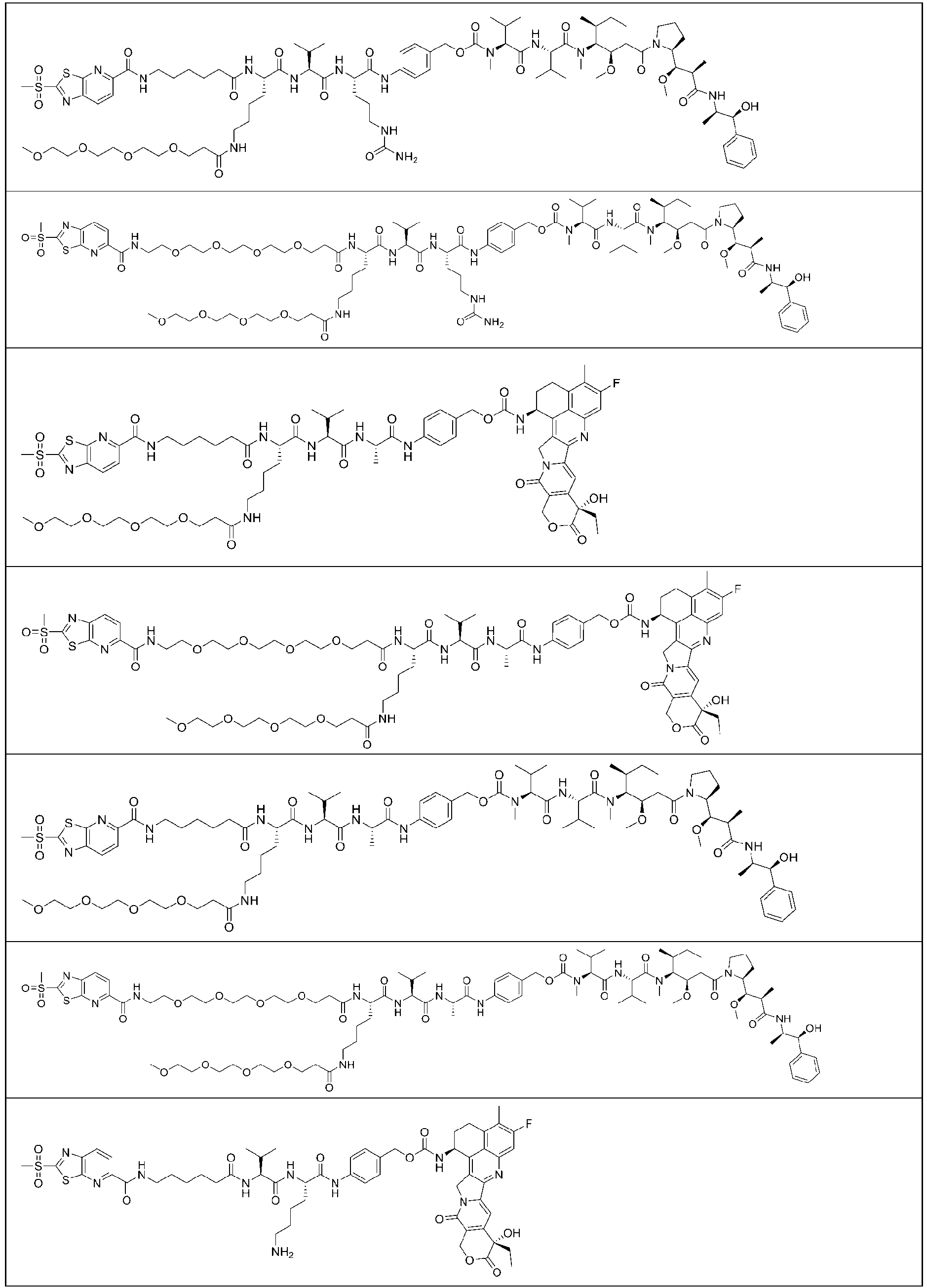

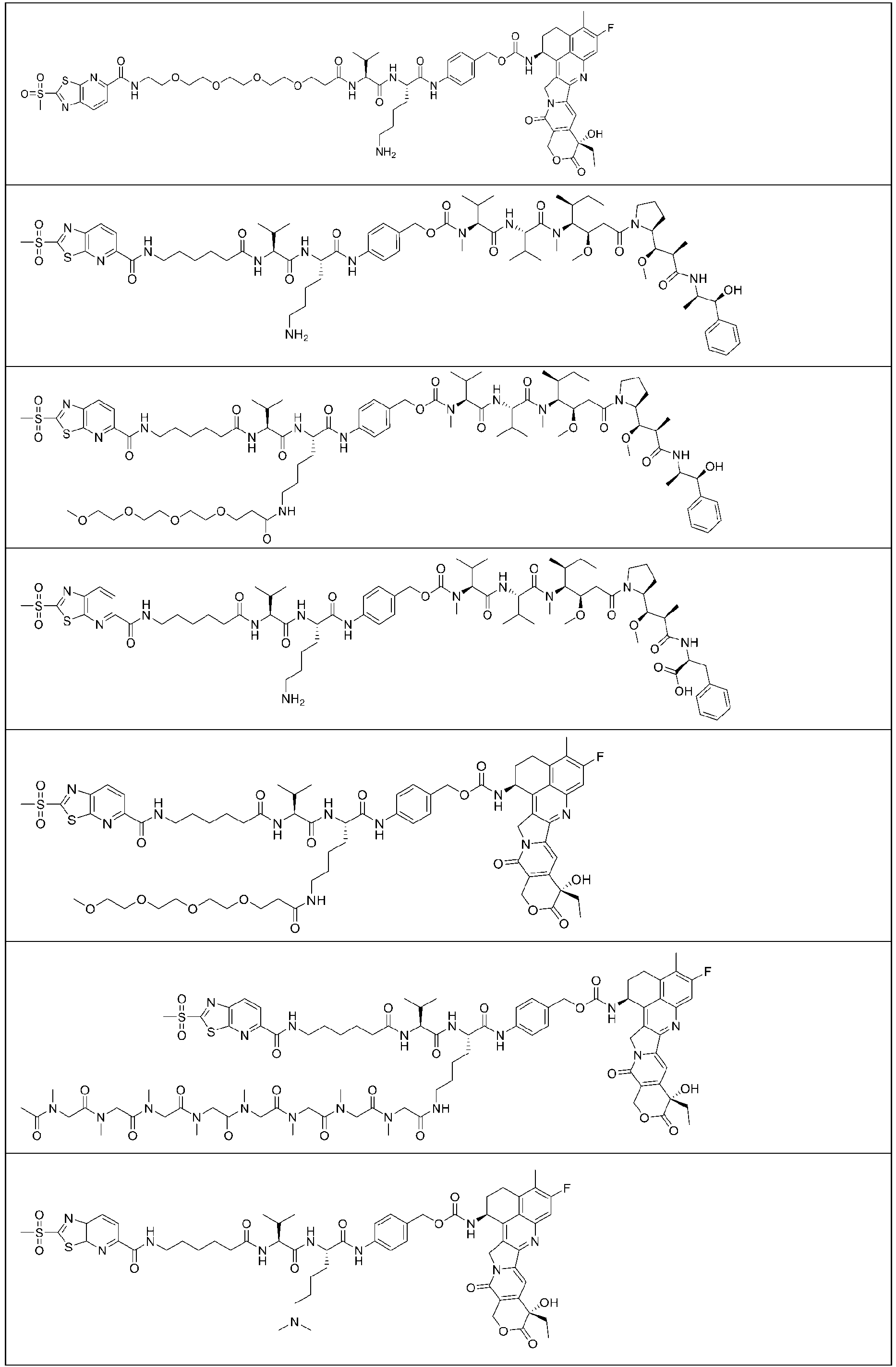

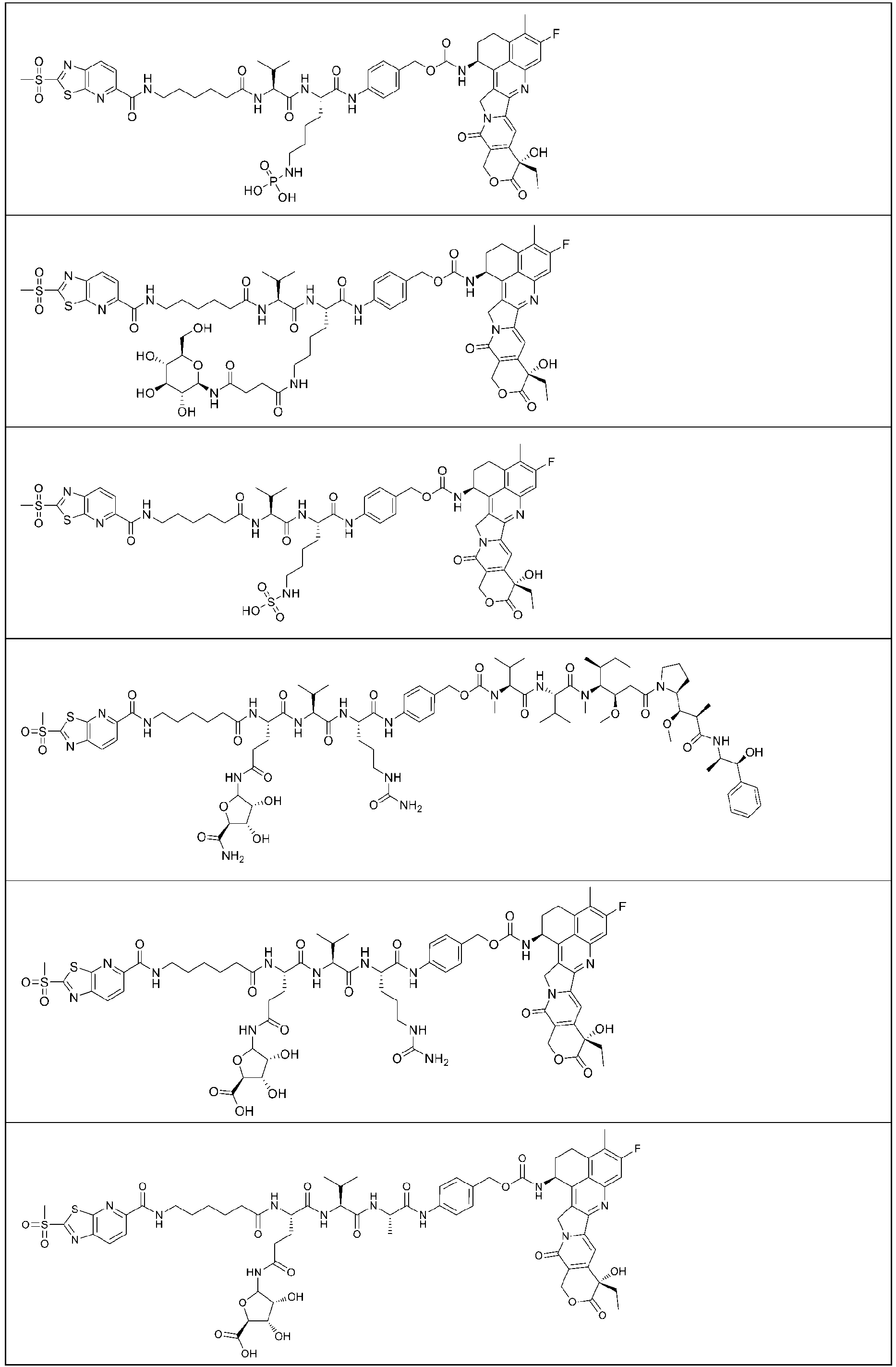

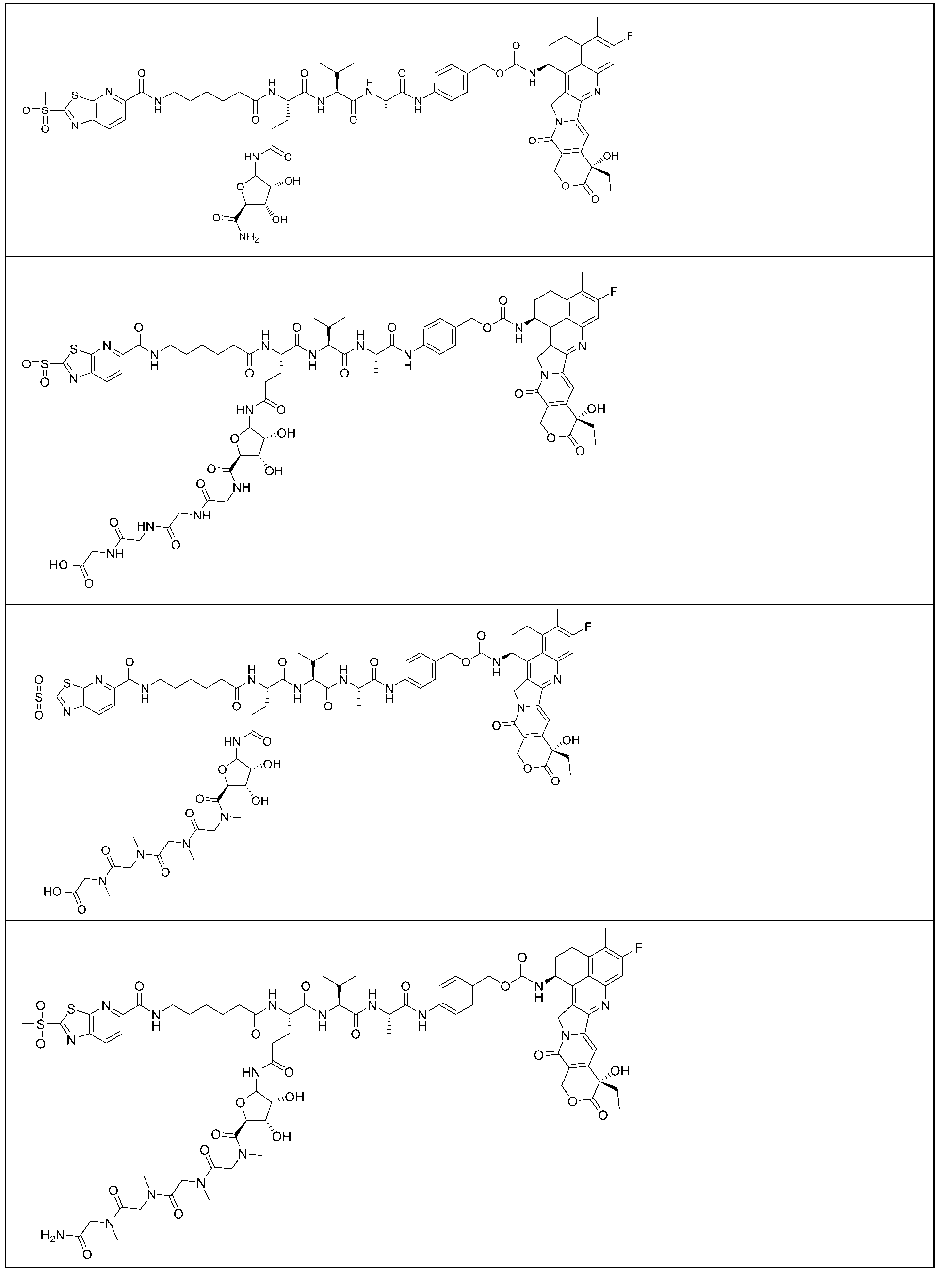

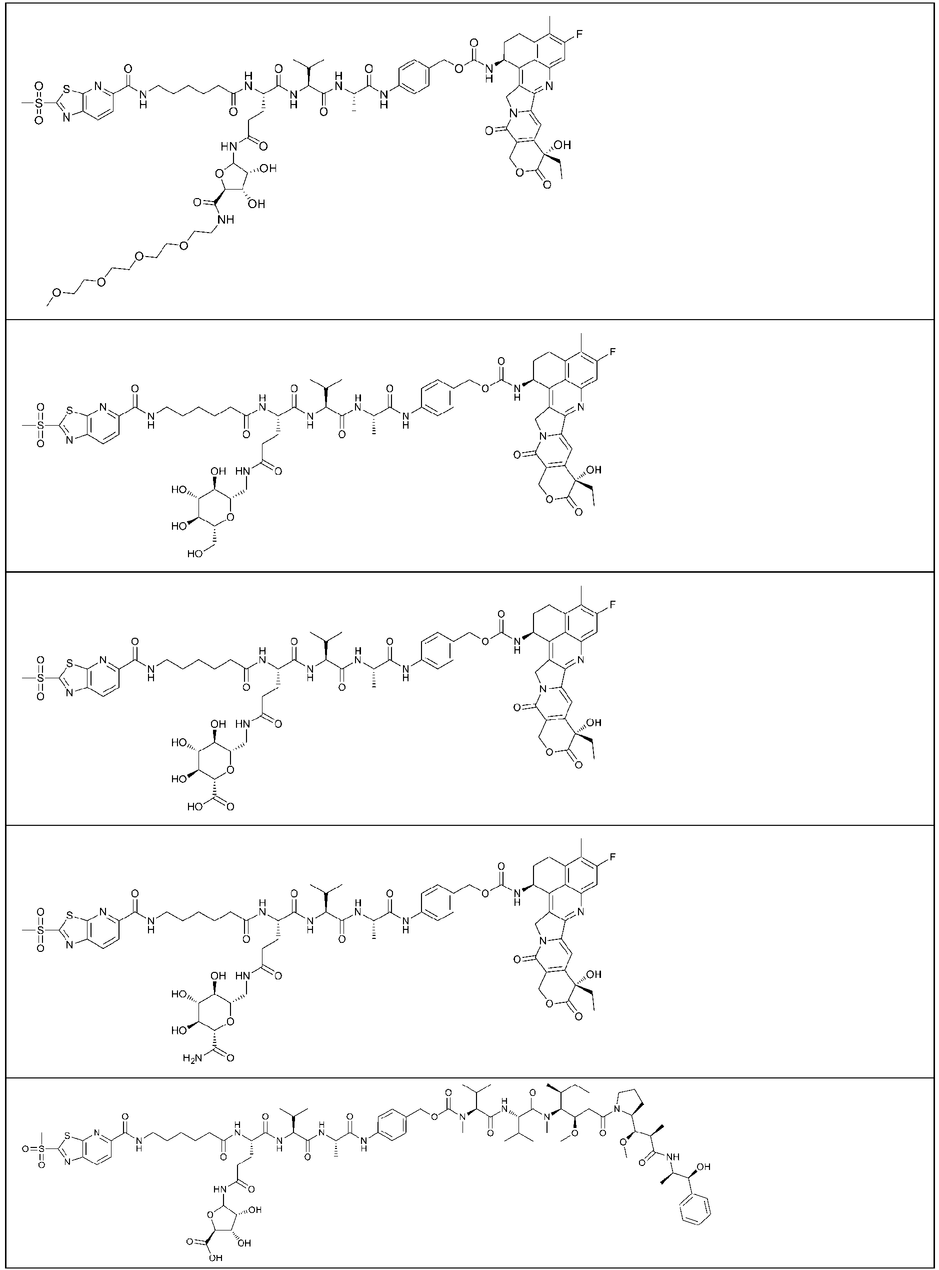

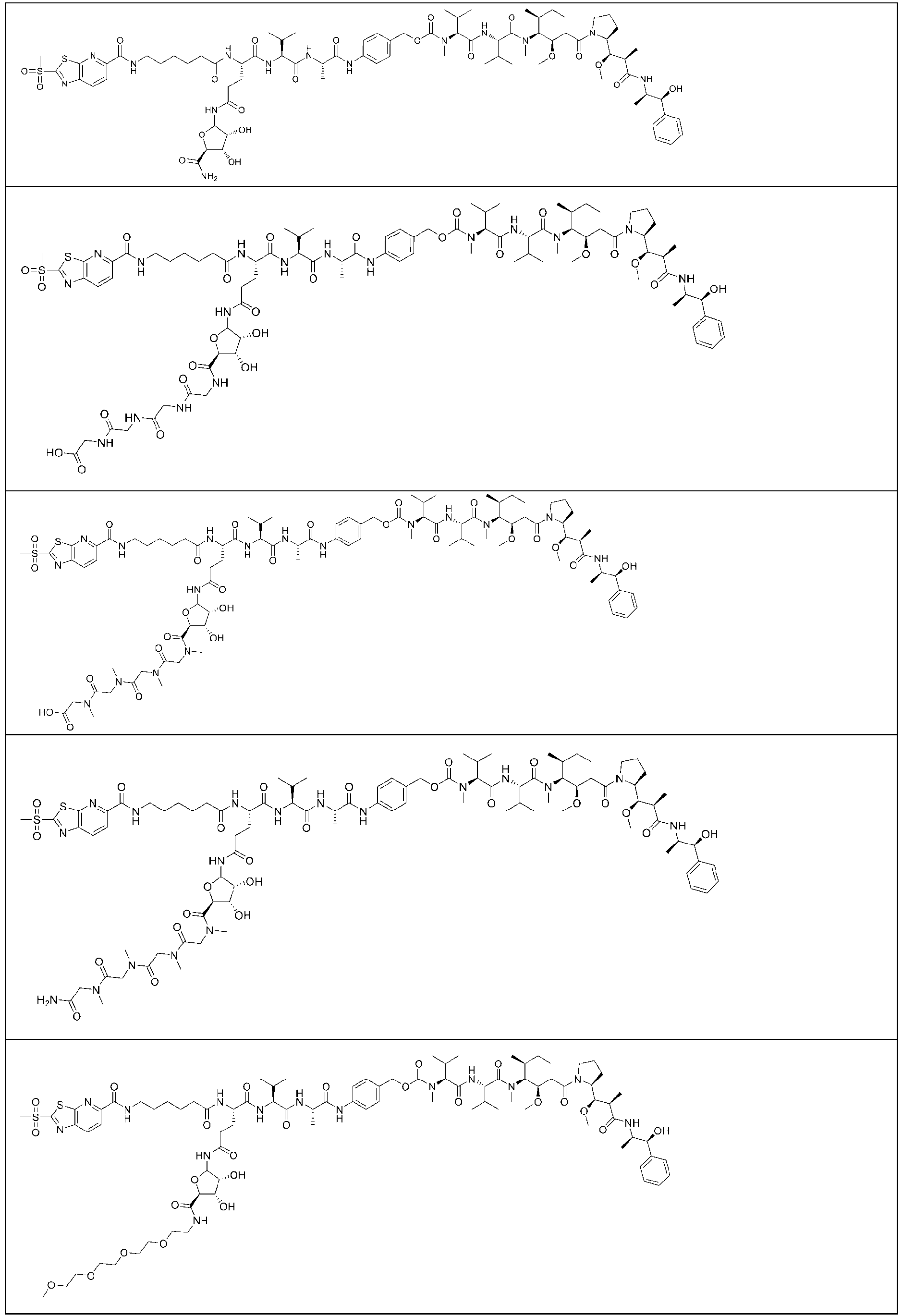

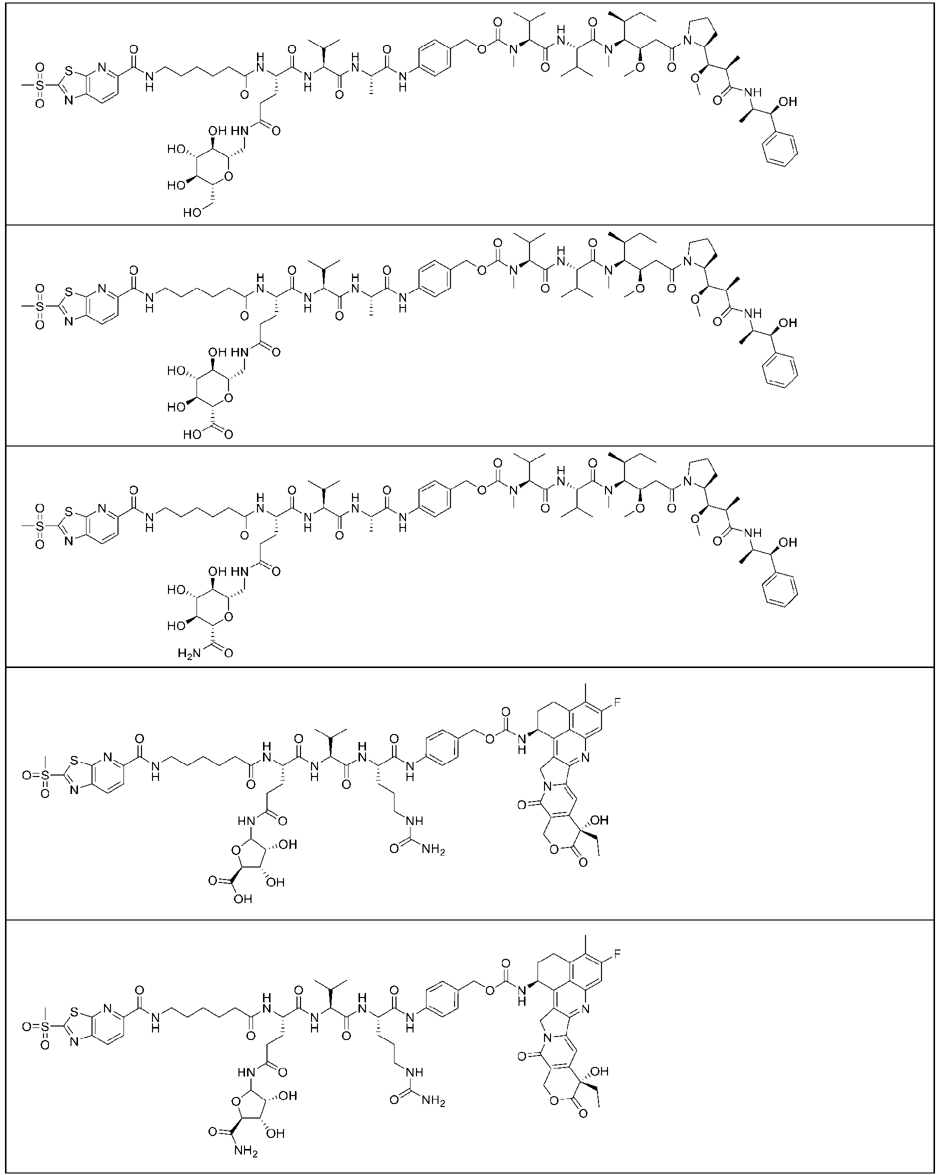

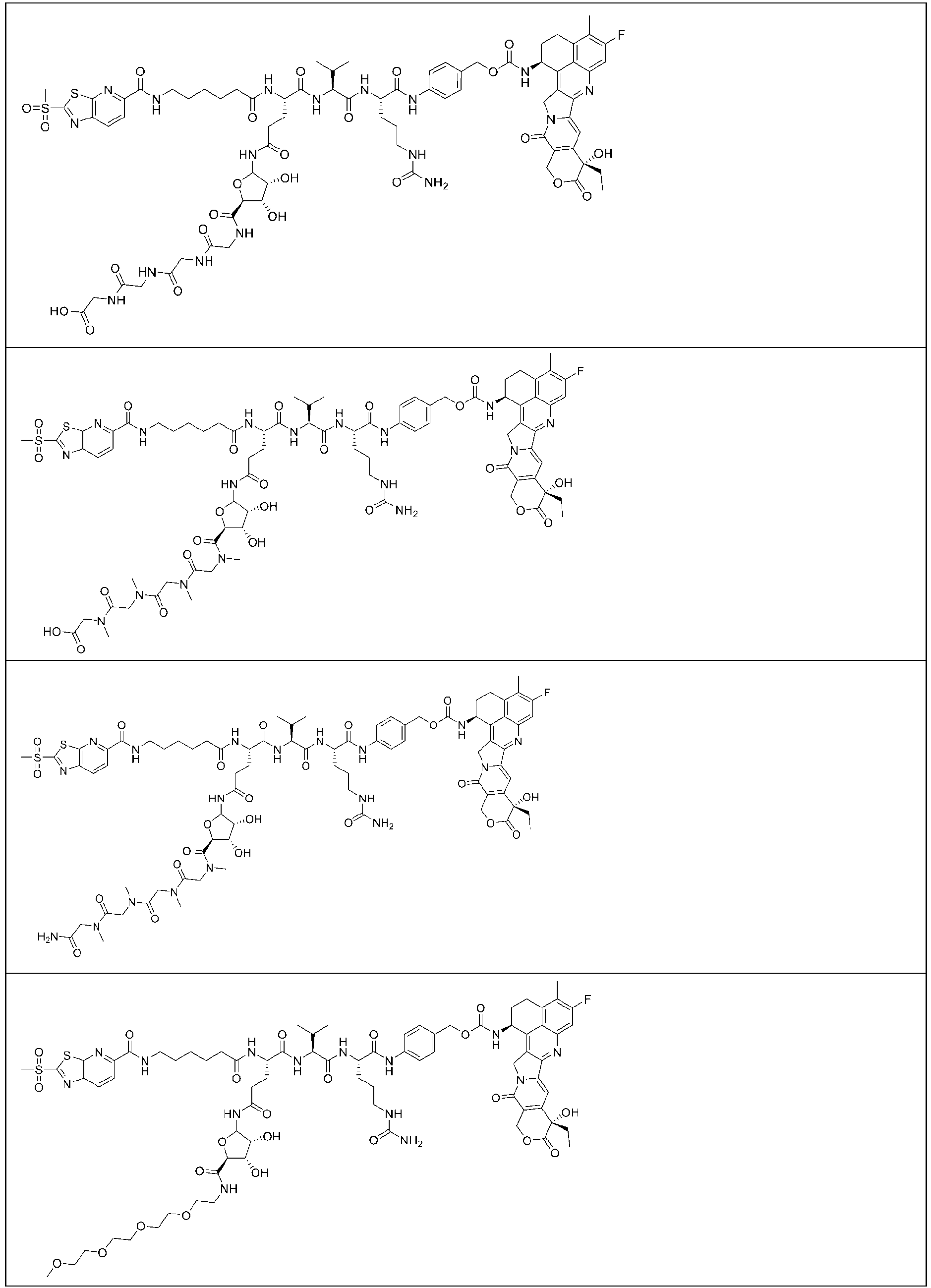

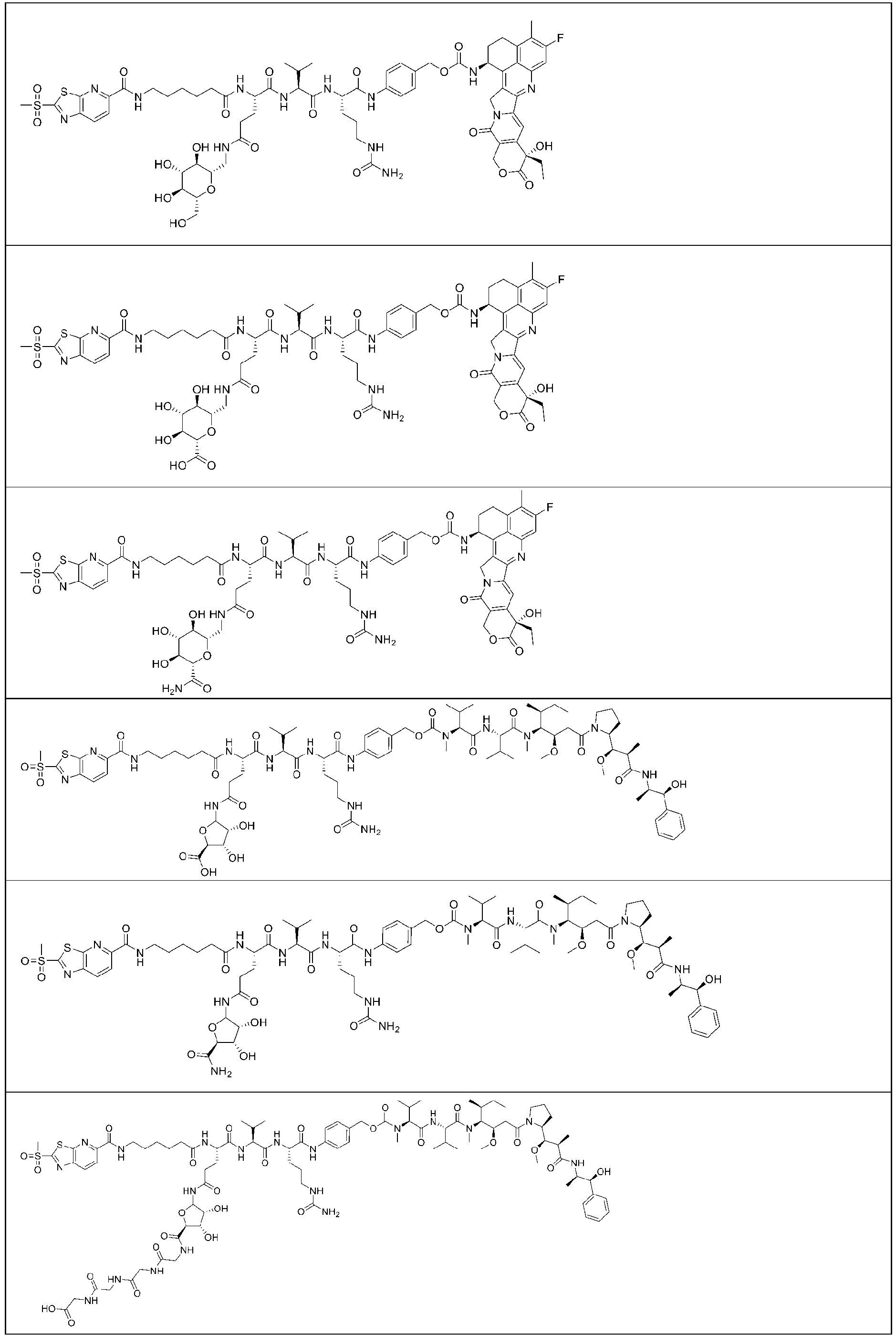

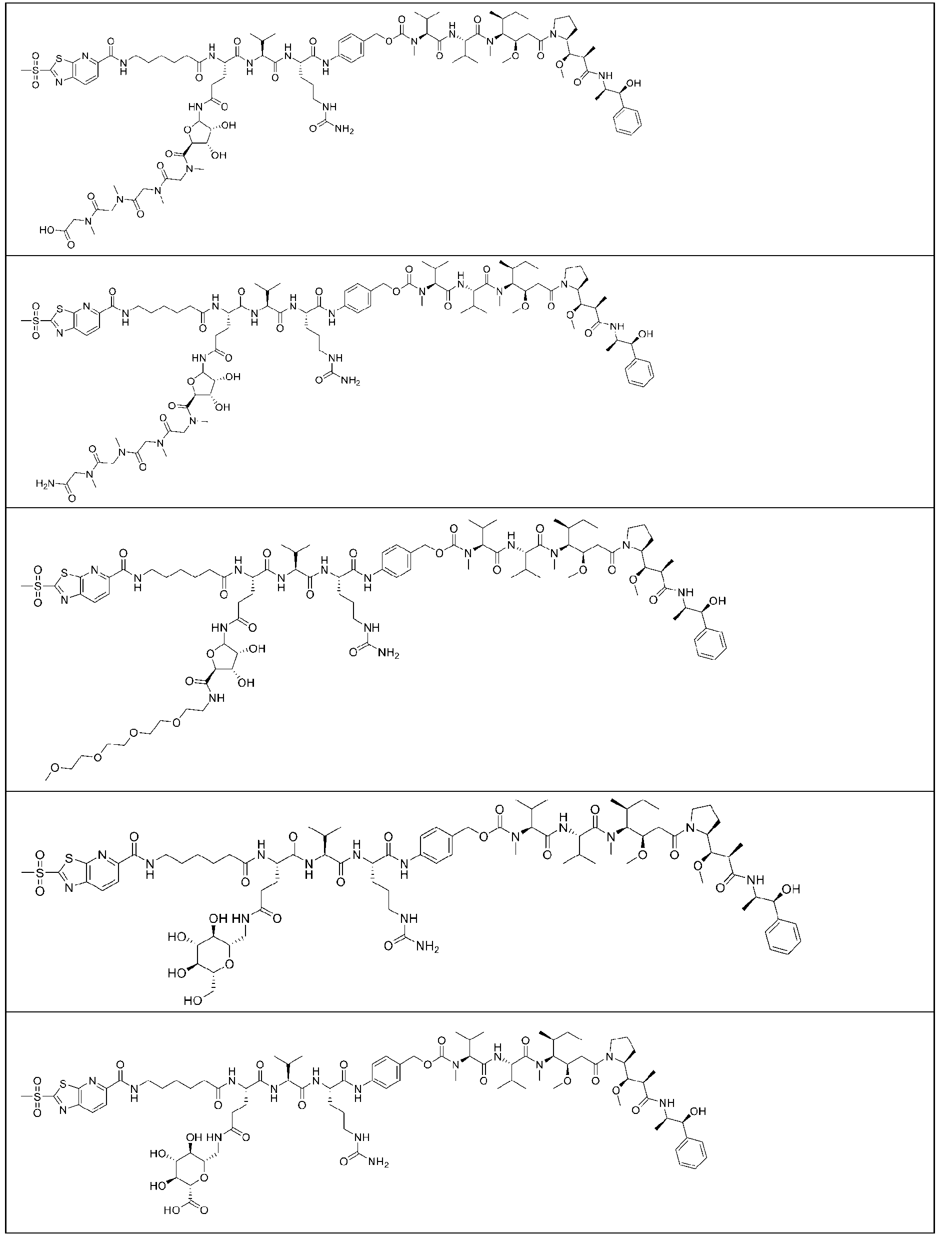

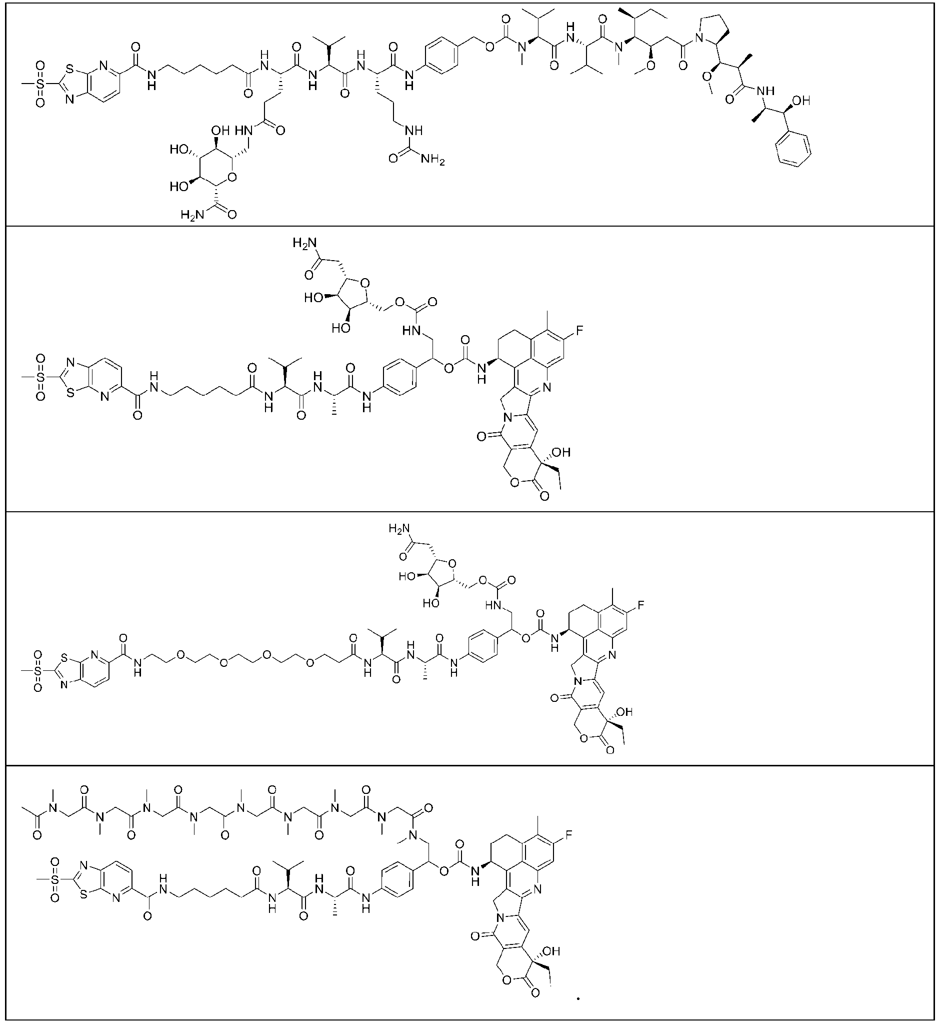

20. A compound of formula (III), or a pharmaceutically acceptable salt, prodrug, hydrate, solvate, enantiomer, diastereomer, mesomer, racemate, or tautomer thereof, having the following general formula: 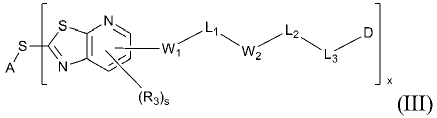 wherein,
A is a targeting molecule;
x = 1, 2, 3, 4, 5, 6, 7 or 8;
other groups are defined as any one of claims 1-19.

21. The compound of formula (III) of claim 20, or a pharmaceutically acceptable salt, prodrug, hydrate, solvate, enantiomer, diastereomer, mesomer, racemate, or tautomer thereof, wherein A is selected from proteins, antibodies, antibody fragments, fusion proteins, polypeptides, enzymes, and small molecules; alternatively, A is an antibody, alternatively a monoclonal antibody, such as a monospecific monoclonal antibody and a bispecific monoclonal antibody; alternatively, A is an IgG antibody or an HCAb antibody targeting tumor-associated antigens.

22. The compound of formula (III) of claim 21, or a pharmaceutically acceptable salt, prodrug, hydrate, solvate, enantiomer, diastereomer, mesomer, racemate, or tautomer thereof, wherein the tumor-associated antigens targeted by A include, but are not limited to: HER2, Trop-2, Claudin-6, Claudin-9, Claudin-18.2, EGFR, c-Met, CD19, PSMA, Muc1, BCMA, PD-L1, CD33, CD30, CD22, CD79b, Nectin-4, CD19, tissue factor, FRα, B7-H3, B7-H4, CDH3, CDH6, CDH17, ALPP, CD56, CD37, HER3, ROR1, MSLN, TNF-α, CD25, ENPP3, Muc1, Axl, CD20, ROR2, GPNMB, CEACAM5, CEACAM6, CD138, GC-C, LIV-1, CA6, FUT3, IGF-1R, CTLA4, RNF43, DPEP3, 5T4, ITGB6, EFNA4, CD228, Notch3, CD46, CAIX, SLAMF6, ADAM9, GD3, TDGF1, SLAMF2, CLL-1, CD123, FCRL5, TIM1, sTn, ETB, Globo H, CD38, Ly6E, SLITRK6, GPR20, FGFR2, Muc16, CD51, SLAMF7, LAMP-1, CD74, CCR7, PTK7, SEZ6, LYFD3, TAA, PRLreceptor, FGFR3, KAAG1, STEAP1, Flt3, Muc1, LRRC15, CD44, CD70, EphA2, CXCR4, DDR1, DKL1, FOLR, CD45, DSG2, ALK, TRAIL, EpCAM, VEGFR2, CD47, CD49, SSEA-4, DCLK1, OAcGD2, CD73, ENO1, BSG, CD24, GLUT1, and GPRC5D, alternatively HER2, HER3, EGFR, Trop-2, Claudin-6, Claudin-18.2, B7-H3, B7-H4, CDH3, CDH6, CDH17, FRα, ROR1, ALPP, CEACAM5, CEACAM6, or FOLR.

23. The compound of formula (III) of any one of claims 20-22, or a pharmaceutically acceptable salt, prodrug, hydrate, solvate, enantiomer, diastereomer, mesomer, racemate, or tautomer thereof, wherein x = 2, 3, 4, 5, 6, 7, or 8; alternatively x = 2, 4, 6, or 8; x = 4, 6, or 8.

24. The compound of any one of claims 20-23, or a pharmaceutically acceptable salt, prodrug, hydrate, solvate, enantiomer, diastereomer, mesomer, racemate, or tautomer thereof, having the following general formula: 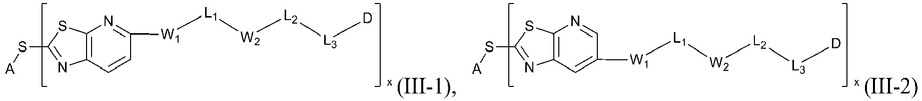 or 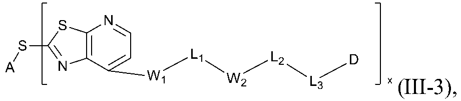 wherein each of the groups is defined as any one of claims 1-23.

25. The compound of any one of claims 20-24, or a pharmaceutically acceptable salt, prodrug, hydrate, solvate, enantiomer, diastereomer, mesomer, racemate, or tautomer thereof, wherein
A represents an antibody, alternatively a monoclonal antibody, such as a monospecific monoclonal antibody and a bispecific monoclonal antibody;
x = 2, 3, 4, 5, 6, 7 or 8; alternatively x = 2, 4, 6 or 8; x = 4, 6 or 8;
W₁ is -C(O)O- or -C(O)NH-;
L₁ is selected from -(CH₂CH₂O)ₙ₁₁-(CH₂)ₘ₁₁-, or -(CH₂)ₘ₂₁-;
wherein n11 = 1, 2, 3, 4, 5, 6, 7, 8, 9 or 10;
m11 = 0, 1, 2, 3, 4, 5, 6, 7, 8, 9 or 10;
m21 = 1, 2, 3, 4, 5, 6, 7, 8, 9 or 10;
W₂ is -C(O)-;
L₂ is selected from dipeptide, tripeptide, or tetrapeptide residues consisting of the following amino acids, wherein the amino acid is selected from valine, citrulline, alanine, glycine, phenylalanine, lysine, arginine, aspartic acid, glutamic acid, and serine, and the amino acid is optionally substituted with 1, 2, 3, 4, 5, or 6 R₄;
alternatively, L₂ is selected from valine-citrulline, valine-alanine, valine-lysine, phenylalanine-lysine, lysine-lysine, alanine-lysine, phenylalanine-citrulline, leucine-citrulline, isoleucine-citrulline, phenylalanine-alanine, lysine-valine-citrulline, lysine-valine-alanine, valine-lysine-glycine, glycine-valine-lysine, glycine-valine-alanine, glutamine-valine-alanine, glutamine-valine-citrulline, glutamic acid-valine-alanine, glutamic acid-valine-citrulline, alanine-alanine-alanine, alanine-alanine-asparagine, phenylalanine-phenylalanine-lysine, glycine-phenylalanine-lysine, leucine-alanine-leucine, isoleucine-alanine-leucine, valine-alanine-valine, glycine-glycine-phenylalanine-glycine, alanine-leucine-alanine-leucine, and glycine-phenylalanine-leucine-glycine, and the amino acid is optionally substituted with 1, 2, 3, 4, 5, or 6 R₄;
wherein N-terminal of amino acid residue or oligopeptide residue is connected to W₂, and the C-terminal is connected to L₃;
R₄ is selected from H, D, halogen, NO₂, -ORₐ, -NR_{b}R_{c}, C₁₋₆ alkyl, C₁₋₆ haloalkyl, polyethylene glycol, polysarcosine, pentose, hexose, sulfonic group, methylsulfonyl, phosphate group, phosphite group, quaternary ammonium salt, or selected from the following groups: 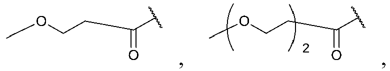 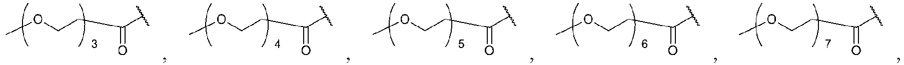 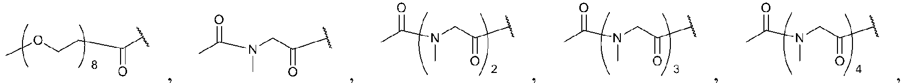 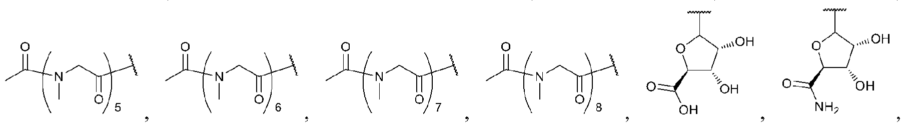 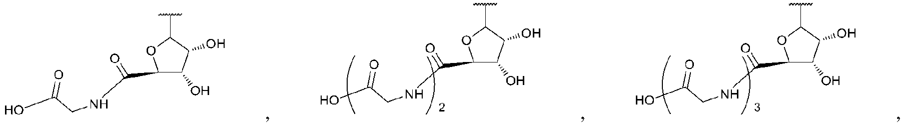 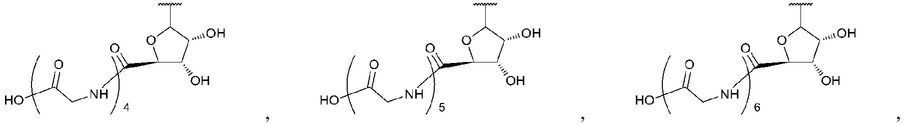 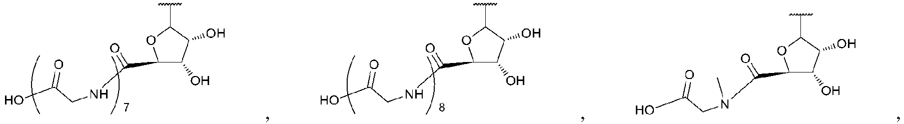 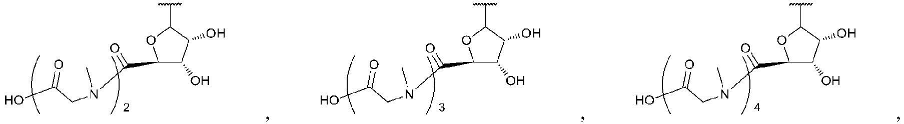 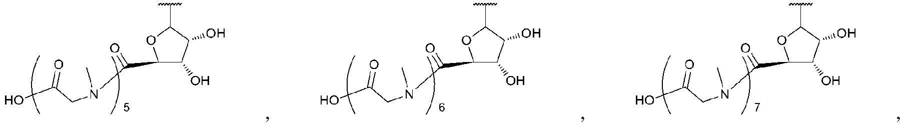 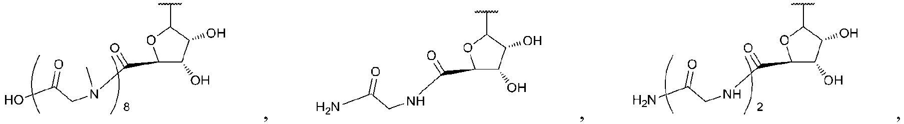 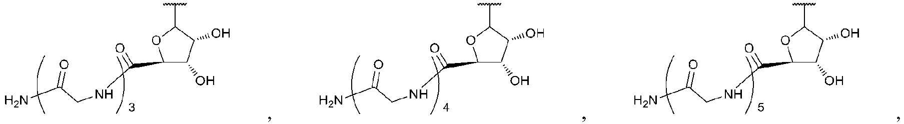 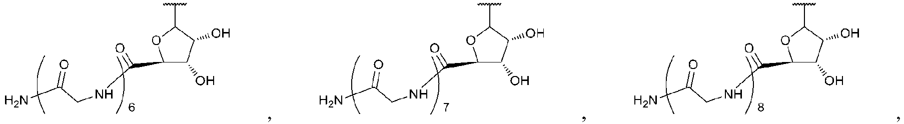 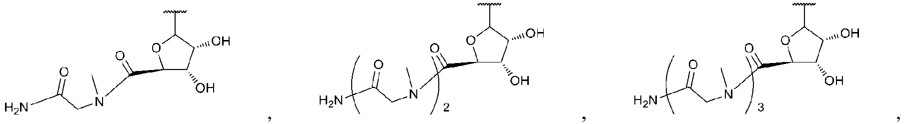 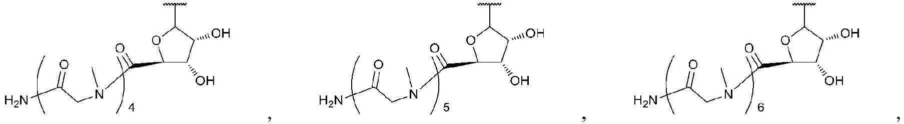 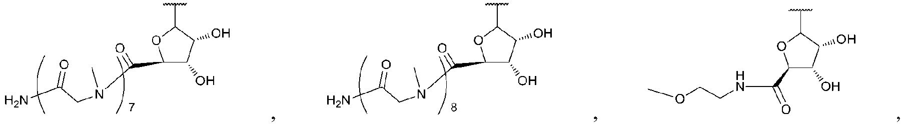 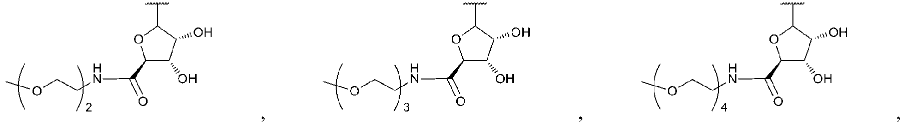 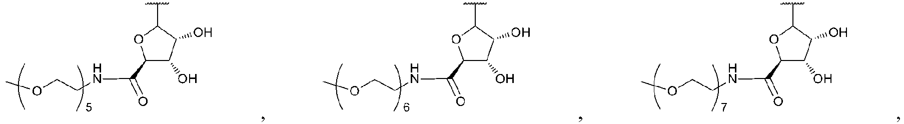 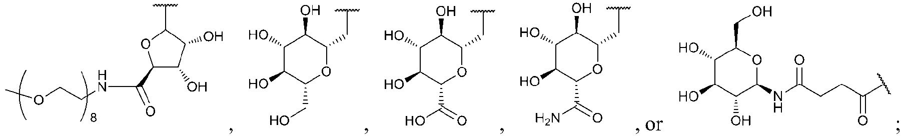
L₃ is selected from the following structures: 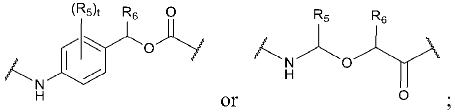
wherein NH is connected to L₂, and C(O) is connected to D;
R₅ is selected from H, D, halogen, C₁₋₆ alkyl, or C₁₋₆ haloalkyl;
t = 0, 1, 2, 3, or 4;
R₆ is selected from H, 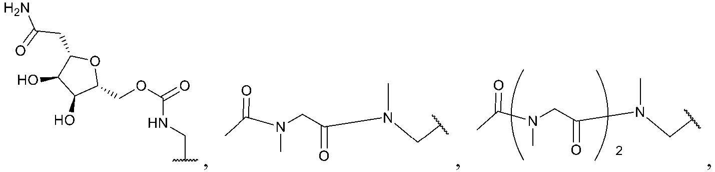 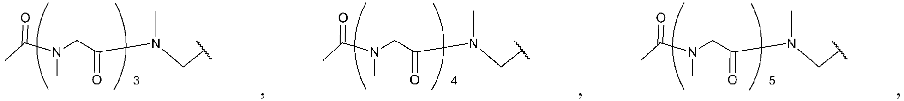 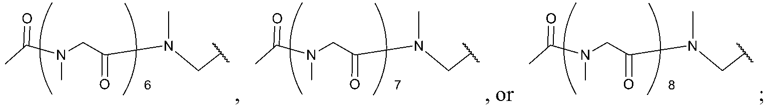
D is selected from microtubule inhibitors, topoisomerase I inhibitors, and DNA alkylating agents, such as MMAE, MMAF, Dxd, or Dx-8951f.

26. The compound of any one of claims 20-25, or a pharmaceutically acceptable salt, prodrug, hydrate, solvate, enantiomer, diastereomer, mesomer, racemate, or tautomer thereof, wherein the compound is selected from: 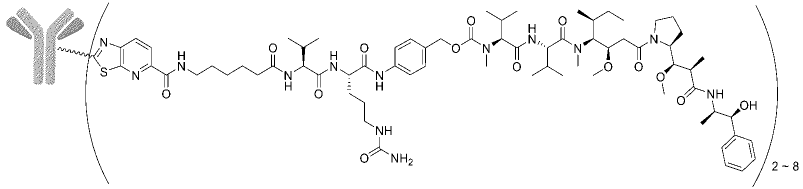 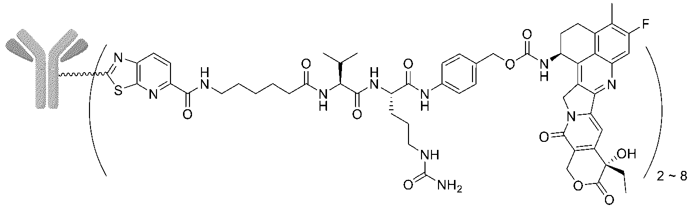 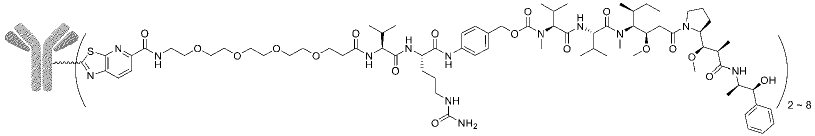 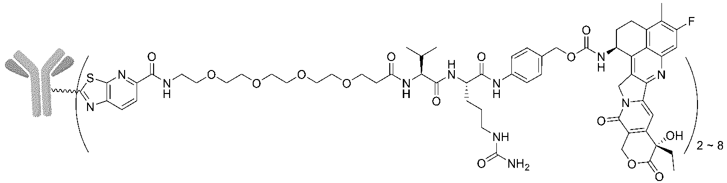 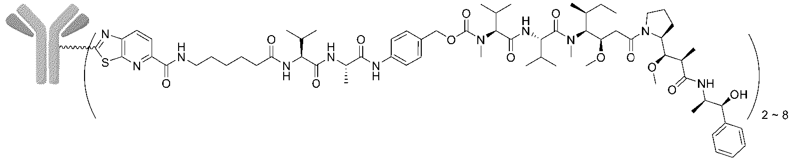 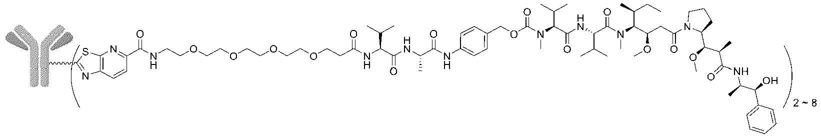 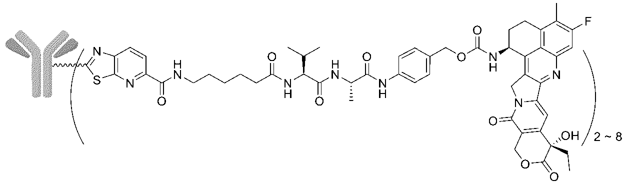 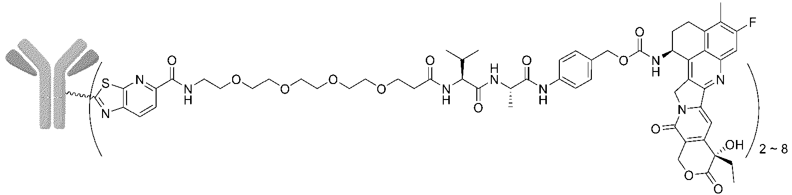 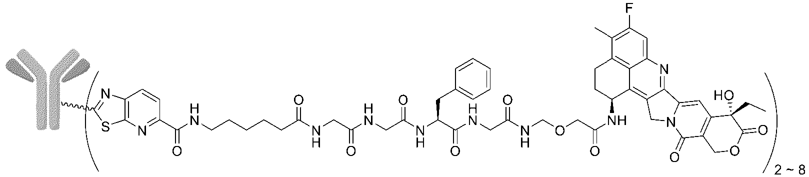 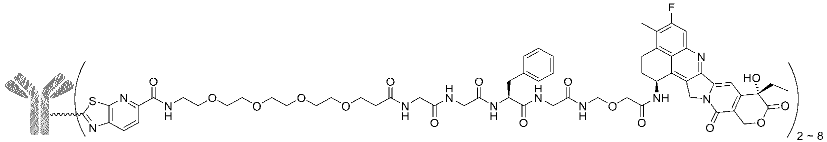 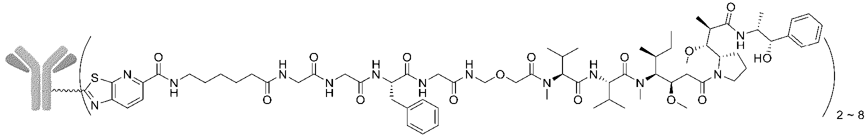 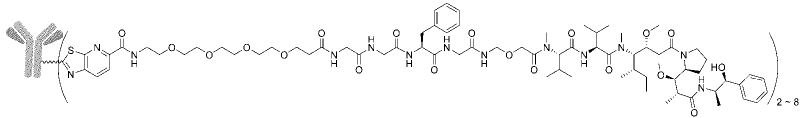 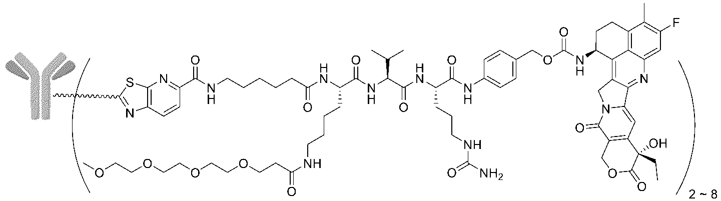 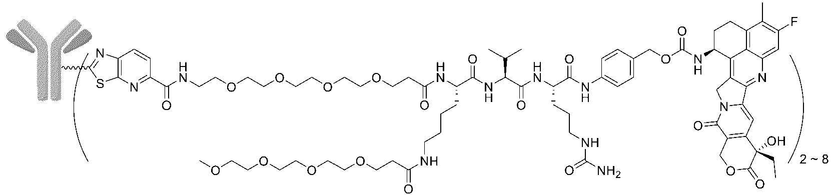 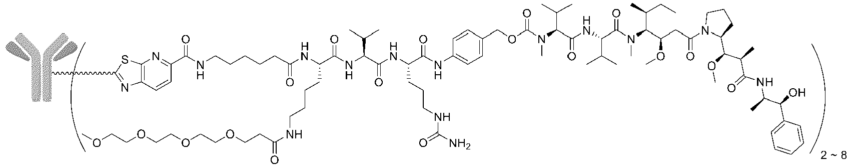 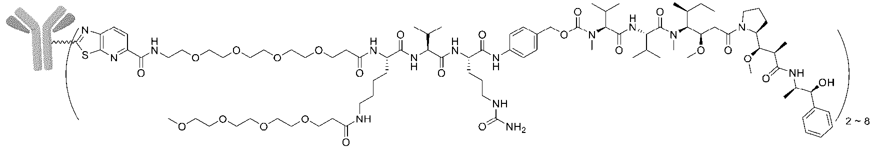 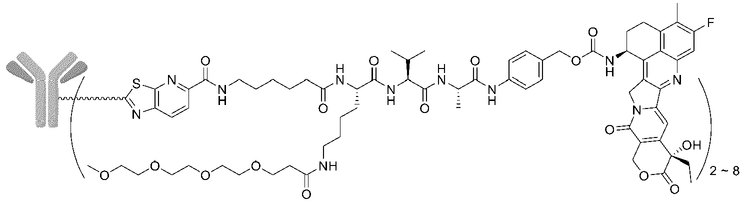 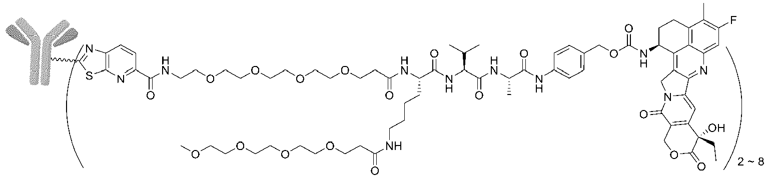 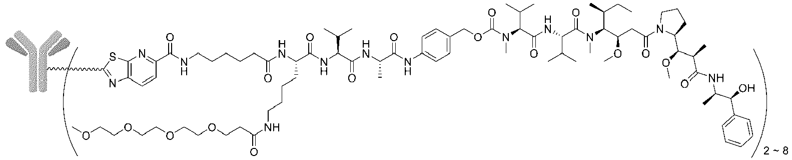 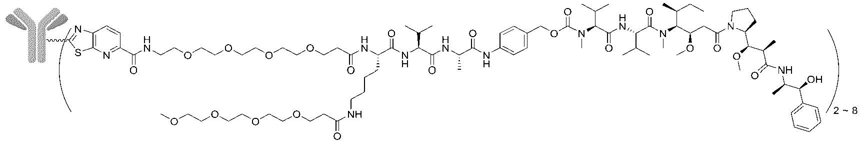 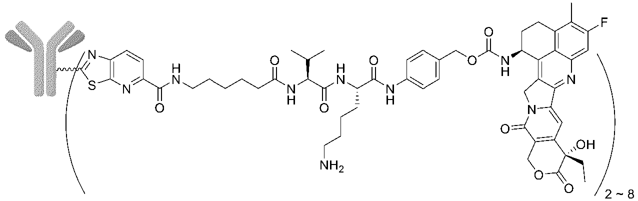 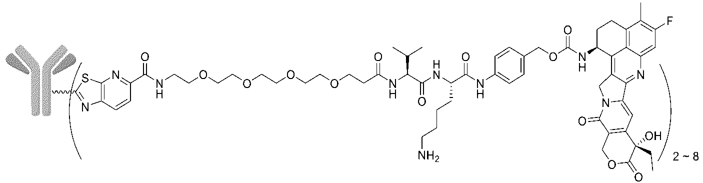 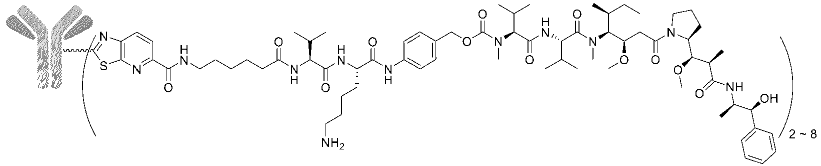 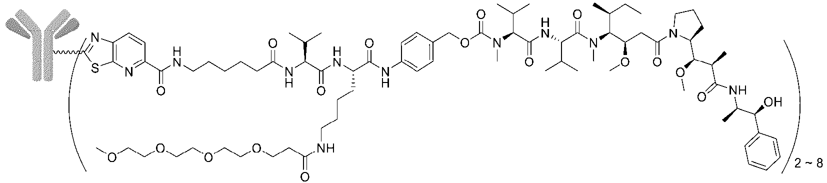 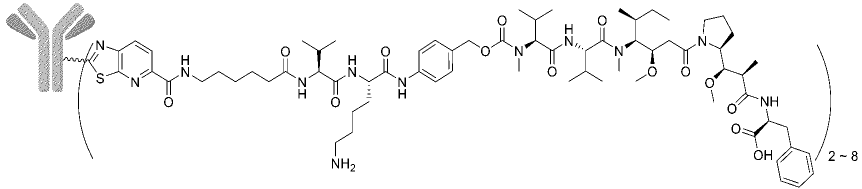 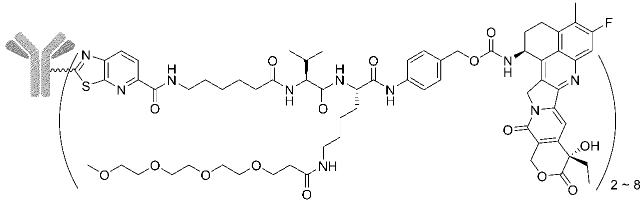 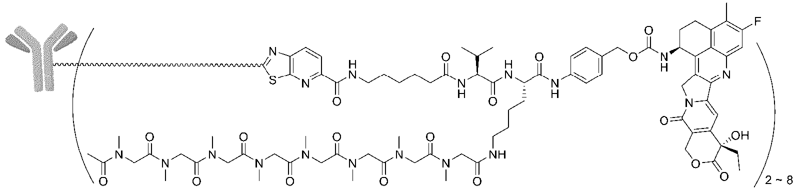 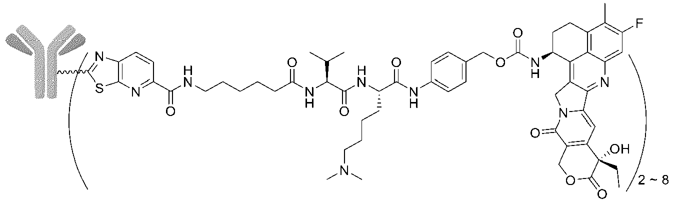 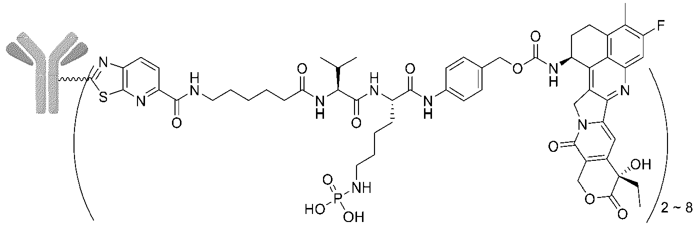 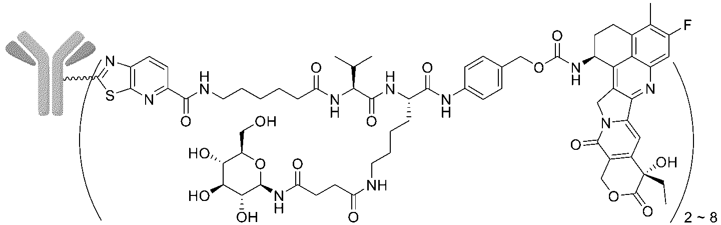 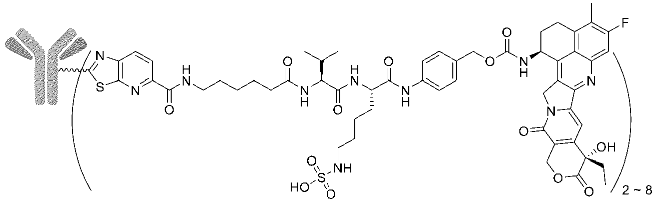 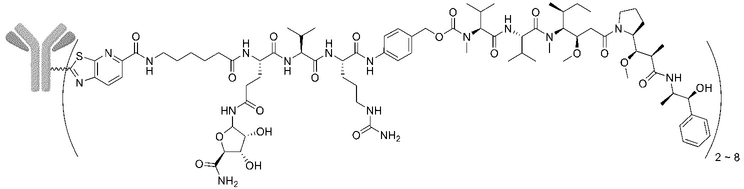 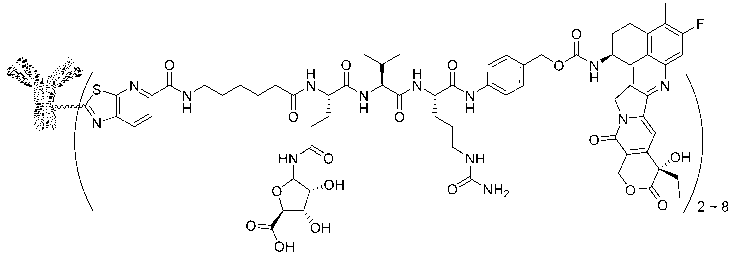 wherein, 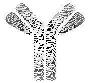 indicates a monoclonal antibody, alternatively an IgG1 or HCAb type monoclonal antibody against HER2 and B7-H3.

27. A pharmaceutical composition comprising the compound of any one of claims 20-26 or a pharmaceutically acceptable salt, prodrug, hydrate, solvate, enantiomer, diastereomer, mesomer, racemate or tautomer thereof, and a pharmaceutically acceptable excipient or adjuvant.

28. Use of the compound of any one of claims 20-26 or a pharmaceutically acceptable salt, prodrug, hydrate, solvate, enantiomer, diastereomer, mesomer, racemate or tautomer thereof in the manufacture of a medicament for treating a disease or condition and for reducing the severity of said disease or condition.

29. The compound of any one of claims 20-26 or a pharmaceutically acceptable salt, prodrug, hydrate, solvate, enantiomer, diastereomer, mesomer, racemate, or tautomer, or the pharmaceutical composition of claim 27, for treating a disease or condition and for reducing the severity of said disease or condition.

30. A method of treating a disease or condition and reducing the severity of said disease or condition in a subject, comprising administering to the subject the compound of any one of claims 20-26 or a pharmaceutically acceptable salt, prodrug, hydrate, solvate, enantiomer, diastereomer, mesomer, racemate, or tautomer, or the pharmaceutical composition of claim 27.

31. The use of claim 28, or the compound for use of claim 29, or the method of claim 30, wherein the disease or condition is selected from tumor, cancer, autoimmune diseases, infectious diseases, hematological diseases, metabolic diseases, and inflammation;
preferably, the cancer is selected from breast cancer, lung cancer, squamous cell carcinoma, peritoneal cancer, liver cancer, stomach cancer, cancer of the esophagus, gastrointestinal cancer, adenocarcinoma of the membrane, glioblastoma, cervical cancer, ovarian cancer, bladder cancer, urethral cancer, intestinal cancer, uterine cancer, salivary gland cancer, kidney cancer, prostate cancer, vulvar cancer, carcinoma of penis, thyroid cancer, anal cancer, melanoma, brain cancer, gallbladder cancer, esophageal cancer, bile duct cancer, head and neck cancer, lymphoma, multiple myeloma, blastoma, leukemia, myeloma, plasmacytoma, sarcoma, pancreatic cancer, mesothelioma, and nasopharyngeal carcinoma.
